# EUROPEAN PATENT APPLICATION

(11) **EP 2 460 794 A1**
(43) Date of publication of application: **06.06.2012**
(21) Application number: 10804498.3
(22) Date of filing: 29.07.2010
(51) Int. Cl.: C07D 209/42, A61K 31/404, A61K 31/496, A61K 31/4985, A61P 11/06, A61P 17/04, A61P 29/00, A61P 43/00, C07D 209/52, C07D 209/70, C07D 235/24, C07D 403/06, C07D 471/04, C07D 487/04, C07D 491/052

(54) **PHARMACEUTICAL COMPOSITION CONTAINING FUSED HETERO-RING DERIVATIVE**

(30) Priority: 31.07.2009 JP 2009178508
(71) Applicant: Shionogi & Co., Ltd., Osaka 541-0045 (JP)
(72) Inventor: TACHIBANA, Yuuki, Toyonaka-shi Osaka 561-0825 (JP); MIYAGAWA, Masayoshi, Toyonaka-shi Osaka 561-0825 (JP); MASUDA, Tsutomu, Toyonaka-shi Osaka 561-0825 (JP); HASEGAWA, Tsuyoshi, Toyonaka-shi Osaka 561-0825 (JP)
(74) Representative: HOFFMANN EITLE
(86) International application number: PCT/JP2010/062804
(87) International publication number: WO 2011/013752

(57) **Abstract**

The present invention provides a compound which indicates a histamine H4 receptor modulating activity.

A compound represented by a formula (I): 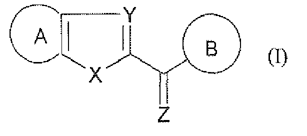 wherein
A ring is a ring represented by the following formula: 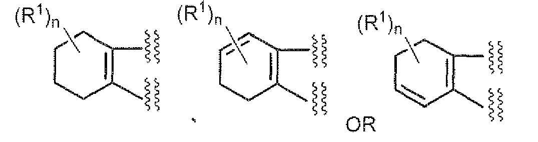 wherein
R¹ is substituted or unsubstituted alkyl, etc., W is -O-, etc., n is an integer of 0 to 6; X is N(R⁷)- or -O-; R⁷ is hydrogen, substituted or unsubstituted alkyl, etc.; Y is-C(R⁸)= or -N=; R⁸ is hydrogen, substituted or unsubstituted alkyl, etc.; Z is =O, =S, etc.;
B ring is a ring represented by the following formula 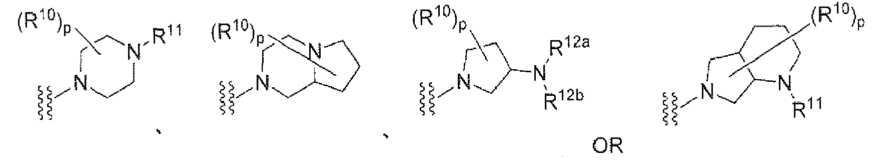 wherein
R¹⁰ is each independently substituted or unsubstituted alkyl, halogen, hydroxy, substituted or unsubstituted alkyloxy, substituted or unsubstituted amino; R¹¹, R^{12a} and R^{12b} are each independently hydrogen or substituted or unsubstituted alkyl, etc.; p is an integer of 0 to 4, or its pharmaceutically acceptable salt, or a solvate thereof.

## Description

### [TECHNICAL FIELD]

The invention relates to a compound, or its pharmaceutically acceptable salt, or a solvate thereof, and pharmaceutical composition comprising them useful for treating a disease or condition associated with histamine H4 receptor.

### [BACKGROUND ART]

Histamine interacts with four recepters of G protein coupled superfamily (histamine H1, H2, H3 and H4 receptor) to express a variety of physiology, H1 receptor-mediated allergic reaction, H2 receptor-mediated gastric acid secretion effect and H3 receptor-mediated neurotransmission effect in central nervous system, etc.
Histamine H4 receptor is G protein coupled-receptor of seven-transmembrance consisting of 390 amino acids which has homology of approximately 40 % with H3 reseptor. Histamine H4 receptor is mainly expressed in hemocyte cells such as eosinophil, mast cells, dendritic cells, and T-cells, especially hyperexpressed in eosinophil and mast cells (Non-Patent Document 1 and Non-Patent Document 2).
Additionally, histamine H4 receptor is confirmed that it expressed in synovial cells obtained from patients suffering from rheumatism (Non-Patent Document 3 and Non-Patent Document 4), in synovial cells obtained from patients suffering from osteoarthritis (Non-Patent Document 5) and in bowel cells (Non-Patent Document 6). Moreover, it is reported that expression level of histamine H4 receptor increases in intranasal polyp (Non-Patent Document 7).
The pharmacological nature of histamine H4 receptor is brought out by some specific ligand. For example, it is known that histamine binded with H4 receptor evokes the effect of eosinophil migration and eosinophil shape change, and increased expression of CD11b/CD18, CD54, etc. (Non-Patent Document 8). In addition, it is known that histamine H4 receptor mediates calcium mobilization of mast cells (Non-Patent Document 9) and modulation of cytokine production from dendritic cells (Non-Patent Document 10), etc., and a wide variety of its action is known.
It is reported that histamine H4 receptor mediates mast cells accumulation induced by histamine (Non-Patent Document 9), neutrophil infiltration in zymosan-induced peritonitis model (Non-Patent Document 11) and pleurisy model (Non-Patent Document 12), neutrophil infiltration by egg albumin in asthma model (Non-Patent Document 10) and itch (Non-Patent Document 13) in vivo.
It is described that aromatic heterocyclic derivatives act on histamine H4 receptor in Patent Document 1, 2 and 3, but the compound of the present invention is not described and suggested in any documents.
The compounds described in Patent Document 4 to 8 and Non-Patent document 14 and 15 are known as fused hetero-ring derivatives, but histamine H4 receptor modulating effect is not known in these compounds.

### [PRIOR ART DOCUMENTS]

### [PATENT DOCUMENTS]

[Patent Document 1]WO2004/022060
[Patent Document 2]WO2004/022537
[Patent Document 3]WO2005/033088
[Patent Document 4]JP2001-294572
[Patent Document 5]WO2000/009480
[Patent Document 6]WO2002/030935
[Patent Document 7]WO1997/025331
[Patent Document 8]US2009/0163545

### [NON-PATENT DOCUMENTS]

[Non-Patent Document 1]J.Biol.Chem., 2000, 275, 3678
[Non-Patent Document 2]Mol Pharmacol., 2001, 59, 427.
[Non-Patent Document 3]Ann. Rheum. Dis., 2006, 65(Suppl II), 129
[Non-Patent Document 4]Bio. Pharm. Bull., 2005, 28, 2016.
[Non-Patent Document 5]European Histamine Research Society XXXVI Annual meeting, Florence, Italy, 2007, P-11
[Non-Patent Document 6]Gut, 2006, 55, 438.
[Non-Patent Document 7]Cell Biol. Int., 2007, 31, 1367.
[Non-Patent Document 8]Br. J. Pharmacol., 2004, 142, 161.
[Non-Patent Document 9]J. Pharmcol. Exp. Ther., 2003, 305, 1212.
[Non-Patent Document 10]J. Immunol., 2006, 176, 7062.
[Non-Patent Document 11]J. Pharmcol. Exp. Ther., 2004, 309, 404.
[Non-Patent Document 12]J. Pharmcol. Exp. Ther., 2003, 307, 1072.
[Non-Patent Document 13]J. Allergy. Clin. Immunol., 2007, 119, 176.
[Non-Patent Document 14]J. Med. Chem., 2004, 47, 5167-5182.
[Non-Patent Document 15]Indian Journal of Chemistry Section B: Organic Chemistry Including Medicinal Chemistry, 1982, 21B(9), 852-856.

### [DISCLOSURE OF INVENTION]

### [PROBLEM TO BE RESOLVED BY THE INVENTION]

The present invention provides a novel compound having histamine H4 receptor modulating effect.

### [MEANS OF SOLVING THE PROBLEMS]

During studies to solve the problems described above, the inventors have discovered novel compounds that has histamine H4 receptor modulating effect and are useful for preventing and/or treating a deseases associated with histamine H4 receptor, and thus, archieved the inventions described bellow.

Accordingly, for example, the present invention relates to the following item.
(1) A compound represented by a formula (I): 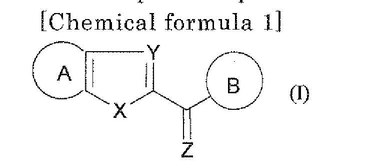 wherein
   A ring is a ring represented by the following formula: 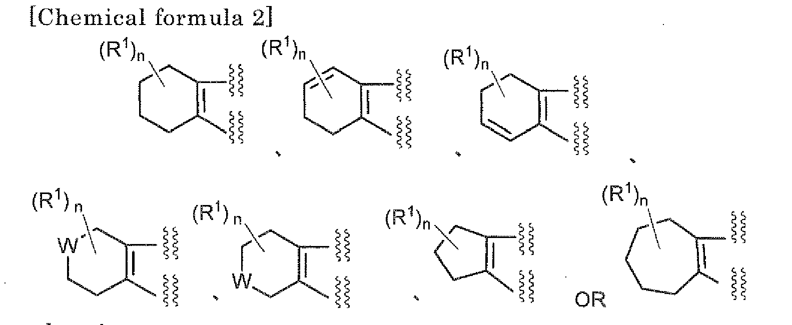 wherein
   R¹ is each independently one or more group(s) selected from the group consisting of following group a) to group e) defined below;
   a)halogen, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylthio, substituted or unsubstituted alkenylthio, substituted or unsubstituted alkynylthio, acyl, hydroxy, formyl, carboxy, substituted or unsubstituted alkyloxycarbonyl, substituted or unsubstituted carbamoyl, substituted or unsubstituted sulfamoyl, substituted or unsubstituted alkylaxysulfonyl, substituted or unsubstituted aryloxysulfonyl, substituted or unsubstituted alkylsulfinyl, substituted or unsubstituted arylsulfinyl, cyano, nitro, substituted or unsubstituted amino, substituted or unsubstituted cycloalkyl, substituted or unsubstituted cycloalkenyl, a substituted or unsubstituted non-aromatic heterocyclic group, substituted or unsubstituted aryl, substituted or unsubstituted heteroaryl, substituted or unsubstituted cycloalkyloxy, substituted or unsubstituted cycloalkenyloxy, substituted or unsubstituted non-aromatic heterocyclic ring-oxy, substituted or unsubstituted aryloxy, and substituted or unsubstituted heteroaryloxy,
   b) two R¹ on the same carbon atom are taken together =O and =NR³,
   c) two R¹ on the same carbon atom are taken together -(CR^{4a}R^{4b})ₓ-,
   d) two R¹ on the adjacent carbon atom are taken together -(CR^{5a}R^{5b})_{y}-, and
   e) two R¹ on the non-adjacent carbon atom are taken together -(CR^{6a}R^{6b})_{z}-, provided that R¹ is not selected from the group consisting of group c), group d) and group e) at the same time;
   W is -O-, -N(R²)- or -S(O)ₘ -;
   R² is hydrogen, substituted or unsubstituted alkyl, substituted or unsubstituted alkenyl, substituted or unsubstituted alkynyl, acyl, substituted or unsubstituted cycloalkyl, substituted or unsubstituted cycloalkenyl, substituted or unsubstituted alkyl non-aromatic heterocyclic group, substituted or unsubstituted aryl, or substituted or unsubstituted heteroaryl;
   m is an integer of 0 to 2;
   R³ is hydrogen, substituted or unsubstituted alkyl, hydroxy or substituted or unsubstituted alkyloxy;
   R^{4a}, R^{4b}, R^{5a}, R^{5b}, R^{6a} and R^{6b} are each in dependently hydrogen, halogen or substituted or unsubstituted alkyl;
   n is an integer of 0 to 6;
   x is an integer of 2 to 7;
   y is an integer of 1 to 5;
   z is an integer of 1 to 3;
   X is -N(R⁷)- or -O-;
   R⁷ is hydrogen or substituted or unsubstituted alkyl or substituted or unsubstituted cycloalkyl;
   Y is -C(R⁸)= or -N=;
   R⁸ is hydrogen, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylthio, acyl, hydroxy, formyl, carboxy, substituted or unsubstituted alkyloxycarbonyl, substituted or unsubstituted carbamoyl, substituted or unsubstituted sulfamoyl, cyano, nitro, or substituted or unsubstituted amino;
   Z is =O, =S, or =NR⁹;
   R⁹ is hydrogen, substituted or unsubstituted alkyl, hydroxy or substituted or unsubstituted alkyloxy;
   B ring is a ring represented by the following formula: 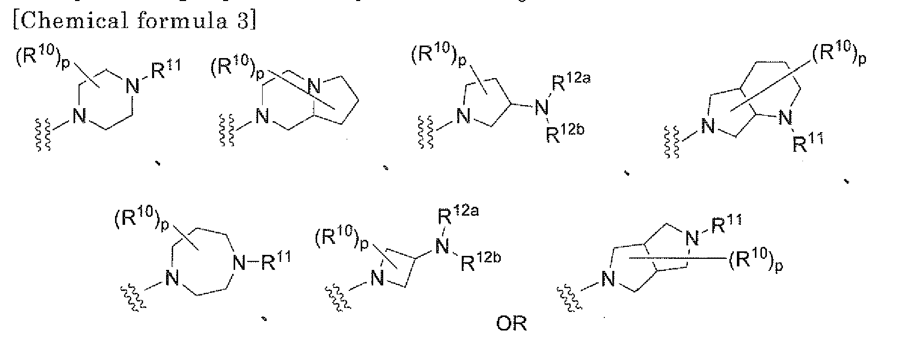 wherein
   R¹⁰ is each independently substituted or unsubstituted alkyl, halogen, hydroxy, substituted or unsubstituted alkyloxy or substituted or unsubstituted amino;
   R¹¹ is hydrogen or substituted or unsubstituted alkyl;
   R¹² a and R^{12b} are each independently hydrogen or substituted or unsubstituted alkyl;
   p is an integer of 0 to 4;
   provided that R⁸ is not methyl when X is -O- and A ring is a ring represented by the following formula: 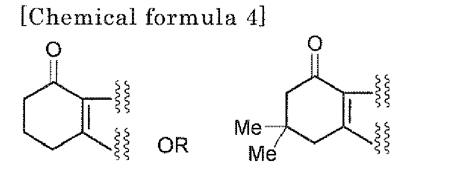 , or its pharmaceutically acceptable salt, or a solvate thereof.
(2) The compound according to the above (I), wherein Z is =O, or its pharmaceutically acceptable salt, or a solvate thereof.
(3) The compound according to the above (1) or (2), wherein X is -N(R⁷)-, wherein R⁷ is as defined in the above (1), or its pharmaceutically acceptable salt, or a solvate thereof.
(4) The compound according to any one of the above (1) to (3), wherein A ring is a ring represented by the following formula: 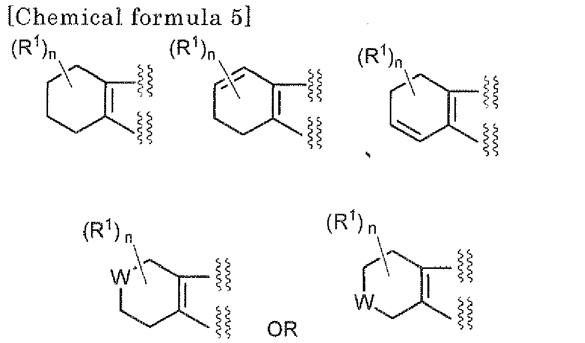 wherein R¹, W and n are as defined in the above (1), or its pharmaceutically acceptable salt, or a solvate thereof.
(5) The compound according to any one of the above (1) to (4), wherein W is -O- or-N(R²)-, wherein R² is as defined in the above (1), or its pharmaceutically acceptable salt, or a solvate thereof.
(6) The compound according to the above (1) to (5), wherein R¹ is one or more group(s) selected from the group consisting of group a) and group b), or its pharmaceutically acceptable salt, or a solvate thereof.
(7) The compound according to any one of the above (1) to (6), wherein B ring is a ring represented by the following formula: 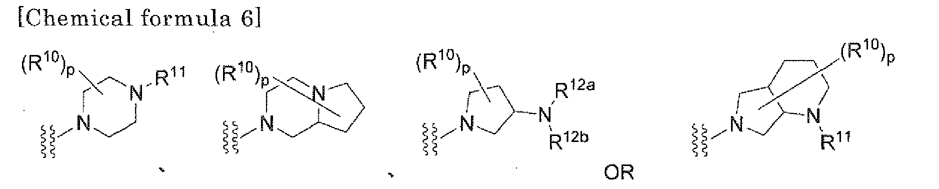 wherein R¹⁰, R¹¹, R^{12a}, R^{12b} and p are as defined in the above (1), or its pharmaceutically acceptable salt, or a solvate thereof.
(8) A pharmaceutical composition comprising the compound according to any one of the above (1) to (7), or its pharmaceutically acceptable salt, or a solvate thereof.
(9) The pharmaceutical composition according to the above (8), wherein the composition is for histamine H4 modulator.
(10) The pharmaceutical composition according to the above (9), wherein the histamine H4 modulator is histamine H4 receptor antagonist.
(11) The pharmaceutical composition according to the above (9), wherein the histamine H4 modulator is histamine H4 receptor partial agonist.
(12) The pharmaceutical composition according to the above (9), wherein the histamine H4 modulator is histamine H4 receptor inverse agonist.
(13) A method of preventing and/or treating a disease related to histamine H4 receptor comprising administrating the compound according to any one of the above (1) to (7), or its pharmaceutically acceptable salt, or a solvate thereof.
(14) Use of the compound according to any one of the above (1) to (7), or its pharmaceutically acceptable salt, or a solvate thereof in the manufacturing of an agent for treating and/or preventing a disease related to histamine H4 receptor.
(15) The compound, or its pharmaceutically acceptable salt, or a solvate thereof according to any one of the above (1) to (7) for treating and/or preventing a disease related to histamine H4 receptor.

In addition, for example, the present invention relates to the following item.
(1α) A compound represented by a formula (I): 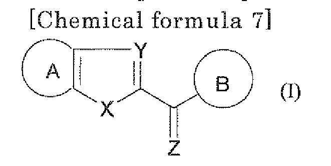 wherein
   A ring is a ring represented by the following formula: 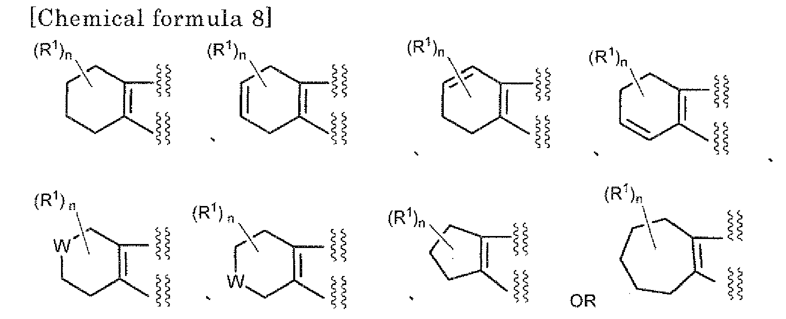 wherein
   R¹ is each independently one or more group(s) selected from the group consisting of group a) to group e) defined below;
   a)halogen, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, hydroxy, formyl, carboxy, substituted or unsubstituted alkyloxycarbonyl, substituted or unsubstituted carbamoyl, substituted or unsubstituted sulfamoyl, substituted or unsubstituted alkylsulfonyloxy, substitute or unsubstituted arylsulfonyloxy, substituted or unsubstituted alkylsulfinyl, substituted or unsubstituted arylsulfinyl, cyano, nitro, substituted or unsubstituted amino, substituted or unsubstituted cycloalkyl, substituted or unsubstituted cycloalkenyl, a substituted or unsubstituted non-aromatic heterocyclic group, substituted or unsubstituted aryl, substituted or unsubstituted heteroaryl, substituted or unsubstituted cycloalkyloxy, substituted or unsubstituted cycloalkenyloxy, substituted or unsubstituted non-aromatic heterocyclic ring-oxy, substituted or unsubstituted aryloxy, and substituted or unsubstituted heteroaryloxy,
   b) two R¹ on the same carbon atom are taken together =O and =NR³,
   c) two R¹ on the same carbon atom are taken together -(CR^{4a}R^{4b})ₓ-,
   d) two R¹ on the adjacent carbon atom are taken together -(CR^{5a}R^{5b})_{y}-, and
   e) two R¹ on the non-adjacent carbon atom are taken together -(CR^{6a}R^{6b})_{z}-, provided that R¹ is not selected from the group consisting of group c), group d) and group e) at the same time;
   W is -O-, -N(R²)- or -S(O)ₘ -;
   R² is hydrogen, substituted or unsubstituted alkyl, substituted or unsubstituted alkenyl, substituted or unsubstituted alkynyl, acyl, substituted or unsubstituted cycloalkyl, substituted or unsubstituted cycloalkenyl, substituted or unsubstituted non-aromatic heterocyclic group, substituted or unsubstituted aryl, or substituted or unsubstituted heteroaryl;
   m is an integer of 0 to 2;
   R³ is hydrogen, substituted or unsubstituted alkyl, hydroxy or substituted or unsubstituted alkyloxy;
   R^{4a}, R^{4b}, R^{5a}, R^{5b}, R^{6a} and R^{6b} are each independently hydrogen, halogen or substituted or unsubstituted alkyl;
   n is an integer of 0 to 6;
   x is an integer of 2 to 7;
   y is an integer of 1 to 5;
   z is an integer of 1 to 3;
   X is -N(R⁷)- or -O-;
   R⁷ is hydrogen, substituted or unsubstituted alkyl, substituted or unsubstituted cycloalkyl, substituted or unsubstituted acyl or substituted or unsubstituted alkyloxycarbonyl;
   Y is -C(R⁸)= or -N=;
   R⁸ is hydrogen, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylthio, acyl, hydroxy, formyl, carboxy, substituted or unsubstituted alkyloxycarbonyl, substituted or unsubstituted carbamoyl, substituted or unsubstituted sulfamoyl, cyano, nitro, or substituted or unsubstituted amino;
   Z is =O, =S, or =NR⁹;
   R⁹ is hydrogen, substituted or unsubstituted alkyl, hydroxyl or substituted or unsubstituted alkyloxy;
   B ring is a ring represented by the following formula: 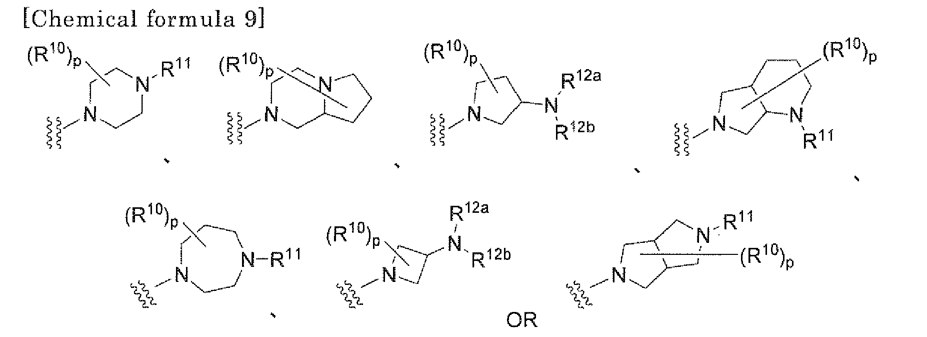 wherein
   R¹⁰ is each independently substituted or unsubstituted alkyl, halogen, hydroxy, substituted or unsubstituted alkyloxy, substituted or unsubstituted amino;
   R¹¹ is hydrogen or substituted or unsubstituted alkyl;
   R^{12a} and R^{12b} are each independently hydrogen or substituted or unsubstituted alkyl;
   p is an integer of 0 to 4;
   provided that R⁸ is not methyl when A ring is a ring represented by the following formula: 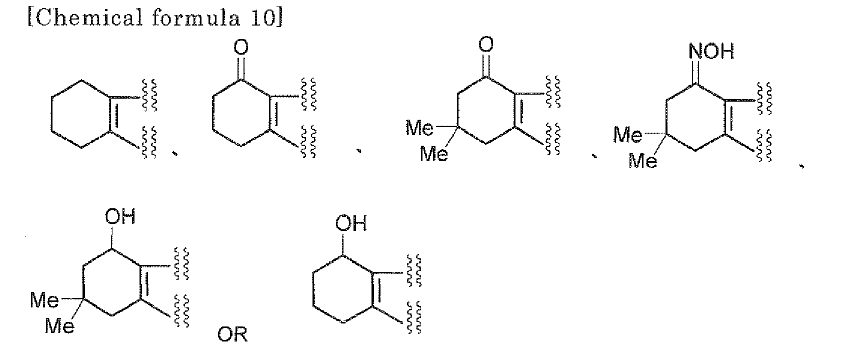 , or its pharmaceutically acceptable salt, or a solvate thereof.
(2α) The compound according to the above (1α), wherein Z is =O, or its pharmaceutically acceptable salt, or a solvate thereof.
(3α) The compound according to the above (1α) or (2α), wherein R¹ is one or more group(s) selected from the group consisting of group a) and group b), or its pharmaceutically acceptable salt, or a solvate thereof.
(4α) The compound according to any one of the above (1α) to (3α), wherein B ring is a ring represented by the following formula: 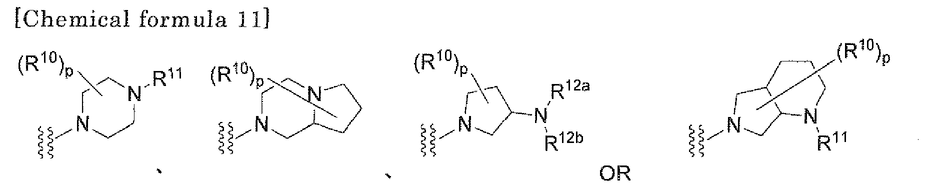 wherein R¹⁰, R¹¹, R^{12a}, R^{12b} and p are as defined in the above (1α), or its pharmaceutically acceptable salt, or a solvate thereof.
(5α) The compound according to any one of the above (1α) to (4α), wherein W is -O- or -N(R²)-, wherein R² is as defined in the above (1α), or its pharmaceutically acceptable salt, or a solvate thereof.
(6α) The compound according to any one of the above (1α) to (5α), wherein A ring is a ring represented by the following formula: 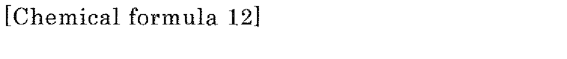 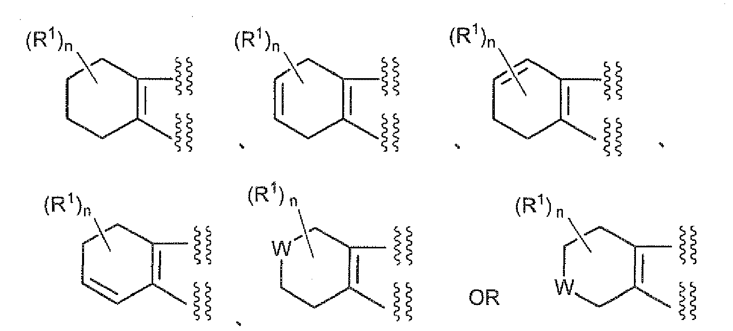 wherein R¹ is one or more group(s) selected from the group consisting of group a) and group b) in the above (1α), W and n are as defined in the above (1α), or its pharmaceutically acceptable salt, or a solvate thereof.
(7α) The compound according to the above (1α) to (6α), wherein X is -N(R⁷)-, wherein R⁷ is as defined in the above (1α), or its pharmaceutically acceptable salt, or a solvate thereof.
(8α) A pharmaceutical composition comprising the compound according to any one of the above (1α) to (7α), or its pharmaceutically acceptable salt, or a solvate thereof.
(9α) The pharmaceutical composition according to the above (8α), wherein the composition is for histamine H4 modulator.
(10α) The pharmaceutical composition according to the above (9α), wherein the histamine H4 modulator is histamine H4 receptor antagonist.
(11α) The pharmaceutical composition according to the above (9α), wherein the histamine H4 modulator is histamine H4 receptor partial agonist.
(12α) The pharmaceutical composition according to the above (9α), wherein the histamine H4 modulator is histamine H4 receptor inverse agonist.
(13α) A method of treating and/or preventing a disease or condition related to histamine H4 receptor comprising administrating the compound according to any one of the above (1α) to (7α), or its pharmaceutically acceptable salt, or a solvate thereof.
(14α) The compound, or its pharmaceutically acceptable salt, or a solvate thereof according to any one of the above (1α) to (7α) for treating and/or preventing a disease or condition related to histamine H4 receptor.

### [EFFECT OF THE INVENTION]

The compound of the present invention represented by the above general formula (I) has modulating effect to histamine H4 receptor (agonist, partial agonist, inverse agonist and antagonist, especially antagonist), is useful for the treatment of disease associated with histamine H4 receptor.
For example, it is efficacious for respiratory disease such as bronchial asthma, allergic rhinitis and chronic obstructive pulmonary disease(COPD); inflammatory disease such as chronic rheumatoid arthritis, atopic dermatitis, allergic conjunctivitis, psoriasis, inflammatory bowel disease, ulcerative colitis, lupus, atherosclerotic cardiovascular disease; pain relief for neurogenic pain and nociceptive pain.

The compound of the present invention can be a drug with reduced side-effect such as effect on motor function, because it has a high affinity to histamine receptor, especially histamine H4 receptor, and a high selectivity to subtype (selectivity to histamine H1, H2 and H3 receptor, especially H3 receptor) and other receptors. In addition, the compound of the present invention is advantageous because of its high stability, high oral absorptivity, high solubility, good bioavailability, low clearance, long half-life, prolonged duration of action, and/or low activity of hepatic enzyme inhibition, etc.

In another embodiment, the present invention provides a pharmaceutical composition comprising an effective amount of the compound of the present invention, in combination with a pharmaceutically acceptable carrier.

For use of the compound of the present invention as a medicament, a pharmaceutical composition can be prepared according to conventional methods, using pharmaceutically acceptable carriers well known in the art, such as excipients, binders, disintegrants, lubricants, colourants, flavors, corrigents, surfactants, etc.

For the pharmaceutical composition of the present invention to be administered in the treatment of mammals including human, an appropriate unit dosage form may be selected depending on the purpose of the treatment and the route of administration. Specifically, such unit dosage form includes oral formulations such as tablet, coated tablet, powder, granule, capsule, liquid, pill, suspension, emulsion, etc., and parenteral formulations such as injectable solution, suppository, ointment, patch, aerosol, etc. Such unit dosage form can be formulated according to methods well known in the art.

The amount of the compound of the present invention in a formulation can be varied depending on its dosage form, route for administration, dosing regimen, etc.

Means for administration of the pharmaceutical composition may be selected depending on dosage form, age, sex, body weight, severity of the disease, and other factors, etc., and route for administration can be selected from various routes such as oral, subcutaneous, transdermal, rectal, intranasal, buccal, etc.

Dose of the compound of the present invention in a pharmaceutical composition of the present invention can be determined depending on the choice of route for administration, age, sex, body weight, severity of the disease, the present compound to be administered, and other factors, etc., and can be generally from 0.05 to 1000 mg/kg/day, preferably from 0.1 to 10 mg/kg/day, for oral administration to adults. For parenteral administration, dose can be varied widely depending on its route but generally from 0.005 to 100 mg/kg/day, preferably from 0.01 to 1 mg/kg/day. Such pharmaceutical composition of the present invention may be administered once a day or in several times at a divided dosage in a day.

### [MODE FOR CARRYING OUT THE INVENTION]

Hereinafter, the present invention is described with reference to embodiments. It should be understood that, throughout the present specification, the expression of a singular form includes the concept of its plural form unless specified otherwise. Accordingly, it should be understood that an article in singular form (for example, in the English language, "a", "an", "the", and the like) includes the concept of its plural form unless specified otherwise. Furthermore, it should be understood that the terms used herein are used in a meaning normally used in the art unless specified otherwise. Thus, unless defined otherwise, all technical and scientific terms used herein have the same meaning as those generally understood by those skilled in the art in the field to which the present invention pertains. If there is a contradiction, the present specification (including definitions) precedes.
Each meaning of terms used herein is described below. Each term has the following meaning unless specifically meditated.

The term "halogen" means fluorine, chlorine, bromine and iodine.

The halogen moiety in said "haloalkyl" and "haloalkyloxy", is as defined, above for "halogen".

The term "alkyl" includes a straight or branched chain monovalent hydrocarbon group containing from I to 10 carbon(s), preferably from 1 to 8 carbon(s), more preferably from 1 to 6 carbon(s). For example, methyl, ethyl, n-propyl, isopropyl, n-butyl, isobutyl, sec-butyl, tert-butyl, n-pentyl, isopentyl, neo-pentyl, n-hexyl, isohexyl, n-heptyl, isoheptyl, n-octyl, isooctyl, n-nonyl, n-decyl, etc. are exemplified.

The alkyl moiety in said "haloalkyl", "alkylamino", "alkylimino", "alkylsulfonyl", "alkylsulfamoyl", "alkylcarbamoyl", "arylalkyl", "arylalkylamino", "alkylsulfinyl" is as defined above for "alkyl".

The alkyl moiety in said "alkyloxy" is as defined above for "alkyl". For example, methoxy, ethoxy, propoxy, isopropoxy, butoxy, isobutoxy, sec-butoxy, tert-butoxy, pentoxy, isopentoxy, neopentoxy, hexyloxy, etc. are exemplified.

The alkyloxy moiety in said "haloalkyloxy", "alkyloxyimino" is as defined above for "alkyloxy".

The alkyl moiety in said "alkylthio" is as defined above for "alkyl". For example, methylthio, ethylthio, propylthio, isopropylthio, butylthio, isobutylthio, sec-butylthio, tert-butylthio, pentylthio, isopentylthio, neopentylthio, hexylthio, etc. are exemplified.

The alkyloxy moiety in said "alkyloxycarbonyl" is as defined above for "alkyloxy". For example, methyloxycarbonyl, ethyloxycarbonyl, n-propyloxycarbonyl, isopropyloxycarbonyl, n-butyloxycarbonyl, tert-butyloxycarbonyl, n-pentyloxycarbonyl, etc. are exemplified.

The alkyl moiety in said "alkylcarbamoyl" is as defined above for "alkyl". For example, methylcarbamoyl, ethylcarbamoyl, n-propylearbamoyl, isopropylcarbamoyl, cyclopropylcarbamoyl, n-butylcarbarnoyl, dimethylcarbamoyl, diethylcarbamoyl, dipropylcarbamoyl, etc., mono- or di-alkylcarbamoyl are exemplified.

The alkyl moiety in said "alkylsulfonyloxy" is as defined above for "alkyl". For example, methylsulfonyloxy, ethylsulfonyloxy, n-propylsulfonyloxy, isopropylsulfonyloxy, n-butylsulfonyloxy, tert-butylsulfanyloxy, n-pentylsulfonyloxy, etc. are exemplified.

The term "alkenyl" includes a straight or branched chain alkenyl containing from 2 to 6 carbons, preferably containing from 2 to 3 carbons having one or more double bond(s) at any position. For example, vinyl, propenyl, isopropenyl, butenyl, isobutenyl, prenyl, butadienyl, pentenyl, isopentenyl, pentadienyl, hexenyl, isohexenyl, hexadienyl, etc. are exemplified.

The alkenyl moiety in said "alkenyloxy" is as defined above for "alkenyl". For example, vinyloxy, propenyloxy, isopropenyloxy, butenyloxy, isobutenyloxy, prenyloxy, butadienyloxy, pentenyloxy, isopentenyloxy, pentadienyloxy, hexenyloxy, isohexenyloxy, hexadienyloxy, etc. are exemplified.

The alkenyl moiety in said "alkenylthio," is as defined above for "alkenyl,". For example, vinylthio, propenylthio, isopropenylthio, butenylthio, isobutenylthio, prenylthio, butadienylthio, pentenylthio, isopentenylthio, pentadienylthio, hexenylthio, isohexenylthio, hexadienylthio, etc. are exemplified.

The term "alkynyl" includes a straight or branched chain alkynyl containing from 2 to 6 carbons, preferably containing from 2 to 3 carbons. The term "alkynyl," has one or more triple bond(s) at any position, moreover, it can possess double bond(s). For example, ethynyl, 1-propynyl, 2-propynyl, 1-butynyl, 2-butynyl, 3-butynyl, 1-pentynyl, 2-pentynyl, various pentynyl isomers, etc. are exemplified.

The alkynyl moiety in said "alkynyloxy" is as defined above for "alkynyl". For example, ethynyloxy, propynyloxy, butynyloxy, pentynyloxy, etc. are exemplified. Preferably, C2 to C6 alkynyloxy is exemplified. In particular, C2 to C4 alkynyloxy is preferred.

The alkynyl moiety in said "alkynylthio" is as defined above for "alkynyl". For example, ethynylthio, propynylthio, butynylthio, pentynylthio, etc. are exemplified. Preferably, C2 to C6 alkynylthio is exemplified. In particular, C2 to C4 alkynylthio is preferred.
The term "acyl" includes a group of the formula R-C(=O)-, wherein R is, for example, "alkyl" or "alkenyl", as defined above or "aryl", "a heterocyclic group", "cycloalkyl", "cycloalkenyl", "arylalkyl" or "heteroarylalkyl" as defined below. The each said terms can be optionally substituted with hydroxy, carboxy, "alkyl", "alkenyl" "alkynyl" "halogen", "alkyloxy", "alkenyloxy", "alkynyloxy", "alkylthio", "carbamoyl", "alkyloxycarbonyl", "aryloxycarbonyl", etc. as defined above or below.

The acyl moiety in said "acyl amino" and "acyl imino" is as defined above for "acyl".

The term "sulfinyl" includes a group of the formula -S(=O)-R, wherein R is, "alkyl" or "alkenyl" as defined above or "aryl", "heterocyclic group", "cycloalkyl", "cycloalkenyl ", "arylalkyl" or "heteroarylalkyl" as defined below. The each said terms can be optionally substituted with hydroxy, carboxy, "alkyl", "alkenyl", "alkynyl", "halogen", "alkyloxy", "alkenyloxy", "alkynyloxy", "alkylthio", "carbamoyl", "alkyloxycarbonyl", "aryloxycarbonyl", etc. as defined above or below.

The term "unsubstituted carbamoyl" includes a group of the formula -C(=O)-NH₂.

The term "unsubstituted sulfamoyl" includes a group of the formula -S(=O)₂-NH₂.

The term "cycloalkane" includes a monocyclic or polycyclic saturated carbocyclic ring containing from 3 to 10 carbons. Monocyclic cycloalkane includes, for example, cyclopropane, cyclobutane, cyclopentane, cyclohexane, cycloheptane, cyclooctane, cyclononane, cyclodecane, etc. Polycyclic cycloalkane includes norbornanane, tetrahydronaphthalene, etc.

The term "cycloalkyl" includes a monovalent group derived from "cycloalkane" as defined above. Monocyclic cycloalkyl includes, for example, cyclopropyl, cyclobutyl, cyclopentyl, cyclohexyl, cycloheptyl, cyclooctyl, cyclononyl, cyclodecyl, etc. Polycyclic cycloalkyl incudes norbornanyl, tetrahydronaphthalene-5-yl, tetrahydronaphthalene-6-yl, etc.

The term "cycloalkane-diyl" includes a divalent group derived from "cycloalkane" as defined above. Monocyclic cycloalkane-diyl includes, for example, cyclopropane-diyl, cyclobutane-diyl, cyclapentane-diyl, cyclohexane-diyl, cycloheptane-diyl, cyclooctane-diyl, cyclonone-diyl, cyclodecane-diyl, etc. Polycyclic cycloalkane-diyl includes norbornane-diyl, etc.

The cycloalkyl moiety in said "cycloalkyloxy" is as defined above for "cycloalkyl". For example, cyclopropyloxy, cyclobutyloxy, cyclopentyloxy, cyclohexyloxy, cycloheptyloxy, cyclooctyloxy, cyclononyloxy, cyclodecyloxy, etc. are exemplified.

The term "cycloalkene" includes a non-aromatic monocyclic or polycyclic ring of 3 to 10 carbons containing at least one carbon-carbon double bond. Monocyclic cycloalkene includes cyclopentene, cyclohexene, etc. Polycyclic cycloalkene includes norborne, indene, etc.

The term "cycloalkenyl" includes a monovalent group derived from "cycloalkene" as defined above. Monocyclic cycloalkenyl includes cyclopentenyl, cyclohexenyl, etc. Polycyclic cycloalkenyl includes norbornyl, indene-1-yl, indene-2-yl, indene-3-yl, etc.

The term "cycloalkene-diyl" includes a divalent group derived from "cycloalkane" as defined above. Monocyclic cycloalkene-diyl includes cyclopentene-diyl, cyclohexene-diyl, etc. Polycyclic cycloalkene-diyl includes norbornene-diyl, etc.

The cycloalkenyl moiety in said "cycloalkenyloxy" is as defined above for "cycloalkenyl". For example, monocyclic cycloalkenyloxy includes cyclopentenyloxy, cyclohexenyloxy, etc. Polycyclic cycloalkenyloxy includes norbornyloxy, indenyloxy, etc.

The term "aromatic carbocyclic ring" includes an aromatic hydrocarbocyclic ring which is monocyclic or fused-cyclic. For example, benzene ring, naphthalene ring, anthracene ring, phenanthrene ring, etc. are exemplified.

The "aryl" includes a monovalent group derived from "aromatic carbocyclic ring" as defined above. For example, phenyl, 1-naphthyl, 2-naphthyl, anthryl, phenanthryl, etc. are exemplified.

The aryl moiety in said "arylsylfonyloxy" is as defined above for "aryl". For example, phenylsulfonyloxy, 1- naphthylsulfonyloxy, etc. are exemplified.

The term "arylalkyl" includes a group of "alkyl" as defined above substituted with "aryl" as defined above. For example, benzyl, phenethyl, etc. are exemplified.
The aryl moiety in said "aryloxy", "arylalkylamino", "arylsulfinyl," and "arylalkyl" is as defined above for "aryl".

The term "aromatic carbocyclic ring-diyl" includes a divalent group derived from "aromatic carbocyclic ring" as defined above. For example, 1,2-phenylene, 1,3-phenylene, 1,4-phenylene, 1,2-naphthalene, etc. are exemplified.

The term "heterocyclic ring" includes an aromatic or a non-aromatic monocyclic or fused-cyclic ring, which includes a five- to seven-membered ring having at least one nitrogen atom, oxygen atom, and/or sulphur atom in the ring; a fused ring consisting of two or more said five- to seven-membered rings; or a fused ring consisting of said five- to seven-membered ring having at least one nitrogen atom, oxygen atom, and/or sulphur atom in the ring fused to one or more "aromatic carbocyclic ring", "cycloalkane " or "cycloalkene" as defined above.
For example, a monocyclic non-aromatic heterocyclic ring such as pyrroline, pyrrolidine, piperidine, piperazine, morpholine, thiomorpholine, tetrahydropyrane, dihydropyridine, dihydropyridazine, dioxane, oxathiolane, thiane, tetrahydrofuran, tetrahydropyran, tetrahydrothiazole, tetrahydroisothiazole, etc.;
for example, a monocyclic aromatic heterocyclic ring such as pyrrole, pyrazine, pyrazole, tetrazole, furan, thiophene, pyridine, imidazole, triazole, tetrazole, triazine, pyridazine, pyrimidine, isoxazole, thiazole, isothiazole, thiadiazole, oxazole, oxadiazole, etc; and
for example, a fused heterocyclic ring such as indole, isoindole, indazole, indolizine, indoline, isoindoline, quinoline, isoquinoline, cinnoline, phthalazine, quinazoline, naphthyridine, quinoxaline, purine, pteridine, benzopyrane, benzimidazole, benzisoxazole, benzoxazole, benzoxadiazole, benzisothiazole, benzothiazole, benzothiadiazole, benzofuran, isobenzofuran, benzothiophene, benzotriazole, imidazopyridine, triazolopyridine, imidazothiazole, pyrazinopyridazine, benzimidazole, benzodioxane, tetrahydroquinoline, tetrahydrobenzothiophene, etc. are exemplified.

The term "heterocyclic group" includes a monovalent group derived from "heterocyclic ring" as defined above.
For example, a monocyclic non-aromatic heterocyclic groups such as pyrrolinyl, pyrrolidino, pyrrolidinyl, imidazolynyl, imidazolidinyl, pyrazolinyl, pyrazolidinyl, piperidino, piperidyl, piperadino, piperazinyl, morpholinyl, morpholino, thiomorpholinyl, thiomorpholino, tetrahydropyranyl, dihydropyridyl, dihydropyridazinyl, dihydropyrazinyl, dioxanyl, oxathiolanyl, thianyl, tetrahydrofuryl, tetrahydropyranyl, tetrahydrothiazolinyl, tetrahydroisothiazolinyl, etc.;
for example, a monocyclic aromatic heterocyclic groups such as pyrrolyl, pyrazinyl, pyrazolyl, tetrazolyl, furyl, thienyl, pyridyl, imidazolyl, triazolyl, tetrazolyl, triazinyl, pyridazinyl, pyrimidinyl, pyrazinyl, isoxazolyl, thiazolyl, isothiazolyl, thiadiazolyl, oxazolyl and oxadiazolyl, etc; and
for example, a fused heterocyclic groups such as indolyl, isoindolyl, indazolyl, indolizinyl, indolinyl, isoindolinyl, quinolyl, isoquinolyl, cinnolinyl, phthalazinyl, quinazolinyl, naphthyridinyl, quinoxalinyl, purinyl, pteridinyl, benzopyranyl, benzimidazolyl, benzisoxazolyl, benzoxazolyl, benzoxadiazolyl, benzisothiazolyl, benzothiazolyl, benzothiadiazolyl, benzofuryl, isobenzofuryl, benzothienyl, benzotriazolyl, imidazopyridyl, triazolopyridyl, imidazothiazolyl, pyrazinopyridazinyl, benzimidazolynyl, benzodioxanyl, tetrahydroquinoline, tetrahydrobenzothienyl, etc. are exemplified.

The term "heterocyclic ring-diyl" includes a divalent group derived from "heterocyclic ring" as defined above.
For example, a monocyclic non-aromatic "heterocyclic ring-diyl such as pyrrolin-diyl, pyrrolidin-diyl, imidazolidin-diyl, pyrazolin-diyl, pyrazolidin-diyl, piperidin-diyl, piperazine-diyl, morpholin-diyl, thiomorpholin-diyl, tetrahydropyran-diyl, dihydropyridine-diyl, dihydropyridazin-diyl, dihydropyrazin-diyl, dioxan-diyl, oxathiolan-diyl, thian-diyl, tetrahydrofuran-diyl, tetrahydropyran-diyl, tetrahydrothiazol-diyl, tetrabydroisothiazol-dlyl, etc.;
for example, a monocyclic aromatic heterocyclic ring-diyl such as pyrrole-diyl, pyrazine-diyl, pyrazole-diyl, tetrazole-diyl, furan-diyl, thlophene-diyl, pyridine-diyl, imidazole-diyl, triazole-diyl, tetrazale-diyl, triazine-diyl, pyridazine-diyl, pyrimidinediyl, pyrazine-diyl, isoxazole-diyl, thiazole-diyl, isothiazole-diyl, thiadiazole-diyl, oxazole-diyl, oxadiazole-diyl, etc.; and
for example, a fused heterocyclic ring-diyl such as indole-diyl, isoindole-diyl, indazole-diyl, indolizine-diyl, indoline-diyl, isoindoline-dlyl, quinoline-diyl, isoquinoline-diyl, cinnoline-diyl, phthalazine-diyl, quinazoline-diyl, naphthyridine-diyl, quinoxaline-diyl, purine-diyl, pteridine-diyl, benzopyrane-diyl, benzimidazole-diyl, benzisoxazole-diyl, benzoxazole-diyl, benzoxadiazole-diyl, benzisothiazole-diyl, benzothiazole-diyl, benzothiadiazole-diyl, benzofuran-diyl, isobenzofuran-diyl, benzothiophene-diyl, benzotriazole-diyl, imidazopyridine-diyl, triazolopyridine-diyl, imidazothiazole-diyl, pyrazinopyridazine-diyl, benzimidazole-diyl, benzodioxane-diyl, tetrahydroquinoline-diyl, tetrahydrobenzothiophene-diyl, etc. are exemplified.

The term "non-aromatic carbocyclic ring" includes a ring selected from "cycloalkene" as defined above and "cycloalkene" as defined above. As a fused ring, indene, etc. are exemplified.

The term "aromatic heterocyclic ring" inculeds aromatic rings of "heterocyclic ring" as defined above. "Aromatic heterocyclic ring" includes a five- to seven-membered aromatic ring having at least one nitrogen atom, oxygen atom, and/or sulphur atom in the ring; a fused aromatic ring consisting of two or more said rings; and a fused ring consisting of a five- to seven-membered aromatic ring having at least one nitrogen atom, oxygen atom, and/or sulphur atom in the ring fused to one or more "aromatic carbocyclic ring" as defined above.
For example, a monocyclic aromatic heterocyclic ring such as pyrazine, pyrazole, tetrazole, furan, thiophene, pyridine, imidazole, triazole, triazine, pyridazine, pyrimidine, pyrazine, isoxazole, thiazole, isothiazole, thiadiazole, oxazole, oxadiazole, etc; and
for example, a fused aromatic heterocyclic ring such as indole, isoindole, indazole, indolizine, quinoline, isoquinoline, cinnoline, phthalazine, quinazoline, naphthyridine, quinoxaline, purine, pteridine, benzimidazole, benzisoxazole, benzoxazole, benzoxadiazole, benzisothiazole, benzothiazole, benzothiadiazole, benzofuran, isobenzofuran, benzothiophene, benzotriazole, imidazopyridine, triazolopyridine, imidazothiazole, pyrazinopyridazine, benzixnidazoline, etc. are exemplified.

The term "heteroaryl" includes a monovalent group derived from "aromatic heterocyclic ring" as defined above. "Heteroaryl" includes a five- to seven-membered aromatic group having at least one nitrogen atom, oxygen atom, and/or sulphur atom in the ring;
a fused aromatic group consisting of two or more said five- to seven membered rings;
and a fused ring consisting of said five- to seven-membered aromatic group having at least one nitrogen atom, oxygen atom, and/or sulphur atom in the ring fused to one or more "aromatic carbocyclic ring" as defined above.
For example, a monocyclic heteroaryl such as pyrrolyl, pyrazinyl, pyrazolyl, indolyl, tetrazolyl, furyl, thienyl, pyridyl, imidazolyl, triazolyl, tetrazolyl, triazinyl, pyridazinyl, pyrimidinyl, pyrazinyl, isoxazolyl, thiazolyl, isothiazolyl, thiadiazolyl, oxazolyl, oxadiazolyl, etc; and
for example, a fused heteroaryl such as isoindolyl, indazolyl, indolizinyl, isoindolinyl, quinolyl, isoquinolyl, cinnolinyl, phthalazinyl, quinazolinyl, naphthyridinyl, quinoxalinyl, purinyl, pteridinyl, benzimidazolyl, benzisoxazolyl, benzoxazolyl, benzoxadiazolyl, benzisothiazolyl, benzothiazolyl, benzothiadiazolyl, benzofuryl, isobenzofuryl, benzothienyl, benzotriazolyl, imidazopyridyl, triazolopyridyl, imidazothiazolyl, pyrazinopyridazinyl, benzimidazolynyl, etc. are exemplified.
The heteroaryl moiety in said "heteroaryloxy","heteroarylalkylamino" and "heteroarylalkyl" is as defined above for "heteroaryl".

The term "non-aromatic heterocyclic ring" includes non-aromatic rings of "heterocyclic ring" as defined above.
"Non-aromatic heterocyclic ring" includes, a five- to seven-membered non-aromatic ring having at least one nitrogen atom, oxygen atom, and/or sulphur atom in the ring;
a fused non-aromatic ring consisting of two or more said rings;
a fused ring consisting of a five- to seven-membered aromatic ring having at least one nitrogen atom, oxygen atom, and/or sulphur atom in the ring fused to one or more "cycloalkane" as defined above or "cycloalkane" as defined above;
or a fused ring consisting of a five- to seven-membered non-aromatic heterocyclic ring having at least one nitrogen atom, oxygen atom, and/or sulphur atom in the ring fused to one or more "aromatic carbocyclic ring" as defined above or "non-aromatic carbocyclic ring" as defined above.
For example, a monocyclic non-aromatic heterocyclic ring such as pyrroline, pyrrolidine, imidazoline, imidazolidine, pyrazoline, pyrazolidine, piperidine, piperazine, morpholine, thiomorpholine, thiomorpholine, tetrahydropyrane, dihydropyridine, dihydropyridazine, dihydropyrazine, dioxane, oxathiolane, thiane, tetrahydrofuran, tetrahydropyran, tetrahydrothiazolin, tetrahydroisothiazolin, etc.;
for example, a fused non-aromatic heterocyclic ring such as indoline, isoindoline, benzopyrane, benzodioxane, tetrahydroquinoline, benzo[d]oxazole-2(3H)-one, tetrahydrobenzothiophene, etc. are exemplified.

The term "non-aromatic heterocyclic group" includes a monovalent group derived from "non-aromatic heterocyclic ring" as defined above.
For example, a monocyclic non-aromatic heterocyclic groups such as pyrrolinyl, pyrrolidino, pyrrolidinyl, imidazolynyl, imidazolidinyl, pyrazolinyl, pyrazolidinyl, piperidino, piperidyl, piperadino, piperazinyl, morpholinyl, morpholino, thiomorpholinyl, thiomorpholino, tetrahydropyranyl, dihydropyridyl, dihydropyridazinyl, dihydropyrazinyl, dioxanyl, oxathiolanyl, thianyl, tetrahydrofuryl, tetrahydropyranyl, tetrahydrothiazolinyl, tetrahydroisothiazolinyl, etc.; and fused heterocyclic groups such as benzodioxanyl, tetrahydroquinolinyl, benzo[d]oxazolyl-2(3H)-one, tetrahydrobenzothiophenyl, etc. are exemplified.

The non-aromatic heterocyclic ring moiety in said "non-aromatic heterocyclic ring-oxy", "non-aromatic heterocyclic ring-alkylamino" and "non-aromatic heterocyclic ring-alkyl" is as defined above for "non-aromatic heterocyclic ring".

Substituents for "substituted alkyl", "substituted alkenyl", "substituted alkynyl", "substituted alkyloxy", "substituted alkenyloxy", "substituted alkynyloxy", "substituted alkylthio", "substituted alkenylthio", "substituted alkynylthio", "substituted alkyloxycarbonyl", "substituted alkylcarbamoyl", "substituted alkylsulfonyloxy", and "substituted alkylsulfinyl" include, but are not limited to, one or more same or different substituent(s) selected from the group comprising: deuterium, hydroxy, carboxy, halogen (F, Cl, Br, 1), haloalkyloxy (e.g., CF₃O), cycloalkyl (e.g., cyclopropyl), cycloalkenyl (e.g., cyclopropenyl), alkyloxy (e.g., methoxy, ethoxy, propoxy, butoxy, etc.), alkenyloxy (e.g., vinyloxy, allyloxy, etc.), alkyloxycarbonyl (e.g., methoxycarbonyl, ethoxycarbonyl, tert-butoxycarbonyl, etc.), nitro, nitroso, amino, alkylamino (e.g., methylamino, ethylamino, dimethylamino, etc.), acylamino (e.g., acetylamino, benzoylamino, etc.), arylalkylamino (e.g., benzylamino, tritylamino), hydroxyamino, imino, hydroxyimino, alkylimino (e.g., methylimino, ethylimino, dimethylimino, etc.), alkyloxyimino (e.g., methoxyimino, ethoxyimino, etc.), acylimino (e.g., acetylimino, benzoylimino, etc.), azido, aryl (e.g., phenyl, etc.), arylalkyl (e.g., benzyl, phenylethyl, etc.), arylalkyloxy (e.g., benzyloxy), a non-aromatic heterocyclic group (e.g., pyrrolinyl, piperidyl, piperadino, pyrrolidino, pyrrolidinyl, morpholinyl, morpholino, etc.), heteroaryl (e.g., furyl, thienyl, pyridyl, isoxazolyl, thiazolyl, thiadiazolyl, oxazolyl, oxadiazolyl, etc.), heteroarylalkyl (e.g., pyridylmethyl, pyridylethyl, etc.), cyano, isocyano, isocyanato, thiocyanato, isothiocyanato, mercapto, alkylthio (e.g., methylthio, etc.), alkylsulfonyl (e.g., methanesulfonyl, ethanesulfonyl), carbamoyl, alkylcarbamoyl (e.g., methylcarbamoyl, ethylcarbamoyl, dimethylcarbamoyl, etc.), sulfamoyl, alkylsulfamoyl, formyl, acyl (e.g., acetyl, etc.), formyloxy, thioformyl, thiocarboxy, dithiocarboxy, thiocarbamoyl, sulfino, sulfo, hydrazino, azido, ureido, amidino, guanidino, phthalimido, trialkylsilyl (e.g., trimethylsilyl, etc.), and oxo.

A substituent for "substituted acyl" is selected from the group comprising substituents for "substituted alkyl" as defined above, "alkyl" as defined above, "alkenyl" as defined above, and "alkynyl" as defined above. Especially, when R of acyl (R-C(=O)-) is "aryl", "heterocyclic group", "cycloalkyl", "cycloalkenyl", "arylalkyl" or "heteroarylalkyl", a substituent for each ring is alkyl(e.g., methyl, ethyl, isopropyl, tert-butyl, etc.), haloalkyl(e.g., CF₃, CH₂CF₃, CH₂CCl₃ , etc.), alkenyl, alkynyl(e.g., ethynyl), etc.

A substituent for "substituted carbamoyl" or "substituted sulfamoyl" is, but is not limited to, one or more same or different substituent(s) selected from the group comprising: hydroxy, carboxy, halogen(.F, Cl, Br, I), alkyl(e.g., methyl, ethyl), alkenyl(e.g., vinyl), alkynyl(e.g., ethynyl), cycloalkyl(e.g., cyclopropyl), cycloalkenyl(e.g., cyclopropenyl), alkyloxycarbonyl(e.g., methoxycarbonyl, ethoxycarbonyl, tert-butoxycarbonyl, etc.), amino, alkylamino(e.g., methylamino, ethylamino, dimethylamino, etc.), acylamino(e.g., acetylamino, benzoylamino, etc.), arylalkylamino(e.g., benzylamino, tritylamino), hydroxyamino, aryl(e.g., phenyl, etc.), cyano, isocyano, isocyanato, thiocyanato, isothiocyanato, formyl and acyl(e.g., acetyl, etc.).

A substituent for "substituted amino" is, but is not limited to, one or more same or different substituent(s) selected from the group comprising: alkyl(e.g., methyl, ethyl, isopropyl, tert-butyl, etc.), haloalkyl(e.g., CF₃, CH₂CF₃, CH₂CCl₃, etc.), hydroxyalkyl(e.g., hydroxyethyl, -C(CH₃)₂ CH₂ OH, etc.), alkenyl(e.g., vinyl), alkynyl(e.g., ethynyl), cycloalkyl(e.g., cyclopropyl), cycloalkenyl(e.g., cyclopropenyl), alkyloxy(e.g., methoxy, ethoxy, propoxy, butoxy, etc.), haloalkyloxy(e.g., CF₃O), alkenyloxy(e.g., vinyloxy, allyloxy, etc.), alkyloxycarbonyl(tert-butyloxycarbonyl, etc.), amino, alkylamino(e.g., methylamino, ethylamino, dimethylamino, etc.), acylamino(e.g., acetylamino, benzoylamino, etc.), arylalkylamino(e.g., benzylamino, tritylamino), hydroxyamino, imino, hydroxyimino, alkylimino(e.g., methylimino, ethylimino, dimethylimino, etc.), alkyloxyimino(e.g., methoxyimino, ethoxyimino, etc.), acyl imino(e.g., acetylimino, benzoylimino, etc.), aryl(e.g., phenyl, etc.), arylalkyl(e.g., benzyl, etc.), aryloxy(e.g., phenoxy, etc.), a non-aromatic heterocyclic group(e.g., pyrrolinyl, pyrrolidino, piperidino, piperidyl, piperadino, piperazinyl, morpholinyl, morpholino, etc.), heteroaryl(e.g., pyridyl, thienyl, thiazolyl, furyl, etc.), heteroarylalkyl(e.g., pyridylmethyl., thienylmethyl, thiazolylmethyl, furylmethyl, etc.), non-aromatic heterocyclic ring-oxy(piperadinooxy, piperidinooxy, etc.), heteroaryloxy(pyridyloxy, etc.), hydroxy, halogen(F, Cl, Br, I), cyano, formyl and acyl(e.g., acetyl, etc.).

A substituent for "substituted cycloalkyl", "substituted cycloalkenyl", substituted aryl", "a substituted heterocyclic group", "substituted heteroaryl", "substituted arylalkyl", "substituted heteroarylalkyl", "a substituted non-aromatic heterocyclic group", "substituted phenyl", "substituted pyridyl", "substituted arylsulfonyloxy", "substituted arylsulfinyl", "substituted cycloalkenyloxy", "substituted non-aromatic heterocyclic ring-oxy", "substituted aryloxy"or "substituted heteroaryloxy" is, but is not limited to, one or more same or different substituent(s) selected from the group comprising: alkyl(e.g., methyl, ethyl, isopropyl, tert-butyl, etc.), haloalkyl(e.g., CF₃, CH₂CF₃, CH₂CCl₃, etc.), haloalkyloxy(e.g., CF₃O, CHCF₂O, etc.), alkenyl(e.g., vinyl), alkynyl(e.g., ethynyl), cycloalkyl(e.g., cyclopropyl), cycloalkenyl(e.g., cyclopropenyl), alkyloxy(e.g., methoxy, ethoxy, propoxy, butoxy, etc.), alkenyloxy(e.g., vinyloxy, allyloxy, etc.), alkyloxycarbonyl(e.g., methoxycarbonyl, ethoxycarbonyl, tert-butoxycarbonyl, etc.), nitro, nitroso, amino, alkylamino(e.g., methylamino, ethylamino, dimethylamino, etc.), acylamino(e.g., acetylamino, benzoylamino, etc.), arylalkylamino(e.g., benzylamino, tritylamino), hydroxyamino, imino, hydroxyimino, alkylimino(e.g., methylimino, ethylimino, dimethylimino, etc.), alkyloxyimino(e.g., methoxyimino, ethoxyimino, etc.), acyl imino(e.g., acetylimino, benzoylimino, etc.), azido, aryl(e.g., phenyl, etc.), arylalkyl(e.g., benzyl, etc.), aryloxy(e.g., phenoxy, etc.), arylalkyloxy(e.g., benzyloxy, etc.), a non-aromatic heterocyclic group(e.g., pyrrolinyl, pyrrolidino, piperidino, piperidyl, piperadino, piperazinyl, morpholinyl, morpholino, etc.), heteroaryl(e.g., pyridyl, thienyl, thiazolyl, furyl, etc.), heteroarylalkyl(e.g., pyridylmethyl, thienylmethyl, thiazolylmethyl, furylmethyl, etc.), non-aromatic heterocyclic ring-oxy(piperadinooxy, piperidinooxy, etc.), heteroaryloxy(pyridyloxy, etc.), cyano, isocyano, isocyanato, thiocyanato, isothiocyanato, mercapto, alkylthio(e.g., methylthio, etc.), alkylsulfanyl(e.g., methanesulfonyl, ethanesulfonyl), substituted or unsubstituted carbamoyl(e.g., carbamoyl, N-methyl-N-methoxycarbamoyl, etc.), substituted or unsubstituted alkylcarbamoyl(e.g., methylcarbamoyl, ethylcarbamoyl, dimethylcarbamoyl, hydroxyethylcarbamoyl, trifluoromethylcarbamoyl, trifluoroethylcarbamoyl, etc:), sulfamoyl, alkylsulfamoyl, hydroxy, carboxy, halogen(F, Cl, Br, I), formyl, acyl(e.g., acetyl, etc.), formyloxy, thioformyl, thiocarboxy, dithiocarboxy, thiocarbamoyl, sulfino, sulfo, hydrazino, azido, ureido, amidino, guanidino, phthalimido and oxo.

In the present specification, when a bonding hand of substituent R¹⁰ in the general formula (I) bridges on two rings, R¹⁰ can substitute on each ring. In addition, in the present specification, when a bonding hand of substituent R¹⁰ substitutes on only one ring, R¹⁰ can substitute on the ring.

In the compound of the present invention, a tautomer, regioisomer, and/or optical isomer thereof may exist. The present invention encompasses all possible isomers including these, and mixtures thereof.

Moreover, one or more hydrogen, carbon, or other atoms in the compound of general formula (I) may be replaced with isotopes of hydrogen, carbon, or other atoms respectively. The compound of general formula (I) encompasses all of radiolabeled compounds of the compound of general formula (I). Such "radiolabeling", "a radiolabeled compound", and the like of the compound of general formula (I) are each encompassed by the present invention, and are useful for studies on metabolized drug pharmacokinetics and studies on binding assay, and/or a diagnostic tool.
Examples of isotopes that may be incorporated in the compound of general formula (I) include hydrogen, carbon, nitrogen, oxygen, phosphorus, sulfur, fluorine, and chlorine, such as ²H, ³H, ¹³C, ¹⁴C, ¹⁵N, ¹⁸O, ¹⁷O, ³¹P, ³²P, ³⁵S, ¹⁸F, and ³⁶Cl respectively. A radiolabeled compound of the present invention can be prepared using a well-known method in the relevant technical field. For example, a tritium-labeled compound of general formula (I) can be prepared by introducing a tritium to a certain compound of general formula (I), for example, through a catalytic dehalogenation reaction using a tritium. This method may comprise reacting with an appropriately-halogenated precursor of the compound of general formula (I) with tritium gas in the presence of an appropriate catalyst, such as Pd/C, and in the presence or absent of a base. For another appropriate method of preparing a tritium-labeled compound, the document: Isotopes in the Physical and Biomedical Sciences, Vol. 1, Labeled Compounds (Part A), Chapter 6 (1987) can be referred to. A ¹⁴C-labeled compound can be prepared by using a raw material having ¹⁴C.

The term "solvate" herein includes, for example, solvates with organic solvents and hydrates. In the case that a hydrate is formed, the compound of the present invention may be coordinated with any number of water molecules.

The compound of the present invention includes pharmaceutically acceptable salts thereof. Examples thereof include salts with alkaline metals (e.g. lithium, sodium and potassium), alkaline earth metals (e.g. magnesium and calcium), ammonium, organic bases and amino acids, and salts with inorganic acids (e.g. hydrochloric acid, sulfuric acid, nitric acid, hydrobromic acid, phosphoric acid and hydroiodic acid) and organic acids (e.g. acetic acid, citric acid, lactic acid, tartaric acid, oxalic acid, maleic acid, fumaric acid, mandelic acid, glutaric acid, malic acid, benzoic acid, phthalic acid, benzenesulfonic acid, p-toluenesulfonic acid, methanesulfonic acid and ethanesulfonic acid). Specifically preferable are hydrochloric acid, phosphoric acid, tartaric acid and methanesulfonic acid. These salts may be formed by a conventional method.
Pharmaceutically acceptable salts of the compound of the present invention include the following salts.

Examples of basic salts thereof include: alkali metal salts such as sodium salts, potassium salts, and the like; alkaline earth metal salts such as calcium salts, magnesium salts, and the like; ammonium salts; aliphatic amine salts such as trimethylamine salts, triethylamine salts, dicyclohexylamine salts, ethanolamine salts, diethanolamine salts, triethanolamine salts, procaine salts, meglumine salts, diethanolamine salts, ethylenediamine salts, and the like; aralkylamine salts such as N,N-dibenzylethylenediamine salts, benethamine salts, and the like; heterocyclic ring aromatic amine salts such as pyridine salts, picoline salts, quinoline salts, isoquinoline salts, and the like; quaternary ammonium salts such as tetramethylammonium salts, tetraethylammonium salts, benzyltrimethylammonium salts, benzyltriethylammonium salts, benzyltributylammonium salts, methyltrioctylammonium salts, tetrabutylammonium salts, and the like; basic amino acid salts such as arginine salts, lysine salts; and the like.

Examples of acidic salts include: inorganic acid salts such as hydrochloride, sulfate, nitrate, phosphate, carbonate, bicarbonate, perchlorate, and the like; organic acid salts such as acetate, propionate, lactate, maleate, fumarate, tartrate, malate, citrate, ascorbate, and the like; sulfonate such as methanesulfonate, isethionate, benzenesulfonate, p-toluenesulfonate; acidic amino acids salts such as aspartate, glutamate, and the like.

As a pharmaceutically acceptable prodrug of the present invention, any form known in the art can be adopted. A prodrug refers to a compound that, taking advantage of a metabolic machinery in vivo, does not exhibit a pharmaceutical effect or merely exhibits very low activity in its original form, but is modified so as to, when metabolized in vivo, thereby exhibit or increase pharmacological activity for the first time. Examples of prodrugs can include not only salts, solvates, and the like, but also esters, amides, and the like.

The term "modulator" includes agonist, partial agonist, inverse agonist and antagonist.

In the compound of the present invention, the preferred embodiment is as follows.
In the compound represented by the formula (I); 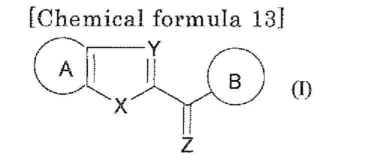 compounds represented by (I-A) to (I-C) are preferred.
The compound (I-A): a compound represented by the above formula (I), A ring is a ring represented by the following formula: 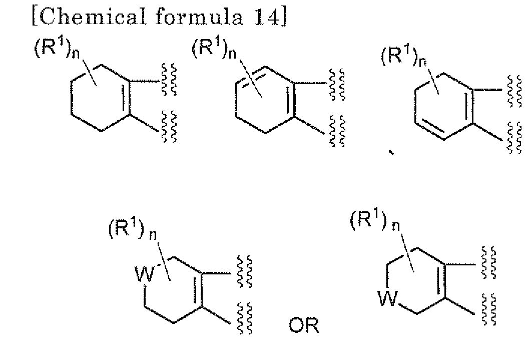 wherein
R¹ is each independently one or two more group(s) selected from the group consisting of group a) and group b) defined below;
a)R¹ is halogen, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylthio, substituted or unsubstituted alkenylthio, substituted or unsubstituted alkynylthio, acyl, hydroxy, formyl, carboxy, substituted or unsubstituted alkyloxycarbonyl, substituted or unsubstituted carbamoyl, substituted or unsubstituted sulfamoyl, substituted or unsubstituted alkyloxysulfonyl, substituted or unsubstituted aryloxysulfonyl, substituted or unsubstituted alkylsulfinyl, substituted or unsubstituted arylsulfinyl, cyano, nitro, substituted or unsubstituted amino, substituted or unsubstituted cycloalkyl, substituted or unsubstituted cycloalkenyl, substituted or unsubstituted non-aromatic heterocyclic group, substituted or unsubstituted aryl, substituted or unsubstituted heteroaryl, substituted or unsubstituted cycloalkyloxy, substituted or unsubstituted cycloalkenyloxy, substituted or unsubstituted non-aromatic heterocyclic ring-flxy, substituted or unsubstituted aryloxy, and substituted or unsubstituted heteroaryloxy,
b) two R¹ on the same carbon atom are taken together =O and =NR³;
W is -O- or -N(R²)-;
Z is =O;
B ring is a ring represented by the following formula: 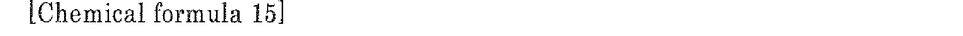 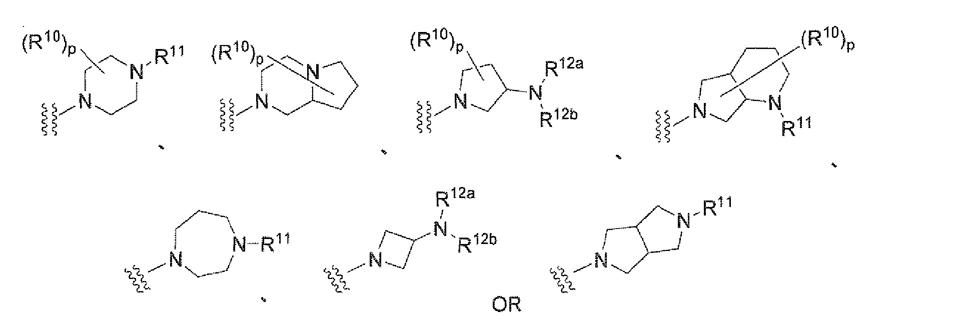 R², R³, R⁵, R⁷, R⁸, R¹⁰, R¹¹, R^{12a}, R^{12b}, X, Y, n and p are as defined in the above (1) or (1α),
or its pharmaceutically acceptable salt, or a solvate thereof.

The compound (I-B): a compound represented by the above formula (I), A ring is a ring represented by the following formula: 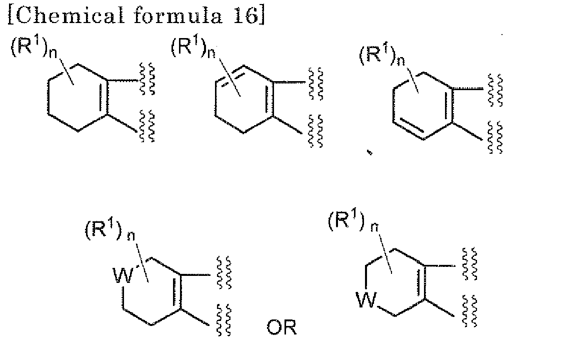 wherein
R¹ is each independently one or two more group(s) selected from the group consisting of group a) and group b) defined below;
a)R¹ is halogen, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylthio, substituted or unsubstituted alkenylthio, substituted or unsubstituted alkynylthio, acyl, hydroxy, formyl, carboxy, substituted or unsubstituted alkyloxycarbonyl, substituted or unsubstituted carbamoyl, substituted or unsubstituted sulfamoyl, substituted or unsubstituted alkyloxysulfonyl, substituted or unsubstituted aryloxysulfonyl, substituted or unsubstituted alkylsulfinyl, substituted or unsubstituted arylsulfinyl, cyano, nitro, substituted or unsubstituted amino, substituted or unsubstituted cycloalkyl, substituted or unsubstituted cycloalkenyl, substituted or unsubstituted non-aromatic heterocyclic group, substituted or unsubstituted aryl, substituted or unsubstituted heteroaryl, substituted or unsubstituted cycloalkyloxy, substituted or unsubstituted cycloalkenyloxy, substituted or unsubstituted non-aromatic heterocyclic ring-oxy, substituted or unsubstituted aryloxy, and substituted or unsubstituted heteroaryloxy,;
b) two R¹ on the same carbon atom are taken together =O and =NR³;
W is -O- or -N(R²);
Z is =O;
B ring is a ring represented by the following formula: 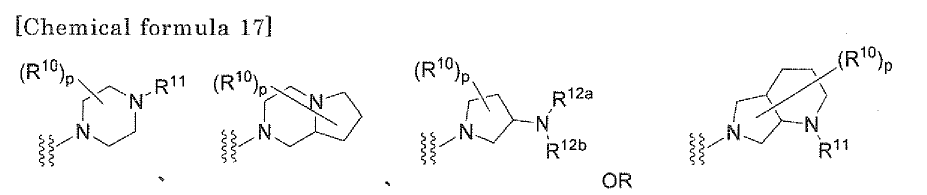 R², R³, R⁵, R⁷, R⁸, R¹⁰, R¹¹, R^{12a}, R^{12b}, X, Y, n and p are as defined in the above (1) or (1α),
or its pharmaceutically acceptable salt, or a solvate thereof.

The compound (I-C): a compound represented by the above formula (I), A ring is a ring represented by the following formula: 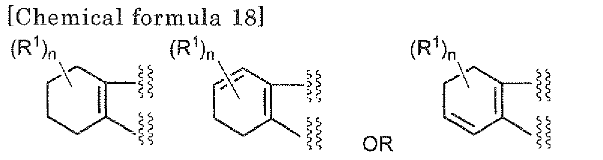 wherein
R¹ is each independently one or two more group(s) selected from the group consisting of group a) and group b) defined below;
a)R¹ is halogen, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylthio, substituted or unsubstituted alkenylthio, substituted or unsubstituted alkynylthio, acyl, hydroxy, formyl, carboxy, substituted or unsubstituted alkyloxycarbonyl, substituted or unsubstituted carbamoyl, substituted or unsubstituted sulfamoyl, substituted or unsubstituted alkyloxysulfonyl, substituted or unsubstituted aryloxysulfonyl, substituted or unsubstituted alkylsulfinyl, substituted or unsubstituted arylsulfinyl, cyano, nitro, substituted or unsubstituted amino, substituted or unsubstituted cycloalkyl, substituted or unsubstituted cycloalkenyl, substituted or unsubstituted non-aromatic heterocyclic group, substituted or unsubstituted aryl, substituted or unsubstituted heteroaryl, substituted or unsubstituted cycloalkyloxy, substituted or unsubstituted cycloalkenyloxy, substituted or unsubstituted non-aromatic heterocyclic ring-oxy, substituted or unsubstituted aryloxy, and substituted or unsubstituted heteroaryloxy,;
b) two R¹ on the same carbon atom are taken together =O and =NR³;
W is -O- or -N(R²)-;
Z is =O;
X is -N(R⁵)-;
Y is -C(R⁸)=;
B ring is a ring represented by the following formula: 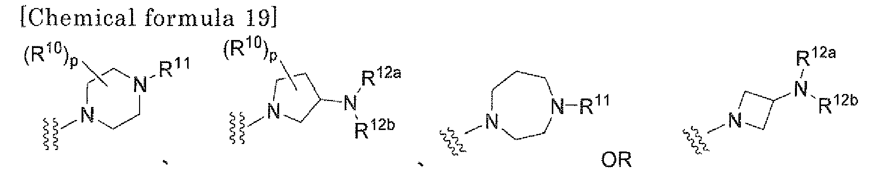 R², R⁵, R⁸, R¹⁰, R¹¹, R^{12a}, R^{12b}, n and p are as defined in the above (1) or (1α), or its pharmaceutically acceptable salt, or a solvate thereof.

In the compound of the present invention represented by the general formula (I), the following compounds represented by a formula (I-D) to (I-F) are also prefered.

In the following formula (I-D), 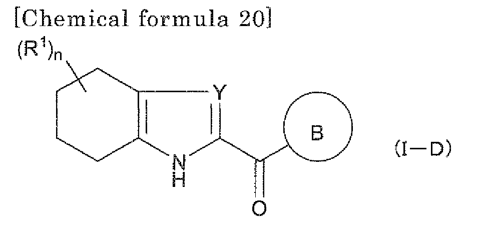
1) n is an integer of 1 to 6, R¹ is each independently one or more group(s) selected from the group consisting of fluorine atom, chlorine atom, hydroxy, cyano, substituted or unsubstituted C1-C6 alkyl, substituted or unsubstituted C1-C6 alkyloxy, substituted or unsubstituted aryl, substituted or unsubstituted carbamoyl and oxo. (Hereinafter, (R¹, n) is defined as a1.)
2) n is an integer of 1 to 6, R^{I} is each independently one or more group(s) selected from the group consisting of fluorine atom, chlorine atom, hydroxy, cyano, unsubstituted C1-C6 alkyl, unsubstituted C1-C6 alkyloxy and oxo. (Hereinafter, (R¹, n) is defined as a2.)
3) n is an integer of 1 or 2, R¹ is a group selected from the group consisting of fluorine atom, chlorine atom, hydroxy, cyano, unsubstituted C1-C6 alkyl, unsubstituted C1-C6 alkyloxy and oxo. (Hereinafter, (R¹, n) is defined as a3.)
4) n is an integer of 1, R¹ is hydroxy or cyano. (Hereinafter, (R¹, n) is defined as a4.)
5) a group represented by a formula (A1): 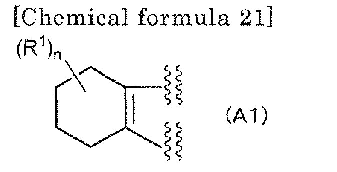 is the following formula: 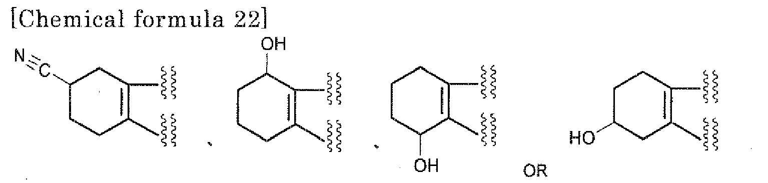 (Hereinafter, (R¹, n) is defined as a5.)
6) n is an integer of 2, R¹ is each independently a group selected from the group consisting of fluorine atom, chlorine atom, hydroxyl, cyano and unsubstituted C1-C6 alkyl, unsubstituted C1-C6 alkyloxy and oxo. (Hereinafter, (R¹, n) is defined as a6.)
7) n is an integer of 2, R¹ is each independently a group selected from the group consisting of fluorine atom, chlorine atom, hydroxyl and unsubstituted C1-C6 alkyl, two R¹ are substituted on the same carbon atom. (Hereinafter, (R¹, n) is defined as a7.)
8) a group represented by the formula (A1): 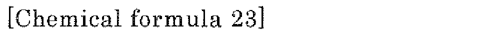 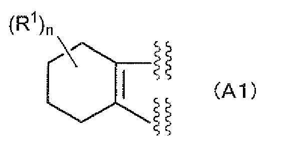 is the following formula: 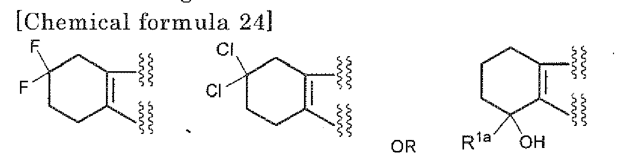 wherein, R^{1a} is unsubstituted alkyl. (Hereinafter, (R¹, n) is defined as a8.)
9) n is an integer of 3, R¹ is each independently a group selected from the group consisting of fluorine atom, chlorine atom, hydroxyl, cyano and unsubstituted C1-C6 alkyl, unsubstituted C1-C6 alkyloxy and oxo. (Hereinafter, (R¹, n) is defined as a9.)
10) n is an integer of 3, R¹ is each independently a group selected from the group consisting of fluorine atom, chlorine atom, hydroxyl and unsubstituted C1-C6 alkyloxy. (Hereinafter, (R¹, n) is defined as a10.)
11) a group represented by the formula (A1): 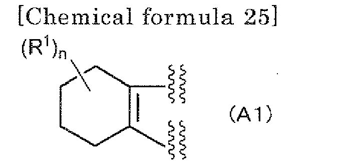 is the following formula: 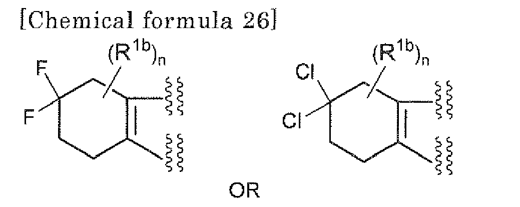 wherein, n is an integer of 1, R^{1b} is hydroxy or unsubstituted C1-C6 alkyloxy.
   (Hereinafter, (R¹, n) is defined as a11.)
12) a group represented by the formula (A1): 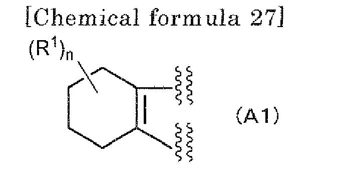 is the following formula: 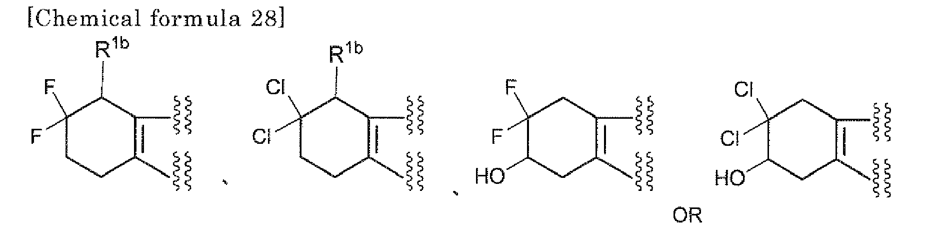 wherein, R^{1b} is hydroxy or unsubstituted C1-C6 alkyloxy. (Hereinafter, (R¹, n) is defined as a12.)
13) n is an integer of 0. (Hereinafter, (R¹, n) is defined as a13.)
14) Y is -CH= or -N=. (Hereinafter, Y is defined as yl.)
15) Y is -CH=. (Hereinafter, Y is defined as y2.)
16) Y is -N=. (Hereinafter, Y is defined as y3.)
17) B ring is a ring represented by the following formula: 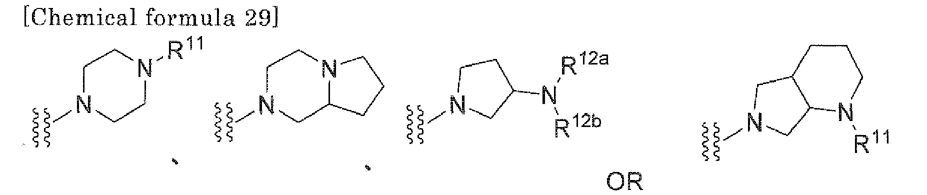 wherein, R¹¹ is hydrogen or substituted or unsubstituted C1-C3 alkyl; R^{12a} and R^{12b} are each independently hydrogen or substituted or unsubstituted C1-C6 alkyl.
   (Hereinafter, B is defined as b1.)
18) B ring is a ring represented by the following formula: 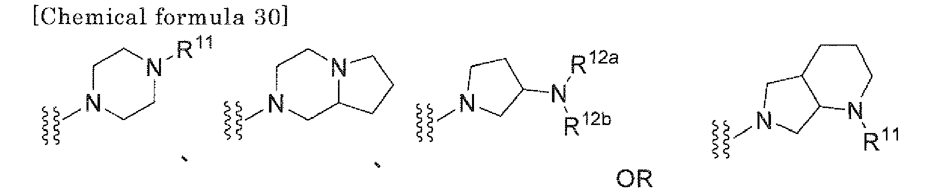 wherein, R¹¹ is hydrogen or unsubstituted C1-C3 alkyl R^{12 a} and R^{12 b} are each independently hydrogen or unsubstituted C1-C3 alkyl. (Hereinafter, B is defined as b2.)
19) B ring is a ring represented by the following formula: 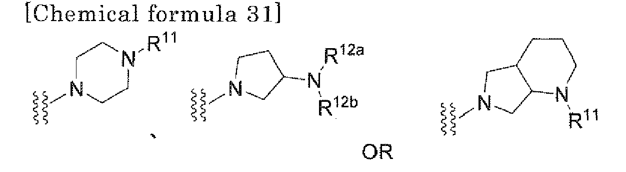 wherein, R¹¹ is hydrogen or unsubstituted C1-C3 alkyl; R^{12 a} and R^{12b} are each independently hydrogen or unsubstituted C1-C3 alkyl. (Hereinafter, B is defined as b3.)
20) B ring is a ring represented by the following formula: 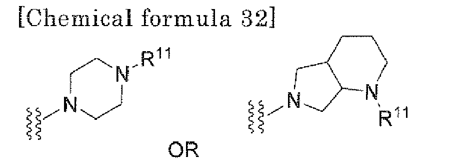 wherein, R¹¹ is hydrogen or unsubstituted C1-C3 alkyl. (Hereinafter, B is defined as b4.)
21) B ring is a ring represented by the following formula: 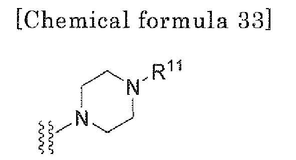 wherein, R¹¹ is hydrogen or unsubstituted C1-C3 alkyl. (Hereinafter, B is defined as b5.)

The following combinations are exemplified as a group of the compound represented by the general formula (I-D).
((R¹, n), Y, B)=
(a1,y1,b1),(a1,y1,b2),(a1,y1,b3),(a1,y1,b4),(a1,y1,b5),(a1,y2,b1),(a1,y2,b2),(a1,y2,b3),(a 1,y2,b4),(a1,y2,b5),(a1,y3,b1),(a1,y3,b2),(a1,y3,b3),(a1,y3,b4),(a1,y3,b5),(a2,y1,b1),(a2, y1,b2),(a2,y1,b3),(a2,y1,b4),(a2,y1,b5),(a2,y2,b1),(a2,y2,b2),(a2,y2,b3),(a2,y2,b4),(a2,y 2,b5),(a2,y3,b1),(a2,y3,b2),(a2,y3,b3),(a2,y3,b4),(a2,y3,b5),(a3,y1,b1),(a3,y1,b2),(a3,yl, b3),(a3,y1,b4),(a3,y1,b5),(a3,y2,b1),(a3,y2,b2),(a3,y2,b3),(a3,y2,b4),(a3,y2,b5),(a3,y3,b) 1),(a3,y3,b2),(a3,y3,b3),(a3,y3,b4),(a3,y3,b5),(a4,y1,b1),(a4,y1,b2),(a4,y1,b3),(a4,y1,b4) ,(a4,y1,b5),(a4,y2,b1),(a4,y2,b2),(a4,y2,b3),(a4,y2,b4),(a4,y2,b5),(a4,y1,b1),(a4,y3,b2),( a4,y3,b3),(a4,y3,b4),(a4,y3,b5),(a5,y1,b1),(a5,y1,b2),(a5,y1,b3),(a5,y1,b4),(a5,y1,b5),(a 5,y2,b1),(a5,y2,b2),(a5,y2,b3),(a5,y2,b4),(a5,y2,b5),(a5,y3,b1),(a5,y3,b2),(a5,y3,b3),(a5, y3,b4),(a5,y3,b5),(a6,y1,b1),(a6,y1,b2),(a6,y1,b3),(a6,y1,b4),(a6,y1,b5),(a6,y2,b1),(a6,y 2,b2),(a6,y2,b3),(a6,y2,b4),(a6,y2,b5),(a6,y3,b1),(a6,y3,b2),(a6,y3,b3),(a6,y3,b4),(a6,y3, b5),(a7,y1,b1),(a7,y1,b2),(a7,y1,b3),(a7,y1,b4),(a7,y1,b5),(a7,y2,b1),(a7,y2,b2),(a7,y2,b 3),(a7,y2,b4),(a7,y2,b5),(a7,y3,b1),(a7,y3,b2),(a7,y3,b3),(a7,y3,b4),(a7,y3,b5),(a8,y1,b1) ,(a8,y1,b2),(a8,y1,b3),(a8,y1,b4),(a8,y1,b5),(a8,y2,b1),(a8,y2,b2),(a8,y2,b3),(a8,y2,b4),( a8,y2,b5),(a8,y3,b1),(a8,y3,b2),(a8,y3,b3),(a8,y3,b4),(a8,y3,b5),(a9,y1,b1),(a9,y1,b2),(a 9,y1,b3),(a9,y1,b4),(a9,y1,b5),(a9,y2,b1),(a9,y2,b2),(a9,y2,b3),(a9,y2,b4),(a9,y2,b5),(a9, y3,b1),(a9,y3,b2),(a9,y3,b3),(a9,y3,b4),(a9,y3,b5),(a10,y1,b1),(a10,y1,b2),(a10,y1,b3),(a 10,y1,b4),(a10,y1,b5),(a10,y2,b1),(a10,y2,b2),(a10,y2,b3),(a10,y2,b4),(a10,y2,b5),(a10,y 3,b1),(a10,y3,b2),(a10,y3,b3),(a10,y3,b4),(a10,y3,b5),(a11,y1,b1),(a11,y1,b2),(a11,y1,b3 ),(a11,y1,b4),(a11,y1,b5),(a11,y2,b1),(a11,y2,b2),(a11,y2,b3),(a11,y2,b4),(a11,y2,b5),(a 11,y3,b1),(a11,y3,b2),(a11,y3,b3),(a11,y3,b4),(a11,y3,b5),(a12,y1,b1),(a12,yl,b2),(a12,y 1,b3),(a12,y1,b4),(a12,y1,b5),(a12,y2,b1),(a12,y2,b2),(a12,y2,b3),(a12,y2,b4),(a12,y2,b5 ),(a12,y3,b1),(a12,y3,b2),(a12,y3,b3),(a12,y3,b4),(a12,y3,b5),(a13,y1,b1),(a13,y1,b2),(a 13,y1,b3),(a13,y1,b4),(a13,y1,b5),(a13,y2,b1),(a13,y2,b2),(a13,y2,b3),(a13,y2,b4),(a13,y 2,b5),(a13,y3,b1),(a13,y3,b2),(a13,y3,b3),(a13,y3,b4),(a13,y3,b5)

In the following formula (I-E), 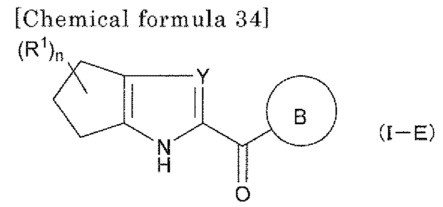
22) n is an integer of 1 to 6, R¹ is each independently one or more group(s) selected from the group consisting of fluorine atom, chlorine atom, hydroxy, cyano, substituted or unsubstituted C1-C6 alkyl, substituted or unsubstituted C1-C6 alkyloxy, substituted or unsubstituted aryl, substituted or unsubstituted carbamoyl and oxo.
   (Hereinafter, (R¹, n) is defined as ab1.)
23) n is an integer of 1 to 6, R¹ is each independently one or more group(s) selected from the group consisting of fluorine atom, chlorine atom, hydroxy, cyano, unsubstituted C1-C6 alkyl, unsubstituted C1-C6 alkyloxy and oxo. (Hereinafter, (R¹, n) is defined as ab2.)
24) n is an integer of lor 2, R¹ is hydroxy or oxo. (Hereinafter, (R¹, n) is defined as ab3.)
25) a group represented by a formula (A2): 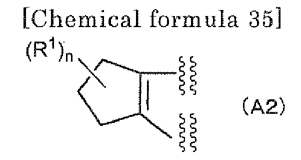 is the following formula: 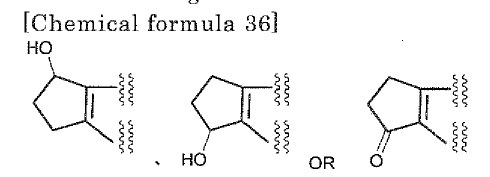 (Hereinafter, (R¹, n) is defined as ab4.)
26) n is an integer of 0. (Hereinafter, (R¹, n) is defined as ab5.)
27) Y is -CH= or -N=. (Hereinafter, Y is defined as yb1.)
28) Y is -CH=. (Hereinafter, Y is defined as yb2.)
29) Y is -N=. (Hereinafter, Y is defined as yb3.)
30) B ring is a ring represented by the following formula: 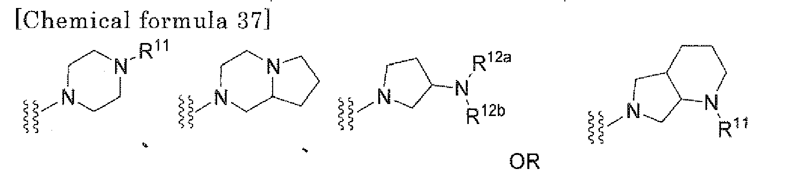 wherein, R¹¹ is hydrogen or substituted or unsubstituted C1-C3 alkyl; R¹² any R^{12b} are each independently hydrogen or substituted or unsubstituted C1-C3 alkyl.
   (Hereinafter, B is defined as bb1.)
31) B ring is a ring represented by the following formula: 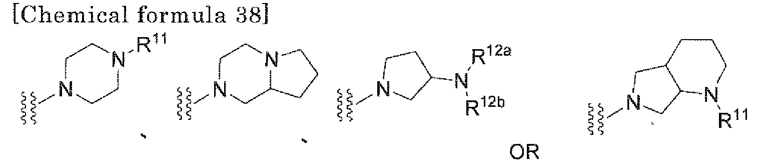 wherein, R¹¹ is hydrogen or unsubstituted C1-C3 alkyl; R¹² and R^{12b} are each independently hydrogen or unsubstituted C1-C3 alkyl. (Hereinafter, B is defined as bb2.)
32) B ring is a ring represented by the following formula: 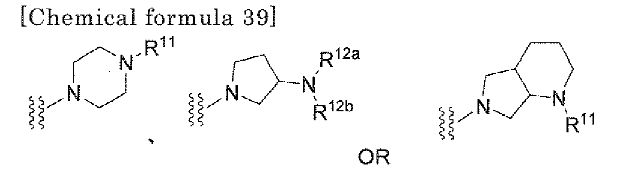 wherein, R¹¹ is hydrogen or unsubstituted C1-C3 alkyl; R¹² and R^{12b} are each independently hydrogen or unsubstituted C1-C3 alkyl. (Hereinafter, B is defined as bb3.)
33) B ring is a ring represented by the following formula: 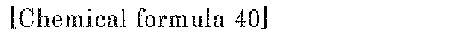 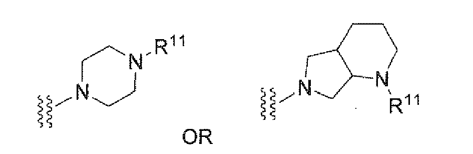 wherein, R¹¹ is hydrogen or unsubstituted C1-C3 alkyl. (Hereinafter, B is defined as bb4.)
34) B ring is a ring represented by the following formula: 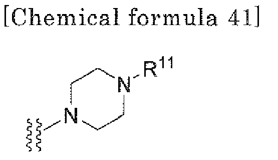 wherein, R¹¹ is hydrogen or unsubstituted C1-C3 alkyl. (Hereinafter, B is defined as bb5.)

The following combinations are exemplified as a group of the compound represented by the general formula (I-E).
((R¹, n), Y, B)=
(ab1,yb1,bb1),(ab1,yb1,bb2),(ab1,yb1,bb3),(ab1,yb1,bb4),(ab1,yb1,bb5),(ab1,yb2,bb1),( ab1,yb2,bb2),(ab1,yb2,bb3),(ab1,yb2,bb4),(ab1,yb2,bb5),(ab1,yb3,bb1),(ab1,yb3,bb2),(a b1,yb3,bb3),(ab1,yb3,bb4),(ab1,yb3,bb5),(ab2,yb1,bb1),(ab2,yb1,bb2),(ab2,yb1,bb3),(ab 2,yb1,bb4),(ab2,yb2,bb5),(ab2,yb2,bb1),(ab2,yb2,bb2),(ab2,yb2,bb3),(ab2,yb2,bb4),(ab2 ,yb2,bb5),(ab2,yb3,bbl),(ab2,yb3,bb2),(ab2,yb3,bb3),(ab2,yb3,bb4),(ab2,yb3,bb5),(ab3,y b1,bb1),(ab3,yb1,bb2),(ab3,yb1,bb3),(ab3,yb1,bb4),(ab3,yb1,bb5),(ab3,yb2,bb1),(ab3,yb 2,bb2),(ab3,yb2,bb3),(ab3,yb2,bb4),(ab3,yb2,bb5),(ab3,yb3,bb1),(ab3,yb3,bb2),(ab3,yb3 ,bb3),(ab3,yb3,bb4),(ab3,yb3,bb,5),(ab4,yb1,bb1),(ab4,ybl,bb2),(ab4,yb1,bb3),(ab4,yb1, bb4),(ab4,yb1,bb5),(ab4,yb2,bb1),(ab4,yb2,bb2),(ab4,yb2,bb3),(ab4,yb2,bb4),(ab4,yb2,b b5),(ab4,yb3,bb1),(ab4,yb3,bb2),(ab4,yb3,bb3),(ab4,yb3,bb4),(ab4,yb3,bb5),(ab5,yb1,bb 1),(ab5,yb1,bb2),(ab5,yb1,bb3),(ab5,yb1,bb4),(ab5,yb1,bbs),(ab5,yb2,bb1),(ab5,yb2,bb2 ),(ab5,yb2,bb3),(ab5,yb2,bb4),(ab5,yb2,bb5),(ab5,yb3,bbl),(ab5,yb3,bb2),(ab5,yb3,bb3), (ab5,yb3,bb4),(ab5,yb3,bb5)

In the following formula (I-F), 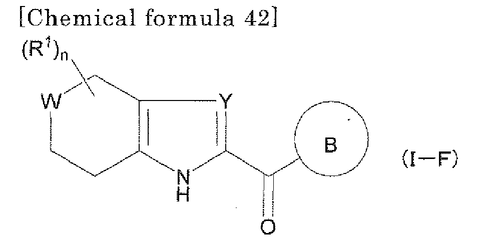
35) n is an integer of 1 to 6, R¹ is each independently one or more group(s) selected from the group consisting of fluorine atom, chlorine atom, hydroxy, cyano, substituted or unsubstituted C1-C6 alkyl, substituted or unsubstituted C1-C6 alkyloxy, substituted or unsubstituted aryl, substituted or unsubstituted carbamoyl and oxo.
   (Hereinafter, (R¹, n) is defined as ac1.)
36) n is an integer of 1 to 6, R¹ is each independently one or more group(s) selected from the group consisting of fluorine atom, chlorine atom, hydroxy, cyano, unsubstituted C1-C6 alkyl, unsubstituted C1-C6 alkyloxy and oxo. (Hereinafter, (R¹, n) is defined as ac2.)
37) n is an integer of 0. (Hereinafter, (R¹, n) is defined as ac3.)
38) W is -O- or -N(R²)-, R² is hydrogen, substituted or unsubstituted C1-C6 alkyl, substituted or unsubstituted cycloalkyl, substituted or unsubstituted aryl, or substituted or unsubstituted heteroaryl. (Hereinafter, W is defined as w1.)
39) W is -O-. (Hereinafter, W is defined as w2.)
40) Y is -CH= or -N=. (Hereinafter, Y is defined as yc1.)
41) Y is -CH=. (Hereinafter, Y is defined as yc2.)
42) Y is -N=. (Hereinafter, Y is defined as yc3.)
43) B ring is a ring represented by the following formula: 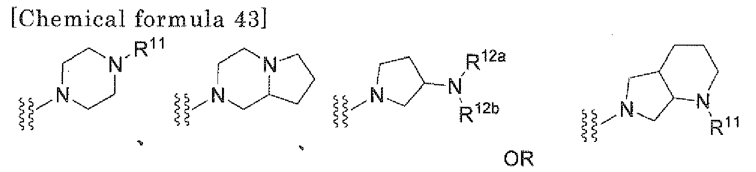 wherein, R¹¹ is hydrogen or substituted or unsubstituted C1-C3 alkyl; R^{12 a} a and R^{12 b} are each independently hydrogen or substituted or unsubstituted C1-C3 alkyl.
   (Hereinafter, B is defined as bc1.)
44) B ring is a ring represented by the following formula: 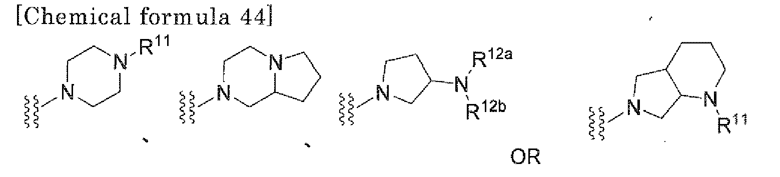 wherein, R¹¹ is hydrogen or unsubstituted C1-C3 alkyl R¹² and R^{12b} are each independently hydrogen or unsubstituted C1-C3 alkyl. (Hereinafter, B is defined as bc2.)
45) B ring is a ring represented by the following formula: 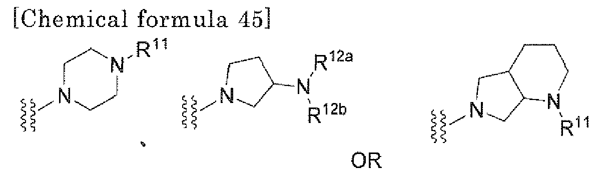 wherein, R¹¹ is hydrogen or unsubstituted C1-C3 alkyl; R^{12a} and R^{12b} are each independently hydrogen or unsubstituted C1-C3 alkyl. (Hereinafter, B is defined as bc3.)
46) B ring is a ring represented by the following formula: 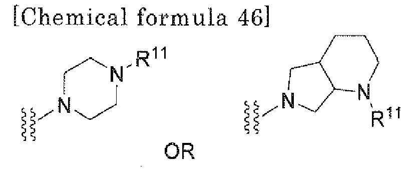 wherein, R¹¹ is hydrogen or unsubstituted C1-C3 alkyl. (Hereinafter, B is defined as bc4.)
47) B ring is a ring represented by the following formula: 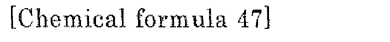 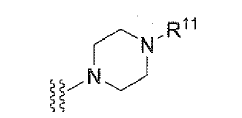
wherein, R¹¹ is hydrogen or unsubstituted C1-C3 alkyl. (Hereinafter, B is defined as bc5.)

The following combinations are exemplified as a group of the compound represented by the general formula (I-F).
((R¹, n),W, Y, B) =
(ac1,w2,yc2,bc4),(ac1,w2,yc2,bc5),(ac1,w2,yc3,bc1),(ac1,w2,yc3,bc2),(ac1,w2,yc3,bc3),( ac1,w2,yc3,bc4),(ac1,w2,yc3,bc5),(ac2,w1,yc1,bc1),(ac2,w1,yc1,bc2),(ac2,w1,yc1,bc3),(a c2,w1,yc1,bc4),(ac2,w1,yc1,bc5),(ac2,w1,yc2,bc1),(ac2,w1,yc2,bc2),(ac2,wl,ye2,bc3),(ac 2,w1,yc2,bc4),(ac2,w1,yc2,bc5),(ac2,w1,yc3,bcl),(ac2,w1,yc3,bc2),(ac2,w1,yc3,bc3),(ac2, w1,yc3,bc4),(ac2,w1,yc3,bc5),(ac2,w2,yc1,bc1),(ac2,w2,yc1,bc2),(ac2,w2,yc1,bc3),(ac2,w 2,yc1,bc4),(ac2,w2,yc1,bc5),(ac2,w2,yc2,bc1),(ac2,w2,yc2,bc2),(ac2,w2,yc2,bc3),(ac2,w2, yc2,bc4),(ac2,w2,yc2,bc5),(ac2,w2,yc3,bcl),(ac2,w2,yc3,bc2),(ac2,w2,yc3,bc3),(ac2,w2,y c3,bc4),(ac2,w2,yc3,bc5),(ac3,wl,yc1,bc1),(ac3,w1,yc1,bc2),(ac3,w1,yc1,bc3),(ac3,w1,yc 1,bc4),(ac3,w1,yc1,bc5),(ac3,w1,yc2,bc1),(ac3,w1,yc2,bc2),(ac3,w1,yc2,bc3),(ac3,w1,yc2, bc4),(ac3,wl,yc2,bc5),(ac3,w1,yc3,bc1),(ac3,w1,yc3,bc2),(ac3,w1,yc3,bc3),(ac3,w1,yc3,b c4),(ac3,w1,yc3,bcs),(ac3,w2,yc1,bc1),(ac3,w2,yc1,bc2),(ac3,w2,yc1,bc3),(ac3,w2,yc1,bc 4),(ac3,w2,yc1,bcs),(ac3,w2,yc2,bc1),(ac3,w2,yc2,bc2),(ac3,w2,yc2,bc3),(ac3,w2,yc2,bc4 ),(ac3,w2,yc2,bc5),(ac3,w2,yc3,bc1),(ac3,w2,yc3,bc2),(ac3,w2,yc3,bc3),(ac3,w2,yc3,bc4), (ac3,w2,yc3,bc5)

In the compounds of the present invention, compopunds having the following group in the general formula (I) are also preferred.

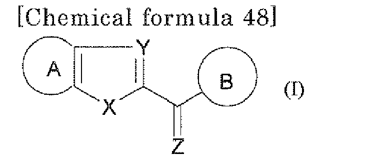

**[Table 1]**

| | A | | A |
|---|---|---|---|
| A1 | 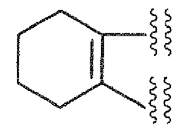 | A6 | 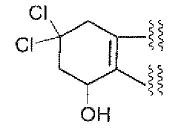 |
| A2 | 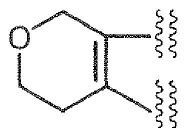 | A7 | 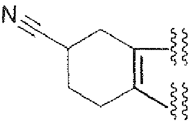 |
| A3 | 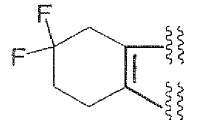 | A8 | 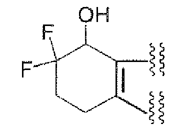 |
| A4 | 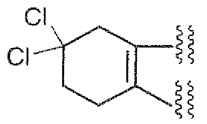 | A9 | 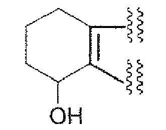 |
| A5 | 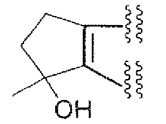 | | |

**[Table 2]**

| | B | | B |
|---|---|---|---|
| B1 | 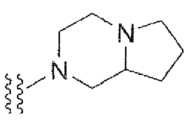 | B5 | 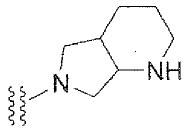 |
| B2 | 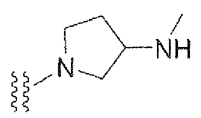 | B6 | 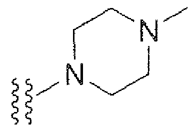 |
| B3 | 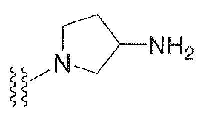 | B7 | 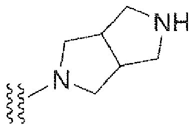 |
| B4 | 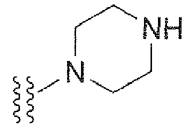 | B8 | 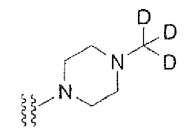 |

**[Table 3]**

| | X | | X |
|---|---|---|---|
| X1 | NH | X5 | 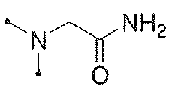 |
| X2 | NAc | X6 | 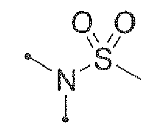 |
| X3 | O | X7 | 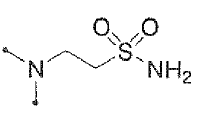 |
| X4 | 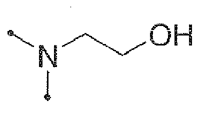 | | |

**[Table 4]**

| | Y |
|---|---|
| Y 1 | N |
| Y 2 | CH |

**[Table 5]**

| | Z |
|---|---|
| Z 1 | O |
| Z 2 | NH |
| Z 3 | NCN |

wherein, Ac is acetyl, D is deuterium.

A compound having the following (A, B, X, Y, Z) in the combination of A, B, X, Y, Z.
(A, B, X, Y, Z)=
(A1,B1,X1,Y1,Z1),(A1,B1,X1,Y1,Z2),(A1,B1,X1,Y1,Z3),(A1,B1,X1,Y2,Z1),(A1,B1,X1,Y2, Z2),(A1,B1,X1,Y2,Z3),(A1,B1,X2,Y1,Z1),(A1,B1,X2,Y1,Z2),(A1,B1,X2,Y1,Z3),(A1,B1,X2 ,Y2,Z1),(A1,B1,X2,Y2,Z2),(A1,B1,X2,Y2,Z3),(A1,B1,X3,Y1,Z1),(A1,B1,X3,Y1,Z2),(A1,B 1,X3,Y1,Z3),(A1,B1,X3,Y2,Z1),(A1,BLX3,Y2,Z2),(A1,B1,X3,Y2,Z3),(A1,B1,X4,Y1,Z1),( A1,B1,X4,Y1,Z2),(A1,B1,X4,Y1,Z3),(A1,B1,X4,Y2,Z1),(A1,B1,X4,Y2,Z2),(A1,B1,X4,Y2, Z3),(A1,B1,X5,Y1,Z1),(A1,B1,X5,Y1,Z2),(A1,B1,X5,Y1,Z3),(A1,B1,X5,Y2,Z1),(A1,B1,X5 ,Y2,Z2),(A1,B1,X5,Y2,Z3),(A1,B1,X6,Y1,Z1),(A1,B1,X6,Y1,Z2),(A1,B1,X6,Y1,Z3),(A1,B 1,X6,Y2,Z1),(A1,B1,X6,Y2,Z2),(A1,B1,X6,Y2,Z3),(A1,B1,X7,Y1,Z1),(A1,B1,X7,Y1,Z2),( A1,B1,X7,Y1,Z3),(A1,B1,X7,Y2,Z1),(A1,B1,X7,Y2,Z2),(A1,B1,X7,Y2,Z3),(A1,B2,X1,Y1, Z1),(A1,B2,X1,Y1,Z2),(A1,B2,X1,Y1,Z3),(A1,B2,X1,Y2,Z1),(A1,B2,X1,Y2,Z2),(A1,B2,X1 ,Y2,Z3),(A1,B2,X2,Y1,Z1),(A1,B2,X2,Y1,Z2),(A1,B2,X2,Y1,Z3),(A1,B2,X2,Y2,Z1),(A1,B 2,X2,Y2,Z2),(A1,B2,X2,Y2,Z3),(A1,B2,X3,Y1,Z1),(A1,B2,X3,Y1,Z2),(A1,B2,X3,Y1,Z3),( A1,B2,X3,Y2,Z1),(A1,B2,X3,Y2,Z2),A1,B2,X3,Y2,Z3),(A1,B2,X4,Y1,Z1),(A1,B2,X4,Y1, Z2),A1,B2,X4,Y1,Z3),(A1,B2,X4,Y2,Z1),(A1,B2,X4,Y2,Z2),(A1,B2,X4,Y2,Z3),(A1,B2,X5 ,Y1,Z1),(A1,B2,X5,Y1,Z2),(A1,B2,X5,Y1,Z3),(A1,B2,X5,Y2,Z1),(A1,B2,X5,Y2,Z2),(A1,B 2,X5,Y2,Z3),(A1,B2,X6,Y1,Z1),(A1,B2,X6,Y1,Z2),(A1,B2,X6,Y1,Z3),(A1,B2,X6,Y2,Z1),( A1,B2,X6,Y2,Z2),(A1,B2,X6,Y2,Z3),(A1,B2,X7,Y1,Z1),(A1,B2,X7,Y1,Z2),(A1,B2,X7,Y1, Z3),(A1,B2,X7,Y2,Z1),(A1,B2,X7,Y2,Z2),(A1,B2,X7,Y2,Z3),(A1,B3,X1,Y1,Z1),(A1,B3,X1 ,Y1,Z2),(A1,B3,X1,Y1,Z3),(A1,B3,X1,Y2,Z1),(A1,B3,X1,Y2,Z2),(A1,B3,X1,Y2,Z3),(A1,B 3,X2,Y1,Z1),(A1,B3,X2,Y1,Z2),(A1,B3,X2,Y1,Z3),(A1,B3,X2,Y2,Z1),(A1,B3,X2,Y2,Z2),( A1,B3,X2,Y2,Z3),(A1,B3,X3,Y1,Z1),(A1,B3,X3,Y1,Z2),(A1,B3,X3,Y1,Z3),(A1,B3,X3,Y2, Z1),(A1,B3,X3,Y2,Z2),(A1,B3,X3,Y2,Z3),(A1,B3,X4,Y1,Z1),(A1,B3,X4,Y1,Z2),(A1,B3,X4 ,Y1,Z3),(A1,B3,X4,Y2,Z1),(A1,B3,X4,Y2,Z2),(A1,B3,X4,Y2,Z3),(A1,B3,X5,Y1,Z1),(A1,B 3,X5,Y1,Z2),(A1,B3,X5,Y1,Z3),(A1,B3,X5,Y2,Z1),(A1,B3,X5,Y2,Z2),(A1,B3,X5,Y2,Z3),( A1,B3,X6,Y1,Z1),(A1,B3,X6,Y1,Z2),(A1,B3,X6,Y1,Z3),(A1,B3,X6,Y2,Z1),(A1,B3,X6,Y2, Z2),(A1,B3,X6,Y2,Z3),(A1,B3,X7,Y1,Z1),(A1,B3,X7,Y1,Z2),(A1,B3,X7,Y1,Z3),(A1,B3,X7 ,Y2,Z1),(A1,B3,X7,Y2,Z2),(A1,B3,X7,Y2,Z3),(A1,B4,X1,Y1,Z1),(A1,B4,X1,Y1,Z2),(A1,B 4,X1,Y1,Z3),(A1,B4,X1,Y2,Z1),(A1,B4,X1,Y2,Z2),(A1,B4,X1,Y2,Z3),(A1,B4,X2,Y1,Z1),( A1,B4,X2,Y1,Z2),(A1,B4,X2,Y1,Z3),(A1,B4,X2,Y2,Z1),(A1,B4,X2,Y2,Z2),(A1,B4,X2,Y2, Z3),(A1,B4,X3,Y1,Z1),(A1,B4,X3,Y1,Z2),(A1,B4,X3,Y1,Z3),(A1,B4,X3,Y2,Z1),(A1,B4,X3 ,Y2,Z2),(A1,B4,X3,Y2,Z3),(A1,B4,X4,Y1,Z1),(A1,B4,X4,Y1,Z2),(A1,B4,X4,Y1,Z3),(A1,B 4,X4,Y2,Z1),(A1,B4,X4,Y2,Z2),(A1,B4,X4,Y2,Z3),(A1,B4,X5,Y1,Z1),(A1,B4,X5,Y1,Z2),( A1,B4,X5,Y1,Z3),(A1,B4,X5,Y2,Z1),(A1,B4,X5,Y2,Z2),(A1,B4,X5,Y2,Z3),(A1,B4,X6,Y1, Z1),(A1,B4,X6,Y1,Z2),(A1,B4,X6,Y1,Z3),(A1,B4,X6,Y2,Z1),(A1,B4,X6,Y2,Z2),(A1,B4,X6 ,Y2,Z3),(A1,B4,X7,Y1,Z1),(A1,B4,X7,Y1,Z2),(A1,B4,X7,Y1,Z3),(A1,B4,X7,Y2,Z1),(A1,B 4,X7,Y2,Z2),(A1,B4,X7,Y2,Z3),(A1,B5,X1,Y1,Z1),(A1,B5,X1,Y1,Z2),(A1,B5,X1,Y1,Z3),( A1,B5,X1,Y2,Z1),(A1,B5,X1,Y2,Z2),(A1,B5,X1,Y2,Z3),(A1,B5,X2,Y1,Z1),(A1,B5,X2,Y1, Z2),(A1,B5,X2,Y1,Z3),(A1,B5,X2,Y2,Z1),(A1,B5,X2,Y2,Z2),(A1,B5,X2,Y2,Z3),(A1,B5,X3 ,Y1,Z1),(A1,B5,X3,Y1,Z2),(A1,B5,X3,Y1,Z3),(A1,B5,X3,Y2,Z1),(A1,B5,X3,Y2,Z2),(A1,B 5,X3,Y2,Z3),(A1,B5,X4,Y1,Z1),(A1,B5,X4,Y1,Z2),(A1,B5,X4,Y1,Z3),(A1,B5,X4,Y2,Z1),( A1,B5,X4,Y2,Z2),(A1,B5,X4,Y2,Z3),(A1,B5,X5,Y1,Z1),(A1,B5,X5,Y1,Z2),(A1,B5,X5,Y1, Z3),(A1,B5,X5,Y2,Z1),(A1,B5,X5,Y2,Z2),(A1,B5,X5,Y2,Z3),(A1,B5,X6,Y1,Z1),(A1,B5,X6 ,Y1,Z2),(A1,B5,X6,Y1,Z3),(A1,B5,X6,Y2,Z1),(A1,B5,X6,Y2,Z2),(A1,B5,X6,Y2,Z3),(A1,B 5,X7,Y1,Z1),(A1,B5,X7,Y1,Z2),(A1,B5,X7,Y1,Z3),(A1,B5,X7,Y2,Z1),(A1,B5,X7,Y2,Z2),( A1,B5,X7,Y2,Z3),(A1,B6,X1,Y1,Z2),(A1,B6,X1,Y1,Z2),(A1,B6,X1,Y1,Z3),(A1,B6,X1,Y2, Z1),(A1,B6,X1,Y2,Z2),(A1,B6,X1,Y2,Z3),(A1,B6,X2,Y1,Z1),(A1,B6,X2,Y1,Z2),(A1,B6,X2 ,Y1,Z3),(A1,B6,X2,Y2,Z1),(A1,B6,X2,Y2,Z2),(A1,B6,X2,Y2,Z3),(A1,B6,X3,Y1,Z1),(A1,B 6,X3,Y1,Z2),(A1,B6,X3,Y1,Z3),(A1,B6,X3,Y2,Z1),(A1,B6,X3,Y2,Z2),(A1,B6,X3,Y2,Z3),( A1,B6,X4,Y1,Z1),(A1,B6,X4,Y1,Z2),(A1,B6,X4,Y1,Z3),(A1,B6,X4,Y2,Z1),(A1,B6,X4,Y2, Z2),(A1,B6,X4,Y2,Z3),(A1,B6,X5,Y1,Z1),(A1,B6,X5,Y1,Z2),(A1,B6,X5,Y1,Z3),(A1,B6,X5 ,Y2,Z1),(A1,B6,X5,Y2,Z2),(A1,B6,X5,Y2,Z3),(A1,B6,X6,Y1,Z1),(A1,B6,X6,Y1,Z2),(A1,B 6,X6,Y1,Z3),(A1,B6,X6,Y2,Z1),(A1,B6,X6,Y2,Z2),(A1,B6,X6,Y2,Z3),(A1,B6,X7,Y1,Z1),( A1,B6,X7,Y1,Z2),(A1,B6,X7,Y1,Z3),(A1,B6,X7,Y2,Z1),(A1,B6,X7,Y2,Z2),(A1,B6,X7,Y2, Z3),(A1,B7,X1,Y1,Z1),(A1,B7,X1,Y1,Z2),(A1,B7,X1,Y1,Z3),(A1,B7,X1,Y2,Z1),(A1,B7,X1 ,Y2,Z2),(A1,B7,X1,Y2,Z3),(A1,B7,X2,Y1,Z1),(A1,B7,X2,Y1,Z2),(A1,B7,X2,Y1,Z3),(A1,B 7,X2,Y2,Z1),(A1,B7,X2,Y2,Z2),(A1,B7,X2,Y2,Z3),(A1,B7,X3,Y1,Z1),(A1,B7,X3,Y1,Z2),( A1,B7,X3,Y1,Z3),(A1,B7,X3,Y2,Z1),(A1,B7,X3,Y2,Z2),(A1,B7,X3,Y2,Z3),(A1,B7,X4,Y1, Z1),(A1,B7,X4,Y1,Z2),(A1,B7,X4,Y1,Z3),(A1,B7,X4,Y2,Z1),(A1,B7,X4,Y2,Z2),(A1,B7,X4 ,Y2,Z3),(A1,B7,X5,Y1,Z1),(A1,B7,X5,Y1,Z2),(A1,B7,X5,Y1,Z3),(A1,B7,X5,Y2,Z1),(A1,B 7,X5,Y2,Z2),(A1,B7,X5,Y2,Z3),(A1,B7,X6,Y1,Z1),(ALB7,X6,Y1,Z2),(A1,B7,X6,Y1,Z3),( A1,B7,X6,Y2,Z1),(A1,B7,X6,Y2,Z2),(A1,B7,X6,Y2,Z3),(A1,B7,X7,Y1,Z1),(A1,B7,X7,Y1, Z2),(A1,B7,X7,Y1,Z3),(A1,B7,X7,Y2,Z1),(A1,B7,X7,Y2,Z2),(A1,B7,X7,Y2,Z3),(A1,B8,X1 ,Y1,Z1),(A1,B8,X1,Y1,Z2),(A1,B8,X1,Y1,Z3),(A1,B8,X1..Y2,Z1),(A1,B8,X1,Y2,Z2),(A1,B 8,X1,Y2,Z3),(A1,B8,X2,Y1,Z1),(A1,B8,X2,Y1,Z2),(A1,B8,X2,Y1,Z3),(A1,B8,X2,Y2,Z1),( A1,B8,X2,Y2,Z2),(A1,B8,X2,Y2,Z3),(A1,B8,X3,Y1,Z1),(A1,B8,X3,Y1,Z2),(A1,B8,X3,Y1, Z3),(A1,B8,X3,Y2,Z1),(A1,B8,X3,Y2,Z2),(A1,B8,X3,Y2,Z3),A1,B8,X4,Y1,Z1),(A1,B8,X4 ,Y1,Z2),(A1,B8,X4,Y1,Z3),(A1,B8,X4,Y2,Z1),(A1,B8,X4,Y2,Z2),(A1,B8,X4,Y2,Z3),(A1,B 8,X5,Y1,Z1),(A1,B8,X5,Y1,Z2),(A1,B8,X5,Y1,Z3),(A1,B8,X5,Y2,Z1),(A1,B8,X5,Y2,Z2),( A1,B8,X5,Y2,Z3),(A1,B8,X6,Y1,Z1),(A1,B8,X6,Y1,Z2),(A1,B8,X6,Y1,Z3),(A1,B8,X6,Y2, Z1),(A1,B8,X6,Y2,Z2),(A1,B8,X6,Y2,Z3),(A1,B8,X7,Y1,Z1),(A1,B8,X7,Y1,Z2),(A1,B8,X7 ,Y1,Z3),(A1,B8,X7,Y2,Z1),(A1,B8,X7,Y2,Z2),(A1,B8,X7,Y2,Z3),(A2,B1,X1,Y1,Z1),(A2,B 1,X1,Y1,Z2),(A2,B1,X1,Y1,Z3),(A2,B1,X1,Y2,Z1),(A2,B1,X1,Y2,Z2),(A2,B1,X1,Y2,Z3),( A2,B1,X2,Y1,Z1),(A2,B1,X2,Y1,Z2),(A2,B1,X2,Y1,Z3),(A2,B1,X2,Y2,Z1),(A2,B1,X2,Y2, Z2),(A2,B1,X2,Y2,Z3),(A2,B1,X3,Y1,Z1),(A2,B1,X3,Y1,Z2),(A2,B1,X3,Y1,Z3),(A2,B1,X3 ,Y2,Z1),(A2,B1,X3,Y2,Z2),(A2,B1,X3,Y2,Z3),(A2,B1,X4,Y1,Z1),A2,B1,X4,Y1,Z2),(A2,B 1,X4,Y1,Z3),(A2,B1,X4,Y2,Z1),(A2,B1,X4,Y2,Z2),(A2,B1,X4,Y2,Z3),(A2,B1,X5,Y1,Z1),( A2,B1,X5,Y1,Z2),(A2,B1,X5,Y1,Z3),(A2,B1,X5,Y2,Z1),(A2,B1,X5,Y2,Z2),(A2,B1,X5,Y2, Z3),(A2,B1,X6,Y1,Z1),(A2,B1,X6,Y1,Z2),(A2,B1,X6,Y1,Z3),(A2,B1,X6,Y2,Z1),(A2,B1,X6 ,Y2,Z2),(A2,B1,X6,Y2,Z3),(A2,B1,X7,Y1,Z1),(A2,B1,X7,Y1,Z2),(A2,B1,X7,Y1,Z3),(A2,B 1,X7,Y2,Z1),(A2,B1,X7,Y2,Z2),(A2,B1,X7,Y2,Z3),(A2,B2,X1,Y1,Z1),(A2,B2,X1,Y1,Z2),( A2,B2,X1,Y1,Z3),(A2,B2,X1,Y2,Z1),(A2,B2,X1,Y2,Z2),(A2,B2,X1,Y2,Z3),(A2,B2,X2,Y1, Z1),(A2,B2,X2,Y1,Z2),(A2,B2,X2,Y1,Z3),(A2,B2,X2,Y2,Z1),(A2,B2,X2,Y2,Z2),(A2,B2,X2 ,Y2,Z3),(A2,B2,X3,Y1,Z1),(A2,B2,X3,Y1,Z2),(A2,B2,X3,Y1,Z3),(A2,B2,X3,Y2,Z1),(A2,B 2,X3,Y2,Z2),(A2,B2,X3,Y2,Z3),(A2,B2,X4,Y1,Z1),(A2,B2,X4,Y1,Z2),(A2,B2,X4,Y1,Z3),( A2,B2,X4,Y2,Z1),(A2,B2,X4,Y2,Z2),(A2,B2,X4,Y2,Z3),(A2,B2,X5,Y1,Z1),(A2,B2,X5,Y1, Z2),(A2,B2,X5,Y1,Z3),(A2,B2,X5,Y2,Z1),(A2,B2,X5,Y2,Z2),(A2,B2,X5,Y2,Z3),(A2,B2,X6 ,Y1,Z1),(A2,B2,X6,Y1,Z2),(A2,B2,X6,Y1,Z3),(A2,B2,X6,Y2,Z1),(A2,B2,X6,Y2,Z2),(A2,B 2,X6,Y2,Z3),(A2,B2,X7,Y1,Z1),(A2,B2,X7,Y1,Z2),(A2,B2,X7,Y1,Z3),(A2,B2,X7,Y2,Z1),( A2,B2,X7,Y2,Z2),(A2,B2,X7,Y2,Z3),(A2,B3,X1,Y1,Z1),(A2,B3,X1,Y1,Z2),(A2,B3,X1,Y1, Z3),(A2,B3,X1,Y2,Z1),(A2,B3,X1,Y2,Z2),(A2,B3,X1,Y2,Z3),(A2,B3,X2,Y1,Z1),(A2,B3,X2 ,Y1,Z2),(A2,B3,X2,Y1,Z3),(A2,B3,X2,Y2,Z1),(A2,B3,X2,Y2,Z2),(A2,B3,X2,Y2,Z3),(A2,B 3,X3,Y1,Z1),(A2,B3,X3,Y1,Z2),(A2,B3,X3,Y1,Z3),(A2,B3,X3,Y2,Z1),(A2,B3,X3,Y2,Z2),( A2,B3,X3,Y2,Z3),(A2,B3,X4,Y1,Z1),(A2,B3,X4,Y1,Z2),(A2,B3,X4,Y1,Z3),(A2,B3,X4,Y2, Z1),(A2,B3,X4,Y2,Z2),(A2,B3,X4,Y2,Z3),(A2,B3,X5,Y1,Z1),(A2,B3,X5,Y1,Z2),(A2,B3,X5 ,Y1,Z3),(A2,B3,X5,Y2,Z1),(A2,B3,X5,Y2,Z2),(A2,B3,X5,Y2,Z3),(A2,B3,X6,Y1,Z1),(A2,B 3,X6,Y1,Z2),(A2,B3,X6,Y1,Z3),(A2,B3,X6,Y2,Z1),(A2,B3,X6,Y2,Z2),(A2,B3,X(3,Y2,Z3),( A2,B3,X7,Y1,Z1),(A2,B3,X7,Y1,Z2),(A2,B3,X7,Y1,Z3),(A2,B3,X7,Y2,Z1),(A2,B3,X7,Y2, Z2),(A2,B3,X7,Y2,Z3),(A2,B4,X1,Y1,Z1),(A2,B4,X1,Y1,Z2),(A2,B4,X1,Y1,Z3),(A2,B4,X1 ,Y2,Z1),(A2,B4,X1,Y2,Z2),(A2,B4,X1,Y2,Z3),(A2,B4,X2,Y1,Z1),(A2,B4,X2,Y1,Z2),(A2,B 4,X2,Y1,23),(A2,B4,X2,Y2,Z1),(A2,B4,X2,Y2,Z2),(A2,B4,X2,Y2,Z3),(A2,B4,X3,Y1,Z1),( A2,B4,X3,Y1,Z2),(A2,B4,X3,Y1,Z3),(A2,B4,X3,Y2,Z1),(A2,B4,X3,Y2,Z2),(A2,B4,X3,Y2, Z3),A2,B4,X4,Y1,Z1),(A2,B4,X4,Y1,2),(A2,B4,X4,Y1,Z3),(A2,B4,X4,Y2,Z1),(A2,B4,X4 ,Y2,Z2),(A2,B4,X4,Y2,Z3),(A2,B4,X5,Y1,Z1),(A2,B4,X5,Y1,Z2),(A2,B4,X5,Y1,Z3),(A2,B 4,X5,Y2,Z1),(A2,B4,X5,Y2,Z2),(A2,B4,X5,Y2,Z3),(A2,B4,X6,Y1,Z1),(A2,B4,X6,Y1,Z2),( A2,B4,X6,Y1,Z3),(A2,B4,X6,Y2,Z1),(A2,B4,X6,Y2,Z2),(A2,B4,X6,Y2,Z3),(A2,B4,X7,Y1, Z1),(A2,B4,X7,Y1,Z2),(A2,B4,X7,Y1,Z3),(A2,B4,X7,Y2,Z1),(A2,B4,X7,Y2,Z2),(A2,B4,X7 ,Y2,Z3),(A2,B5,X1,Y1,Z1),(A2,B5,X1,Y1,Z2),(A2,B5,X1,Y1,Z3),(A2,B5,X1,Y2,Z1),(A2,B 5,X1,Y2,Z2),(A2,B5,X1,Y2,Z3),(A2,B5,X2,Y1,Z1),(A2,B5,X2,Y1,Z2),(A2,B5,X2,Y1,Z3),( A2,B5,X2,Y2,Z1),(A2,B5,X2,Y2,Z2),(A2,B5,X2,Y2,23),(A2,B5,X3,Y1,Z1),(A2,B5,X3,Y1, Z2),(A2,B5,X3,Y1,Z3),(A2,B5,X3,Y2,Z1),(A2,B5,X3,Y2,Z2),(A2,B5,X3,Y2,Z3),(A2,B5,X4 ,Y1,Z1),(A2,B5,X4,Y1,Z2),(A2,B5,X4,Y1,Z3),(A2,B5,X4,Y2,Z1),(A2,B5,X4,Y2,Z2),(A2,B 5,X4,Y2,Z3),(A2,B5,X5,Y1,Z1),(A2,B5,X5,Y1,Z2),(A2,B5,X5,Y1,Z3),(A2,B5,X5,Y2,Z1),( A2,B5,X5,Y2,Z2),(A2,B5,X5,Y2,Z3),(A2,B5,X6,Y1,Z1),(A2,B5,X6,Y1,Z2),(A2,B5,X6,Y1, Z3),(A2,B5,X6,Y2,Z1),(A2,B5,X6,Y2,Z2),(A2,B5,X6,Y2,Z3),(A2,B5,X7,Y1,Z1),(A2,B5,X7 ,Y1,Z2),(A2,B5,X7,Y1,Z3),(A2,B5,X7,Y2,Z1),(A2,B5,X7,Y2,Z2),(A2,B5,X7,Y2,Z3),(A2,B 6,X1,Y1,Z1),(A2,B6,X1,Y1,Z2),(A2,B6,X1,Y1,Z3),(A2,B6,X1,Y2,Z1),(A2,B6,X1,Y2,Z2),( A2,B6,X1,Y2,Z3),(A2,B6,X2,Y1,Z1),(A2,B6,X2,Y1,Z2),(A2,B6,X2,Y1,Z3),(A2,B6,X2,Y2, Z1),(A2,B6,X2,Y2,Z2),(A2,B6,X2,Y2,Z3),(A2,B6,X3,Y1,Z1),(A2,B6,X3,Y1,Z2),(A2,B6,X3 ,Y1,Z3),(A2,B6,X3,Y2,Z1),(A2,B6,X3,Y2,Z2),(A2,B6,X3,Y2,Z3),(A2,B6,X4,Y1,Z1),(A2,B 6,X4,Y1,Z2),(A2,B6,X4,Y1,Z3),(A2,B6,X4,Y2,Z1),(A2,B6,X4,Y2,Z2),(A2,B6,X4,Y2,Z3),( A2,B6,X5,Y1,Z1),(A2,B6,X5,Y1,Z2),(A2,B6,X5,Y1,Z3),(A2,B6,X5,Y2,Z1),(A2,B6,X5,Y2, Z2),(A2,B6,X5,Y2,Z3),(A2,B6,X6,Y1,Z1),(A2,B6,X6,Y1,Z2),(A2,B6,X6,Y1,Z3),(A2,B6,X6 ,Y2,Z1),(A2,B6,X6,Y2,Z2),(A2,B6,X6,Y2,Z3),(A2,B6,X7,Y1,Z1),(A2,B6,X7,Y1,Z2),(A2,B 6,X7,Y1,Z3),(A2,B6,X7,Y2,Z1),(A2,B6,X7,Y2,Z2),(A2,B6,X7,Y2,Z3),(A2,B7,X1,Y1,Z1),( A2,B7,X1,Y1,Z2),(A2,B7,X1,Y1,Z3),(A2,B7,X1,Y2,Z1),(A2,B7,X1,Y2,Z2),(A2,B7,X1,Y2, Z3),(A2,B7,X2,Y1,Z1),(A2,B7,X2,Y1,Z2),(A2,B7,X2,Y1,Z3),(A2,B7,X2,Y2,Z1),(A2,B7,X2 ,Y2,Z2),(A2,B7,X2,Y2,Z3),(A2,B7,X3,Y1,Z1),(A2,B7,X3,Y1,Z2),(A2,B7,X3,Y1,Z3),(A2,B 7,X3,Y2,Z1),(A2,B7,X3,Y2,Z2),(A2,B7,X3,Y2,Z3),(A2,B7,X4,Y1,Z1),(A2,B7,X4,Y1,Z2),( A2,B7,X4,Y1,Z3),(A2,B7,X4,Y2,Z1),(A2,B7,X4,Y2,Z2),(A2,B7,X4,Y2,Z3),(A2,B7,X5,Y1, Z1),(A2,B7,X5,Y1,Z2),(A2,B7,X5,Y1,Z3),(A2,B7,X5,Y2,Z1),(A2,B7,X5,Y2,Z2),(A2,B7,X5 ,Y2,Z3),(A2,B7,X6,Y1,Z1),(A2,B7,X6,Y1,Z2),(A2,B7,X6,Y1,Z3),(A2,B7,XG,Y2,Z1),(A2,B 7,X6,Y2,Z2),(A2,B7,X6,Y2,Z3),(A2,B7,X7,Y1,Z1),(A2,B7,X7,Y1,Z2),(A2,B7,X7,Y1,Z3),( A2,B7,X7,Y2,Z1),(A2,B7,X7,Y2,Z2),(A2,B7,X7,Y2,Z3),(A2,B8,X1,Y1,Z1),(A2,B8,X1,Y1, Z2),(A2,B8,X1,Y1,Z3),(A2,B8,X1,Y2,Z1),(A2,B8,X1,Y2,Z2),(A2,B8,X1,Y2,Z3),(A2,B8,X2 ,Y1,Z1),(A2,B8,X2,Y1,Z2),(A2,B8,X2,Y1,Z3),(A2,B8,X2,Y2,Z1),(A2,B8,X2,Y2,Z2),(A2,B 8,X2,Y2,Z3),(A2,B8,X3,Y1,Z1),(A2,B8,X3,Y1,Z2),(A2,B8,X3,Y1,Z3),(A2,B8,X3,Y2,Z1),( A2,B8,X3,Y2,Z2),(A2,B8,X3,Y2,Z3),(A2,B8,X4,Y1,Z1),(A2,B8,X4,Y1,Z2),(A2,B8,X4,Y1, Z3),(A2,B8,X4,Y2,Z1),(A2,B8,X4,Y2,Z2),(A2,B8,X4,Y2,Z3),(A2,B8,X5,Y1,Z1),(A2,B8,X5 ,Y1,Z2),(A2,B8,X5,Y1,Z3),(A2,B8,X5,Y2,Z1),(A2,B8,X5,Y2,Z2),(A2,B8,X5,Y2,Z3),(A2,B 8,X6,Y1,Z1),(A2,B8,X6,Y1,Z2),(A2,B8,X6,Y1,Z3),(A2,B8,X6,Y2,Z1),(A2,B8,X6,Y2,Z2),( A2,B8,X6,Y2,Z3),(A2,B8,X7,Y1,Z1),(A2,B8,X7,Y1,Z2),(A2,B8,X7,Y1,Z3),(A2,B8,X7,Y2, Z1),(A2,B8,X7,Y2,Z2),(A2,B8,X7,Y2,Z3),(A3,B1,X1,Y1,Z1),(A3,B1,X1,Y1,Z2),(A3,B1,X1 ,Y1,Z3),(A3,B1,X1,Y2,Z1),(A3,B1,X1,Y2,Z2),(A3,B1,X1,Y2,Z3),(A3,B1,X2,Y1,Z1),(A3,B 1,X2,Y1,Z2),(A3,B1,X2,Y1,Z3),(A3,B1,X2,Y2,Z1),(A3,B1,X2,Y2,Z2),(A3,B1,X2,Y2,Z3),( A3,B1,X3,Y1,Z1),(A3,B1,X3,Y1,Z2),(A3,B1,X3,Y1,Z3),(A3,B1,X3,Y2,Z1),(A3,B1,X3,Y2, Z2),(A3,B1,X3,Y2,Z3),(A3,B1,X4,Y1,Z1),(A3,B1,X4,Y1,Z2),(A3,B1,X4,Y1,Z3),(A3,B1,X4 ,Y2,Z1),(A3,B1,X4,Y2,Z2),(A3,B1,X4,Y2,Z3),(A3,B1,X5,Y1,Z1),(A3,B1,X5,Y1,Z2),(A3,B 1,X5,Y1,Z3),(A3,B1,X5,Y2,Z1),(A3,B1,X5,Y2,Z2),(A3,B1,X5,Y2,Z3),(A3,B1,X6,Y1,Z1),( A3,B1,X6,Y1,Z2),(A3,B1,X6,Y1,Z3),(A3,B1,X6,Y2,Z1),(A3,B1,X6,Y2,Z2),(A3,B1,X6,Y2, Z3),(A3,B1,X7,Y1,Z1),(A3,B1,X7,Y1,Z2),(A3,B1,X7,Y1,Z3),(A3,B1,X7,Y2,Z1),(A3,B1,X7 ,Y2,Z2),(A3,B1,X7,Y2,Z3),(A3,B2,X1,Y1,Z1),(A3,B2,X1,Y1,Z2),(A3,B2,X1,Y1,Z3),(A3,B 2,X1,Y2,Z1),(A3,B2,X1,Y2,Z2),(A3,B2,X1,Y2,Z3),(A3,B2,X2,Y1,Z1),(A3,B2,X2,Y1,Z2),( A3,B2,X2,Y1,Z3),(A3,B2,X2,Y2,Z1),(A3,B2,X2,Y2,Z2),(A3,B2,X2,Y2,Z3),(A3,B2,X3,Y1, Z1),(A3,B2,X3,Y1,Z2),(A3,B2,X3,Y1,Z3),(A3,B2,X3,Y2,Z1),(A3,B2,X3,Y2,Z2),(A3,B2,X3 ,Y2,Z3),(A3,B2,X4,Y1,Z1),(A3,B2,X4,Y1,Z2),(A3,B2,X4,Y1,Z3),(A3,B2,X4,Y2,Z1),(A3,B 2,X4,Y2,Z2),(A3,B2,X4,Y2,Z3),(A3,B2,X5,Y1,Z1),(A3,B2,X5,Y1,Z2),(A3,B2,X5,Y1,Z3),( A3,B2,X5,Y2,Z1),(A3,B2,X5,Y2,Z2),(A3,B2,X5,Y2,Z3),(A3,B2,X6,Y1,Z1),(A3,B2,X6,Y1, Z2),(A3,B2,X6,Y1,Z3),(A3,B2,X6,Y2,Z1),(A3,B2,X6,Y2,Z2),(A3,B2,X6,Y2,Z3),(A3,B2,X7 ,Y1,Z1),(A3,B2,X7,Y1,Z2),(A3,B2,X7,Y1,Z3),(A3,B2,X7,Y2,Z1),(A3,B2,X7,Y2,Z2),(A3,B 2,X7,Y2,Z3),(A3,B3,X1,Y1,Z1),(A3,B3,X1,Y1,Z2),(A3,B3,X1,Y1,Z3),(A3,B3,X1,Y2,Z1),( A3,B3,X1,Y2,Z2),(A3,B3,X1,Y2,Z3),(A3,B3,X2,Y1,Z1),(A3,B3,X2,Y1,Z2),(A3,B3,X2,Y1, Z3),(A3,B3,X2,Y2,Z1),(A3,B3,X2,Y2,Z2),(A3,B3,X2,Y2,Z3),(A3,B3,X3,Y1,Z1),(A3,B3,X3 ,Y1,Z2),(A3,B3,X3,Y1,Z3),(A3,B3,X3,Y2,Z1),(A3,B3,X3,Y2,Z2),(A3,B3,X3,Y2,Z3),(A3,B 3,X4,Y1,Z1),(A3,B3,X4,Y1,Z2),(A3,B3,X4,Y1,Z3),(A3,B3,X4,Y2,Z1),(A3,B3,X4,Y2,Z2),( A3,B3,X4,Y2,Z3),(A3,B3,X5,Y1,Z1),(A3,B3,X5,Y1,Z2),(A3,B3,X5,Y1,Z3),(A3,B3,X5,Y2, Z1),(A3,B3,X5,Y2,Z2),(A3,B3,X5,Y2,Z3),(A3,B3,X6,Y1,Z1),(A3,B3,X6,Y1,Z2),(A3,B3,X6 ,Y1,Z3),(A3,B3,X6,Y2,Z1),(A3,B3,X6,Y2,Z2),(A3,B3,X6,Y2,Z3),(A3,B3,X1,Y1,Z1),(A3,B 3,X7,Y1,Z2),(A3,B3,X7,Y1,Z3),(A3,B3,X7,Y2,Z1),(A3,B3,X7,Y2,Z2),(A3,B3,X7,Y2,Z3),( A3,B4,X1,Y1,Z1),(A3,B4,X1,Y1,Z2),(A3,B4,X1,Y1,Z3),(A3,B4,X1,Y2,Z1),(A3,B4,X1,Y2, Z2),(A3,B4,X1,Y2,Z3),(A3,B4,X2,Y1,Z1),(A3,B4,X2,Y1,Z2),(A3,B4,X2,Y1,Z3),(A3,B4,X2 ,Y2,Z1),(A3,B4,X2,Y2,Z2),(A3,B4,X2,Y2,Z3),(A3,B4,X3,Y1,Z1),(A3,B4,X3,Y1,Z2),(A3,B 4,X3,Y1,Z3),(A3,B4,X3,Y2,Z1),(A3,B4,X3,Y2,Z2),(A3,B4,X3,Y2,Z3),(A3,B4,X4,Y1,Z1),( A3,B4,X4,Y1,Z2),(A3,B4,X4,Y1,Z3),(A3,B4,X4,Y2,Z1),(A3,B4,X4,Y2,Z2),(A3,B4,X4,Y2, Z3),(A3,B4,X5,Y1,Z1),(A3,B4,X5,Y1,Z2),(A3,B4,X5,Y1,Z3),(A3,B4,X5,Y2,Z1),(A3,B4,X5 ,Y2,Z2),(A3,B4,X5,Y2,Z3),(A3,B4,X6,Y1,Z1),(A3,B4,X6,Y1,Z2),(A3,B4,X6,Y1,Z3),(A3,B 4,X6,Y2,Z1),(A3,B4,X6,Y2,Z2),(A3,B4,X6,Y2,Z3),(A3,B4,X7,Y1,Z1),(A3,B4,X7,Y1,Z2),( A3,B4,X7,Y1,Z3),(A3,B4,X7,Y2,Z1),(A3,B4,X7,Y2,Z2),(A3,B4,X7,Y2,Z3),(A3,B5,X1,Y1, Z1),(A3,B5,X1,Y1,Z2),(A3,B5,X1,Y1,Z3),(A3,B5,X1,Y2,Z1),(A3,B5,X1,Y2,Z2),(A3,B5,X1 ,Y2,Z3),(A3,B5,X2,Y1,Z1),(A3,B5,X2,Y1,Z2),(A3,B5,X2,Y1,Z3),(A3,B5,X2,Y2,Z1),(A3,B 5,X2,Y2,Z2),(A3,B5,X2,Y2,Z3),(A3,B5,X3,Y1,Z1),(A3,B5,X3,Y1,Z2),(A3,B5,X3,Y1,Z3),( A3,B5,X3,Y2,Z1),(A3,B5,X3,Y2,Z2),(A3, B5,X3,Y2,Z3),(A3,B5,X4,Y1,Z1),(A3,B5,X4,Y1, Z2),(A3,B5,X4,Y1,Z3),(A3,B5,X4,Y2,Z1),(A3,B5,X4,Y2,Z2),(A3,B5,X4,Y2,Z3),(A3,B5,X5 ,Y1,Z1),(A3,B5,X5,Y1,Z2),(A3,B5,X5,Y1,Z3),(A3,B5,X5,Y2,Z1),(A3,B5,X5,Y2,Z2),(A3,B 5,X5,Y2,Z3),(A3,B5,X6,Y1,Z1),(A3,B5,X6,Y1,Z2),(A3,B5,X6,Y1,Z3),(A3,B5,X6,Y2,Z1),( A3,B5,X6,Y2,Z2),(A3,B5,X6,Y2,23),(A3,B5,X7,Y1,Z1),(A3,B5,X7,Y1,Z2),(A3,B5,X7,Y1, Z3),(A3,B5,X7,Y2,Z1),(A3,B5,X7,Y2,Z2),(A3,B5,X7,Y2,Z3),(A3,B6,X1,Y1,Z1),(A3,B6,X1 ,Y1,Z2),(A3,B6,X1,Y1,Z3),(A3,B6,X1,Y2,Z1),(A3,B6,X1,Y2,Z2),(A3,B6,X1,Y2,Z3),(A3,B 6,X2,Y1,Z1),(A3,B6,X2,Y1,Z2),(A3,B6,X2,Y1,Z3),(A3,B6,X2,Y2,Z1),(A3,B6,X2,Y2,Z2),( A3,B6,X2,Y2,Z3),(A3,B6,X3,Y1,Z1),(A3,B6,X3,Y1,Z2),(A3,B6,X3,Y1,Z3),(A3,B6,X3,Y2, Z1),(A3,B6,X3,Y2,Z2),(A3,B6,X3,Y2,Z3),(A3,B6,X4,Y1,Z1),(A3,B6,X4,Y1,Z2),(A3,B6,X4 ,Y1,Z3),(A3,B6,X4,Y2,Z1),(A3,B6,X4,Y2,Z2),(A3,B6,X4,Y2,Z3),(A3,B6,X5,Y1,Z1),(A3,B 6,X5,Y1,Z2),(A3,B6,X5,Y1,Z3),(A3,B6,X5,Y2,Z1),(A3,B6,X5,Y2,Z2),(A3,B6,X5,Y2,Z3),( A3,B6,X6,Y1,Z1),(A3,B6,X6,Y1,Z2),(A3,B6,X6,Y1,Z3),(A3,B6,X6,Y2,Z1),(A3,B6,X6,Y2, Z2),(A3,B6,X6,Y2,Z3),(A3,B6,X7,Y1,Z1),(A3,B6,X7,Y1,Z2),(A3,B6,X7,Y1,Z3),(A3,B6,X7 ,Y2,Z1),(A3,B6,X7,Y2,Z2),(A3,B6,X7,Y2,Z3),(A3,B7,X1,Y1,Z1),(A3,B7,X1,Y1,Z2),(A3,B 7,X1Y1,Z3),(A3,B7,X1,Y2,Z1),(A3,B7,X1,Y2,Z2),(A3,B7,X1,Y2,Z3),(A3,B7,X2,Y1,Z1),( A3,B7,X2,Y1,Z2),(A3,B7,X2,Y1,Z3),(A3,B7,X2,Y2,Z1),(A3,B7,X2,Y2,Z2),(A3,B7,X2,Y2, Z3),(A3,B7,X3,Y1,Z1),(A3,B7,X3,Y1,Z2),(A3,B7,X3,Y1,Z3),(A3,B7,X3,Y2,Z1),(A3,B7,X3 ,Y2,Z2),(A3,B7,X3,Y2,Z3),(A3,B7,X4,Y1,Z1),(A3,B7,X4,Y1,Z2),(A3,B7,X4,Y1,Z3),(A3,B 7,X4,Y2,Z1),(A3,B7,X4,Y2,Z2),(A3,B7,X4,Y2,Z3),(A3,B7,X5,Y1,Z1),(A3,B7,X5,Y1,Z2),( A3,B7,X5,Y1,Z3),(A3,B7,X5,Y2,Z1),(A3,B7,X5,Y2,Z2),(A3,B7,X5,Y2,Z3),(A3,B7,X6,Y1, Z1),(A3,B7,X6,Y1,Z2),(A3,B7,X6,Y1,Z3),(A3,B7,X6,Y2,Z1),(A3,B7,X6,Y2,Z2),(A3,B7,X6 ,Y2,Z3),(A3,B7,X7,Y1,Z1),(A3,B7,X7,Y1,Z2),(A3,B7,X7,Y1,Z3),(A3,B7,X7,Y2,Z1),(A3,B 7,X7,Y2,Z2),(A3,B7,X7,Y2,Z3),(A3,B8,X1,Y1,Z1),(A3,B8,X1,Y1,Z2),(A3,B8,X1,Y1,Z3),( A3,B8,X1,Y2,Z1),(A3,B8,X1,Y2,Z2),(A3,B8,X1,Y2,Z3),(A3,B8,X2,Y1,Z1),(A3,B8,X2,Y1, Z2),(A3,B8,X2,Y1,Z3),(A3,B8,X2,Y2,Z1),(A3,B8,X2,Y2,Z2),(A3,B8,X2,Y2,Z3),(A3,B8,X3 ,Y1,Z1),(A3,B8,X3,Y1,Z2),(A3,B8,X3,Y1,Z3),(A3,B8,X3,Y2,Z1),(A3,B8,X3,Y2,Z2),(A3,B 8,X3,Y2,Z3),(A3,B8,X4,Y1,Z1),(A3,B8,X4,Y1,Z2),(A3,B8,X4,Y1,Z3),(A3,B8,X4,Y2,Z1),( A3,B8,X4,Y2,Z2),(A3,B8,X4,Y2,Z3),(A3,B8,X5,Y1,Z1),(A3,B8,X5,Y1,Z2),(A3,B8,X5,Y1, Z3),(A3,B8,X5,Y2,Z1),(A3,B8,X5,Y2,Z2),(A3,B8,X5,Y2,Z3),(A3,B8,X6,Y1,Z1),(A3,B8,X6 ,Y1,Z2),(A3,B8,X6,Y1,Z3),(A3,B8,X6,Y2,Z1),(A3,B8,X6,Y2,Z2),(A3,B8,X6,Y2,Z3),(A3,B 8,X7,Y1,Z1),(A3,B8,X7,Y1,Z2),(A3,B8,X7,Y1,Z3),(A3,B8,X7,Y2,Z1),(A3,B8,X7,Y2,Z2),( A3,B8,X7,Y2,Z3),(A4,B1,X1,Y1,Z1),(A4,B1,X1,Y1,Z2),(A4,B1,X1,Y1,Z3),(A4,B1,X1,Y2, Z1),(A4,B1,X1,Y2,Z2),(A4,B1,X1,Y2,Z3),(A4,B1,X2,Y1,Z1),(A4,B1,X2,Y1,Z2),(A4,B1,X2 ,Y1,Z3),(A4,B1,X2,Y2,Z1),(A4,B1,X2,Y2,Z2),(A4,B1,X2,Y2,Z3),(A4,B1,X3,Y1,Z1),(A4,B 1,X3,Y1,Z2),(A4,B1,X3,Y1,Z3),(A4,B1,X3,Y2,ZI),(A4,B1,X3,Y2,Z2),(A4,B1,X3,Y2,Z3),( A4,B1,X4,Y1,Z1),(A4,B1,X4,Y1,Z2),(A4,B1,X4,Y1,Z3),(A4,B1,X4,Y2,Z1),(A4,B1,X4,Y2, Z2),(A4,B1,X4,Y2,Z3),(A4,B1,X5,Y1,Z1),(A4,B1,X5,Y1,Z2),(A4,B1,X5,Y1,Z3),(A4,B1,X5 ,Y2,Z1),(A4,B1,X5,Y2,Z2),(A4,B1,X5,Y2,Z3),(A4,B1,X6,Y1,Z1),(A4,B1,X6,Y1,Z2),(A4,B 1,X6,Y1,Z3),(A4,B1,X6,Y2,Z1),(A4,B1,X6,Y2,Z2),(A4,B1,X6,Y2,Z3),(A4,B1,X7,Y1,Z1),( A4,B1,X7,Y1,Z2),(A4,B1,X7,Y1,Z3),(A4,B1,X7,Y2,Z1),(A4,B1,X7,Y2,Z2),(A4,B1,X7,Y2, Z3),(A4,B2,X1,Y1,Z1),(A4,B2,X1,Y1,Z2),(A4,B2,X1,Y1,Z3),(A4,B2,X1,Y2,Z1),(A4,B2,X1 ,Y2,Z2),(A4,B2,X1,Y2,Z3),(A4,B2,X2,Y1,Z1),(A4,B2,X2,Y1,Z2),(A4,B2,X2,Y1,Z3),(A4,B 2,X2,Y2,Z1),(A4,B2,X2,Y2,Z2),(A4,B2,X2,Y2,Z3),(A4,B2,X3,Y1,Z1),(A4,B2,X3,Y1,Z2),( A4,B2,X3,Y1,Z3),(A4,B2,X3,Y2,Z1),(A4,B2,X3,Y2,Z2),(A4,B2,X3,Y2,Z3), (A4,B2,X4,Y1, Z1),(A4,B2,X4,Y1,Z2),(A4,B2,X4,Y1,Z3),(A4,B2,X4,Y2,Z1),(A4,B2,X4,Y2,Z2),(A4,B2,X4 ,Y2,Z3),(A4,B2,X5,Y1,Z1),(A4,B2,X5,Y1,Z2),(A4,B2,X5,Y1,Z3),(A4,B2,X5,Y2,Z1),(A4,B 2,X5,Y2,Z2),(A4,B2,X5,Y2,Z3),(A4,B2,X6,Y1,Z1),(A4,B2,X6,Y1,Z2),(A4,B2,X6,Y1,Z3),( A4,B2,X6,Y2,Z1),(A4,B2,X6,Y2,Z2),(A4,B2,X6,Y2,Z3),(A4,B2,X7,Y1,Z1),(A4,B2,X7,Y1, Z2),(A4,B2,X7,Y1,Z3),(A4,B2,X7,Y2,Z1),(A4,B2,X7,Y2,Z2),(A4,B2,X7,Y2,Z3),(A4,B3,X ,Y1,Z1),(A4,B3,X1,Y1,Z2),(A4,B3,X1,Y1,Z3),(A4,B3,X1,Y2,Z1),(A4,B3,X1,Y2,Z2),(A4,B 3,X1,Y2,Z3),(A4,B3,X2,Y1,Z1),(A4,B3,X2,Y1,Z2),(A4,B3,X2,Y1,Z3),(A4,B3,X2,Y2,Z1),( A4,B3,X2,Y2,Z2),(A4,B3,X2,Y2,Z3),(A4,B3,X3,Y1,Z1),(A4,B3,X3,Y1,Z2),(A4,B3,X3,Y1, Z3),(A4,B3,X3,Y2,Z1),A4,B3,X3,Y2,Z2),(A4,B3,X3,Y2,Z3),(A4,B3,X4,Y1,Z1),(A4,B3,X4 ,Y1,Z2),(A4,B3,X4,Y1,Z3),(A4,B3,X4,Y2,Z1),(A4,B3,X4,Y2,Z2),(A4,B3,X4,Y2,Z3),(A4,B 3,X5,Y1,Z1),(A4,B3,X5,Y1,Z2),(A4,B3,X5,Y1,Z3),(A4,B3,X5,Y2,Z1),(A4,B3,X5,Y2,Z2),( A4,B3,X5,Y2,Z3),(A4,B3,X6,Y1,Z1),(A4,B3,X6,Y1,Z2),(A4,B3,X6,Y1,Z3),(A4,B3,X6,Y2, Z1),(A4,B3,X6,Y2,Z2),(A4,B3,X6,Y2,Z3),(A4,B3,X7,Y1,Z1),(A4,B3,X7,Y1,Z2),(A4,B3,X7 ,Y1,Z3),(A,4,B3,X7,Y2,Z1),(A4,B3,X7,Y2,Z2),(A4,B3,X7,Y2,Z3),(A4,B4,X1,Y1,Z1),(A4,B 4,X1,Y1,Z2),(A4,B4,X1,Y1,Z3),(A4,B4,X1,Y2,Z1),(A4,B4,X1,Y2,Z2),(A4,B4,X1,Y2,Z3),( A4,B4,X2,Y1,Z1),(A4,B4,X2,Y1,Z2),(A4,B4,X2,Y1,Z3),(A4,B4,X2,Y2,Z1),(A4,B4,X2,Y2, Z2),(A4,B4,X2,Y2,Z3),(A4,B4,X3,Y1,Z1),(A4,B4,X3,Y1,Z2),(A4,B4,X3,Y1,Z3),(A4,B4,X3 ,Y2,Z1),(A4,B4,X3,Y2,Z2),(A4,B4,X3,Y2,Z3),(A4,B4,X4,Y1,Z1),(A4,B4,X4,Y1,Z2),(A4,B 4,X4,Y1,Z3),(A4,B4,X4,Y2,Z1),(A4,B4,X4,Y2,Z2),(A4,B4,X4,Y2,Z3),(A4,B4,X5,Y1,Z1),( A4,B4,X5,Y1,Z2),(A4,B4,X5,Y1,Z3),(A4,B4,X5,Y2,Z1),(A4,B4,X5,Y2,Z2),(A4,B4,X5,Y2, Z3),(A4,B4,X6,Y1,Z1),(A4,B4,X6,Y1,Z2),(A4,B4,X6,Y1,Z3),(A4,B4,X6,Y2,Z1),(A4,B4,X6 ,Y2,Z2),(A4,B4,X6,Y2,Z3),(A4,B4,X7,Y1,Z1),(A4,B4,X7,Y1,Z2),(A4,B4,X,7,Y1,Z3),(A4,B 4,X7,Y2,Z1),(A4,B4,X7,Y2,Z2),(A4,B4,X7,Y2,Z3),(A4,B5,X1,Y1,Z1),(A4,B5,X1,Y1,Z2),( A4,B5,X1,Y1,Z3),(A4,B5,X1,Y2,Z1),(A4,B5,X1,Y2,Z2),(A4,B5,X1,Y2,Z3),(A4,B5,X2,Y1, Z1),(A4,B5,X2,Y1,Z2),(A4,B5,X2,Y1,Z3),(A4,B5,X2,Y2,Z1),(A4,B5,X2,Y2,Z2),(A4,B5,X2 ,Y2,Z3),(A4,B5,X3,Y1,Z1),(A4,B5,X3,Y1,Z2),(A4,B5,X3,Y1,Z3),(A4,B5,X3,Y2,Z1),(A4,B 5,X3,Y2,Z2),(A4,B5,X3,Y2,Z3),(A4,B5,X4,Y1,Z1),(A4,B5,X4,Y1,Z2),(A4,B5,X4,Y1,Z3),( A4,B5,X4,Y2,Z1),(A4,B5,X4,Y2,Z2),(A4,B5,X4,Y2,Z3),(A4,B5,X5,Y1,Z1),(A4,B5,X5,Y1, Z2),(A4,B5,X5,Y1,Z3),(A4,B5,X5,Y2,Z1),(A4,B5,X5,Y2,Z2),(A4,B5,X5,Y2,3),(A4,B5,X6 ,Y1,Z1),(A4,B5,X6,Y1,Z2),(A4,B5,X6,Y1,Z3),(A4,B5,X6,Y2,Z1),(A4,B5,X6,Y2,Z2),(A4,B 5,X6,Y2,Z3),(A4,B5,X7,Y1,Z1),(A4,B5,X7,Y1,Z2),(A4,B5,X7,Y1,Z3),(A4,B5,X7,Y2,Z1),( A4,B5,X7,Y2,Z2),(A4,B5,X7,Y2,Z3),(A4,B6,X1,Y1,Z1),(A4,B6,X1,Y1,Z2),(A4,B6,X1,Y1, Z3),(A4,B6,X1,Y2,Z1),(A4,B6,X1,Y2,Z2),(A4,B6,X1,Y2,Z3),(A4,B6,X2,Y1,Z1),(A4,B6,X2 ,Y1,Z2),(A4,B6,X2,Y1,Z3),(A4,B6,X2,Y2,Z1),(A4,B6,X2,Y2,Z2),(A4,B6,X2,Y2,Z3),(A4,B 6,X3,Y1,Z1),(A4,B6,X3,Y1,Z2),(A4,B6,X3,Y1,Z3),(A4,B6,X3,Y2,Z1),(A4,B6,X3,Y2,Z2),( A4,B6,X3,Y2,Z3),(A4,B6,X4,Y1,Z1),(A4,B6,X4,Y1,Z2),(A4,B6,X4,Y1,Z3),(A4,B6,X4,Y2, Z1),(A4,B6,X4,Y2,Z2),(A4,B6,X4,Y2,Z3),(A4,B6,X5,Y1,Z1),(A4,B6,X5,Y1,Z2),(A4,B6,X5 ,Y1,Z3),(A4,B6,X5,Y2,Z1),(A4,B6,X5,Y2,Z2),(A4,B6,X5,Y2,Z3),(A4,B6,X6,Y1,Z1),(A4,B 6,X6,Y1,Z2),(A4,B6,X6,Y1,Z3),(A4,B6,X6,Y2,Z1),(A4,B6,X6,Y2,Z2),(A4,B6,X6,Y2,Z3),( A4,B6,X7,Y1,Z1),(A4,B6,X7,Y1,Z2),(A4,B6,X7,Y1,Z3),(A4,B6,X7,Y2,Z1),(A4,B6,X7,Y2, Z2),(A4,B6,X7,Y2,Z3),(A4,B7,X1,Y1,Z1),(A4,B7,X1,Y1,Z2),(A4,B7,X1,Y1,Z3),(A4,B7,X1 ,Y2,Z1),(A4,B7,X1,Y2,Z2),(A4,B7,X1,Y2,Z3),(A4,B7,X2,Y1,Z1),(A4,B7,X2,Y1,Z2),(A4,B 7,X2,Y1,Z3),(A4,B7,X2,Y2,Z1),(A4,B7,X2,Y2,Z2),(A4,B7,X2,Y2,Z3),(A4,B7,X3,Y1,Z1),( A4,B7,X3,Y1,Z2),(A4,B7,X3,Y1,Z3),(A4,B7,X3,Y2,Z1),(A4,B7,X3,Y2,Z2),(A4,B7,X3,Y2, Z3),(A4,B7,X4,Y1,Z1),(A4,B7,X4,Y1,Z2),(A4,B7,X4,Y1,Z3),(A4,B7,X4,Y2,Z1),(A4,B7,X4 ,Y2,Z2),(A4,B7,X4,Y2,Z3),(A4,B7,X5,Y1,Z1),(A4,B7,X5,Y1,Z2),(A4,B7,X5,Y1,Z3),(A4,B 7,X5,Y2,Z1),(A4,B7,X5,Y2,Z2),(A4,B7,X5,Y2,Z3),(A4,B7,X6,Y1,Z1),(A4,B7,X6,Y1,Z2),( A4,B7,X6,Y1,Z3),(A4,B7,X6,Y2,Z1),(A4,B7,X6,Y2,Z2),(A4,B7,X6,Y2,Z3),(A4,B7,X7,Y1, Z1),(A4,B7,X7,Y1,Z2),(A4,B7,X7,Y1,Z3),(A4,B7,X7,Y2,Z1),(A4,B7,X7,Y2,Z2),(A4,B7,X7 ,Y2,Z3),(A4,B8,X1,Y1,Z1),(A4,B8,X1,Y1,Z2),(A4,B8,X1,Y1,Z3),(A4,B8,X1,Y2,Z1),(A4,B 8,X1,Y2,Z2),(A4,B8,X1,Y2,Z3),(A4,B8,X2,Y1,Z1),(A4,B8,X2,Y1,Z2),(A4,B8,X2,Y1,Z3),( A4,B8,X2,Y2,Z1),(A4,B8,X2,Y2,Z2),(A4,B8,X2,Y2,Z3),(A4,B8,X3,Y1,Z1),(A4,B8,X3,Y1, Z2),(A4,B8,X3,Y1,Z3),(A4,B8,X3,Y2,Z1),(A4,B8,X3,Y2,Z2),(A4,B8,X3,Y2,Z3),(A4,B8,X4 ,Y1,Z1),(A4,B8,X4,Y1,Z2),(A4,B8,X4,Y1,Z3),(A4,B8,X4,Y2,Z1),(A4,B8,X4,Y2,Z2),(A4,B 8,X4,Y2,23),(A4,B8,X5,Y1,Z1),(A4,B8,X5,Y1,Z2),(A4,B8,X5,Y1,Z3),(A4,B8,X5,Y2,Z1),( A4,B8,X5,Y2,Z2),(A4,B8,X5,Y2,Z3),(A4,B8,X6,Y1,Z1),(A4,B8,X6,Y1,Z2),(A4,B8,X6,Y1, Z3),(A4,B8,X6,Y2,Z1),(A4,B8,X6,Y2,Z2),(A4,B8,X6,Y2,z3),(A4,B8,X7,Y1,Z1),(A4,B8,X7 ,Y1,Z2),(A4,B8,X7,Y1,Z3),(A4,B8,X7,Y2,Z1),(A4,B8,X7,Y2,Z2),(A4,B8,X7,Y2,Z3),(A5,B 1,X1,Y1,Z1),(A5,B1,X1,Y1,Z2),(A5,B1,X1,Y1,Z3),(A5,B1,X1,Y2,Z1),(A5,B1,X1,Y2,Z2),( A5,B1,X1,Y2,Z3),(A5,B1,X2,Y1,Z1),(A5,B1,X2,Y1,Z2),(A5,B1,X2,Y1,Z3),(A5,B1,X2,Y2, Z1),(A5,B1,X2,Y2,Z2),(A5,B1,X2,Y2,Z3),(A5,B1,X3,Y1,Z1),(A5,B1,X3,Y1,Z2),(A5,B1,X3 ,Y1,Z3)7(A5,B1,X3,Y2,Z1),(A5,B1,X3,Y2,Z2),(A5,B1,X3,Y2,Z3),(A5,B1,X4,Y1,Z1),(A5,B 1,X4,Y1,Z2),(A5,B1,X4,Y1,Z3),(A5,B1,X4,Y2,Z1),(A5,B1,X4,Y2,Z2),(A5,B1,X4,Y2,Z3),( A5,B1,X5,Y1,Z1),(A5,B1,X5,Y1,Z2),(A5,B1,X5,Y1,Z3),(A5,B1,X5,Y2,Z1),(A5,B1,X5,Y2, Z2),(A5,B1,X5,Y2,Z3),(A5,B1,X6,Y1,Z1),(A5,B1,X6,Y1,Z2),(A5,B1,X6,Y1,Z3),(A5,B1,X6 ,Y2,Z1),(A5,B1,X6,Y2,Z2),(A5,B1,X6,Y2,Z3),(A5,B1,X7,Y1,Z1),(A5,B1,X7,Y1,Z2),(A5,B 1,X7,Y1,Z3),(A5,B1,X7,Y2,Z1),(A5,B1,X7,Y2,Z2),(A5,B1,X7,Y2,Z3),(A5,B2,X1,Y1,Z1), A5,B2,X1,Y1,Z2),(A5,B2,X1,Y1,Z3),(A5,B2,X1,Y2,Z1),(A5,B2,X1,Y2,Z2),(A5,B2,X1,Y2, 23),(A5,B2,X2,Y1,Z1),(A5,B2,X2,Y1,Z2),(A5,B2,X2,Y1,23),(A5,82,X2,Y2,Z1),(A5,82,X2 ,Y2,Z2),(A5,B2,X2,Y2,Z3),(A5,B2,X3,Y1,Z1),(A5,B2,X3,Y1,Z2),(A5,B2,X3,Y1,Z3),(A5,B 2,X3,Y2,Z1),(A5,B2,X3,Y2,Z2),(A5,B2,X3,Y2,Z3),(A5,B2,X4,Y1,Z1),(A5,B2,X4,Y1,Z2),( A5,B2,X4,Y1,Z3),(A5,B2,X4,Y2,Z1),(A5,B2,X4,Y2,Z2),(A5,B2,X4,Y2,Z3),A5,B2,X5,Y1, Z1),(A5,B2,X5,Y1,Z2),(A5,B2,X5,Y1,Z3),(A5,B2,X5,Y2,Z1),(A5,B2,X5,Y2,Z2),(A5,B2,X5 ,Y2,23),(A5,B2,X6,Y1,Z1),(A5,B2,X6,Y1,Z2),(A5,B2,X6,Y1,Z3),(A5,B2,X6,Y2,Z1),(A5,B 2,X6,Y2,Z2),(A5,B2,XG,Y2,Z3),(A5,B2,X7,Y1,Z1),(A5,B2,X7,Y1,Z2),(A5,B2,X7,Y1,Z3),( A5,B2,X7,Y2,Z1),(A5,B2,X7,Y2,Z2),(A5,B2,X7,Y2,Z3),(A5,B3,X1,Y1,Z1),(A5,B3,X1,Y1, Z2),(A5,B3,X1,Y1,Z3),(A5,B3,X1,Y2,Z1),(A5,B3,X1,Y2,Z2),(A5,B3,X1,Y2,Z3),(A5,B3,X2 ,Y1,Z1),(A5,B3,X2,Y1,Z2),(A5,B3,X2,Y1,Z3),(A5,B3,X2,Y2,Z1),(A5,B3,X2,Y2,Z2),(A5,B 3,X2,Y2,Z3),(A5,B3,X3,Y1,Z1),(A5,B3,X3,Y1,Z2),(A5,B3,X3,Y1,Z3),(A5,B3,X3,Y2,Z1),( A5,B3,X3,Y2,Z2),(A5,B3,X3,Y2,Z3),(A5,B3,X4,Y1,Z1),(A5,B3,X4,Y1,Z2),(A5,B3,X4,Y1, Z3),(A5,B3,X4,Y2,Z1),(A5,B3,X4,Y2,Z2),(A5,B3,X4,Y2,Z3),(A5,B3,X5,Y1,Z1),(A5,B3,X5 ,Y1,Z2),(A5,B3,X5,Y1,Z3),(A5,B3,X5,Y2,Z1),(A5,B3,X5,Y2,Z2),(A5,B3,X5,Y2,Z3),(A5,B 3,X6,Y1,Z1),(A5,B3,X6,Y1,Z2),(A5,B3,X6,Y1,Z3),(A5,B3,X6,Y2,Z1),(A5,B3,X6,Y2,Z2),( A5,B3,X6,Y2,Z3),(A5,B3,X7,Y1,Z1),(A5,B3,X7,Y1,Z2),(A5,B3,X7,Y1,Z3),(A5,B3,X7,Y2, Z1),(A5,B3,X7,Y2,Z2),(A5,B3,X7,Y2,Z3),(A5,B4,X1,Y1,Z1),(A5,B4,X1,Y1,Z2),(A5,B4,X1 ,Y1,Z3),(A5,B4,X1,Y2,Z1),(A5,B4,X1,Y2,Z2),(A5,B4,X1,Y2,Z3),(A5,B4,X2,Y1,Z1),(A5,B 4,X2,Y1,Z2),(A5,B4,X2,Y1,Z3),(A5,B4,X2,Y2,Z1),(A5,B4,X2,Y2,Z2),(A5,B4,X2,Y2,Z3),( A5,B4,X3,Y1,Z1),(A5,B4,X3,Y1,Z2),(A5,B4,X3,Y1,Z3),(A5,B4,X3,Y2,Z1),(A5,B4,X3,Y2, Z2),(A5,B4,X3,Y2,Z3),(A5,B4,X4,Y1,Z1),(A5,B4,X4,Y1,Z2),(A5,B4,X4,Y1,Z3),(A5,B4,X4 ,Y2,Z1),(A5,B4,X4,Y2,Z2),(A5,B4,X4,Y2,Z3),(A5,B4,X5,Y1,Z1),(A5,B4,X5,Y1,Z2),(A5,B 4,X5,Y1,Z3),(A5,B4,X5,Y2,Z1),(A5,B4,X5,Y2,Z2),(A5,B4,X5,Y2,Z3),(A5,B4,X6,Y1,Z1),( A5,B4,X6,Y1,Z2),(A5,B4,X6,Y1,Z3),(A5,B4,X6,Y2,Z1),(A5,B4,X6,Y2,Z2),(A5,B4,X6,Y2, Z3),(A5,B4,X7,Y1,Z1),(A5,B4,X7,Y1,Z2),(A5,B4,X7,Y1,Z3),(A5,B4,X7,Y2,Z1),(A5,B4,X7 ,Y2,Z2),(A5,B4,X7,Y2,Z3),(A5,B5,X1,Y1,Z1),(A5,B5,X1,Y1,Z2),(A5,B5,X1,Y1,Z3),(A5,B 5,X1,Y2,Z1),(A5,B5,X1,Y2,Z2),(A5,B5,X1,Y2,Z3),(A5,B5,X2,Y1,Z1),(A5,B5,X2,Y1,Z2),( A5,B5,X2,Y1,Z3),(A5,B5,X2,Y2,Z1),(A5,B5,X2,Y2,Z2),(A5,B5,X2,Y2,Z3),(A5,B5,X3,Y1, Z1),(A5,B5,X3,Y1,Z2),(A5,B5,X3,Y1,Z3),(A5,B5,X3,Y2,Z1),(A5,B5,X3,Y2,Z2),(A5,B5,X3 ,Y2,Z3),(A5,B5,X4,Y1,Z1),(A5,B5,X4,Y1,Z2),(A5,B5,X4,Y1,Z3),(A5,B5,X4,Y2,Z1),(A5,B 5,X4,Y2,Z2),(A5,B5,X4,Y2,Z3),(A5,B5,X5,Y1,Z1),(A5,B5,X5,Y1,Z2),(A5,B5,X5,Y1,Z3),( A5,B5,X5,Y2,Z1),(A5,B5,X5,Y2,Z2),(A5,B5,X5,Y2,Z3),(A5,B5,X6,Y1,Z1),(A5,B5,X6,Y1, Z2),(A5,B5,X6,Y1,Z3),(A5,B5,X6,Y2,Z1),(A5,B5,X6,Y2,Z2),(A5,B5,X6,Y2,Z3),(A5,B5,X7 ,Y1,Z1),(A5,B5,X7,Y1,Z2),(A5,B5,X7,Y1,Z3),(A5,B5,X7,Y2,Z1),(A5,B5,X7,Y2,Z2),(A5,B 5,X7,Y2,Z3),(A5,B6,X1,Y1,Z1),(A5,B6,X1,Y1,Z2),(A5,B6,X1,Y1,Z3),(A5,B6,X1,Y2,Z1),( A5,B6,X1,Y2,Z2),(A5,B6,X1,Y2,Z3),(A5,B6,X2,Y1,Z1),(A5,B6,X2,Y1,Z2),(A5,B6,X2,Y1, Z3),(A5,B6,X2,Y2,Z1),(A5,B6,X2,Y2,Z2),(A5,B6,X2,Y2,Z3),(A5,B6,X3,Y1,Z1),(A5,B6,X3 ,Y1,Z2),(A5,B6,X3,Y1,Z3),(A5,B6,X3,Y2,Z1),(A5,B6,X3,Y2,Z2),(A5,B6,X3,Y2,Z3),(A5,B 6,X4,Y1,Z1),(A5,B6,X4,Y1,Z2),(A5,B6,X4,Y1,Z3),(A5,B6,X4,Y2,Z1),(A5,B6,X4,Y2,Z2),( A5,B6,X4,Y2,Z3),(A5,136,X5,Y1,Z1),(A5,B6,X5,Y1,Z2),(A5,B6,X5,Y1,Z3),(A5,B6,X5,Y2, Z1),(A5,B6,X5,Y2,Z2),(A5,B6,X5,Y2,Z3),(A5,B6,X6,Y1,Z1),(A5,B6,X6,Y1,Z2),(A5,B6,X6 ,Y1,Z3),(A5,B6,X6,Y2,Z1),(A5,B6,X6,Y2,Z2),(A5,B6,X6,Y2,Z3),(A5,B6,X7,Y1,Z1),(A5,B 6,X7,Y1,Z2),(A5,B6,X7,Y1,Z3),(A5,B6,X7,Y2,Z1),(A5,B6,X7,Y2,Z2),(A5,B6,X7,Y2,Z3),( A5,B7,X1,Y1,Z1),(A5,B7,X1,Y1,Z2),(A5,B7,X1,Y1,Z3),(A5,B7,X1,Y2,Z1),(A5,B7,X1,Y2, Z2),(A5,B7,X1,Y2,Z3),(A5,B7,X2,Y1,Z1),(A5,B7,X2,Y1,Z2),(A5,B7,X2,Y1,Z3),(A5,B7,X2 ,Y2,Z1),(A5,87,X2,Y2,Z2),(A5,B7,X2,Y2,23),(A5,B7,X3,Y1,Z1),(A5,B7,X3,Y1,Z2),(A5,B 7,X3,Y1,Z3),(A5,B7,X3,Y2,Z1),(A5,B7,X3,Y2,Z2),(A5,B7,X3,Y2,Z3),(A5,B7,X4,Y1,Z1),( A5,B7,X4,Y1,Z2),(A5,B7,X4,Y1,Z3),(A5,B7,X4,Y2,Z1),(A5,B7,X4,Y2,Z2),(A5,B7,X4,Y2, Z3),(A5,B7,X5,Y1,Z1),(A5,B7,X5,Y1,Z2),(A5,B7,X5,Y1,Z3),(A5,B7,X5,Y2,Z1),(A5,B7,X5 ,Y2,Z2),(A5,B7,XS,Y2,Z3),(A5,B7,X6,Y1,Z1),(A5,B7,X6,Y1,Z2),(A5,B7,X6,Y1,Z3),(A5,B 7,X6,Y2,Z1),(A5,B7,X6,Y2,Z2),(A5,B7,X6,Y2,Z3),(A5,B7,X7,Y1,Z1),(A5,B7,X7,Y1,Z2),( A5,B7,X7,Y1,Z3),(A5,B7,X7,Y2,Z1),(A5,B7,X7,Y2,Z2),(A5,B7,X7,Y2,Z3),(A5,B8,X1,Y1, Z1),(A5,B8,X1,Y1,Z2),(A5,B8,X1,Y1,Z3),(A5,B8,X1,Y2,Z1),(A5,B8,X1,Y2,Z2),(A5,B8,X1 ,Y2,Z3),(A5,B8,X2,Y1,Z1),(A5,B8,X2,Y1,Z2),(A5,B8,X2,Y1,Z3),(A5,B8,X2,Y2,Z1),(A5,B 8,X2,Y2,Z2),(A5,B8,X2,Y2,Z3),(A5,B8,X3,Y1,Z1),(A5,B8,X3,Y1,Z2),(A5,B8,X3,Y1,Z3),( A5,B8,X3,Y2,Z1),(A5,B8,X3,Y2,Z2),(A5,B8,X3,Y2,Z3),(A5,B8,X4,Y1,Z1),(A5,B8,X4,Y1, Z2),(A5,B8,X4,Y1,Z3),(A5,B8,X4,Y2,Z1),(A5,B8,X4,Y2,Z2),(A5,B8,X4,Y2,Z3),(A5,B8,X5 ,Y1,Z1),(A5,B8,X5,Y1,Z2),(A5,B8,X5,Y1,Z3),(A5,B8,X5,Y2,Z1),(A5,B8,X5,Y2,Z2),(A5,B 8,X5,Y2,Z3),(A5,B8,X6,Y1,Z1),(A5,B8,X6,Y1,Z2),(A5,B8,X6,Y1,Z3),(A5,B8,X6,Y2,Z1),( A5,B8,X6,Y2,Z2),(A5,B8,X6,Y2,Z3),(A5,B8,X7,Y1,Z1),(A5,B8,X7,Y1,Z2),(A5,B8,X7,Y1, Z3),(A5,B8,X7,Y2,Z1),(A5,B8,X7,Y2,Z2),(A5,B8,X7,Y2,Z3),(A6,B1,X1,Y1,Z1),(A6,B1,X1 ,Y1,Z2),(A6,B1,X1,Y1,Z3),(A6,B1,X1,Y2,Z1),(A6,B1,X1,Y2,Z2),(A6,B1,X1,Y2,Z3),(A6,B 1,X2,Y1,Z1),(A6,B1,X2,Y1,Z2),(A6,B1,X2,Y1,Z3),(A6,B1,X2,Y2,Z1),(A6,B1,X2,Y2,Z2),( A6,B1,X2,Y2,Z3),(A6,B1,X3,Y1,Z1),(A6,B1,X3,Y1,Z2),(A6,B1,X3,Y1,Z3),(A6,B1,X3,Y2, Z1),(A6,B1,X3,Y2,Z2),(A6,B1,X3,Y2,Z3),(A6,B1,X4,Y1,Z1),(A6,B1,X4,Y1,Z2),(A6,B1,X4 ,Y1,Z3),(A6,B1,X4,Y2,Z1),(A6,B1,X4,Y2,Z2),(A6,B1,X4,Y2,Z3),(A6,B1,X5,Y1,Z1),(A6,B 1,X5,Y1,Z2),(A6,B1,X5,Y1,Z3),(A6,B1,X5,Y2,Z1),(A6,B1,X5,Y2,Z2),(A6,B1,X5,Y2,Z3),( A6,B1,X6,Y1,Z1),(A6,B1,X6,Y1,Z2),(A6,B1,X6,Y1,Z3),(A6,B1,X6,Y2,Z1),(A6,B1,X6,Y2, Z2),(A6,B1,X6,Y2,Z3),(A6,B1,X7,Y1,Z1),(A6,B1,X7,Y1,Z2),(A6,B1,X7,Y1,Z3),(A6,B1,X7 ,Y2,Z1),(A6,B1,X7,Y2,Z2),(A6,B1,X7,Y2,Z3),(A6,B2,X1,Y1,Z1),(A6,B2,X1,Y1,Z2),(A6,B 2,X1,Y1,Z3),(A6,B2,X1,Y2,Z1),(A6,B2,X1,Y2,Z2),(A6,B2,X1,Y2,Z3),(A6,B2,X2,Y1,Z1),( A6,B2,X2,Y1,Z2),(A6,B2,X2,Y1,Z3),(A6,B2,X2,Y2,Z1),(A6,B2,X2,Y2,Z2),(A6,B2,X2,Y2, Z3),(A6,B2,X3,Y1,Z1),(A6,B2,X3,Y1,Z2),(A6,B2,X3,Y1,Z3),(A6,B2,X3,Y2,Z1),(A6,B2,X3 ,Y2,Z2),(A6,B2,X3,Y2,Z3),(A6,B2,X4,Y1,Z1),(A6,B2,X4,Y1,Z2),(A6,B2,X4,Y1,Z3),(A6,B 2,X4,Y2,Z1),(A6,B2,X4,Y2,Z2),(A6,B2,X4,Y2,Z3),(A6,B2,X5,Y1,Z1),(A6,B2,X5,Y1,Z2),( A6,B2,X5,Y1,Z3),(A6,B2,X5,Y2,Z1),(A6,B2,X5,Y2,Z2),(A6,B2,X5,Y2,Z3),(A6,B2,X6,Y1, Z1),(A6,B2,X6,Y1,Z2),(A6,B2,X6,Y1,Z3),(A6,B2,X6,Y2,Z1),(A6,B2,X6,Y2,Z2),(A6,B2,X6 ,Y2,Z3),(A6,B2,X7,Y1,Z1),(A6,B2,X7,Y1,Z2),(A6,B2,X7,Y1,Z3),(A6,B2,X7,Y2,Z1),(A6,B 2,X7,Y2,Z2),(A6,B2,X7,Y2,Z3),(A6,B3,X1,Y1,Z1),(A6,B3,X1,Y1,Z2),(A6,B3,X1,Y1,Z3),( A6,B3,X1,Y2,Z1),(A6,B3,X1,Y2,Z2),(A6,B3,X1,Y2,Z3),(A6,B3,X2,Y1,Z1),(A6,B3,X2,Y1, Z2),(A6,B3,X2,Y1,Z3),(A6,B3,X2,Y2,Z1),(A6,B3,X2,Y2,Z2),(A6,B3,X2,Y2,Z3),(A6,B3,X3 ,Y1,Z1),(A6,B3,X3,Y1,Z2),(A6,B3,X3,Y1,Z3),(A6,B3,X3,Y2,Z1),(A6,B3,X3,Y2,Z2),(A6,B 3,X3,Y2,Z3),(A6,B3,X4,Y1,Z1),(A6,B3,X4,Y1,Z2),(A6,B3,X4,Y1,Z3),(A6,B3,X4,Y2,Z1),( A6,B3,X4,Y2,Z2),(A6,B3,X4,Y2,Z3),(A6,B3,X5,Y1,Z1),(A6,B3,X5,Y1,Z2),(A6,B3,X5,Y1, Z3),(A6,B3,X5,Y2,Z1),(A6,B3,X5,Y2,Z2),(A6,B3,X5,Y2,Z3),(A6,B3,X6,Y1,Z1),(A6,B3,X6 ,Y1,Z2),(A6,B3,X6,Y1,Z3),(A6,B3,X6,Y2,Z1),(A6,B3,X6,Y2,Z2),(A6,B3,X6,Y2,Z3),(A6,B 3,X7,Y1,Z1),(A6,B3,X7,Y1,Z2),(A6,B3,X7,Y1,Z3),(A6,B3,X7,Y2,Z1),(A6,B3,X7,Y2,Z2),( A6,B3,X7,Y2,Z3),(A6,B4,X1,Y1,Z1),(A6,B4,X1,Y1,Z2),(A6,B4,X1,Y1,Z3),(A6,B4,X1,Y2, Z1),(A6,B4,X1,Y2,Z2),(A6,B4,X1,Y2,Z3),(A6,B4,X2,Y1,Z1),(A6,B4,X2,Y1,Z2),(A6,B4,X2 ,Y1,Z3),(A6,B4,X2,Y2,Z1),(A6,B4,X2,Y2,Z2),(A6,B4,X2,Y2,Z3),(A6,B4,X3,Y1,Z1),(A6,B 4,X3,Y1,Z2),(A6,B4,X3,Y1,Z3),(A6,B4,X3,Y2,Z1),(A6,B4,X3,Y2,Z2),(A6,B4,X3,Y2,Z3),( A6,B4,X4,Y1,Z1),(A6,B4,X4,Y1,Z2),(A6,B4,X4,Y1,Z3),(A6,B4,X4,Y2,Z1),(A6,B4,X4,Y2, Z2),(A6,B4,X4,Y2,Z3),(A6,B4,X5,Y1,Z1),(A6,B4,X5,Y1,Z2),(A6,B4,X5,Y1,Z3),(A6,B4,X5 ,Y2,Z1),(A6,B4,X5,Y2,Z2),(A6,B4,X5,Y2,Z3),(A6,B4,X6,Y1,Z1),(A6,B4,X6,Y1,Z2),(A6,B 4,X6,Y1,Z3),(A6,B4,X6,Y2,Z1),(A6,B4,X6,Y2,Z2),(A6,B4,X6,Y2,Z3),(A6,B4,X-1,Y1,Z1),( A6,B4,X7,Y1,Z2),(A6,B4,X7,Y1,Z3),(A6,B4,X7,Y2,Z1),(A6,B4,X7,Y2,Z2),(A6,B4,X7,Y2, Z3),(A6,B5,X1,Y1,Z1),(A6,B5,X1,Y1,Z2),(A6,B5,X1,Y1,Z3),(A6,B5,X1,Y2,Z1),(A6,B5,X1 ,Y2,Z2),(A6,B5,X1,Y2,Z3),(A6,B5,X2,Y1,Z1),(A6,B5,X2,Y1,Z2),(A6,B5,X2,Y1,Z3),(A6,B 5,X2,Y2,Z1),(A6,B5,X2,Y2,Z2),(A6,B5,X2,Y2,Z3),(A6,B5,X3,Y1,Z1),(A6,B5,X3,Y1,Z2),( A6,B5,X3,Y1,Z3),(A6,B5,X3,Y2,Z1),(A6,B5,X3,Y2,Z2),(A6,B5,X3,Y2,Z3),(A6,B5,X4,Y1, Z1),(A6,B5,X4,Y1,Z2),(A6,B5,X4,Y1,Z3),(A6,B5,X4,Y2,Z1),(A6,B5,X4,Y2,Z2),(A6,B5,X4 ,Y2,Z3),(A6,B5,X5,Y1,Z1),(A6,B5,X5,Y1,Z2),(A6,B5,X5,Y1,Z3),(A6,B5,X5,Y2,Z1),(A6,B 5,X5,Y2,Z2),(A6,B5,X5,Y2,Z3),(A6,B5,X6,Y1,Z1),(A6,B5,X6,Y1,Z2),(A6,B5,X6,Y1,Z3),( A6,B5,X6,Y2,Z1),(A6,B5,X6,Y2,Z2),(A6,B5,X6,Y2,Z3),(A6,B5,X7,Y1,Z1),(A6,B5,X7,Y1, Z2),(A6,B5,X7,Y1,Z3),(A6,B5,X7,Y2,Z1),(A6,B5,X7,Y2,Z2),(A6,B5,X7,Y2,Z3),(A6,B6,X ,Y1,Z1),(A6,B6,X1,Y1,Z2),(A6,B6,X1,Y1,Z3),(A6,B6,X1,Y2,Z1),(A6,B6,X1,Y2,Z2),(A6,B 6,X1,Y2,Z3),(A6,B6,X2,Y1,Z1),(A6,B6,X2,Y1,Z2),(A6,B6,X2,Y1,Z3),(A6,B6,X2,Y2,Z1),( A6,B6,X2,Y2,Z2),(A6,B6,X2,Y2,Z3),(A6,B6,X3,Y1,Z1),(A6,B6,X3,Y1,Z2),(A6,B6,X3,Y1, Z3),(A6,B6,X3,Y2,Z1),(A6,B6,X3,Y2,Z2),(A6,B6,X3,Y2,Z3),(A6,B6,X4,Y1,Z1),(A6,B6,X4 ,Y1,Z2),(A6,B6,X4,Y1,Z3),(A6,B6,X4,Y2,Z1),(A6,B6,X4,Y2,Z2),(A6,B6,X4,Y2,Z3),(A6,B 6,X5,Y1,Z1),(A6,B6,X5,Y1,Z2),(A6,B6,X5,Y1,Z3),(A6,B6,X5,Y2,Z1),(A6,B6,X5,Y2,Z2),( A6,B6,X5,Y2,Z3),(A6,B6,X6,Y1,Z1),(A6,B6,X6,Y1,Z2),(A6,B6,X6,Y1,Z3),(A6,B6,X6,Y2, Z1),(A6,B6,X6,Y2,Z2),(A6,B6,X6,Y2,Z3),(A6,B6,X7,Y1,Z1),(A6,B6,X7,Y1,Z2),(A6,B6,X7 ,Y1,Z3),(A6,B6,X7,Y2,Z1),(A6,B6,X7,Y2,Z2),(A6,B6,X7,Y2,Z3),(A6,B7,X1,Y1,Z1),(A6,B 7,X1,Y1,Z2),(A6,B7,X1,Y1,Z3),(A6,B7,X1,Y2,Z1),(A6,B7,X1,Y2,Z2),(A6,B7,X1,Y2,Z3),( A6,B7,X2,Y1,Z1),(A6,B7,X2,Y1,Z2),(A6,B7,X2,Y1,Z3),(A6,B7,X2,Y2,Z1),(A6,B7,X2,Y2, Z2),(A6,B7,X2,Y2,Z3),(A6,B7,X3,Y1,Z1),(A6,B7,X3,Y1,Z2),(A6,B7,X3,Y1,Z3),(A6,B7,X3 ,Y2,Z1),(A6,B7,X3,Y2,Z2),(A6,B7,X3,Y2,Z3),(A6,B7,X4,Y1,Z1),(A6,B7,X4,Y1,Z2),(A6,B 7,X4,Y1,Z3),(A6,B7,X4,Y2,Z1),(A6,B7,X4,Y2,Z2),(A6,B7,X4,Y2,Z3),(A6,B7,X5,Y1,Z1),( A6,B7,X5,Y1,Z2),(A6,B7,X5,Y1,Z3),(A6,B7,X5,Y2,Z1),(A6,B7,X5,Y2,Z2),(A6,B7,X5,Y2, Z3),(A6,B7,X6,Y1,Z1),(A6,B7,X6,Y1,Z2),(A6,B7,X6,Y1,Z3),(A6,B7,X6,Y2,Z1),(A6,B7,X6 ,Y2,Z2),(A6,B7,X6,Y2,Z3),(A6,B7,X7,Y1,Z1),(A6,B7,X7,Y1,Z2),(A6,B7,X7,Y1,Z3),(A6,B 7,X7,Y2,Z1),(A6,B7,X7,Y2,Z2),(A6,B7,X7,Y2,Z3),(A6,B8,X1,Y1,Z1),(A6,B8,X1,Y1,Z2),( A6,B8,X1,Y1,Z3),(A6,B8,X1,Y2,Z1),(A6,B8,X1,Y2,Z2),(A6,B8,X1,Y2,Z3),(A6,B8,X2,Y1, Z1),(A6,B8,X2,Y1,Z2),(A6,B8,X2,Y1,Z3),(A6,B8,X2,Y2,Z1),(A6,B8,X2,Y2,Z2),(A6,B8,X2 ,Y2,Z3),(A6,B8,X3,Y1,Z1),(A6,B8,X3,Y1,Z2),(A6,B8,X3,Y1,Z3),(A6,B8,X3,Y2,Z1),(A6,B 8,X3,Y2,Z2),(A6,B8,X3,Y2,Z3),(A6,B8,X4,Y1,Z1),(A6,B8,X4,Y1,Z2),(A6,B8,X4,Y1,Z3),( A6,B8,X4,Y2,Z1),(A6,B8,X4,Y2,Z2),(A6,B8,X4,Y2,Z3),(A6,B8,X5,Y1,Z1),(A6,B8,X5,Y1, Z2),(A6,B8,X5,Y1,Z3),(A6,B8,X5,Y2,Z1),(A6,B8,X5,Y2,Z2),(A6,B8,X5,Y2,Z3),(A6,B8,X6 ,Y1,Z1),(A6,B8,X6,Y1,Z2),(A6,B8,X6,Y1,Z3),(A6,B8,X6,Y2,Z1),(A6,B8,X6,Y2,Z2),(A6,B 8,X6,Y2,Z3),(A6,B8,X7,Y1,Z1),(A6,B8,X7,Y1,Z2), (A6,B8,X7,Y1,Z3),(A6,B8,X7,Y2,Z1),( A6,B8,X7,Y2,Z2),(A6,B8,X7,Y2,Z3),(A7,B1,X1,Y1,Z1),(A7,B1,X1,Y1,Z2),(A7,B1,X1,Y1, Z3),(A7,B1,X1,Y2,Z1),(A7,B1,X1,Y2,Z2),(A7,B1,X1,Y2,Z3),(A7,B1,X2,Y1,Z1),(A7,B1,X2 ,Y1,Z2),(A7,B1,X2,Y1,Z3),(A7,B1,X2,Y2,Z1),(A7,B1,X2,Y2,Z2),(A7,B1,X2,Y2,Z3),(A7,B 1,X3,Y1,Z1),(A7,B1,X3,Y1,Z2),(A7,B1,X3,Y1,Z1),(A7,B1,X3,Y2,Z1),(A7,B1,X3,Y2,Z2),( A7,B1,X3,Y2,Z3),(A7,B1,X4,Y1,Z1),(A7,B1,X4,Y1,Z2),(A7,B1,X4,Y1,Z3),(A7,B1,X4,Y2, Z1),(A7,B1,X4,Y2,Z2),(A7,B1,X4,Y2,Z3),(A7,B1,X5,Y1,Z1),(A7,B1,X5,Y1,Z2),(A7,B1,X5 ,Y1,Z3),(A7,B1,X5,Y2,Z1),(A7,B1,X5,Y2,Z2),(A7,B1,X5,Y2,Z3),(A7,B1,X6,Y1,Z1),(A7,B 1,X6,Y1,Z2),(A7,B1,X6,Y1,Z3),(A7,B1,X6,Y2,Z1),(A7,B1,X6,Y2,Z2),(A7,B1,X6,Y2,Z3),( A7,B1,X7,Y1,Z1),(A7,B1,X7,Y1,Z2),(A7,B1,X7,Y1,Z3),(A7,B1,X7,Y2,Z1),(A7,B1,X7,Y2, Z2),(A7,B1,X7,Y2,Z3),(A7,B2,X1,Y1,Z1),(A7,B2,X1Y1,Z2),(A7,B2,X1,Y1,Z3),(A7,B2,X1 ,Y2,Z1),(A7,B2,X1,Y2,Z2),(A7,B2,X1,Y2,Z3),(A7,B2,X2,Y1,Z1),(A7,B2,X2,Y1,Z2),(A7,B 2,X2,Y1,Z3),(A7,B2,X2,Y2,Z1),(A7,B2,X2,Y2,Z2),(A7,B2,X2,Y2,Z3),(A7,B2,X3,Y1,Z1),( A7,B2,X3,Y1,Z2),(A7,B2,X3,Y1,Z3),(A7,B2,X3,Y2,Z1),(A7,B2,X3,Y2,Z2),(A7,B2,X3,Y2, Z3),(A7,B2,X4,Y1,Z1),(A7,B2,X4,Y1,Z2),(A7,B2,X4,Y1,Z3),(A7,B2,X4,Y2,Z1),(A7,B2,X4 ,Y2,Z2),(A7,82,X4,Y2,23),(A7,82,X5,Y1,Z1),(A7,B2,X5,Y1,Z2),(A7,B2,X5,Y1,Z3),(A7,B 2,X5,Y2,Z1),(A7,B2,X5,Y2,Z2),(A7,B2,X5,Y2,Z3),(A7,B2,X6,Y1,Z1),(A7,B2,X6,Y1,Z2),( A7,B2,X6,Y1,Z3),(A7,B2,X6,Y2,Z1),(A7,B2,X6,Y2,Z2),(A7,B2,X6,Y2,Z3),(A7,B2,X7,Y1, Z1),(A7,B2,X7,Y1,Z2),(A7,B2,X7,Y1,Z3),(A7,B2,X7,Y2,Z1),(A7,B2,X7,Y2,Z2),(A7,B2,X7 ,Y2,Z3),(A7,B3,X1,Y1,Z1),(A7,B3,X1,Y1,Z2),(A7,B3,X1,Y1,Z3),(A7,B3,X1,Y2,Z1),(A7,B 3,X1,Y2,Z2),(A7,B3,X1,Y2,Z3),(A7,B3,X2,Y1,Z1),(A7,B3,X2,Y1,Z2),(A7,B3,X2,Y1,Z3),( A7,B3,X2,Y2,Z1),(A7,B3,X2,Y2,Z2),(A7,B3,X2,Y2,Z3),(A7,B3,X3,Y1,Z1),(A7,B3,X3,Y1, Z2),(A7,B3,X3,Y1,Z3),(A7,B3,X3,Y2,Z1),(A7,B3,X3,Y2,Z2),(A7,B3,X3,Y2,Z3),(A7,B3,X4 ,Y1,Z1),(A7,B3,X4,Y1,Z2),(A7,B3,X4,Y1,Z3),(A7,B3,X4,Y2,Z1),(A7,B3,X4,Y2,Z2),(A7,B 3,X4,Y2,Z3),(A7,B3,X5,Y1,Z1),(A7,B3,X5,Y1,Z2),(A7,B3,X5,Y1,Z3),(A7,B3,X5,Y2,Z1),( A7,B3,X5,Y2,Z2),(A7,B3,X5,Y2,Z3),(A7,B3,X6,Y1,Z1),(A7,B3,X6,Y1,Z2),(A7,B3,X6,Y1, Z3),(A7,B3,X6,Y2,Z1),(A7,B3,X6,Y2,Z2),(A7,B3,X6,Y2,Z3),(A7,B3,X7,Y1,Z1),(A7,B3,X7 ,Y1,Z2),(A7,B3,X7,Y1,Z3),(A7,B3,X7,Y2,Z1),(A7,B3,X7,Y2,Z2),(A7,B3,X7,Y2,Z3),(A7,B 4,X1,Y1,Z1),(A7,B4,X1,Y1,Z2),(A7,B4,X1,Y1,Z3),(A7,B4,X1,Y2,Z1),(A7,B4,X1,Y2,Z2),( A7,B4,X1,Y2,Z3),(A7,B4,X2,Y1,Z1),(A7,B4,X2,Y1,Z2),(A7,B4,X2,Y1,Z3),(A7,B4,X2,Y2, Z1),(A7,B4,X2,Y2,Z2),(A7,B4,X2,Y2,Z3),(A7,B4,X3,Y1,Z1),(A7,B4,X3,Y1,Z2),(A7,B4,X3 ,Y1,Z3),(A7,B4,X3,Y2,Z1),(A7,B4,X3,Y2,Z2),(A7,B4,X3,Y2,Z3),(A7,B4,X4,Y1,Z1),(A7,B 4,X4,Y1,Z2),(A7,B4,X4,Y1,Z3),(A7,B4,X4,Y2,Z1),(A7,B4,X4,Y2,Z2),(A7,B4,X4,Y2,Z3),( A7,B4,X5,Y1,Z1),(A7,B4,X5,Y1,Z2),(A7,B4,X5,Y1,Z3),(A7,B4,X5,Y2,Z1),(A7,B4,X5,Y2, Z2),(A7,B4,X5,Y2,Z3),(A7,B4,X6,Y1,Z1),(A7,B4,X6,Y1,Z2),(A7,B4,X6,Y1,Z3),(A7,B4,X6 ,Y2,Z1),(A7,B4,X6,Y2,Z2),(A7,B4,X6,Y2,Z3),(A7,B4,X7,Y1,Z1),(A7,B4,X7,Y1,Z2),(A7,B 4,X7,Y1,Z3),(A7,B4,X7,Y2,Z1),(A7,B4,X7,Y2,Z2),(A7,B4,X7,Y2,Z3),(A7,B5,X1,Y1,Z1),( A7,B5,X1,Y1,Z2),(A7,B5,X1,Y1,Z3),A7,B5,X1,Y2,Z1),(A7,B5,X1,Y2,Z2),(A7,B5,X1,Y2, Z3),(A7,B5,X2,Y1,Z1),(A7,B5,X2,Y1,Z2),(A7,B5,X2,Y1,Z3),(A7,B5,X2,Y2,Z1),(A7,B5,X2 ,Y2,Z2),(A7,B5,X2,Y2,Z3),(A7,B5,X3,Y1,Z1),CA7,B5,X3,Y1,Z2),(A7,B5,X3,Y1,Z3),(A7,B 5,X3,Y2,Z1),(A7,B5,X3,Y2,Z2),(A7,B5,X3,Y2,Z3),(A7,B5,X4,Y1,Z1),(A7,B5,X4,Y1,Z2),( A7,B5,X4,Y1,Z3),(A7,B5,X4,Y2,Z1),(A7,B5,X4,Y2,Z2),(A7,B5,X4,Y2,Z3),(A7,B5,X5,Y1, Z1),(A7,B5,X5,Y1,Z2),(A7,B5,X5,Y1,Z3),(A7,B5,X5,Y2,Z1),(A7,B5,X5,Y2,Z2),(A7,B5,X5 ,Y2,Z3),(A7,B5,X6,Y1,Z1),(A7,B5,X1,Y1,Z2),(A7,B5,X6,Y1,Z3),(A7,B5,X6,Y2,Z1),(A7,B 5,X6,Y2,Z2),(A7,B5,X6,Y2,Z3),(A7,85,X7,Y1,Z1),(A7,85,X7,Y1,22),(A7,85,X7,Y1,Z3),( A7,B5,X7,Y2,Z1),(A1,B5,X7,Y2,Z2),(A7,B5,X7,Y2,Z3),(A7,B6,X1,Y1,Z1),(A7,B6,X3,Y1, Z2),(A7,B6,X1,Y1,Z3),(A7,B6,X1,Y2,Z1),(A7,B6,X1,Y2,Z2),(A7,B6,X1,Y2,Z3),(A7,B6,X2 ,Y1,Z1),(A7,B6,X2,Y1,X2),(A7,B6,X2,Y1,Z3),(A7,B6,X2,Y2,Z1),(A7,B6,X2,Y2,Z2),(A7,B 6,X2,Y2,Z3),(A7,B3,X3,Y1,Z1),(A7,B6,X3,Y1,Z2),(A7,B6,X3,Y1,Z3),(A7,B6,X3,Y2,Z1),( A7,B6,X3,Y2,Z2),(A7,B6,X3,Y2,Z3),(A7,B6,X4,Y1,Z1),(A7,B6,X4,Y1,Z2),(A7,B6,X4,Y1, Z3),(A7,B6,X4,Y2,Z1),(A7,B6,X4,Y2,Z2),(A7,B6,X4,Y2,Z3),(A7,B6,X5,Y1,Z1),(A7,B6,X5 ,Y1,Z2),(A7,B6,X5,Y1,Z3),(A7,B6,X5,Y2,Z2),(A7,B6,X5,Y2,Z1),(A7,B6,X5,Y2,3),(A7,B 6,X6,Y1,Z1),(A7,B6,X6,Y1,Z2),(A7,B6,X6,Y1,Z3),(A7,B6,X6,Y2,Z1),(A7,B6,X6,Y2,Z2),( A7,B6,X6,Y2,Z3),(A7,B6,X7,Y1,Z1),(A7,B6,X1,Y1,Z2),(A7,B8,X7,Y1,Z3),(A7,B6,X7,Y2, Z1),(A7,B6,X7,Y2,Z2),(A7,B6,X7,Y2,Z3),(A7,B7,X1,Y1,Z1),(A7,B7,X1,Y1,Z2),(A7,B7,X1 ,Y1,Z3),(A7,B7,X1,Y2,Z1),(A7,B7,X1,Y2,Z2),(A7,B7,X1,Y2,Z3),(A7,B7,X2,Y1,Z1),(A7,B 7,X2,Y1,Z2),(A7,B7,X2,Y1,Z3),(A7,B7,X2,Y2,Z1),(A7,B7,X2,Y2,Z2),(A7,B7,X2,Y2,Z3),( A7,B7,X3,Y1,Z1),(A7,B7,X3,Y1,Z2),(A7,B7,X3,Y1,Z3),(A7,B7,X3,Y2,Z1),(A7,B7,X3,Y2, Z2),(A7,B7,X3,Y2,Z3),(A7,B7,X4,Y1,Z1),(A7,B7,X4,Y1,Z2),(A7,B7,X4,Y1,Z3),(A7,B7,X4 ,Y2,Z1),(A7,B7,X4,Y2,Z2),(A7,B7,X4,Y2,Z3),(A7,B7,X5,Y1,Z1),(A7,B7,X5,Y1,Z2),(A7,B 7,X5,Y1,Z3),(A7,B7,X5,Y2,Z1),(A7,B7,X5,Y2,Z2),(A7,B7,X5,Y2,Z3),(A7,B7,X6,Y1,Z1),( A7,B7,X6,Y1,Z2),(A7,B7,X6,Y1,Z3),(A7,B7,X6,Y2,Z1),(A7,B7,X6,Y2,Z2),(A7,B7,X6,Y2, Z3),(A7,B7,X7,Y1,Z1),(A7,B7,X7,Y1,Z2),(A7,B7,X7,Y1,Z3),(A7,B7,X7,Y2,Z1),(A7,B7,X7 ,Y2,Z2),(A7,B7,X7,Y2,Z3),(A7,B8,X1,Y1,Z1),(A7,B8,X1,Y1,Z2),(A7,B8,X1,Y1,Z3),(A7,B 8,X1,Y2,Z1),(A7,B8,X1,Y2,Z2),(A7,B8,X1,Y2,Z3),(A7,B8,X2,Y1,Z1),(A7,B8,X2,Y1,Z2),( A7,B8,X2,Y1,Z3),(A7,B8,X2,Y2,Z1),(A7,B8,X2,Y2,Z2),(A7,B8,X2,Y2,Z3),(A1,B8,X3,Y1, Z1),(A7,B8,X3,Y1,Z2),(A7,B8,X3,Y1,Z3),(A7,B8,X3,Y2,Z1),(A7,B8,X3,Y2,Z2),(A7,B8,X3 ,Y2,Z3),(A7,B8,X4,Y1,Z1),(A7,B8,X4,Y1,Z2),(A7,B8,X4,Y1,Z3),(A7,B8,X4,Y2,Z1),(A7,B 8,X4,Y2,Z2),(A7,B8,X4,Y2,23),(A7,B8,X5,Y1,Z1),(A7,B8,X5,Y1,Z2),(A7,B8,X5,Y1,Z3),( A7,B8,X5,Y2,Z1),(A7,B8,X5,Y2,Z2),(A7,B8,X5,Y2,Z3),(A7,B8,X6,Y1,Z1),(A7,B8,X6,Y1, Z2),(A7,B8,X6,Y1,Z3),(A7,B8,X6,Y2,Z1),(A7,B8,X6,Y2,Z2),(A7,B8,X6,Y2,Z3),(A7,B8,X7 ,Y1,Z1),(A7,B8,X7,Y1,Z2),(A7,B8,X7,Y1,Z3),(A7,B8,X7,Y2,Z1),(A7,B8,X7,Y2,Z2),(A7,B 8,X7,Y2,Z3),(A8,B1,X1,Y1,Z1),(A8,B1,X1,Y1,Z2),(A8,B1,X1,Y1,Z3),(A8,B1,X1,Y2,Z1),( A8,B1,X1,Y2,Z2),(A8,B1,X1,Y2,Z3),(A8,B1,X2,Y1,Z1),(A8,B1,X2,Y1,Z2),(A8,B1,X2,Y1, Z8),(A8,B1,X2,Y2,Z1),(A8,B1,X2,Y2,Z2),(A8,B1,X2,Y2,Z3),(A8,B1,X3,Y1,Z1),(A8,B1,X3 ,Y1,Z2),(A8,B1,X8,Y1,Z3),(A8,B1,X3,Y2,Z1),(A8,B1,X8,Y2,Z2),(A8,B1,X3,Y2,Z3),(A8,B 1,X4,Y1,Z1),(A8,B1,X4,Y1,Z2),(A8,B1,X4,Y1,Z3),(A8,B1,X4,Y2,Z1),(A8,B1,X4,Y2,Z2),( A8,B1,X4,Y2,Z3),(A8,B1,X5,Y1,Z1),(A8,B1,X5,Y1,Z2),(A8,B1,X5,Y1,Z3),(A8,B1,X5,Y2, Z1),(A8,B1,X5,Y2,Z2),(A8,B1,X5,Y2,Z3),(A8,B1,X6,Y1,Z1),(A8,B1,X6,Y1,Z2),(A8,B1,X6 ,Y1,Z3),(A8,B1,X6,Y2,Z1),(A8,B1,X6,Y2,Z2),(A8,B1,X6,Y2,Z3),(A8,B1,X7,Y1,Z1),(A8,B 1,X7,Y1,Z2),(A8,B1,X7,Y1,Z3),(A8,B1,X7,Y2,Z1),(A8,B1,X7,Y2,Z2),(A8,B1,X7,Y2,Z3),( A8,B2,X1,Y1,Z1),(A8,B2,X1,Y1,Z2),(A8,B2,X1,Y1,Z8),(A8,B2,X1,Y2,Z1),(A8,B2,X1,Y2, Z2),(A8,B2,X1,Y2,Z3),(A8,B2,Z2,Y1,Z2),(A8,B2,X2,YZ,Z2),(A8,B2,X2,Y1,Z3),(A8,B2,X2 ,Y2,Z1),(A8,B2,X2,Y2,Z2),(A8,B2,X2,Y2,Z3),(A8,B2,X3,Y1,Z1),(A8,B2,X1,Y1,Z2),(A8,B 2,X3,Y1,23),(A8,B2,X3,Y2,Z1),(A8,B2,X3,Y2,Z2),(A8,B2,X3,Y2,Z3),(A8,B2,X4,Y1,Z1),( A8,B2,X1,Y1,Z2),(A8,B2,X4,Y1,Z3),(A8,B2,X1,Y2,Z1),(A8,B2,X4,Y2,Z2),(A8,B2,X4,Y2, Z3),(A8,B2,X5,Y1,Z1),(A8,B2,X5,Y1,Z2),(A8,B2,X5,Y1,Z3),(A8,B2,X5,Y2,Z1),(A8,B2,X5 ,Y2,Z2),(A8,B2,X5,Y2,Z3),(A8,B2,X6,Y1,Z1),(A8,B2,X6,Y1,Z2),(A8,B2,X6,Y1,Z3),(A8,B 2,X6,Y2,Z1),(A8,B2,X6,Y2,Z2),(A8,B2,X6,Y2,Z3),(A8,B2,X7,Y1,Z1),(A8,B2,X7,Y1,Z2),( A8,B2,X7,Y1,Z3),(A8,B2,X7,Y2,Z1),(A8,B2,X7,Y2,Z2),(A8,B2,X7,Y2,Z3),(A8,B3,X1,Y1, Z1),(A8,B3,X1,Y1,Z2),(A8,B3,X1,Y1,Z3),(A8,B3,X1,Y2,Z1),(A8,B3,X1,Y2,Z2),(A8,B3,X1 ,Y2,Z3),(A8,B3,X2,Y1,Z1),(A8,B3,X2,Y1,Z2),(A8,B3,X2,Y1,Z3),(A8,B3,X2,Y2,Z1),(A8,B 3,X2,Y2,Z2),(A8,B3,X2,Y2,Z3),(A8,B3,X3,Y1,Z1),(A8,B3,X8,Y1,Z2),(A8,B3,X3,Y1,Z3),( A8,B3,X3,Y2,Z1),(A8,B3,X3,Y2,Z2),(A8,B3,X3,Y2,Z3),(A8,B3,X4,Y1,Z1),(A8,B3,X4,Y1, Z2),(A8,B3,X4,Y1,Z3),(A8,B3,X4,Y2,Z1),(A8,B3,X4,Y2,Z2),(A8,B3,X4,Y2,Z3),(A8,B3,X5 ,Y1,Z1), (A8,B3,X5,Y1,Z2),(A8,B3,X5,Y1,Z3),(A8,B3,X5,Y2,Z1),(A8,B3,X5,Y2,Z2),(A8,B 3,X5,Y2,Z3),(A8,B3,X6,Y1,Z1),(A8,B3,X6,Y1,Z2),(A8,B3,X6,Y1,Z3),(A8,B3,X6,Y2,Z1),( A8,B3,X6,Y2,Z2),(A8,B3,X6,Y2,Z3),(A8,B3,X7,Y1,Z1),(A8,B3,X7,Y1,Z2),(A8,B3,X7,Y1, Z3),(A8,B3,X7,Y2,Z1),(A8,B3,X7,Y2,Z2),(A8,B3,X7,Y2,Z3),(A8,B4,X1,Y1,Z1),(A8,B4,X1 ,Y1,Z2),(A8,B4,X1,Y1,Z3),(A8,B4,X1,Y2,Z1),(A8,B4,X1,Y2,Z2),(A8,B4,X1,Y2,Z3),(A8,B 4,X2,Y1,Z1),(A8,B4,X2,Y1,Z2),(A8,B4,X2,Y1,Z,3),(A8,B4,X2,Y2,Z1),(A8,B4,X2,Y2,Z2), A8,B4,X2,Y2,Z3),(A8,B4,X3,Y1,Z1),(A8,B4,X3,Y1,Z2),(A8,B4,X3,Y1,Z3),(A8,B4,X3,Y2, Z1),(A8,B4,X3,Y2,Z2),(A8,B4,X3,Y2,Z3),(A8,B4,X4,Y1,Z1),(A8,B4,X4,Y1,Z2),(A8,B4,X4 ,Y1,Z3),(A8,B4,X4,Y2,Z1),(A8,4,X4,Y2,Z2),(A8,B4,X4,Y2,Z3),(A8,B4,X5,Y1,Z1),(A8,B 4,X5,Y1,Z2),(A8,B4,X5,Y1,Z3),(A8,B4,X5,Y2,Z1),(A8,B4,X5,Y2,Z2),(A8,B4,X5,Y2,Z3),( A8,B4,X6,Y1,Z1),(A8,B4,X6,Y1,Z2),(A8,B4,X6,Y1,Z3),(A8,B4,X6,Y2,Z1),(A8,B4,X6,Y2, Z2),(A8,B4,X6,Y2,Z3),(A8,B4,X7,Y1,Z1),(A8,B4,X7,Y1,Z2),(A8,B4,X7,Y1,Z3),(A8,B4,X7 ,Y2,Z1),(A8,B4,X7,Y2,Z2),(A8,B4,X7,Y2,Z3),(A8,B5,X1,Y1,Z1),(A8,B5,X1,Y1,Z2),(A8,B 5,X1,Y1,Z3),(A8,B5,X1,Y2,Z1),(A8,B5,X1,Y2,Z2),(A8,B5,X1,Y2,Z3),(A8,B5,X2,Y1,Z1),( A8,B5,X2,Y1,Z2),(A8,B5,X2,Y1,Z3),(A8,B5,X2,Y2,Z1),(A8,B5,X2,Y2,Z2),(A8,B5,X2,Y2, Z3),(A8,B5,X3,Y1,Z1),(A8,B5,X3,Y1,Z2),(A8,B5,X3,Y1,Z3),(A8,B5,X3,Y2,Z1),(A8,B5,X3 ,Y2,Z2),(A8,B5,X3,Y2,Z3),(A8,B5,X4,Y1,Z1),(A8,B5,X4,Y1,Z2),(A8,B5,X4,Y1,Z3),(A8,B 5,X4,Y2,Z1),(A8,B5,X4,Y2,Z2),(A8,B5,X4,Y2,Z3),(A8,B5,X5,Y1,Z1),(A8,B5,X5,Y1,Z2),( A8,B5,X5,Y1,Z3),(A8,B5,X5,Y2,Z1),(A8,B5,X5,Y2,Z2),(A8,B5,X5,Y2,23),(A8,B5,X6,Y1, Z1),(A8,B5,X6,Y1,Z2),(A8,B5,X6,Y1,Z3),(A8,B5,X6,Y2,Z1),(A8,B5,X6,Y2,Z2),(A8,B5,X6 ,Y2,Z3),(A8,B5,X7,Y1,Z1),(A8,B5,X7,Y1,Z2),(A8,B5,X7,Y1,Z3),(A8,B5,X7,Y2,Z1),(A8,B 5,X7,Y2,Z2),(A8,B5,X7,Y2,Z3),(A8,B6,X1,Y1,Z1),(A8,B6,X1,Y1,Z2),(A8,B6,X1,Y1,Z3),( A8,B6,X1,Y2,Z1),(A8,B6,X1,Y2,Z2),(A8,B6,X1,Y2,Z3),(A8,B6,X2,Y1,Z1),(A8,B6,X2,Y1, Z2),(A8,B6,X2,Y1,Z3),(A8,B6,X2,Y2,Z1),(A8,B6,X2,Y2,Z2),(A8,B6,X2,Y2,Z3),(A8,B6,X3 ,Y1,Z1),(A8,B6,X3,Y1,Z2),(A8,B6,X3,Y1,Z3),(A8,B6,X3,Y2,Z1),(A8,B6,X3,Y2,Z2),(A8,B 6,X3,Y2,Z3),(A8,B6,X4,Y1,Z1),(A8,B6,X4,Y1,Z2),(A8,B6,X4,Y1,Z3),(A8,B6,X4,Y2,Z1),( A8,B6,X4,Y2,Z2),(A8,B6,X4,Y2,Z3),(A8,B6,X5,Y1,Z1),(A8,B6,X5,Y1,Z2),(A8,B6,X5,Y1, Z3),(A8,B6,X5,Y2,Z1),(A8,B6,X5,Y2,Z2),(A8,B6,X5,Y2,Z3),(A8,B6,X6,Y1,Z1),(A8,B6,X6 ,Y1,Z2),(A8,B6,X6,Y1,Z3),(A8,B6,X6,Y2,Z1),(A8,B6,X6,Y2,Z2),(A8,B6,X6,Y2,Z3),(A8,B 6,X7,Y1,Z1),(A8,B6,X7,Y1,Z2),(A8,B6,X1,Y1,Z3), (A8,B6,X7,Y2,Z1),(A8,B6,X2,Y2,Z2),( A8,B6,X7,Y2,Z3),(A8,B7,X1,Y1,Z1),(A8,B7,X1,Y1,Z2),(A8,B7,X1,Y1,Z3),(A8,B7,X1,Y2, Z1),(A8,B7,X1,Y2,Z2),(A8,B7,X1,Y2,Z3),(A8,B7,X2,Y1,Z1),(A8,B7,X2,Y1,Z2),(A8,B7,X2 ,Y1,Z3),(A8,B7,X2,Y2,Z1),(A8,B7,X2,Y2,Z2),(A8,B7,X2,Y2,Z3),(A8,B7,X3,Y1,Z1),(A8,B 7,X3,Y1,Z2),(A8,B7,X3,Y1,Z3),(A8,B7,X3,Y2,Z1),(A8,B7,X3,Y2,Z2),(A8,B7,X3,Y2,Z3),( A8,B7,X4,Y1,Z1),(A8,B7,X4,Y1,Z2),(A8,B7,X4,Y1,Z3),(A8,B7,X4,Y2,Z1),(A8,B7,X4,Y2, Z2),(A8,B7,X4,Y2,Z3),(A8,B7,X5,Y1,Z1),(A8,B7,X5,Y1,Z2),(A8,B7,X5,Y1,Z3),(A8,B7,X5 ,Y2,Z1),(A8,B7,X5,Y2,Z2),(A8,B7,X5,Y2,Z3),(A8,B7,X6,Y1,Z1),(A8,B7,X6,Y1,Z2),(A8,B 7,X6,Y1,Z3),(A8,B7,X6,Y2,Z1),(A8,B7,X6,Y2,Z2),(A8,B7,X6,Y2,Z3),(A8,B7,X7,Y1,Z1),( A8,B7,X7,Y1,Z2),(A8,B7,X7,Y1,Z3),(A8,B7,X7,Y2,Z1),(A8,B7,X7,Y2,Z2),(A8,B7,X7,Y2, Z3),(A8,B8,X1,Y1,Z1),(A8,B8,X1,Y1,Z2),(A8,B8,X1,Y1,Z3),(A8,B8,X1,Y2,Z1),(A8,B8,X1 ,Y2,Z2),(A8,B8,X1,Y2,Z3),(A8,B8,X2,Y1,Z1),(A8,B8,X2,Y1,Z2),(A8,B8,X2,Y1,Z3),(A8,B 8,X2,Y2,Z1),(A8,B8,X2,Y2,Z2),(A8,B8,X2,Y2,Z3),(A8,B8,X3,Y1,Z1),(A8,B8,X3,Y1,Z2),( A8,B8,X3,Y1,Z3),(A8,B8,X3,Y2,Z1),(A8,B8,X3,Y2,Z2),(A8,B8,X3,Y2,Z3),(A8,B8,X4,Y1, Z1),(A8,B8,X4,Y1,Z2),(A8,B8,X4,Y1,Z3),(A8,B8,X4,Y2,Z1),(A8,B8,X4,Y2,Z2),(A8,B8,X4 ,Y2,Z3),(A8,B8,X5,Y1,Z1),(A8,B8,X5,Y1,Z2),(A8,B8,X5,Y1,Z3),(A8,B8,X5,Y2,Z1),(A8,B 8,X5,Y2,Z2),(A8,B8,X5,Y2,Z3),(A8,B8,X6,Y1,Z1),(A8,B8,X6,Y1,Z2),(A8,B8,X6,Y1,Z3),( A8,B8,X6,Y2,Zl),(A8,B8,X6,Y2,22),(A8,B8,X6,Y2,23),(A8,B8,X7,Y1,Z1),(A8,B8,X7,Y1, Z2),(A8,B8,X7,Y1,Z3),(A8,B8,X7,Y2,Z1),(A8,B8,X7,Y2,Z2),(A8,B8,X7,Y2,Z3),(A9,B1,X1 ,Y1,Z1),(A9,B1,X1,Y1,Z2),(A9,B1,X1,Y1,Z3),(A9,B1,X1,Y2,Z1),(A9,B1,X1,Y2,Z2),(A9,B 1,X1,Y2,Z3),(A9,B1,X2,Y1,Z1),(A9,B1,X2,Y1,Z2),(A9,B1,X2,Y1,Z3),(A9,B1,X2,Y2,Z1),( A9,B1,X2,Y2,Z2),(A9,B1,X2,Y2,Z3),(A9,B1,X3,Y1,Z1),(A9,B1,X3,Y1,Z2),(A9,B1,X3,Y1, Z3),(A9,B1,X3,Y2,Z1),(A9,B1,X3,Y2,Z2),(A9,B1,X3,Y2,Z3),(A9,B1,X4,Y1,Z1),(A9,B1,X4 ,Y1,Z2),(A9,B1,X4,Y1,Z3),(A9,B1,X4,Y2,Z1),(A9,B1,X4,Y2,Z2),(A9,B1,X4,Y2,Z3),(A9,B 1,X5,Y1,Z1),(A9,B1,X5,Y1,Z2),(A9,B1,X5,Y1,Z3),(A9,B1,X5,Y2,Z1),(A9,B1,X5,Y2,Z2),( A9,B1,X5,Y2,Z3),(A9,B1,X6,Y1,Z1),(A9,B1,X6,Y1,Z2),(A9,B1,X6,Y1,Z3),(A9,B1,X6,Y2, Z1),(A9,B1,X6,Y2,Z2),(A9,B1,X6,Y2,Z3),(A9,B1,X7,Y1,Z1),(A9,B1,X7,Y1,Z2),(A9,B1,X7 ,Y1,Z3),(A9,B1,X7,Y2,Z1),(A9,B1,X7,Y2,Z2),(A9,B1,X7,Y2,Z3),(A9,B2,X1,Y1,Z1),(A9,B 2,X1,Y1,Z2),(A9,B2,X1,Y1,Z3),(A9,B2,X1,Y2,Z1),(A9,B2,X1,Y2,Z2),(A9,B2,X1,Y2,Z3),( A9,B2,X2,Y1,Z1),(A9,B2,X2,Y1,Z2),(A9,B2,X2,Y1,Z3),(A9,B2,X2,Y2,Z1),(A9,B2,X2,Y2, Z2),(A9,B2,X2,Y2,Z3),(A9,B2,X3,Y1,Z1),(A9,B2,X3,Y1,Z2),(A9,B2,X3,Y1,Z3),(A9,B2,X3 ,Y2,Z1),(A9,B2,X3,Y2,Z2),(A9,B2,X3,Y2,Z3),(A9,B2,X4,Y1,Z1),(A9,B2,X4,Y1,Z2),(A9,B 2,X4,Y1,Z3),(A9,B2,X4,Y2,Z1),(A9,B2,X4,Y2,Z2),(A9,B2,X4,Y2,Z3),(A9,B2,X5,Y1,Z1),( A9,B2,X5,Y1,Z2),(A9,B2,X5,Y1,Z3),(A9,B2,X5,Y2,Z1),(A9,B2,X5,Y2,Z2),(A9,B2,X5,Y2, Z3),(A9,B2,X6,Y1,Z1),(A9,B2,X6,Y1,Z2),(A9,B2,X6,Y1,Z3),(A9,B2,X6,Y2,Z1),(A9,B2,X6 ,Y2,Z2),(A9,B2,X6,Y2,Z3),(A9,B2,X7,Y1,Z1),(A9,B2,X7,Y1,Z2),(A9,B2,X7,Y1,Z3),(A9,B 2,X7,Y2,Z1),*(A9,*B2,X7,Y2,Z2),(A9,B2,X7,Y2,Z3),(A9,B3,X1,Y1,Z1),(A9*,*B3,X1,Y1,Z2),( A9,B3,X1,Y1,Z3),(A9,B3,X1,Y2,Z1),(A9,B3,X1,Y2,Z2),(A9,B3,X1,Y2,Z3),(A9,B3,X2,Y1, Z1),(A9,B3,X2,Y1,Z2),(A9,B3,X2,Y1,Z3),(A9,B3,X2,Y2,Z1),(A9,B3,X2,Y2,Z2),(A9,B3,X2 ,Y2,23),(A9,B3,X3,Y1,Z1),(A9,B3,X3,Y1,Z2),(A9,B3,X3,Y1,Z3),(A9,B3,X3,Y2,Z1),(A9,B 3,X3,Y2,Z2),(A9,B3,X3,Y2,Z3),(A9,B3,X4,Y1,Z1),(A9,B3,X4,Y1,Z2),(A9,B3,X4,Y1,Z3),( A9,B3,X4,Y2,Z1),(A9,B3,X4,Y2,Z2),(A9,B3,X4,Y2,Z3),(A9,B3,X5,Y1,Z1),(A9,B3,X5,Y1, Z2),(A9,B3,X5,Y1,Z3),(A9,B3,X5,Y2,Z1),(A9,B3,X5,Y2,Z2),(A9,B3,X5,Y2,Z3),(A9,B3,X6 ,Y1,Z1),(A9,B3,X6,Y1,Z2),(A9,B3,X6,Y1,Z3),(A9,B3,X6,Y2,Z1),(A9,B3,X6,Y2,Z2),(A9,B 3,X6,Y2,Z3),(A9,B3,X7,Y1,Z1),(A9,B3,X7,Y1,Z2),(A9,B3,X7,Y1,Z3),(A9,B3,X7,Y2,Z1),( A9,B3,X7,Y2,Z2),(A9,B3,X7,Y2,Z3),(A9,B4,X1,Y1,Z1),(A9,B4,X1,Y1,Z2),(A9,B4,X1,Y1, Z3),(A9,B4,X1,Y2,Z1),(A9,B4,X1,Y2,Z2),(A9,B4,X1,Y2,Z3),(A9,B4,X2,Y1,Z1),(A9,B4,X2 ,Y1,Z2),(A9,B4,X2,Y1,Z3),(A9,B4,X2,Y2,Z1),(A9,B4,X2,Y2,Z2),(A9,B4,X2,Y2,Z3),(A9,B 4,X3,Y1,Z1),(A9,B4,X3,Y1,Z2),(A9,B4,X3,Y1,Z3),(A9,B4,X3,Y2,Z1),(A9,B4,X,3,Y2,Z2),( A9,B4,X3,Y2,Z3),(A9,B4,X4,Y1,Z1),(A9,B4,X4,Y1,Z2),(A9,B4,X4,Y1,Z3),(A9,B4,X4,Y2, Z1),(A9,B4,X4,Y2,Z2),(A9,B4,X4,Y2,Z3),(A9,B4,X5,Y1,Z1),(A9,B4,X5,Y1,Z2),(A9,B4,X5 ,Y1,Z3), (A9,B4,X5,Y2,Z1),(A9,B4,X5,Y2,Z2),(A9,B4,X5,Y2,Z3),(A9,B4,X6,Y1,Z1),(A9,B 4,X6,Y1,Z2),(A9,B4,X6,Y1,Z3),(A9,B4,X6,Y2,Z1),(A9,B4,X6,Y2,Z2),(A9,B4,X6,Y2,Z3),( A9,B4,X7,Y1,Z1),(A9,B4,X7,Y1,Z2),(A9,B4,X7,Y1,Z3),(A9,B4,X7,Y2,Z1),(A9,B4,X7,Y2, Z2),(A9,B4,X7,Y2,Z3),(A9,B5,X1,Y1,Z1),(A9,B5,X1,Y1,Z2),(A9,B5,X1,Y1,Z3),(A9,B5,X1 ,Y2,Z1),(A9,B5,X1,Y2,Z2),(A9,B5,X1,Y2,Z3),(A9,B5,X2,Y1,Z1),(A9,B5,X2,Y1,Z2),A9,B 5,X2,Y1,Z3),(A9,B5,X2,Y2,Z1),(A9,B5,X2,Y2,Z2),(A9,B5,X2,Y2,Z3),(A9,B5,X3,Y1,Z1),( A9,B5,X3,Y1,Z2),(A9,B5,X3,Y1,Z3),(A9,B5,X3,Y2,Z1),(A9,B5,X3,Y2,Z2),(A9,B5,X3,Y2, Z3),(A9,B5,X4,Y1,Z1),(A9,B5,X4,Y1,Z2),(A9,B5,X4,Y1,Z3),(A9,B5,X4,Y2,Z1),(A9,B5,X4 ,Y2,Z2),(A9,B5,X4,Y2,Z3),(A9,B5,X5,Y1,Z1),(A9,B5,X5,Y1,Z2),(A9,B5,X5,Y1,Z3),(A9,B 5,X5,Y2,Z1),(A9,B5,X5,Y2,Z2),(A9,B5,X5,Y2,Z3),(A9,B5,X6,Y1,Z1),(A9,B5,X6,Y1,Z2),( A9,B5,X6,Y1,Z3),(A9,B5,X6,Y2,Z1),(A9,B5,X6,Y2,Z2),(A9,B5,X6,Y2,Z3),(A9,B5,X7,Y1, Z1),(A9,B5,X7,Y1,Z2),(A9,B5,X7,Y1,Z3),(A9,B5,X7,Y2,Z1),(A9,B5,X7,Y2,Z2),(A9,B5,X7 ,Y2,Z3),(A9,B6,X1,Y1,Z1),(A9,B6,X1,Y1,Z2),(A9,B6,X1,Y1,Z3),(A9,B6,X1,Y2,Z1),(A9,B 6,X1,Y2,Z2),(A9,B6,X1,Y2,Z3),(A9,B6,X2,Y1,Z1),(A9,B6,X2,Y1,Z2),(A9,B6,X2,Y1,Z3),( A9,B6,X2,Y2,Z1),(A9,B6,X2,Y2,Z2),(A9,B6,X2,Y2,Z3),(A9,B6,X3,Y1,Z1),(A9,B6,X3,Y1, Z2),(A9,B6,X3,Y1,Z3),(A9,B6,X3,Y2,Z1),CA9,B6,X3,Y2,Z2),(A9,B6,X3,Y2,Z3),(A9,B6,X4 ,Y1,Z1),(A9,B6,X4,Y1,Z2),(A9,B6,X4,Y1,Z3),(A9,B6,X4,Y2,Z1),(A9,B6,X4,Y2,Z2),(A9,B 6,X4,Y2,Z3),(A9,B6,X5,Y1,Z1),(A9,B6,X5,Y1,Z2),(A9,B6,X5,Y1,Z9),(A9,B6,X5,Y2,Z1),( A9,B6,X5,Y2,Z2),(A9,B6,X5,Y2,Z3),(A9,B6,X6,Y1,Z1),(A9,B6,X6,Y1,Z2),(A9,B6,X6,Y1, Z3),(A9,B6,X6,Y2,Z1),(A9,B6,X6,Y2,Z2),(A9,B6,X6,Y2,Z3),(A9,B6,X7,Y1,Z1),(A9,B6,X7 ,Y1,Z2),(A9,B6,X7,Y1,Z3),(A9,B6,X7,Y2,Z1),(A9,B6,X7,Y2,Z2),(A9,B6,X7,Y2,Z3),(A9,B 7,X1,Y1,Z1),(A9,B7,X1,Y1,Z2),(A9,B7,X1,Y1,Z3),(A9,B7,X1,Y2,Z1),(A9,B7,X1,Y2,Z2),( A9,B7,X1,Y2,Z3),(A9,B7,X2,Y1,Z1),(A9,B7,X2,Y1,Z2),(A9,B7,X2,Y1,Z3),(A9,B7,X2,Y2, Z1),(A9,B7,X2,Y2,Z2),(A9,B7,X2,Y2,Z3),(A9,B7,X3,Y1,Z1),(A9,B7,X3,Y1,Z2),(A9,B7,X3 ,Y1,Z3),(A9,B7,X3,Y2,Z1),(A9,B7,X3,Y2,Z2),(A9,B7,X3,Y2,Z3),(A9,B7,X4,Y1,Z1),(A9,B 7,X4,Y1,Z2),(A9,B7,X4,Y1,Z3),(A9,B7,X4,Y2,Z1),(A9,B7,X4,Y2,Z2),(A9,B7,X4,Y2,Z3),( A9,B7,X5,Y1,Z1),(A9,B7,X5,Y1,Z2),(A9,B7,X5,Y1,Z3),(A9,B7,X5,Y2,Z1),(A9,B7,X5,Y2, Z2),(A9,B7,X5,Y2,Z3),(A9,B7,X6,Y1,Z1),(A9,B7,X6,Y1,Z2),(A9,B7,X6,Y1,Z3),A9,B7,X6 ,Y2,Z1),(A9,B7,X6,Y2,Z2),(A9,B7,X6,Y2,Z3),(A9,B7,X7,Y1,Z1),(A9,B7,X7,Y1,Z2),(A9,B 7,X7,Y1,Z3),(A9,B7,X7,Y2,Z1),(A8,B7,X7,Y2,Z2),(A9,B7,X7,Y2,Z3),(A9,B8,X1,Y1,Z1),( A9,B8,X1,Y1,Z2),(A9,B8,X1.Y1,Z3),(A9,B8,X1,Y2,Z1),(A9,B8,X1,Y2,Z2),(A9,B8,X1,Y2, Z3),(A9,B8,X2,Y1,Z1),(A9,B8,X2,Y1,Z2),(A9,B8,X2,Y1,Z3),(A9,B8,X2,Y2,Z1),(A9,B8,X2 ,Y2,Z2),(A9,B8,X2,Y2,Z3),(A9,B8,X3,Y1,Z1),(A9,B8,X3,Y1,Z2),(A9,B8,X3,Y1,Z3),(A9,B 8,X3,Y2,Z1),(A9,B8,X3,Y2,Z2),(A9,B8,X3,Y2,Z3),(A9,B8,X4,Y1,Z1),(A9,B8,X4,Y1,Z2),( A9,B8,X4,Y1,Z3),(A9,B8,X4,Y2,Z1),(A9,B8,X4,Y2,Z2),(A9,B8,X4,Y2,Z3),(A9,B8,X5,Y1, Z1),(A9,B8,X5,Y1,Z2),(A9,B8,X5,Y1,Z3),(A9,B8,X5,Y2,Z1),(A9,B8,X5,Y2,Z2),(A9,B8,X5 ,Y2,Z3),(A9,B8,X6,Y1,Z1),(A9,B8,X6,Y1,Z2),(A3,B8,X6,Y1,ZB),(A9,B8,X6,Y2,Z1),(A9,B B,X6,Y2,Z2),(A9,B8,X6,Y2,Z8),(A9,B8,X7,Y1,Z1),(A9,B8,X7,Y1,Z2),(A9,B8,X7,Y1,Z3),( A9,B8,X7,Y2,Z1),(A9,B8,X7,Y2,Z2),(A9,B8,X7,Y2,Z3)

General procedures for the synthesis of the compound of the present invention are described bellow. Starting materials and reaction reagents used in such synthesis are commercially available or can be prepared according to methods well known in the art using compounds commercially available.

The compound of the present invention represented by the general formula (I) may be prepared in accordance with the synthetic methods as described bellow.

### [Method A]

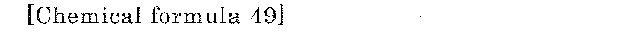

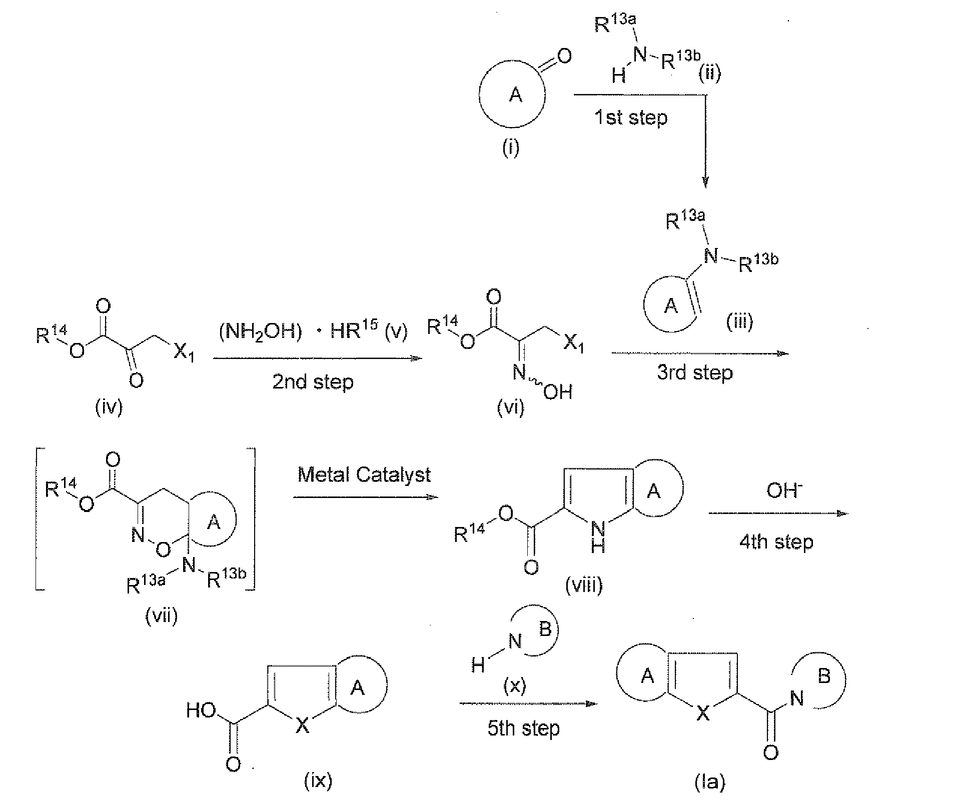

wherein, R^{13a} and R^{13b} are each independently hydrogen or substituted or unsubstituted alkyl; R¹⁴ is substituted or unsubstituted alkyl ; R¹⁵ is halide ion such as chloride ion or bromide ion, etc.; X¹ is leaving group such as halogen, mesyloxy group or tosyloxy group, etc.; all other variables are as defined above (1) or (1α).

### (1st step)

Enamine (iii) may be prepared by the condensation of ketone (i) with amine (ii) in the presense of acid catalyst or metal catalyst.
Solvent that may be used includes toluene, dichloromethane, 1, 2-dichloroethane, chloroform, etc.
Condensing agent that may be used includes p-toluenesulfonic acid hydrate, titanium tetrachloride, tetraisopropyl orthotitanate, etc. Refluxing only may be used.
Temperature for such reaction may be 0 °C to refluxing temperature of the solvent.
Reaction time may be 0.5 to 24 hours.
The obtained enamine (iii) may be purified according to conventional method (e.g., column chromatography, recrystallization, etc.).

### (2nd step)

Oxime (vi) may be prepared by the condensation of ketoester (iv) with hydroxylamine salt (v).
Solvent that may be used includes chloroform, dichloromethane, 1, 2-dichloroethane, water, and the mixture thereof, etc.
Temperature for such reaction may be 0 °C to refluxing temperature of the solvent.
Reaction time may be 0.5 to 24 hours.
The obtained oxime (vi) may be purified according to conventional method (e.g., column chromatography, recrystallization, etc.).

### (3rd step)

Oxazine (vii) may be prepared by the condensation of enamine (iii) with oxime (vi), followed by reduction of oxazine (vii) by using metal catalyst to afford a compound (viii).
Metal catalyst that may be used includes zinc dust, aluminium amalgam, iron carbonyl complex, etc.
Solvent that may be used includes toluene, dichloromethane, 1, 2-dichloroethane, tetrahydrofuran, ether, acetonitrile, etc.
Temperature for such reaction may be 0 °C to refluxing temperature of the solvent.
Reaction time may be 0.5 to 24 hours.
The obtained the compound (viii) may be purified according to conventional method (e.g., column chromatography, recrystallization, etc.).

### (4th step)

Carboxylic acid (ix) may be prepared by the hydrolysis of the compound (viii).
Lithium hydroxide, sodium hydroxide, potassium hydroxide, etc. may be used in an amount of 1 to 5 mol equivalent(s) in respect of the compound (viii).
Solvent that may be used includes methanol, ethanol, propanol, isopropanol, butanol, tetrahydrofuran, water, and the mixture thereof, etc.
Temperature for such reaction may be 0 °C to refluxing temperature of the solvent.
Reaction time may be 0.5 to 24 hours.
The obtained carboxylic acid. (ix) may be purified according to conventional method (e.g., column chromatography, recrystallization, etc.).

### (5th step)

Amide compound represented by the general formula (Ia) may be prepared by the condensation of carboxylic acid (ix) with amine (x) in the presence of condensing agent.
Amine (x) may be used in an amount of 0.5 to 4 mol equivalent(s) in respect of carboxylic acid (ix).
Condensing agent that may be used includes dicyclohexylcarbodiimide, 1-ethyl-3-(3-dimethylaminopropyl) carbodiimide hydrochloride, N, N'-carbonyldiimidazole, HBTU (2-(1H-benzotrzazole-1-yl)-1,1,3,3-tetramethyluronium hexafluorophosphate), HATU (2-(7-Azabenzotriazole-1-yl)-1,1,3,3-tetramethyluronium hexafluorophosphate), ethyl chlorocarbonate, isobutyl chlorocarbonate, thionyl chloride, oxalyl chloride, etc., it may be used in an amount of 0.5 to 2 mol equivalent(s) in respect of carboxylic acid (ix). 1-Hydroxybenzotriazole may be used in an amount of 0.5 to 2 mol equivalent(s) as supplemental condensing agent.
Base that may be used includes triethylamine, N,N-diisopropylethylamine, N-methylmorpholine, 4-dimethylaminopyridine, and the mixture thereof, etc. Base may be used in an amount of 0.05 to 4 mol equivalent(s) in respect of carboxylic acid (ix).
Solvent that may be used includes methylene chloride, tetrahydrofuran, N,N-dimethylformamide, and the mixture thereof, etc.
Temperature for such reaction may be 0 °C to refluxing temperature of the solvent.
Reaction time may be 0.5 to 72 hours.
The obtained compound represented by the general formula (Ia) may be purified according to conventional method (e.g., column chromatography, recrystallization, etc.).

### (Method B)

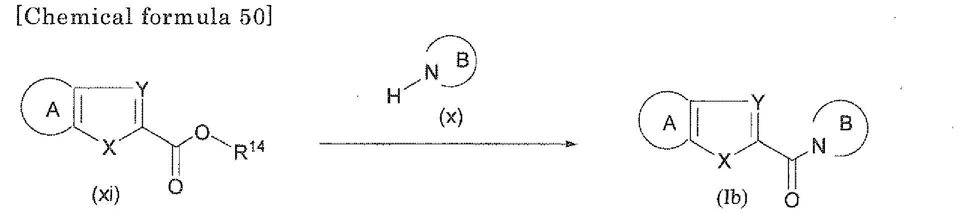

wherein, all variables in the scheme are as defined above.
Amide compound represented by the general formula (Ib) may also be prepared by the condensation of compound (xi) with amine (x).
The compound amine (x) may be used in an amount of 0.5 to 4 mol equivalent(s) in respect of the compound (xi).
Condensing agent that may be used includes trimethylaluminium, dimethylaluminum chloride, etc. Such reaction may be performed without condensing agent.
Solvent that may be used includes methylene chloride, hexane, toluene, and the mixture thereof, etc.
Temperature for such reaction may be 0 °C to refluxing temperature of the solvent.
Reaction time may be 0.5 to 24 hours.
The obtained compound represented by the general formula (Ib) may be purified according to conventional method (e.g., column chromatography, recrystallization, etc.).

### (Method C)

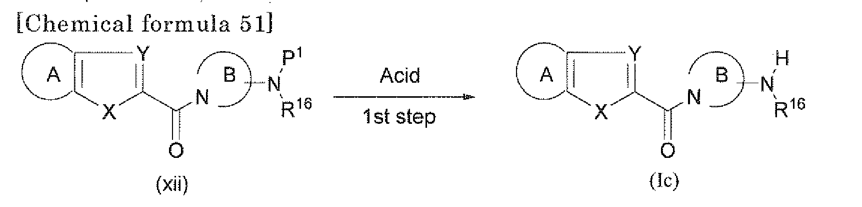

wherein, P¹ is an appropriate protecting group of amino group; R¹⁶ is hydrogen or substituted or unsubstituted alkyl; all other variables are as defined above.

### (1st step)

Amine compound represented by the general formula (Ic) may be prepared by the treatment of a compound (xii) with acid.
Trifluoroacetic acid, hydrochloric acid, etc. may be used in an amount of 1 or more mol equivalent(s) in respect of the compound (xii), or as solvent.
Solvent that may be used includes methanol, ethanol, propanol, isopropanol, butanol, dioxane, ethyl acetate, methylene chloride, chloroform, water and the mixture thereof, etc.
Temperature for such reaction may be 0 °C to 100 °C.
Reaction time may be 0.5 to 24 hours.
The obtained compound represented by the general formula (Ic) may be purified according to conventional method (e.g., column chromatography, recrystallization, etc.).

### (Method D)

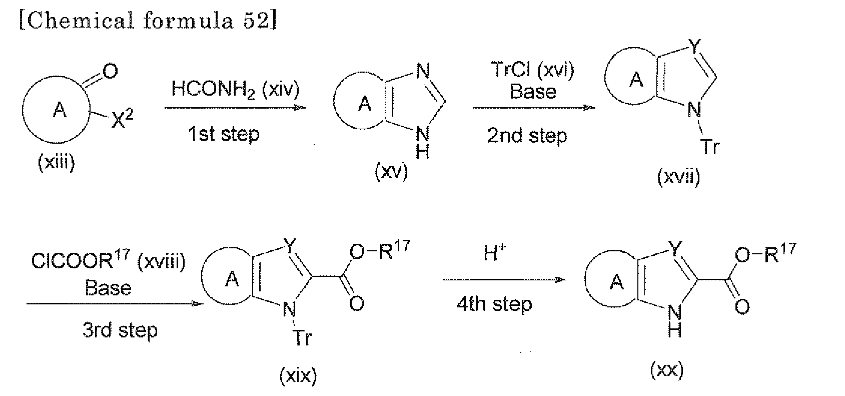

wherein, X² is leaving group such as halogen, mesyloxy group or tosyloxy group, etc.; Tr is trityl; R¹⁷ is substituted or unsubstituted alkyl; all other variables are as defined above.)

### (1st step)

Imidazole (xv) may be prepared by the condensation of a compound (xiii) with formamide (xiv).
Formamide (xiv) may be used in an amount of 5 or more mol equivalents in respect of the compound (xiii), or as solvent.
Temperature for such reaction may be 100 °C to refluxing temperature of the solvent.
Reaction time may be 0.5 to 24 hours.
The obtained imidazole (xv) may be purified according to conventional method (e.g., column chromatography, recrystallization, etc.).

### (2nd step)

A compound (xvii) may be prepared by the reaction of imidazole (xv) with trityl chloride (xvi) in the presence of base.
Trityl chloride (xvi) may be used in an amount of 1 to 5 mol equivalent(s) in respect of imidazole (xv).
Base that may be used includes triethylamine, N,N-diisopropylethylamine, N-methylmorpholine, 4-dimethylaminopyridine, and the mixture thereof, etc.
Solvent that may be used includes methylene chloride, tetrahydrofuran, N,N-dimethylformamide, and the mixture thereof, etc.
Temperature for such reaction may be 0 °C to 60 °C.
Reaction time may be 0.5 to 24 hours.
The obtained compound (xvii) may be purified according to conventional method (e.g., column chromatography, recrystallization, etc.).

### (3rd step)

A compound (xix) may be prepared by the reaction of the compound (xvii) with carbonochloridic acid ester (xviii).
Carbonochloridic acid ester may be used in an amount of 1 to 5 mol equivalent(s) in respect of the compound (xvii).
Base that may be used includes sodium methoxide, pottasium tert-butoxide, n-butyllithium, lithium hexamethyldisilazide, sodium hexamethyldisilazide, potassium hexamethyldisilazide, sodium amide, etc., it may be used in an amount of 1 to 5 mol equivalent(s) in respect of the compound (xvii).
Solvent that may be used includes tetrahydrofuran, diethylether, N,N-dimethylformamide, and the mixture thereof, etc.
Temperature for such reaction may be -78 °C to 50 °C.
Reaction time may be 0.5 to 24 hours.
The obtained compound (xix) may be purified according to conventional method (e.g., column chromatography, recrystallization, etc.).

### (4th step)

A compound (xx) may be prepared by the treatment of the compound (xix) with acid.
Trifluoroacetic acid, hydrochloric acid, etc. may be used in an amount of 1 or more mol equivalent(s) in respect of the compound (xix), or as solvent.
Solvent that may be used includes methanol, ethanol, propanol, isopropanol, butanol, dioxane, ethyl acetate, methylene chloride, chloroform, water, and the mixture thereof, etc.
Temperature for such reaction may be 0 °C to 100 °C.
Reaction time may be 0.5 to 24 hours.
The obtained compound (xx) may be purified according to conventional method (e.g., column chromatography, recrystallization, etc.).

### (Method E)

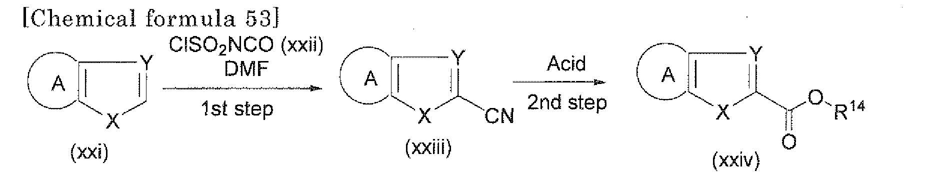

wherein, all variables are as defined above.

### (1st step)

Chlorosulfonyl isocyanate (xxii) is added dropwise to a compound (xxi), then N,N-dimethylformamide is added to the above to afford nitrile compound (xxiii).
Chlorosulfonyl isocyanate (xxii) may be used in an amount of 1 to 5 mol equivalent(s) in respect of the compound (xxi). In addition, N,N-dimethylformamide may be used in an amount of 1 or more mol equivalent(s), or as solvent.
Solvent that may be used includes acetonitrile, etc.
Temperature for such reaction may be -78 °C to 40 °C.
Reaction time may be 0.5 to 24 hours.
The obtained nitrile compound (xxiii) may be purified according to conventional method (e.g., column chromatography, recrystallization, etc.).

### (2nd step)

A compound (xxiv) may be prepared by alcoholysis of nitrile compound (xxiii) in the acidic condition.
Acid such as hydrochloric acid, sulfuric acid, boron trifluoride, etc. may be used in an amount of 1 or more equivalent(s) in respect of nitrile compound (xxiii), or as solvent.
Solvent that may be used includes methanol, ethanol, propanol, isopropanol, butanol, tetrahydrofuran, water, and the mixture thereof, etc.
Temperature for such reaction may be 0 °C to 200 °C.
Reaction time may be 0.5 hours to 7 days.
The obtained compound (xxiv) may be purified according to conventional method (e.g., column chromatography, recrystallization, etc.).

### (Method F)

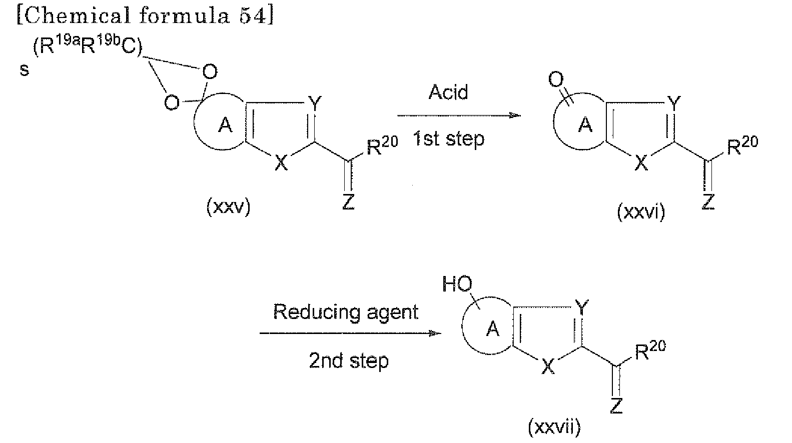

wherein, R^{19a} and R^{19a} are each independently hydrogen, substituted or unsubstituted alkyl; R²⁰ is substituted or unsubstituted alkyloxy or B ring; s is an integer of 1 to 3; all other variables are as defined above.

### (1st step)

A compound (xxv) may be converted to a compound (xxvi) in the acidic condition.
Acid such as hydrochloric acid, sulfuric acid, acetic acid, trifluoroacetic acid, 4-toluenesulfonic acid, etc. may be used in an amount of 1 or more equivalent(s) in respect of the compound (xxv), or as solvent.
Solvent that may be used includes methanol, ethanol, propanol, isopropanol, butanol, methylene chloride, chloroform, tetrahydrofuran, water, and the mixture thereof, etc.
Temperature for such reaction may be 0 °C to refluxing temperature of the solvent.
Reaction time may be 0.5 to 48 hours.
The obtained compound (xxvi) may be purified according to conventional method (e.g., column chromatography, recrystallization, etc.).

### (2nd step)

The compound (xxvi) may be converted to an alcohol (xxvii) by the treatment of reducing agent.
Reducing agent that may be used includes sodium borohydride, lithium borohydride, lithium aluminium hydride, etc., it may be used in an amount of 1 to 10 mol equivalent(s) in respect of the compound (xxvi).
Solvent that may be used includes methanol, ethanol, propanol, isopropanol, butanol, tetrahydrofuran, diethylether, methylene chloride, water, and the mixture thereof, etc.
Temperature for such reaction may be -78 °C to refluxing temperature of the solvent.
Reaction time may be 0.5 to 48 hours.
The obtained alcohol (xxvii) may be purified according to conventional method (e.g., column chromatography, recrystallization, etc.).

### (Method G)

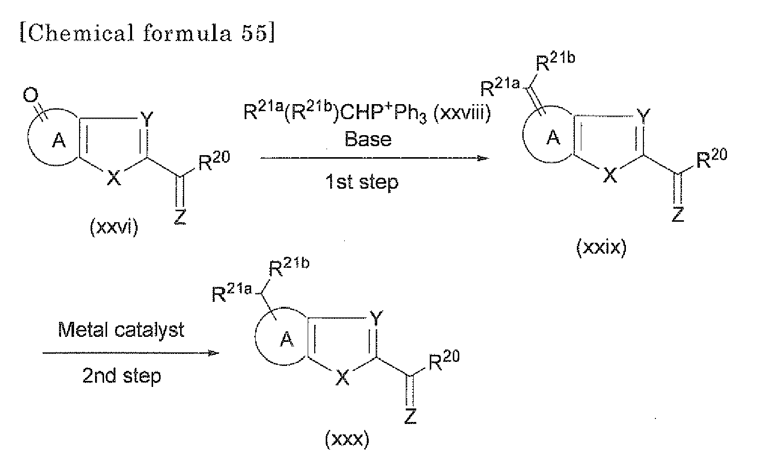

wherein, R^{21a} and R^{21b} are each independently hydrogen, substituted or unsubstituted alkyl, substituted or unsubstituted alkenyl, substituted or unsubstituted alkynyl, acyl, substituted or unsubstituted cycloalkyl, substituted or unsubstituted cycloalkenyl, substituted or unsubstituted alkyl non-aromatic heterocyclic group, substituted or unsubstituted aryl, or substituted or unsubstituted heteroaryl; all other variables are as defined above.

### (1st step)

A compound (xxix) may be prepared by the condensation of the compound (xxvi) with an organophosphorus compound (xxviii) in the basic condition.
An organophosphorus compound (xxviii) may be used in an amount of 1 to 5 mol equivalent(s) in respect of the compound (xxvi).
Solvent that may be used includes tetrahydrofuran, diethylether, toluene, dimethylsulfoxide, N,N'dimethylformamide, and the mixture thereof, etc.
Base that may be used includes lithium hydroxide, sodium hydroxide, potassium hydroxide, sodium hydride, potassium hydride, sodium methoxide, pottasium tert-butoxide, n-butyllithium, lithium hexamethyldisilazide, sodium hexamethyldisilazide, potassium hexamethyldisilazide, sodium amide, etc., it may be used in an amount of 1 to 5 mol equivalent(s) in respect of the compound (xxvi).
Temperature for such reaction may be -78 °C to refluxing temperature of the solvent.
Reaction time may be 0.5 to 24 hours.
The obtained compound (xxix) may be purified according to conventional method (e.g., column chromatography, recrystallization, etc.).

### (2nd step)

A compound (xxx) may be prepared by the hydrogenation of the compound (xxix) in the presence of metal catalyst.
Solvent that may be used includes methanol, ethanol, propanol, isopropanol, butanol, tetrahydrofuran, diethyl ether, toluene, ethyl acetate, and the mixture thereof, etc.
Metal catalyst that may be used includes palladium-carbon, platinum oxide, chlorotris(triphenylphosphine)rhodium(I), etc., it may be used in an amount of 0.01 to 0.5 weight percent in respect of the compound (xxix).
Hydrogen pressure for such reaction may be at 1 to 50 atm(s).
Temperature for such reaction may be 0 °C to refluxing temperature of the solvent.
Reaction time may be 0.5 to 72 hours.
The obtained compound (xxx) may be purified according to conventional method (e.g., column chromatography, recrystallization, etc.).

### (Method H)

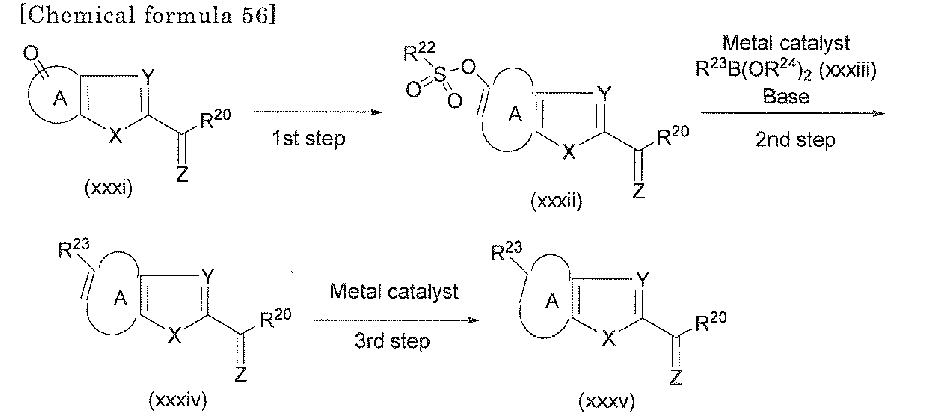

wherein, R²² is substituted or unsubstituted alkyl or substituted or unsubstituted aryl; R²³ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted alkyl non-aromatic heterocyclic group, substituted or unsubstituted aryl, or substituted or unsubstituted heteroaryl; two R²⁴ are each independently hydrogen, or two R²⁴ are e taken together -(CR^{25a} R^{25b})t-, t is an integer of 1 to 3; all other variables are as defined above.

### (1st step)

A compound (xxxii) may be prepared by the condensation of a compound (xxxi) with sulfonylation agent in the presence of base.
Solvent that may be used includes tetrahydrofuran, diethylether, toluene, N,N-dimethylformamide, methylene chloride, and the mixture thereof, etc.
Base that may be used includes lithium hydroxide, sodium hydroxide, potassium hydroxide, sodium hydride, pottasium tert-butoxide, n-butyllithium, lithium hexamethyldisilazide, sodium hexamethyldisilazide, potassium hexamethyldisilazide, sodium amide, etc., it may be used in an amount of 1 to 5 mol equivalent(s) in respect of the compound (xxxi).
Sulfonylation agent that may be used includes 4-toluenesulfonyl chloride, 4-toluenesulfonyl anhydride, methanesulfonyl chloride, methanesulfonyl anhydride, trifluoromethanesulfonyl chloride, trifluoromethanesulfonyl anhydride, N-phenyltrifluoromethanesulfonimide, etc., it may be used in an amount of 1 to 5 mol equivalent(s) in respect of the compound (xxxi).
Temperature for such reaction may be -78 °C to 50 °C.
Reaction time may be 0.5 to 48 hours.
The obtained compound (xxxii) may be purified according to conventional method (e.g., column chromatography, recrystallization, etc.).

### (2nd step)

A compound (xxxiv) may be prepared by the reaction of the compound (xxxii) with boronic acid or boronic acid ester (xxxiii) in the presence of base.
Solvent that may be used includes tetrahydrofuran, toluene, N,N-dimethylformamide, dioxane, water, and the mixture thereof, etc.
Metal catalyst that may be used includes palladium acetate, bis(dibenzylideneacetone)palladium, tetrakis(triphenylphosphine) palladium, bis(triphenylphosphine)palladium(II) chloride, bis(tri-tert-butylphosphine)palladium, etc., it may be used in an amount of 0.001 to 0.5 mol equivalents in respect of the compound (xxxii).
Base that may be used includes lithium hydroxide, sodium hydroxide, potassium hydroxide, pottasium tert-butoxide, sodium tert-butoxide, sodium carbonate, potassium carbonate, sodium hydrogen carbonate, sodium phosphate, sodium hydrogenphosphate, potassium phosphate, potassium hydrogenphosphate, etc., it may be used in an amount of 1 to 10 mol equivalent(s) in respect of the compound (xxxii).
Boronic acid or boronic acid ester (xxxiii) may be used in an amount of 1 to 10 mol equivalent(s) in respect of the compound (xxxii).
Temperature for such reaction may be 20 °C to refluxing temperature of the solvent.
Reaction time may be 0.5 to 48 hours.
The obtained compound (xxxiv) may be purified according to conventional method (e.g., column chromatography, recrystallization, etc.).

### (3rd step)

A compound (xxxv) may be prepared by the hydrogenation of the compound (xxxiv) in the presence of metal catalyst.
Solvent that may be used includes methanol, ethanol, propanol, isopropanol, butanol, tetrahydrofuran, diethyl ether, toluene, ethyl acetate, and the mixture thereof, etc.
Metal catalyst that may be used includes palladium-carbon, platinum oxide, chlorotris(triphenylphosphine)rhodium(I), etc., it may be used in an amount of 0.01 to 0.5 weight percent in respect of the compound (xxxiv).
Hydrogen pressure for such reaction may be at 1 to 50 atm(s).
Temperature for such reaction may be 0 °C to refluxing temperature of the solvent. Reaction time may be 0.5 to 72 hours.

The obtained compound (xxxv) may be purified according to conventional method (e.g., column chromatography, recrystallization, etc.).

### (Method I)

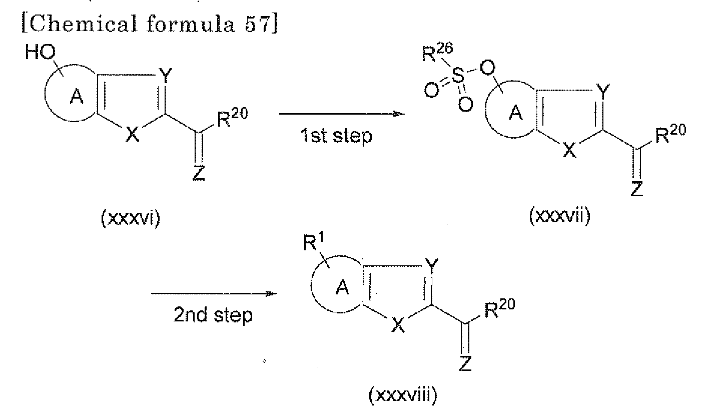

wherein, R²⁶ is substituted or unsubstituted alkyl or substituted or unsubstituted aryl; R¹ is halogen, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylthio, substituted or unsubstituted alkenylthio, substituted or unsubstituted alkynylthio, acyl, hydroxy, formyl, carboxy, substituted or unsubstituted alkyloxycarbonyl, substituted or unsubstituted carbamoyl, substituted or unsubstituted sulfamoyl, substituted or unsubstituted alkyloxysulfonyl, substituted or unsubstituted aryloxysulfonyl, substituted or unsubstituted alkylsulfinyl, substituted or unsubstituted arylsulfinyl, cyano, nitro, substituted or unsubstituted amino, substituted or unsubstituted cycloalkyl, substituted or unsubstituted cycloalkenyl, substituted or unsubstituted non-aromatic heterocyclic group, substituted or unsubstituted aryl, substituted or unsubstituted heteroaryl, substituted or unsubstituted cycloalkyloxy, substituted or unsubstituted cycloalkenyloxy, substituted or unsubstituted non-aromatic heterocyclic ring-oxy, substituted or unsubstituted aryloxy, substituted or unsubstituted heteroaryloxy; all other variables are as defined above.

### (1st step)

A compound (xxxvii) may be prepared by the condensation of a compound (xxxvi) with sulfonylation agent in the presence of base.
Solvent that may be used includes tetrahydrofuran, N,N-dimethylformamide, methylene chloride, chloroform, pyridine, and the mixture thereof, etc.
Base that may be used includes lithium hydroxide, sodium hydroxide, potassium hydroxide, sodium hydride, potassium hydride, pottasium tert-butoxide, n-butyllithium, lithium hexamethyldisilazide, pyridine, triethylamine, N,N-diisopropylethylamine, N-methylmorpholine, 4-dimethylaminopyridine, etc., it may be used in an amount of 1 to 5 mol equivalent(s) in respect of the compound (xxxvi).
Sulfonylation agent that may be used includes 4-toluenesulfonyl chloride, 4-toluenesulfonyl anhydride, methanesulfonyl chloride, methanesulfonyl anhydride, trifluoromethanesulfonyl chloride, trifluoromethanesulfonyl anhydride, N-phenyltrifluoromethanesulfonimide, etc., it may be used in an amount of 1 to 5 mol equivalent(s) in respect of the compound (xxxvi).
Temperature for such reaction may be -78 °C to 50 °C.
Reaction time may be 0.5 to 48 hours.
The obtained compound (xxxvii) may be purified according to conventional method (e.g., column chromatography, recrystallization, etc.).

### (2nd step)

A compound (xxxviii) may be prepared by the condensation of the compound (xxxvii) with nucleophile.
Solvent that may be used includes tetrahydrofuran, N,N-dimethylformamide, methylene chloride, chloroform, pyridine, methanol, ethanol, N-methylpyrrolidinone, dimethylsulfoxide, and the mixture thereof, etc.
Nucleophile that may be used includes sodium methoxide, sodium ethoxide, lithium chloride, pyridinium chloride, sodium chloride, potassium chloride, lithium bromide, sodium bromide, potassium bromide, pyridinium bromide, lithium iodide, sodium iodide, potassium iodide, pyridinium iodide, etc., it may be used in an amount of 1 to 10 mol equivalent(s) in respect of the compound (xxxvii).
Temperature for such reaction may be 0 °C to refluxing temperature of the solvent.
Reaction time may be 0.5 to 72 hours.
The obtained compound (xxxviii) may be purified according to conventional method (e.g., column chromatography, recrystallization, etc.).

### (Method J)

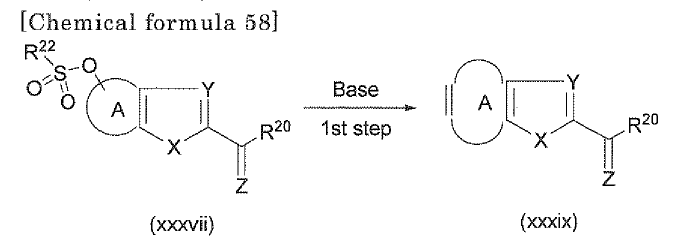

wherein, all variables are as defined above.

### (1st step)

A compound (xxxix) may be prepared by the elimination reaction of a compound (xxxvii) in the presence of the base.
Solvent that may be used includes tetrahydrofuran, N,N-dimethylformamide, methylene chloride, chloroform, methanol, ethanol, N-methylpyrrolidinone, dimethylsulfoxide, and the mixture thereof, etc.
Base that may be used includes pyridine, pottasium tert-butoxide, sodium tert-butoxide, etc., and it may be used in an amount of 1 to 10 mol equivalent(s) in respect of the compound (xxxvii).
Temperature for such reaction may be 0 °C to refluxing temperature of the solvent.
Reaction time may be 0.5 to 48 hours.
The obtained compound (xxxix) may be purified according to conventional method (e.g., column chromatography, recrystallization, etc.).

### (Method K)

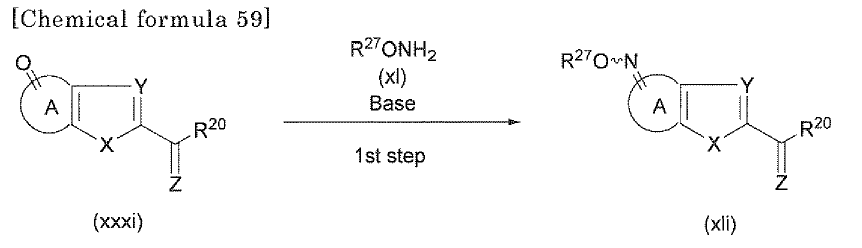

wherein, R²⁷ is substituted or unsubstituted alkyl, substituted or unsubstituted alkenyl, substituted or unsubstituted alkynyl, acyl, substituted or unsubstituted cycloalkyl, substituted or unsubstituted cycloalkenyl, substituted or unsubstituted alkyl non-aromatic heterocyclic group, substituted or unsubstituted aryl, or substituted or unsubstituted heteroaryl; all other variables are as defined above.

### (1st step)

A compound (xli) may be prepared by the condensation of a compound (xxxi) with hydroxylamine (xl) or its salt thereof in the presence of the base.
Solvent that may be used includes tetrahydrofuran, diethylether, toluene, N,N-dimethylformamide, methylene chloride, methanol, ethanol, N-methylpyrrolidinone, dimethylsulfoxide, and the mixture thereof, etc.
Base that may be used includes pyridine, triethylamine, N,N-diisopropylethylamine, N-methylmorpholine, 4-dimethylaminopyridine, etc., it may be used in an amount of 1 to 10 mol equivalent(s) in respect of the compound (xxxi).
Hydroxylamine (xl) or its salt thereof may be used in an amount of 1 to 5 mol equivalent(s) in the respect of the compound (xxxi).
Temperature for such reaction may be 0 °C to refluxing temperature of the solvent.
Reaction time may be 0.5 to 72 hours.
The obtained compound (xli) may be purified according to conventional method (e.g., column chromatography, recrystallization, etc.).

### (Method L)

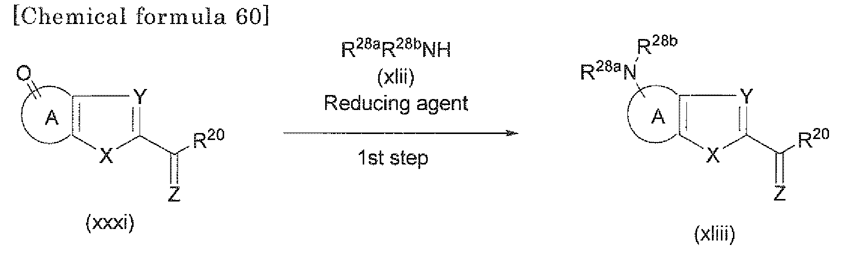

wherein, R^{28a} and R^{28b} are each independently hydrogen, substituted or unsubstituted alkyl, substituted or unsubstituted alkenyl, substituted or unsubstituted alkynyl, acyl, substituted or unsubstituted cycloalkyl, substituted or unsubstituted cycloalkenyl, substituted or unsubstituted alkyl non-aromatic heterocyclic group, substituted or unsubstituted aryl, or substituted or unsubstituted heteroaryl; all other variables are as defined above.

### (1st step)

A compound (xliii) was prepared by the condensation of a compound (xxxi) with amine (xlii) or its salt thereof in the presence of the condensing agent or without condensing agent, followed by the reduction by reducing agent.
Solvent that may be used includes tetrahydrofuran, toluene, methylene chloride, chloroform, methanol, ethanol, and the mixture thereof, etc.
Condensing agent that may be used includes acetic acid, 4-toluenesulfonic acid, methanesulfonic acid, anhydrous magnesium sulfate, tetraisopropyl orthotitanate, titanium tetrachloride, molecular sieve, etc., it may be used in an amount of 1 to 10 mol equivalent(s) in respect of the compound (xxxi).
Amine or its salt thereof may be used in an amount of 1 to 10 mol equivalent(s) in respect of the compound (xxxi).
Reducing agent that may be used includes sodium borohydride, sodium cyanoborohydride, sodium triacetoxyborohydride, borane and its complex thereof, lithium borohydride, potassium borohydride, diisobutylaluminium hydride, etc., it may be used in an amount of 1 to 10 mol equivalent(s) in respect of the compound (xxxi).
Temperature for such reaction may be -78 °C to refluxing temperature of the solvent.
Reaction time may be 0.5 to 48 hours.
The obtained compound (xliii) may be purified according to conventional method (e.g., column chromatography, recrystallization, etc.).

### (Method M)

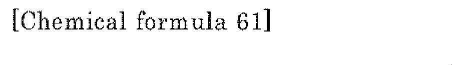

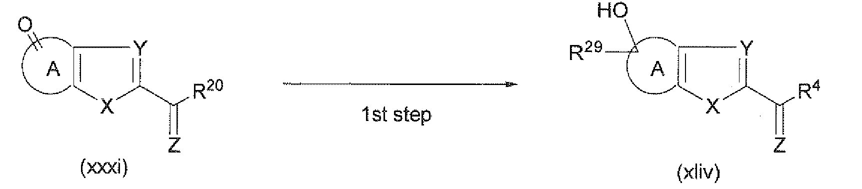

wherein, R^{28a} and R^{28b} are each independently hydrogen, substituted or unsubstituted alkyl, substituted or unsubstituted alkenyl, substituted or unsubstituted alkynyl, acyl, substituted or unsubstituted cycloalkyl, substituted or unsubstituted cycloalkenyl, substituted or unsubstituted alkyl non-aromatic heterocyclic group, substituted or unsubstituted aryl, or substituted or unsubstituted heteroaryl; all other variables are as defined above.

### (1st step)

A compound (xliv) may be prepared from the compound (xxxi) in the presence of the nucleophile.
Solvent that may be used includes tetrahydrofuran, hexane, diethylether, methyl tert-butyl ether, and the mixture thereof, etc.
Nucleophile that may be used includes lithium reagent such as methyllithium, ethyllithium, etc., Grignard reagent such as methylmagnesium bromide, methylmagnesium chloride, methylmagnesium iodide, ethylmagnesium bromide, ethylmagunesium chloride, ethylmagnesium iodide, etc., and its mixed reagent of metallic salt thereof, it may be used in an amount of 1 to 5 mol equivalent(s) in respect of the compound (xxxi).
Temperature for such reaction may be -78 °C to refluxing temperature of the solvent.
Reaction time may be 0.5 to 24 hours.
The obtained compound (xliv) may be purified according to conventional method (e.g., column chromatography, recrystallization, etc.).

### (Method N)

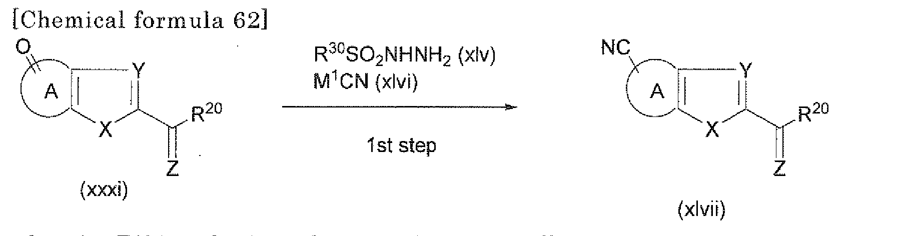

wherein, R³⁰ is substituted or unsubstituted alkyl, substituted or unsubstituted aryl or substituted or unsubstituted heteroaryl; all other variables are as defined above.

### (1st step)

A compound (xlvii) may be prepared by the condensation of the compound (xxxi) with the reagent (xlv), followed by reaction with a cyanide salt (xlvi).
Solvent that may be used includes methanol, ethanol, propanol, isopropanol, butanol, tetrahydrofuran, diethyl ether, toluene, ethyl acetate, and the mixture thereof, etc.
Reacent (xlv) may be used in an amount of 1 to 5 mol equivalent(s) in respect of the compound (xxxi).
Cyanide salt (xlvi) that may be used includes potassium cyanide, sodium cyanide, tetrabutylammonium cyanide, etc. it may be used in an amount of 1 to 5 mol equivalent(s) in respect of the compound (xxxi).
Temperature for such reaction may be 0 °C to refluxing temperature of the solvent.
Reaction time may be 0.5 to 48 hours.
The obtained compound (xlvii) may be purified according to conventional method (e.g., column chromatography, recrystallization, etc.).

### (Method O)

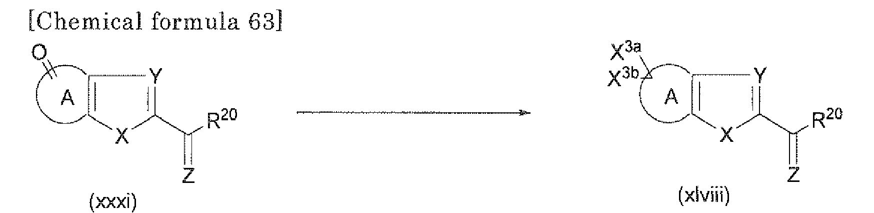

wherein, X^{3a} and X^{3b} are halogen; all other variables are as defined above.
A compound (xlviii) may be prepared by the reaction of the compound (xxxi) with halogenation reagent.
Solvent that may be used includes methylene chloride, chloroform, toluene, and the mixture thereof, etc.
Halogenation reagent that may be used includes diethylaminosulfur trifluoride, morpholinosulfur trifluoride, bis(2-methoxyethyl)aminosulfur trifluoride, 2,2-difluoro-1,3-dimethylimidazolidine, phosphorous pentachloride, phosphorous trichloride, tungsten hexachloride etc., it may be used in an amount of 1 to 10 mol equivalent(s) in respect of the compound (xxxi).
Temperature for such reaction may be -78 °C to refluxing temperature of the solvent.
Reaction time may be 0.5 to 48 hours.
The obtained compound (xlviii) may be purified according to conventional method (e.g., column chromatography, recrystallization, etc.).

### (Method P)

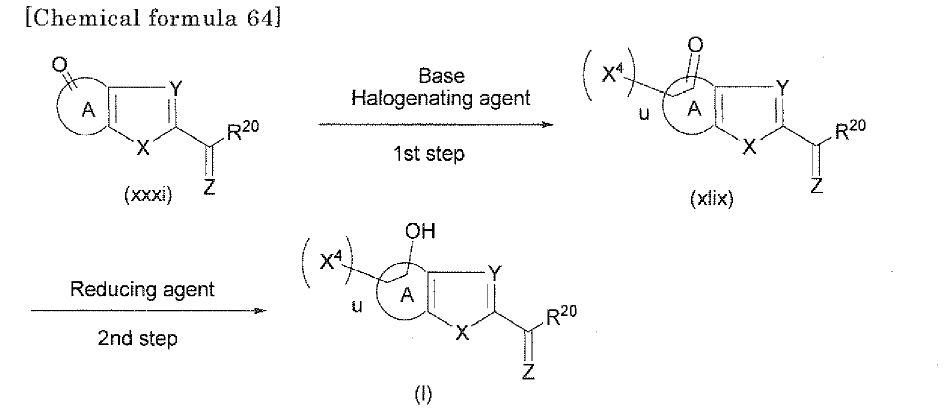

wherein, X⁴ is halogen; u is an integer of 1 to 3; all other variables are as defined above.

### (1st step)

A compound (xlix) may be prepared by the reaction of the compound (xxxi) with halogenation reagent.
Base that may be used includes lithium diisopropylamide, lithium hexamethyldisilazide, sodium hexamethyldisilazide, potassium hexamethyldisilazide, sodium amide, etc., it may be used in an amount of 1 to 5 mol equivalent(s) in respect of the compound (xxxi).
Halogenation reagent that may be used includes diethylaminosulfur trifluoride, morpholinosulfur trifluoride, bis(2-methoxyethyl)aminosulfur trifluoride, 2,2-difluoro-1,3-dimethylimidazolidine, phosphorous pentachloride, phosphorous trichloride, tungsten hexachloride etc., it may be used in an amount of 1 to 10 mol equivalent(s) in respect of the compound (xxxi).
Solvent that may be used includes tetrahydrofuran, diethylether, N,N-dimethylformamide, and the mixture thereof, etc.
Temperature for such reaction may be -78 °C to 50 °C.
Reaction time may be 0.5 to 24 hours.
The obtained compound (xlix) may be purified according to conventional method (e.g., column chromatography, recrystallization, etc.).

### (2nd step)

The compound (xlix) may be converted to an alcohol compound (1) by reducing agent.
Reducing agent that may be used includes sodium borohydride, lithium borohydride, lithium aluminium hydride, etc., it may be used in an amount of 1 to 10 mol equivalent(s) in respect of the compound (xlix).
Solvent that may be used includes methanol, ethanol, propanol, isopropanol, butanol, tetrahydrofuran, diethylether, methylene chloride, water, and the mixture thereof, etc.
Temperature for such reaction may be -78 °C to refluxing temperature of the solvent.
Reaction time may be 0.5 to 48 hours.
The obtained compound (l) may be purified according to conventional method (e.g., column chromatography, recrystallization, etc.).

### (Method Q)

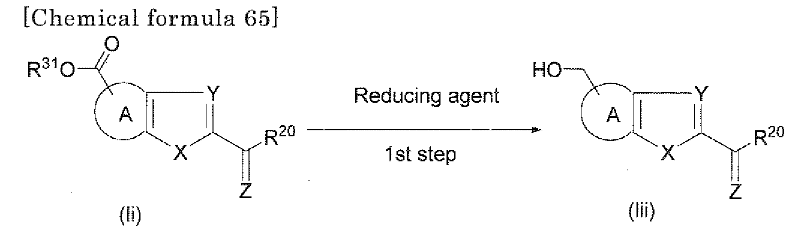

wherein, R³¹ is substituted or unsubstituted alkyl; all other variables are as defined above.
A compound (li) may be converted to an alcohol compound (lii) by reducing agent.
Reducing agent that may be used includes lithium borohydride, sodium borohydride, diisopropylaluminium hydride, sodium bis(2-methoxyethoxy)aluminium hydride, etc., it may be used in an amount of 1 to 10 mol equivalent(s) in respect of the compound (li).
Solvent that may be used includes methanol, ethanol, propanol, isopropanol, butanol, tetrahydrofuran, diethylether, methylene chloride, water and the mixture thereof, etc.
Temperature for such reaction may be -78 °C to refluxing temperature of the solvent.
Reaction time may be 0.5 to 48 hours.
The obtained alcohol compound (lii) may be purified according to conventional method (e.g., column chromatography, recrystallization, etc.).

### (Method R)

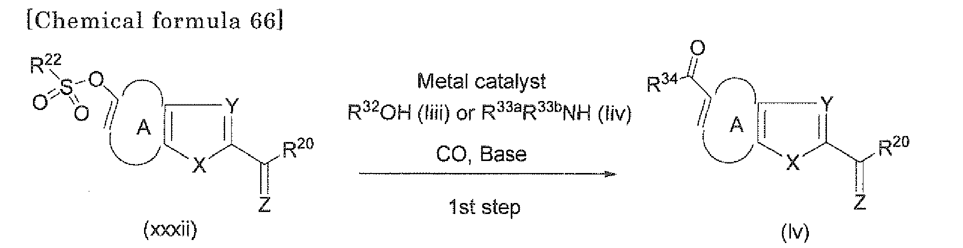

wherein, R³², R^{33a} and R^{33b} are each independently hydrogen, substituted or unsubstituted alkyl, substituted or unsubstituted alkenyl, substituted or unsubstituted alkynyl, acyl, substituted or unsubstituted cycloalkyl, substituted or unsubstituted cycloalkenyl, substituted or unsubstituted alkyl non-aromatic heterocyclic group, substituted or unsubstituted aryl, or substituted or unsubstituted heteroaryl; R³⁴ is hydroxy, substituted or unsubstituted alkyloxy or substituted or unsubstituted amino; all other variables are as defined above.
A compound (lv) may be prepared by the reaction of the compound (xxxii) with alcohol (liii) or amine (liv) by using metal catalyst in the presence of the base under carbon monoxide atmosphere.
Solvent that may be used includes tetrahydrofuran, toluene, N,N-dimethylformamide, dioxane, water, methanol, ethanol, propanol, isopropanol, butanol, and the mixture thereof, etc.
Metal catalyst that may be used includes palladium acetate, tetrakis(triphenylphosphine) palladium, bis(triphenylphosphine)palladium(II) chloride, bis(tri-tert-butylphosphine)palladium, 1,1'-bis(diphenylphosphino)ferrocenepalladium(II) chloride, etc., it may be used in an amount of 0.001 to 0.5 mol equivalents in respect of the compound (xxxii).
Base that may be used includes triethylamine, N,N-diisopropylethylamine, N-methylmorpholine, pottasium tert-butoxide, sodium tert-butoxide, sodium carbonate, potassium carbonate, sodium hydrogen carbonate, sodium phosphate, sodium hydrogenphosphate, potassium phosphate, potassium hydrogenphosphate, etc., it may be used in an amount of 1 to 10 mol equivalent(s) in respect of the compound (xxxii).
Alcohol or amine may be used in an amount of 1 to 5 mol equivalent(s) in respect of the compound (xxxii).
Temperature for such reaction may be 0 °C to refluxing temperature of the solvent.
Reaction time may be 0.5 to 72 hours.
The obtained compound (lv) may be purified according to conventional method (e.g., column chromatography, recrystallization, etc.).

### (Method S)

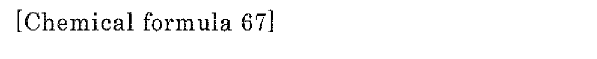

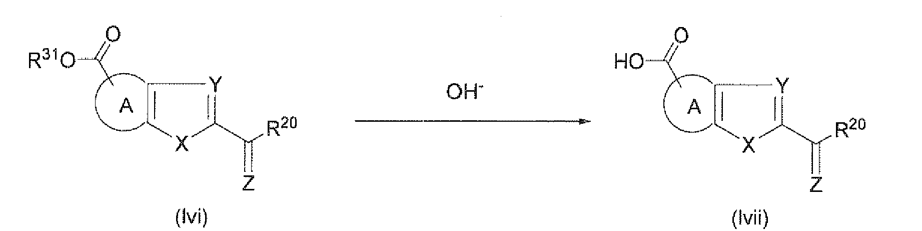

wherein, all variables in the formula are as defined above.
Carboxylic acid (lvii) may be prepared by the hydrolysis of the compound (lvi).
Lithium hydroxide, sodium hydroxide, potassium hydroxide, etc. may be used in an amount of 1 to 5 mol equivalent(s) in respect of the compound (lvi).
Solvent that may be used includes methanol, ethanol, propanol, isopropanol, butanol, tetrahydrofuran, water, and the mixture thereof, etc.
Temperature for such reaction may be 0 °C to refluxing temperature of the solvent.
Reaction time may be 0.5 to 24 hours.
The obtained compound (lvii) may be purified according to conventional method (e.g., column chromatography, recrystallization, etc.).

### (Method T)

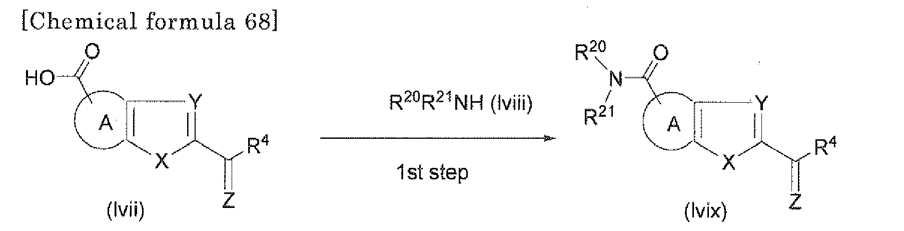

wherein, all variables in the formula are as defined above.
An amide compound (lvix) may be prepared by the condensation of carboxylic acid (lvii) with amine (lviii) in the presence of the condensing agent.
Amine (lviii) may be used in an amount of 0.5 to 4 mol equivalent(s) in respect of carboxylic acid (lvii).
Condensing agent that may be used includes dicyclohexylcarbodiimide, 1-(3-dimethylaminopropyl)-3-ethylcarbodiimide hydrochloride, N, N'-carbonyldiimidazole, HBTU, HATU, ethyl chlorocarbonate, isobutyl chlorocarbonate, thionyl chloride, oxalyl chloride, etc., it may be used in an amount of 0.5 to 2 mol equa.valent(s) in respect of carboxylic acid (lvii). 1-Hydroxybenzotriazole may be used in an amount of 0.5 to 2 mol equivalent(s) as supplemental condensing agent.
Base that may be used includes triethylamine, N,N-diisopropylethylamine, N-methylmorpholine, 4-dimethylaminopyridine, and the mixture thereof, etc. Base may be used in an amount of 0.05 to 4 mol equivalent(s) in respect of carboxylic acid (lvii).
Solvent that may be used includes methylene chloride, tetrahydrofuran, N,N-dimethylformamide, and the mixture thereof, etc.
Temperature for such reaction may be 0 °C to refluxing temperature of the solvent.
Reaction time may be 0.5 to 72 hours.
The obtained compound (lvix) may be purified according to conventional method (e.g., column chromatography, recrystallization, etc.).

### (Method U)

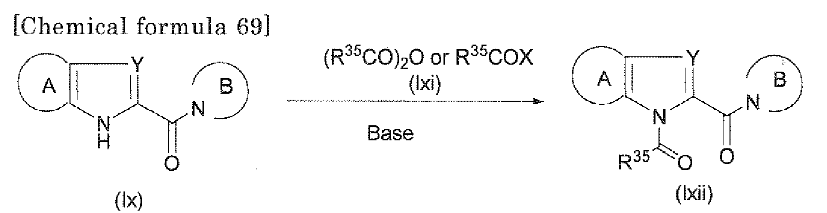

wherein, R³⁵ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted alkyl non-aromatic heterocyclic group, substituted or unsubstituted aryl, or substituted or unsubstituted heteroaryl; all other variables are as defined above.
An amide compound represented by the general formula (lxii) may be prepared by the condensation of the compound (lx) with acid anhydride or acid halide (lxi) in the presence of the base.
Acid anhydride or acid halide (lxi) may be used in an amount of 1 to 10 mol equivalent(s) in respect of the compound (lx).
Base that may be used includes lithium hydroxide, sodium hydroxide, potassium hydroxide, sodium hydride, potassium hydride, sodium methoxide, pottasium tert-butoxide, n-butyllithium, lithium hexamethyldisilazide, sodium hexamethyldisilazide, potassium hexamethyldisilazide, sodium amide, potassium carbonate, sodium carbonate, cesium carbonate, etc., it may be used in an amount of 1 to 10 mol equivalent(s) in respect of the compound represented by the general formula (lx).
Solvent that may be used includes tetrahydrofuran, diethylether, toluene, N,N-dimethylformamide, dichloromethane, 1,2-dichloroethane, chloroform, and the mixture thereof, etc.
Temperature for such reaction may be 0 °C to refluxing temperature of the solvent.
Reaction time may be 0.5 to 24 hours.
The obtained compound represented by the general formula (lxii) may be purified according to conventional method (e.g., column chromatography, recrystallization, etc.).

### (Method V)

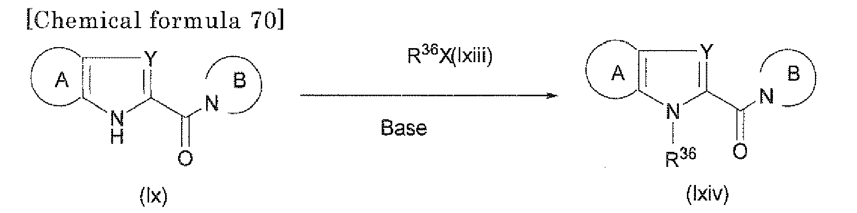

wherein, R³⁶ is substituted or unsubstituted alkyl, substituted or unsubstituted cycloalkyl, substituted or unsubstituted alkyl non-aromatic heterocyclic group; all other variables are as defined above.
A compound represented by the general formula (lxiv) may be prepared by the condensation of the compound (lx) with alkyl halide (lxiii) in the presence of the base.
Alkyl halide (lxiii) may be used in an amount of 1 to 10 mol equivalent(s) in respect of the compound (lx).
Base that may be used includes lithium hydroxide, sodium hydroxide, potassium hydroxide, sodium hydride, potassium hydride, sodium methoxide, pottasium tert-butoxide, n-butyllithium, lithium hexamethyldisilazide, sodium hexamethyldisilazide, potassium hexamethyldisilazide, sodium amide, potassium carbonate, sodium carbonate, cesium carbonate, etc., it may be used in an amount of 1 to 10 mol equivalent(s) in respect of the compound represented by the general formula (lx).
Solvent that may be used includes tetrahydrofuran, diethylether, toluene, N,N-dimethylformamide, dichloromethane, 1,2-dichloroethane, chloroform, and the mixture thereof, etc.
Temperature for such reaction may be 0 °C to refluxing temperature of the solvent.
Reaction time may be 0.5 to 24 hours.
The obtained compound represented by the general formula (lxiv) may be purified according to conventional method (e.g., column chromatography, recrystallization, etc.).

### (Method W)

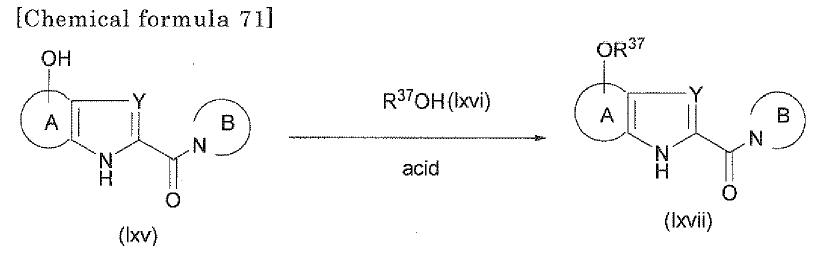

wherein, R³⁷ is substituted or unsubstituted alkyl, substituted or unsubstituted cycloalkyl, substituted or unsubstituted alkyl non-aromatic heterocyclic group, substituted or unsubstituted aryl, or substituted or unsubstituted heteroaryl; all other variables are as defined above.
A compound represented by the general formula (lxvii) may be prepared by the reaction of the compound (lxv) with alcohol (lxvi) in the acidic condition.
Alcohol (lxvi) may be used in an amount of 1 or more equivalent(s) in respect of the compound (lxv), or as solvent.
Acid that may be used includes hydrochloric acid, sulfuric acid, boron trifluoride, titanium tetrachloride, tin tetrachloride, tetraisopropyloxy titanium, 4-toluenesulfonic acid, pyridinium 4-toluenesulfonate, methanesulfonic acid, trifluoroacetic acid, etc., it may be used in an amount of 0.1 to 10 mol equivalent(s) in respect of the compound represented by the general formula (lxv).
Solvent that may be used includes tetrahydrofuran, diethylether, toluene, N,N-dimethylformamide, dichloromethane, 1,2-dichloroethane, chloroform, methanol, ethanol, propanol, isopropanol, butanol, and the mixture thereof, etc.
Temperature for such reaction may be 0 °C to refluxing temperature of the solvent.
Reaction time may be 0.5 to 24 hours.
The obtained compound represented by the general formula (lxvii) may be purified according to conventional method (e.g., column chromatography, recrystallization, etc.).

### (Method X)

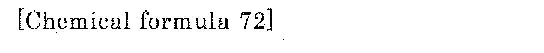

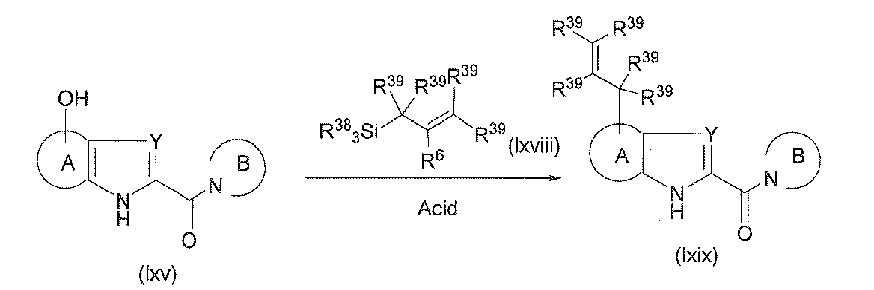

wherein, R³⁸ is substituted or unsubstituted alkyl, substituted or unsubstituted cycloalkyl, substituted or unsubstituted aryl, R³⁹ are each independently hydrogen, substituted or unsubstituted alkyl, substituted or unsubstituted aryl, or substituted or unsubstituted heteroaryl; all other variables are as defined above.
A compound represented by the general formula (lxix) may be prepared by the reaction of the compound (lxv) with allylsilane (lxviii) in the acidic condition.
Allylsilane (lxviii) may be used in an amount of 1 to 10 mol equivalent(s) in respect of the compound (lxv).
Acid that may be used includes hydrochloric acid, sulfuric acid, boron trifluoride, titanium tetrachloride, tin tetrachloride, tetraisopropyloxy titanium, 4-toluenesulfonic acid, pyridinium 4-toluenesulfonate, methanesulfonic acid, trifluoroacetic acid, etc., it may be used in an amount of 0.1 to 10 mol equivalent(s) in respect of the compound represented by the general formula (lxv).
Solvent that may be used includes tetrahydrofuran, diethylether, toluene, N,N-dimethylformamide, dichloromethane, 1,2-dichloroethane, chloroform, and the mixture thereof, etc.
Temperature for such reaction may be 0 °C to refluxing temperature of the solvent.
Reaction time may be 0.5 to 24 hours.
The obtained compound represented by the general formula (lxix) may be purified according to conventional method (e.g., column chromatography, recrystallization, etc.).

### (Method Y)

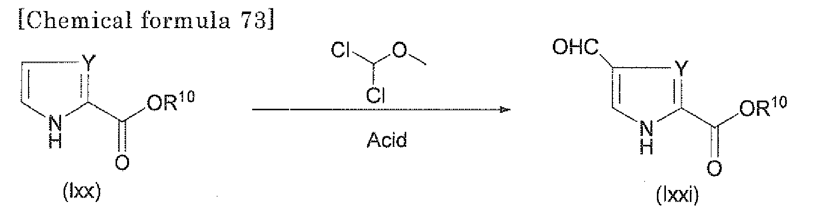

wherein, all variables in the formula are as defined above.
A compound represented by the general formula (lxxi) may be prepared by the reaction of the compound (lxx) with dichloromethoxymethane in the acidic condition.
Dichloromethoxymethane may be used in an amount of 1 to 5 mol equivalent(s) in respect of the compound (lxx).
Acid that may be used includes boron trifluoride, titanium tetrachloride, tin tetrachloride, tetraisopropyloxy titanium, aluminum chloride, etc., it may be used in an amount of 1 to 5 mol equivalent(s) in respect of the compound represented by the general formula (lxx).
Solvent that may be used includes nitromethane, toluene, dichloromethane, 1,2-dichloroethane, chloroform, and the mixture thereof, etc.
Temperature for such reaction may be -78 °C to refluxing temperature of the solvent.
Reaction time may be 0.5 to 24 hours.
The obtained compound represented by the general formula (lxxi) may be purified according to conventional method (e.g., column chromatography, recrystallization, etc.).

### (Method Z)

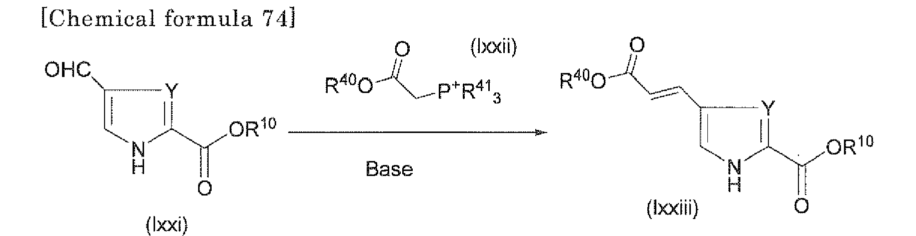

wherein, R⁴⁰ is substituted or unsubstituted benzyl, substituted or unsubstituted cycloalkyl, substituted or unsubstituted aryl, R⁴¹ is substituted or unsubstituted aryl; all other variables are as defined above.
A compound represented by the general formula (lxxiii) may be prepared by the reaction of the compound (lxxi) with phosphonium salt (lxxii) in the presence of the base.
Phosphonium salt (lxxii) may be used in an amount of 1 to 5 mol equivalent(s) in respect of the compound (lxxi).
Base that may be used includes lithium hydroxide, sodium hydroxide, potassium hydroxide, sodium hydride, potassium hydride, sodium methoxide, pottasium tert-butoxide, n-butyllithium, lithium hexamethyldisilazide, sodium hexamethyldisilazide, potassium hexamethyldisilazide, sodium amide, potassium carbonate, sodium carbonate, cesium carbonate, etc., it may be used in an amount of 1 to 10 mol equivalent(s) in respect of the compound represented by the general formula (lxxi).
Solvent that may be used includes tetrahydrofuran, diethylether, toluene, N,N-dimethylformamide, and the mixture thereof, etc.
Temperature for such reaction may be -78 °C to refluxing temperature of the solvent.
Reaction time may be 0.5 to 24 hours.
The obtained compound represented by the general formula (lxxiii) may be purified according to conventional method (e.g., column chromatography, recrystallization, etc.).

### (Method AA)

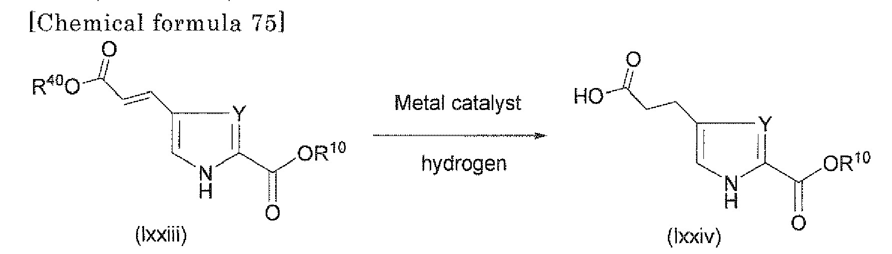

wherein, all variables in the formula are as defined above.
A compound represented by the general formula (lxxiv) may be prepared by the reaction of the compound (lxxiii) with hydrogen in the presence of the metal catalyst.
Metal catalyst that may be used includes palladium-carbon, palladium hydroxide, platinum oxide, chlorotris(triphenylphosphine)rhodium(I), etc., it may be used in an amount of 0.01 to 0.5 weight percent in respect of the compound represented by the general formula (lxxiii).
Solvent that may be used includes methanol, ethanol, propanol, isopropanol, butanol, tetrahydrofuran, diethylether, toluene, ethyl acetate, and the mixture thereof, etc.
Temperature for such reaction may be 0 °C to refluxing temperature of the solvent.
Reaction time may be 0.5 to 72 hours.
The obtained compound represented by the general formula (lxxiv) may be purified according to conventional method (e.g., column chromatography, recrystallization, etc.).

### (Method AB)

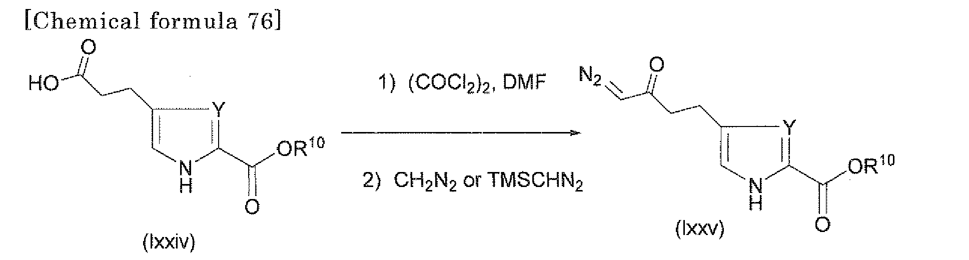

wherein, all variables in the formula are as defined above.
An acid chloride of the compound (lxxiv) may be prepared by the reaction of the compound (lxxiv) with oxalyl chloride in the presence of N,N-dimethylformamide, then the obtained product was treated with diazomethane or trimethylsilyldiazomethane to afford a compound represented by the general formula (lxxv).
Solvent that may be used includes tetrahydrofuran, diethylether, toluene, dichloromethane, 1,2-dichloroethane, chloroform, and the mixture thereof, etc.
Temperature for such reaction may be -78 °C to refluxing temperature of the solvent.
Reaction time may be 0.5 to 48 hours.
The obtained compound represented by the general formula (lxxv) may be purified according to conventional method (e.g., column chromatography, recrystallization, etc.).

### (Method AC)

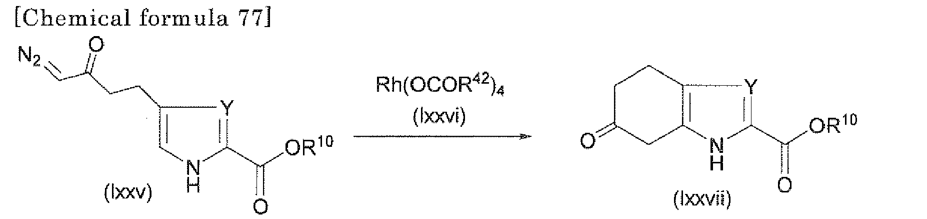

wherein, R⁴² is substituted or unsubstituted alkyl, substituted or unsubstituted cycloalkyl; all other variables are as defined above.
A cyclized compound represented by the general formula (lxxvii) may be prepared by the cyclization of the compound represented by the general formula (lxxv) in the presence of the rhodium catalyst represented by the general formula (lxxvi).
Solvent that may be used includes toluene, dichloromethane, 1,2-dichloroethane, chloroform, and the mixture thereof, etc.
Temperature for such reaction may be -78 °C to refluxing temperature of the solvent.
Reaction time may be 0.5 to 72 hours.
The obtained compound represented by the general formula (lxxvii) may be purified according to conventional method (e.g., column chromatography, recrystallization, etc.).

### (Method AD)

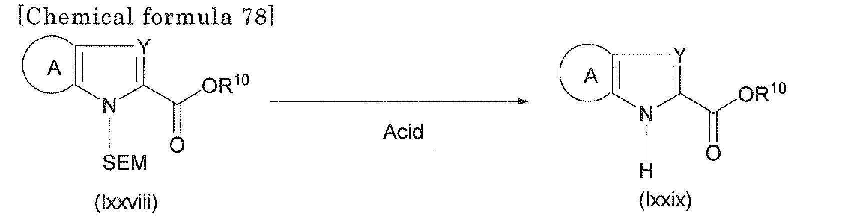

wherein, all variables in the formula are as defined above.
A compound represented by the general formula (lxxix) may be prepared from the compound (lxxviii) in the acidic condition.
Acid that may be used includes hydrochloric acid, sulfuric acid, acetic acid, 4-toluenesulfonic acid, methanesulfonic acid, trifluoroacetic acid, etc., it may be used i an amount of 1 or more mol equivalent(s) in respect of the compound represented by the general formula (lxxviii), or as solvent.
Solvent that may be used includes tetrahydrofuran, diethylether, toluene, dichloromethane, 1,2-dichloroethane, chloroform, ethyl acetate, and the mixture thereof, etc.
Temperature for such reaction may be -20 °C to refluxing temperature of the solvent.
Reaction time may be 0.5 to 24 hours.
The obtained compound represented by the general formula (lxxix) may be purified according to conventional method (e.g., column chromatography, recrystallization, etc.).

### (Method AE)

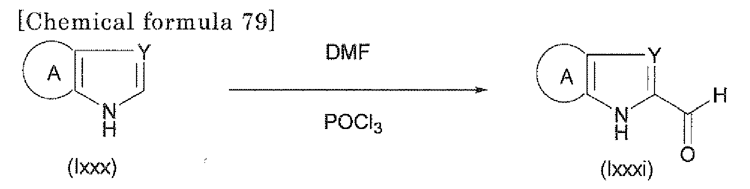

wherein, all variables in the formula are as defined above.
A compound represented by the general formula (lxxi) may be prepared by the reaction of the compound (lxxx) with phosphorous oxychloride.
Phosphorous oxychloride may be used in an amount of I or more equivalent(s) in respect of the compound (lxxx), or as solvent.
Solvent that may be used includes N,N-dimethylformamide.
Temperature for such reaction may be -78 °C to refluxing temperature of the solvent.
Reaction time may be 0.5 to 24 hours.
The obtained compound represented by the general formula (lxxxi) may be purified according to conventional method (e.g., column chromatography, recrystallization, etc.).

### (Method AF)

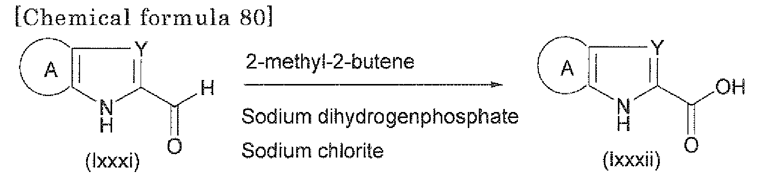

wherein, all variables in the formula are as defined above.
A compound represented by the general formula (lxxxii) may be prepared by the reaction of the compound (Ixxxi) with 2-methyl-2-butene, sodium dihydrogenphosphate and sodium chlorite.
2-methyl-2-butene may be used in an amount of 1 to 50 mol equivalent(s), sodium dihydrogenphosphate may be used in an amount of 1 to 50 mol equivalent(s) in respect of the compound (lxxxi), and sodium chlorite may be used in an amount of 1 or more equivalent(s) in respect of the compound (lxxxi), or as solvent.
Solvent that may be used includes tetrahydrofuran, water, diethylether, toluene, ethanol, ethanol, propanol, isopropanol, butanol, and the mixture thereof, etc.
Temperature for such reaction may be -20 °C to refluxing temperature of the solvent.
Reaction time may be 0.5 to 24 hours.
The obtained compound represented by the general formula (lxxxii) may be purified according to conventional method (e.g., column chromatography, recrystallization, etc.).

### Reference example 1

### Preparation of II-006 (compound IX)

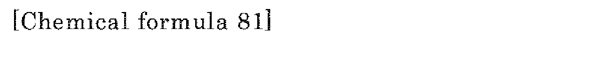

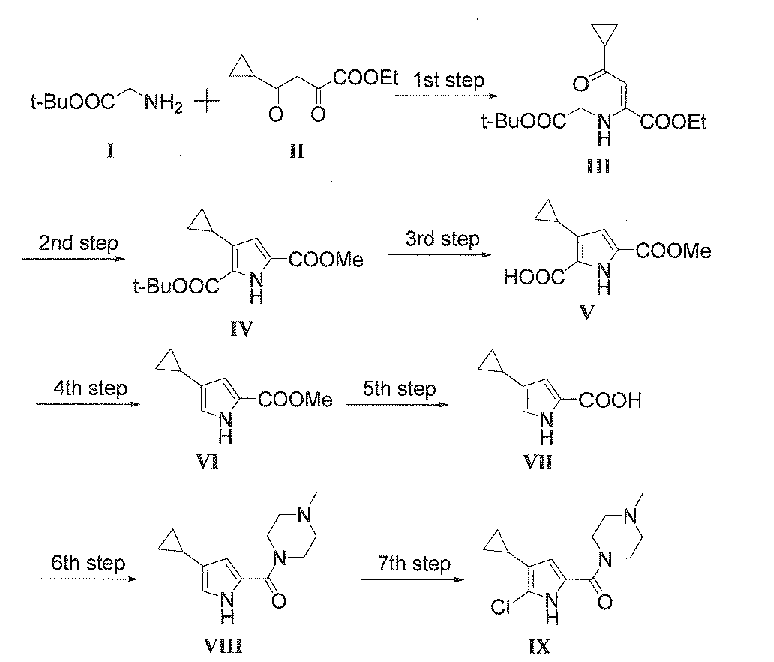

### 1st step

To a solution of a compound 1 (262 mg, 2.0 mmol) in toluene (5 mL) were added a compound II (368 mg, 2.0 mmol) and p-toluenesulfonic acid monohydrate (19 mg, 0.1 mmol), and the resulting solution was stirred at 130 °C for 4 hours. The reaction mixture was poured into saturated sodium bicarbonate aqueous solution and extracted with ethyl acetate, then the organic layer was washed with saturated brine. The organic layer was dried over anhydrous magnesium sulfate, concentrated *in vacuo* to give a compound III (281 mg, Yield 47%) as yellow oil. The obtained compound III was used for the next reaction without further purification. ¹ H NMR (DMSO-d6) δ: 0.78-0.84 (m, 4H), 1.24-1.28 (m, 3H), 1.40 (s, 9H), 1.90-2.00 (m, 1H), 4.10-4.25 (m ,4.H), 5.78 (d, J = 1.2 Hz, 1H), 10.01 (brs, 1H).

### 2nd step

To a solution of the compound III (275 mg, 0.93 mmol) in methanol (3 mL) was added 1 mol/L sodium methoxide in methanol solution (1.02 mL, 1.02 mmol), and the resulting solution was stirred at 60 °C for 4 hours. The reaction solution was poured into 1 mol/L hydrochloric acid aqueous solution, extracted with ethyl acetate, then the organic layer was washed with saturated brine. The organic layer was dried over anhydrous magnesium sulfate, concentrated *in vacuo.* The residue was purified by silicagel column chromatography eluting with hexane/ethyl acetate=40/60. The eluate was collected and concentrated *in vacuo* to give a IV (120 mg, Yield 49 %) as colorless solid.
¹H NMR (DMSO-d6) δ: 0.56-0.61 (m, 2H), 0.87-0.93 (m, 2H), 1.56 (s, 9H), 2.35-2.44 (m, 1H), 3.79 (s, 3H), 6.41 (s, 1H), 11.96 (brs, 1H).

### 3rd step

To a solution of the compound IV (294 mg, 1.11 mmol) in methylene chloride (3mL) was added trifluoroacetic acid (1.5 mL), and the resulting solution was stirred at room temperature overnight. The reaction mixture was concentrated *in vacuo* to give a V (227 mg, Yield 98 %) as pink solid. The obtained compound V was used for the next reaction without further purification.
¹H NMR (DMSO-d6) δ: 0.55-0.59 (m, 2H), 0.85-0.92 (m, 2H), 2.40-2.50 (m, 1H), 3.75 (s, 3H), 6.37 (s, 1H), 11.87 (brs, 1H).

### 4th step

To a solution of the compound V (224 mg, 1.07 mmol) in quinoline (3 mL) was added copper chromite (40 mg), and the resulting solution was stirred at 180 °C for 1 hour under microwave irradiation. The reaction mixture was purified by silicagel column chromatography eluting with chloroform. The eluate was collected and concentrated *in vacuo* to give a VI (117 mg, Yield 66 %) as yellow solid.
¹H NMR (DMSO-d6) δ: 0.43-0.48 (m, 2H), 0.73-0.80 (m, 2H), 1.65-1.73 (m, 1H), 3.71 (s, 3H), 6.50 (s, 1H), 6.81. (d, J = 1.4 Hz, 1H), 11.57 (brs, 1H).

### 5th step

To a solution of the compound VI (30 mg, 0.18 mmol) in methanol (1 mL) was added 2 mol/L sodium hydroxide aqueous solution (0.27 mL, 0.54 mmol), and the resulting solution was stirred at 60 °C for 4 hours. The reaction mixture was poured into 1 mol/L hydrochloric acid aqueous solution, extracted with ethyl acetate, then the organic layer was washed with saturated brine. The organic layer was dried over anhydrous magnesium sulfate, concentrated *in vacuo* to give a VII (26 mg, Yield 95 %) as colorless solid. The obtained compound VII was used for the next reaction without further purification.
¹H NMR (DMSO-d6) δ: 0.42-0.48 (m, 2H), 0.72-0.80 (m, 2H), 1.64-1.73 (m, 1H), 6.44 (s, 1H), 6.74 (s, 1H), 11.37 (brs, 1H).

### 6th step

To a solution of the compound VII(25 mg, 0.17 mmol) in N-methylpyrrolidone (1 mL) were added (7-azabenzotriazol-1-yl)-N,N,N',N'-tetramethyluronium hexafluorophosphate (94 mg, 0.25 mmol), N-methylpiperazine (0.028 mL, 0.25 mmol), and triethylamine (0.057 mL, 0.41 mmol) under ice cooling bath, and the resulting solution was stirred at room temperature for 2 hours. The reaction mixture was poured into water, extracted with ethyl acetate, then the organic layer was washed with water, saturated sodium hydrogen carbonate aqueous solution and saturated brine. The organic layer was dried over anhydrous magnesium sulfate, concentrated *in vacuo.* The residue was purified by silicagel column chromatography eluting with chloroform/methanol =99/1. The eluate was collected and concentrated *in vacuo* to give a VIII (15 mg, Yield 34 %) as yellow solid.
¹H NMR (DMSO-d6) δ: 0.47-0.50 (m, 2H), 0.74-0.80 (m, 2H), 1.67-1.75 (m, 1H), 2.22 (s, 3H), 2.33 (t, J = 5.0 Hz, 2H), 3.67 (t, J = 5.0 Hz, 2H), 6.25 (s, 1H), 6.67 (s, 1H), 11.11 (s, 1H).

### 7th step

To a solution of the compound VIII (100 mg, 0.43 mmol) in carbon tetrachloride (5 mL) was added tert-butyl hypochlorite (0.039 mL, 0.34 mmol), and the resulting solution was stirred at 50 °C for 2 hours. The reaction mixture was concentrated *in vacuo.* The residue was purified by silicagel column chromatography eluting with chloroform/methanol =99/1. The eluate was collected and concentrated *in vacuo.* The residue was recrystallized from hexane/ethyl acetate to give a compound IX (40 mg, Yield 35 %) as colorless solid.
¹H NMR (DMSO-d6) δ: 0.53-0.59 (m, 2H), 0.80-0.86 (m, 2H), 1.62-1.67 (m, 1H), 2.22 (s, 3H), 2.34 (t, J = 5.0 Hz, 2H), 3.64 (t, J = 5.0 Hz, 2H), 6.16 (d, J = 2.9 Hz, 1H), 11.97 (s, 1H).

### Reference example 2 to 6

Compound II-001. to II-005 were prepared according to the similar reaction previously described.

**[Table 6]**

| Compound No. | Structure | LC/MS Retention time (min) | MS[M+H]⁺ | LC method |
|---|---|---|---|---|
| II-001 | 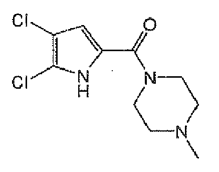 | 0.77 | 262 | B |
| II-002 | 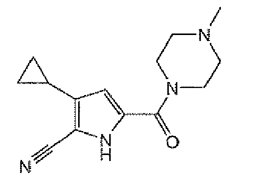 | 0.7 | 258 | A |
| II-003 | 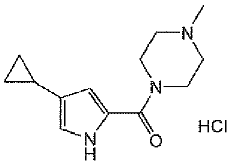 | 0.56 | 234 | A |
| II-004 | 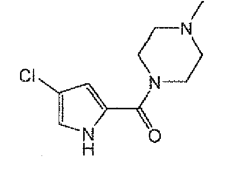 | 0.47 | 228,230 | A |
| II-005 | 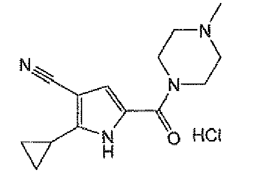 | 0.59 | 259 | A |
| II-006 | 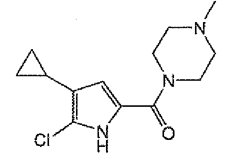 | 2.51 | 268, 70 | F |

Identification method of the compound II-001 to II-006 is as defined below LC/MS measurement.

Following examples illustrate the present invention in more detail, but the present invention is not limited by these examples. The meaning of each abbreviation is following.
Me: methyl
Et: ethyl
Bu: butyl
Ac: acetyl
PMB: p-methoxybenzyl
TMS: tetramethylsilane
TMS-Cl: trimethylsilylchloride
DMSO: dimethyl sulfoxide
DMF dimethylformamide
THF: tetrahydrofuran
DBU: 1,8-diazabicyclo[5.4.0]undee-7-ene
NMM N-methylmorpholine
NMP: N-methyl-2-pyrrolidone
DDQ: 2,3-dichloro-5,6-dicyano-p-benzoquinone
DMAP: 4-(dimethylamino)pyridi.ne
LHMDS: lithium hexamethyldisilazide HOAt: 1-hydroxy-7-azabenzotriazole
HATU: 2-(7-Azabenzotriazole-1-yl)-1,1,3,3-tetramethyluronium hexafluorophosphate
HATU: 2-(1H-benzotriazole-1-yl)-1,1,3,3-tetramethyluronium hexafluorophosphate
PyBOP: benzotriazol-1-yl-oxy)tripyrrolidinophosphonium hexafluorophosphate
TFA: trifluoroacetic acid
Fe₃(CO)₁₂: triiron dodecacarbonyl
PdCl₂(dppf)CH₂Cl₂: 1,1'-bis(diphenylphasphino)ferrocene-palladium(II)dichoride dichloromethane, complex
Ti(O-iPr)₄: tetraisopropyl orthotitanate,
rt: room temperature

### [Example 1]

### Preparation of 1-016 (Compound 6)

### 1st step

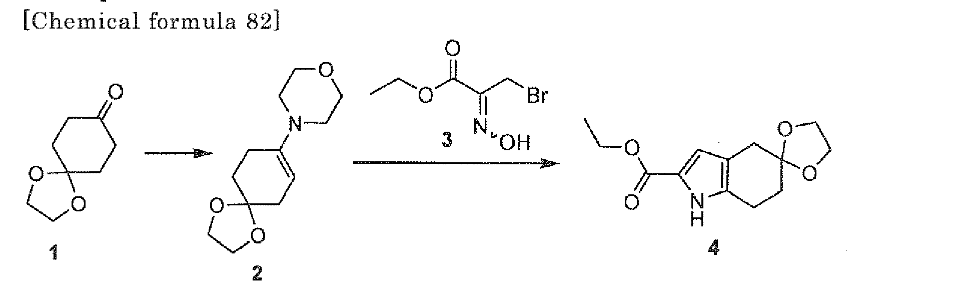

Compound 1 (10.0 g, 64.0 mmol) was dissolved in toluene (100 mL). Morpholine (8.37 mL, 96 mmol), p-toluenesulfonic acid monohydrate (487 mg, 2.56 mmol) and molecular sieves 4A (3 g) were added to the solution, and the resulting solution was refluxed for 7 hours attached with Dean-Stark trap. After cooling to room temperature, the reaction mixture was filtered and the filtrate was concentrated under reduced pressure. The residue was dissolved in toluene (500 mL), and a compound 3(6.70 g, 32.0 mmol) was added to the solution, then the resulting solution was stirred at room temperature for 3 hours. The reaction mixture was washed with water (250 mL) and saturated brine (250 mL), the organic layer was dried over anhydrous magnesium sulfate. Fe₃(CO)₁₂ (27.0 g, 53.6 mmol) and trifluoroacetic acid (8.25 mL, 107 mmol) were added to the solution, then the resulting mixture was refluxed for 3hours. After cooling to room temperature, the mixture was filtered and the filtrate was concentrated under reduced pressure. The residue was purified by silicagel column chromatography (hexane-ethyl acetate) to give Compound 4 (3.23 g, Yield 37%).
¹H-NMR(DMSO-d₆) δppm: 1.24 (t, J = 6.8Hz, 3H), 1.83 (t, J = 6.0Hz, 2H), 2.60 (s, 2H), 2.63 (t, J = 6.8Hz, 2H), 3.91 (s, 4H), 4.19 (q, J = 6.8Hz, 2H), 6.48 (s, 1H), 11.39 (br, 1H)

### 2nd step

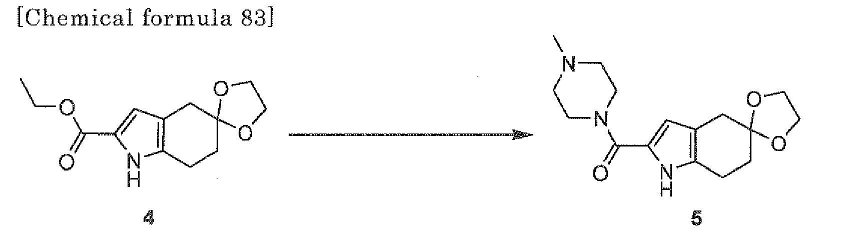

Compound 4(2.50 g, 9.95 mmol) was dissolve in toluene (75.0 mL) under nitrogen atmosphere, 1-methyl piperazine (1.66 mL, 14.9 mmol) was added to the solution. Trimethyl aluminum (1.8M in toluene solution, 8.29 mL, 14.9 mmol) was added slowly to the solution under ice cooling bath, the resulting solution was refluxed overnight. After cooling to room temperature, saturated Rochelle salt aqueous solution (80 mL) was added to the solution, the resulting mixture was stirred for 1hour, and extracted with ethyl acetate. The organic layer was washed with saturated brine, dried over anhydrous magnesium sulfate, then the solvent was removed under reduced pressure to give Compound 5 (2.32 g, Yield 76%). ¹H-NMR(DMSO-d₆) δppm: 1.82 (br, 2H), 2.19 (s, 3H), 2.30 (br, 4H), 2.61 (br, 4H), 3.65 (br, 4H), 3.90 (s, 4H), 6.17 (s, 1H), 10.99 (br, 1H)

### 3rd step

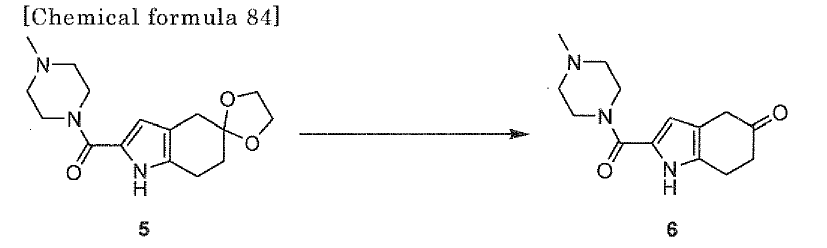

Compound 5 (2.27 g, 7.43 mmol) was dissolved in 2 mol/L hydrochloric acid aqueous solution(15 mL), the resulting solution was stirred at room temperature for 5 hours. The reaction solution was neutralized with 2 mol/L sodium hydroxide aqueous solution, the solvent was concentrated *in vacuo.* Heated methanol-ethyl acetate (1/1) solution(80 mL) was added to the obtained solid and the soluble material was dissolved, then filtered. The filtrate was concentrated under reduced pressure and solidified with hexaae-ethyl acetate to give Compound 6 (1.83 g, Yield 94%).
¹H-NMR(DMSO-d₆) δppm: 2.35 (s, 3H), 2.54-2.60 (m, 6H), 2.91 (t, J = 6.4Hz, 2H), 3.30 (s, 2H), 3.74 (br, 4H), 6.30 (s, 1H), 11.26 (br, 1H)

### [Example 2]

### Preparation of 1-018 (Compound 7)

### 1st step

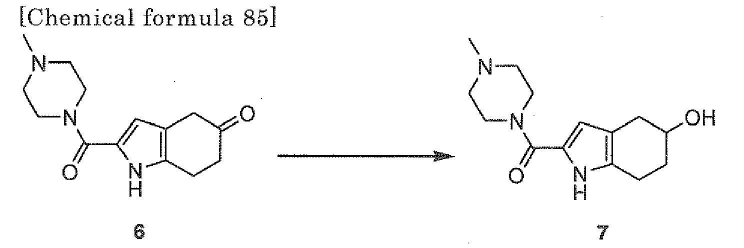

Compound 6 (300 mg, 1.15 mmol) was dissolved in methanol (10 mL), sodium borohydride (65.1 mg, 1.72 mmol) was added to the solution and stirred under ice cooling bath for 30 minutes. Saturated sodium bicarbonate water (10 mL) was added to the solution, extracted with chloroform and dried over anhydrous magnesium sulfate, the solvent was removed under reduced pressure to give Compound 7 (221 mg, Yield 73%)
¹H-NMR(DMSO-d₆) δppm, 1.62 (br, 1H), 1.86 (br, 1H), 2.18 (s, 3H), 2.24-2.34 (br, 6H), 2.58-2.70 (m, 2H), 3.64 (br, 4H), 3.83 (br, 1H), 6.17 (s, 1H), 10.89 (s, 1H)

### [Example 3]

### Preparation of 1-019 ( Compound 8)

### 1st step

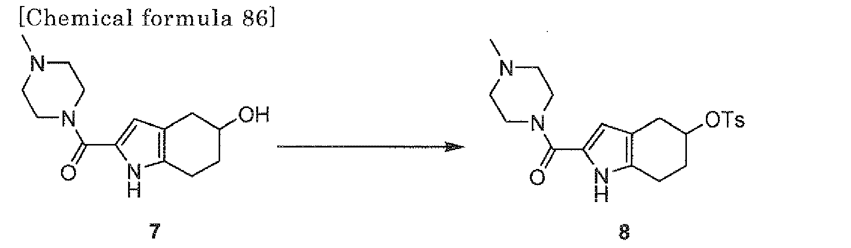

Compound 7 (100 mg, 0.380 mmol) was suspended in methylene chloride (2.0 mL), tosylchloride (145 mg, 0.759 mmol) and DMAP(116 mg, 0.949 mmol) were added to the mixture, then the resulting mixture was stirred at room temperature for 5 hours. The reaction mixture was purified by silicagel column chromatography (chloroform-methanol) to give Compound 8 (114 mg, Yield 72%)
¹H-NMR(DMSO-d₆) δppm: 1.76-1.95 (br, 2H), 2.17 (s, 3H), 2.25-2.33 (br, 4H), 2.42 (s, 3H), 2.56 (br, 4H), 3.63 (br, 4H), 4.85 (br, 1H), 6.18 (s, 1H), 7.48 (br, 2H), 7.81 (br, 2H), 11.03 (br, 1H)

### [Example 4]

### Preparation of I-020(Compound 9) and I-021(Compound 10)

### 1st step

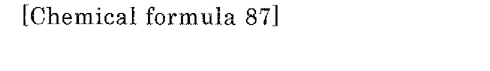

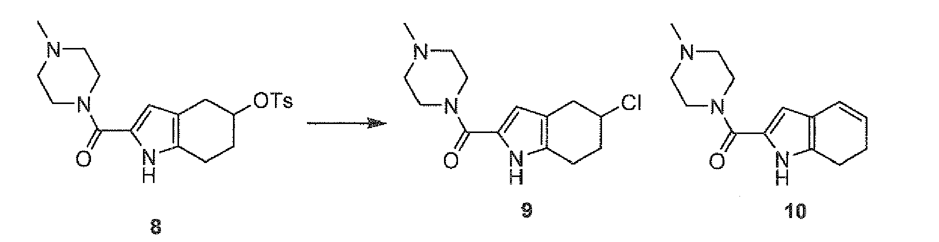

Compound 8 (20 mg, 0.048 mmol) was dissolved in pyridine (0.5 mL), pyridinium chloride (27.7 mg, 0.240 mmol) was added to the solution, then the resulting solution was stirred at 150 °C for 30 minutes under microwave irradiation. The solvent was removed under reduced pressure, the residue was purified by preparative LCMS (method I) to give Compound 9 (3.6 mg, Yield 27%) and a compound 10 (3.2 mg, Yield 27%).
Compound 9
¹H-NMR(DMSO-d₆)δppm:2.12-2.22 (br, 5H), 2.30 (br, 4H), 2.60-2.2.77 (br, 4H), 3.64 (br, 4H), 4.55 (br, 1H)> 6.23 (s, 1H), 11.03 (br, 1H)
Compound 10
¹H-NMR(DMSO-d₆)δppm: 2.20 (s, 3H), 2.33 (br, 4H), 3.06-3.23 (m, 4H), 3.67 (br, 4H), 5.84 (s, 1H), 6.27 (s, 1H), 11.01 (br, 1H)

### [Example 5]

### Preparation of I-023(Compound 11)

### 1st step

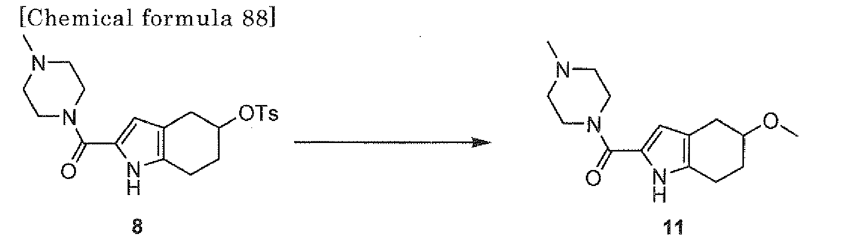

Compound 8 (20 mg, 0.048 mmol) was dissolved in methanol (0.5 mL), sodium methoxide (1.0 mol/L in methanol solution, 0.144 mL, 0.144 mmol) was added to the solution, then the resulting mixture was stirred under ice cooling bath for 30 minutes. Then, after stirred at 40°C for 4 hours, sodium methoxide (1.0 mol/L in methanol solution, 0.144 mL, 0.144 mmol) was added to the mixture, then stirred at 40°C for 2 hours. Saturated sodium bicarbonate water (2.0 mL) was added to the mixture, and extracted with ethyl acetate. The organic layer was washed with saturated brine, dried over anhydrous magnesium sulfate. The solvent was removed under reduced pressure to give Compound 11 (6.0 mg, Yield 45%). ¹H-NMR(DMSO-d₆) δppm: 1.71 (br, 1H), 1.95 (br, 1H), 2.18 (s, 3H), 2.24-2.40 (m, 6H), 2.57 (m, 1H), 2.70-2.80 (m, 1H), 3.33 (s, 3H), 3.55 (br, 1H), 3.65 (br, 4H), 6.18 (s, 1H), 10.95 (br, 1H)

### [Example 6]

### Preparation of I-025(Compound 12)

### 1st step

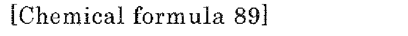

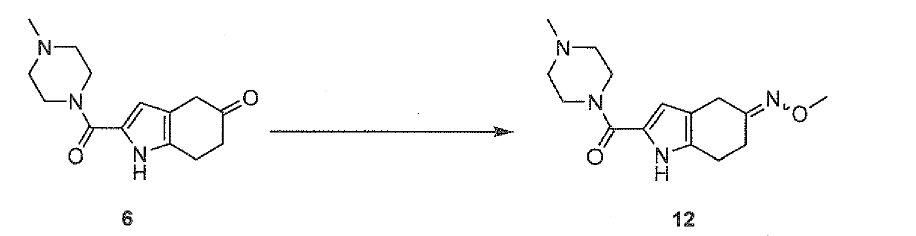

Compound 6 (50 mg, 0.191 mmol) and O-methylhydroxylamine hydrochloride (19.2 mg, 0.230 mmol) were dissolved in ethanol (1.0 mL), triethylamine (0.133 mL, 0.957 mmol) was added to the solution, then the resulting mixture was refluxed for 3 hours. After cooling to room temperature, water was added to the mixture, extracted with ethyl acetate. The organic layer was washed with saturated brine, dried over anhydrous magnesium sulfate, the solvate was removed under reduced pressure to give Compound 12 (20 mg, Yield 36%).
¹H-NMR(DMSO-d₆) δppm: 2.24 (s, 3H), 2.39 (br, 4H), 2.44-2.54 (m, 2H), 2.62-2.74 (m, 2H), 2.99-3.09 (m, 1H), 3.22 (s, 1H), 3.68 (br, 4H), 3.73-3.79 (m, 3H), 6.27 (s, 1H), 11.13 (br, 1H)

### [Example 7]

### Preparation of 1-027 (Compound 14)

### 1st step

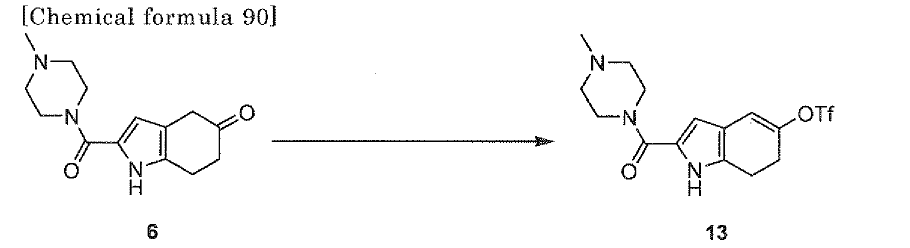

Compound 16(800 mg, 3.06 mmol) was suspended in THF (30 mL) and cooled to - 78 °C under dry ice-acetone cooling bath. Then, LHMDS (1 mol/L in THF solution, 7.35 mL, 7.35 mmol) was added dropwise to the suspension, and stirred at -78°C for 30 minutes. N-phenyl-bis(trifluoromethyl)sulfonimide (2.40 g, 6.74 mmol) in THF solution (10.0 mL) was added dropwise to the mixture, then the resulting mixture was stirred at -78 °C for 1 hour, then stirred under ice cooling bath for 1 hour. Water (10 mL) was added to the mixture, and extracted with chloroform. The organic layer was washed with saturated brine, dried over anhydrous magnesium sulfate, the solvate was removed under reduced pressure. The residue was purified by silicagel column chromatography (chloroform-methanol) to give Compound 13 (500 mg, Yield 42 %).
¹H-NMR(DMSO-de) δppm: 2.19 (s, 3H), 2.31 (br, 4H), 2.73 (t, J = 8.8Hz, 2H), 2.93 (t, J = 8.8Hz, 2H), 3.66 (br, 4H), 6.41 (s, 1H), 6.52 (s, 1H), 11.61 (br, 1H)

### 2nd step

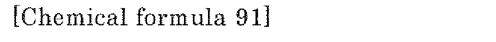

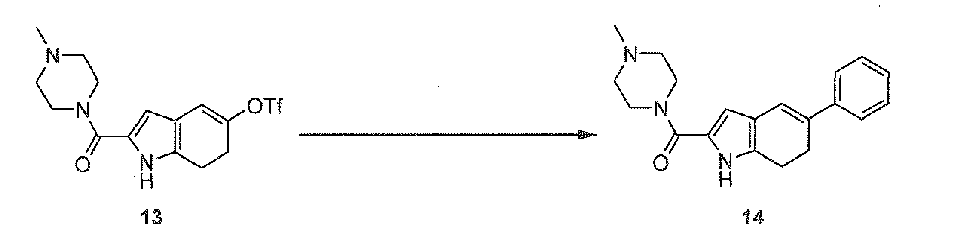

Compound 13 (30 mg, 0.076 mmol), phenylboronic acid (14.0 mg, 0.114mmol), and PdCl₂ (dppf)CH₂ Cl₂ (6.23 mg, 7.63 µmol) were dissolved in THF (1.0 mL) under nitrogen atmosphere, 2 mol/L sodium carbonate aqueous solution (0.153 mL, 0.305 mmol) was added to the solution, and the resulting mixture was stirred at 80°C for 3 hours. Water (3.0 mL) was added to the mixture, and extracted with ethyl acetate. The organic layer was washed with saturated brine, dried over anhydrous magnesium sulfate, the solvent was removed under reduced pressure. The residue was purified by silicagel column chromatography (chloroform-methanol) to give Compound 14 (10 mg, Yield 41 %).
¹H-NMR(DMSO-d₆₎ δppm: 2.21 (s, 3H), 2.34 (br, 4H), 2.74-2.85 (m, 4H), 6.40 (s, 1H), 6.78 (s, 1H), 7.20 (t, J = 7.6Hz, 1H), 7.32 (t, J = 7.2Hz, 2H), 7.67 (d, J = 6.8Hz, 2H), 11.46 (br, 1H)

### [Example 8]

### Preparation of I-028 (Compound 15)

### 1st step

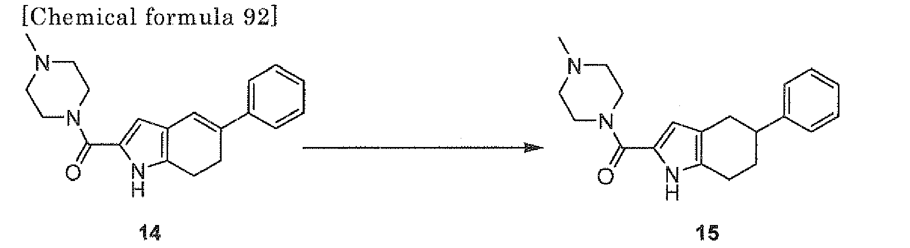

Compound 14 (8 mg, 0.025 mmol) was dissolved in methanol (3.0 mL), 10% palladium-carbon (4.0 mg) was added to the solution. The atmosphere within the reaction system was replaced with hydrogen gas, the reaction mixture was stirred at room temperature overnight. The reaction mixture was filtered through Celite pad, the filtrate was concentrated to give Compound 15 (3.0 mg, Yield 37%). ¹H-NMR(DMSO-d₆) δppm: 1.85-2.04 (br, 2H), 2.12 (s, 3H), 2.30 (br, 4H), 2.55-2.70 (m, 4H), 2.80-2.95 (br, 1H), 3.66 (br, 4H), 6.22 (s, 1H), 7.10-7.35 (m, 5H), 11.00 (br, 1H)

### [Example 9]

### Preparation of 1-024 (Compound 18)

### 1st step

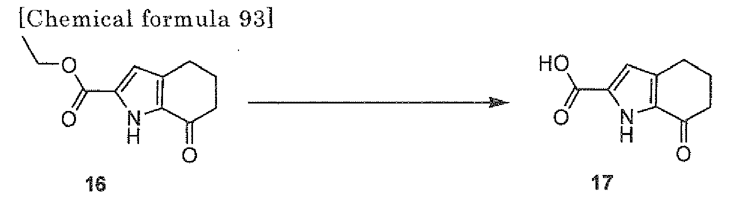

Compound 16 was prepared according to the method described in Journal of Medicinal Chemistry, 1995,38(2),86. Compound 16 (100 mg, 0.483 mmol) was dissolved in methanol-THF (1/1) solution (2.0 mL), 2 mol/L sodium hydroxide aqueous solution (0.724 mL, 1.45 mmol) was added to the solution, and the resulting mixture was stirred at 60°C for 1.5 hours. The reaction mixture was neutralized with 2 mol/L hydrochloric acid aqueous solution, extracted with ethyl acetate. The organic layer was washed with saturated brine, dried over anhydrous magnesium sulfate, the solvent was removed under reduced pressure to give Compound 17 (72 mg, Yield 83 %).
¹H-NMR(DMSO-d₆) δppm: 2.01 (t, J = 6.0Hz, 2H), 2.43 (t, J = 5.6Hz, 2H), 2.68 (t, J = 5.2Hz, 2H), 6.62 (s, 4H), 12.22 (s, 1H), 12.84 (br, 1H)

### 2nd step

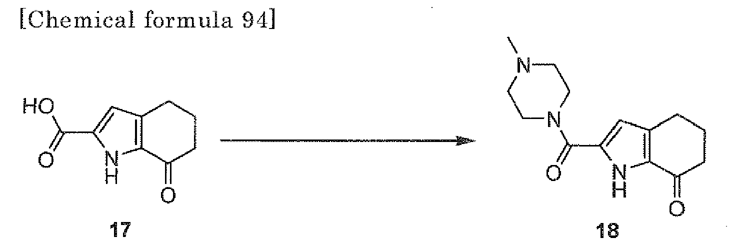

Compound 17 (50 mg, 0.279 mmol) was suspended in methylene chloride (1.0 mL) under nitrogen atmosphere, DMF (10 µL) and oxalyl chloride (0.029 mL, 0.335 mmol) were added to the suspension under ice cooling bath, and the resulting mixture was stirred at room temperature for 15 minutes. The solvent was removed under reduced pressure, toluene (1.0 mL) was added to the residue, then the solvent removed under reduced pressure. The obtained residue was dissolved in methylene chloride (1.0 mL), 1-methyl piperazine (0.093 mL, 0.837 mmol) was added to the solution, and the resulting mixture was stirred at room temperature for 30 minutes. Saturated sodium bicarbonate water (1.0 mL) was added to the mixture, then extracted with chloroform. The organic layer was washed with saturated brine, dried over anhydrous magnesium sulfate, the solvent was removed under reduced pressure to give Compound 18 (50 mg, Yield 69%).
¹H-NMR(DMSO-d₆) δppm: 2.00 (br, 2H), 2.19 (s, 3H), 2.31 (br, 4H), 2.40 (br, 2H), 2.68 (br, 2H), 3.56 (br, 4H), 6.27 (s, 1H), 11.99 (s, 1H)

### [Example 10]

### Preparation of I-044 (Compound 23)

### 1st step

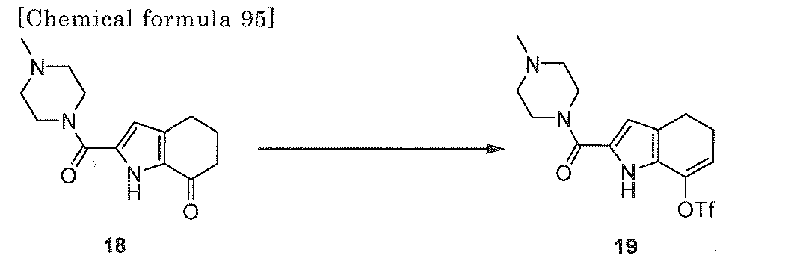

Compound 19 was prepared from the compound 18 according to the Example 6.
¹H-NMR(DMSO-d₆) δppm : 2.24 (s, 3H), 2.38 (br, 4H), 2.45-2.52 (m, 2H), 2.58 (t, J = 8.4Hz, 2H), 3.66 (br, 4H), 5.79 (s, 1H), 6.36 (s, 1H), 12.07 (s, 1H)

### 2nd step

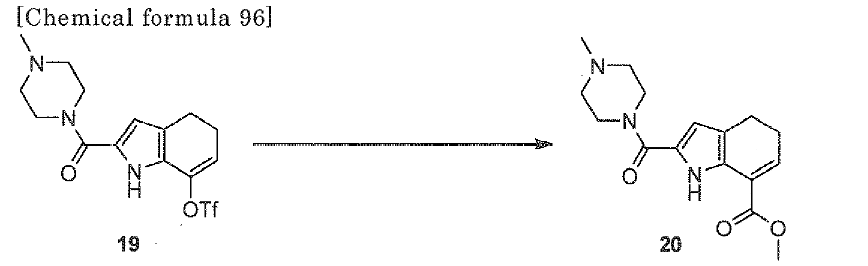

Compound 19 (500 mg, 1.27 mmol) and PdCl₂ (dppf)CH₂ Cl₂ (51.9 mg, 0.064 mmol) were dissolved in DMF -methanol (10/3)solution (13.0 mL). Triethylamine (0.529 mL, 3.81 mmol) was added to the solution, and the atmosphere within the reaction system was replaced with carbon monoxide. The reaction solution was stirred at 60°C for 3.5 hours. The solvent was removed under reduced pressure and the residue was purified by silicagel column chromatography (chloroform-methanol) to give Compound 20 (220 mg, Yield 57 %).
¹H-NMR(DMSO-d₆) δppm: 2.19 (s, 3H), 2.32 (br, 4H), 2.45-2.52 (m, 2H), 2.60 (t, J = 7.6Hz, 2H), 3.67 (br, 4H), 3.79 (s, 3H), 6.40 (s, 1H), 6.87 (s, 1H), 10.18 (s, 1H)

### 3rd step

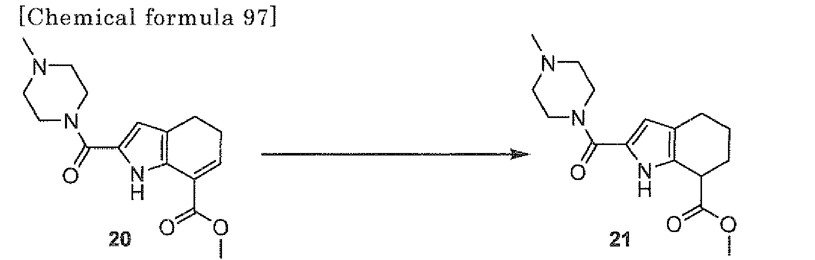

Compound 20 (48 mg, 0.158 mmol) was dissolved in methanol (4.0 mL), and 10 % palladium-carbon (5.0 mg) was added to the solution. The atmosphere within the reaction system was replaced with hydrogen gas, and the reaction mixture was stirred at room temperature for 2.5 hours. The mixture was filtered through Celite pad, the filtrate was concentrated togive Compound 21 (40.0 mg, Yield 83 %).
¹H-NMR(DMSO-d₆) δppm: 1.67 (br, 2H), 1.93 (br, 2H), 2.19 (s, 3H), 2.31 (br, 4H), 2.39-2.46 (m, 2H), 3.46 (m, 1H), 3.62 (s, 3H), 3.66 (br, 4H), 6.22 (s, 1H), 10.89 (s, 1H)

### 4th step

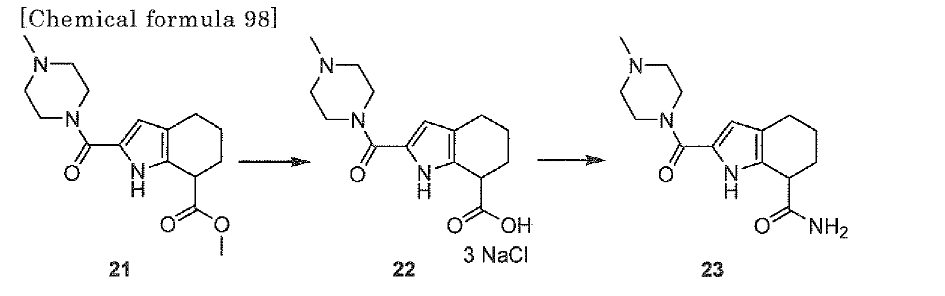

Compound 21 (14 mg, 0.045 mmol) was dissolved in THF -methanol (1/1) solution (0.5 mL), 2 mol/L sodium hydroxide aqueous solution (0.68 mL, 0.135 mmol) was added to the solution, and the resulting solution was stirred at 60°C for 3 hours. The reaction solution was neutralized with 2 mol/L hydrochloric acid aqueous solution, and the solvent was removed under reduced pressure. The obtained solid was suspended in DMF (0.5 mL), 28 % aqueous ammonia (5.20 µL, 0.067 mmol), N - methylmorpholine (12.0 µL, (3.112 mmol) and HATU (22.2 mg, 0.058 mmol) were added to the solution and the resulting mixture was stirred at room temperature overnight. Water (0.3 mL) was added to the mixture to dissolve an insoluble material. The mixture was purified by preparative LCMS (method J) to give Compound 23 (5.1 mg, Yield 39 %).
¹H-NMR(DMSO-d₆) δppm: 1.62 (br, 1H)*,* 1.77 (br, 1H), 1.84-2.02 (br, 2H), 2.1.9 (s, 3H), 2.31 (br, 4H), 2.39 (br, 2H), 3.46 (br, 1H), 3.67 (br, 4H), 6.23 (s, 1H), 6.93 (s, 1H), 7.21 (s, 1H), 10.74 (s, 1H)

### [Example 11]

### Preparation of 1-040 (Compound 24)

### 1st step

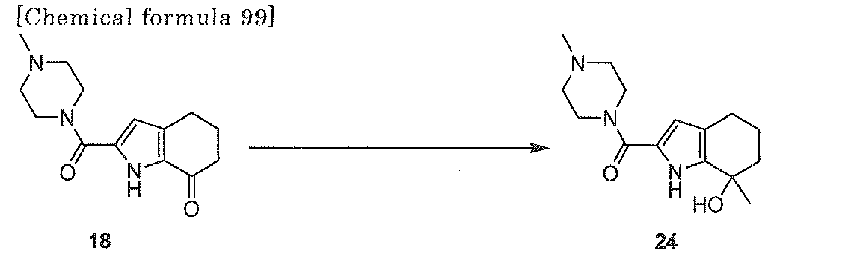

Compound 18 (30 mg, 0.115 mmol) was suspended in THF (8.0 mL) under nitrogen atmosphere, methyllithium (1.09 mol/L in diethyl ether solution, 1.58 mL, 1.722 mmol.) was added dropwise at -10 °C (NaCl-ice), and the resulting mixture was stirred at -10 °C for 30 minutes. Saturated aqueous ammonia chloride solution (2.0 mL) was added to the mixture and extracted with chloroform. The organic layer was washed with saturated brine, dried over anhydrous magnesium sulfate. The solvent was removed under reduced pressure to give Compound 24 (22 mg, Yield 69 %).
¹H-NMR(DMSO-d₆) δppm: 1.44 (s, 3H), 1.55-1.70 (m, 2H), 1.70-1.79 (m, 1H), 1.79-1.90 (m, 1H), 2.19 (s, 3H), 2.30 (br, 4H), 2.38 (br, 2H), 3.66 (br, 4H), 4.65 (s, 1H), 6.15 (s, 1H), 10.77 (s, 1H)

### [Example 12]

### Preparation of 1-043 (Compound 26)

### 1st step

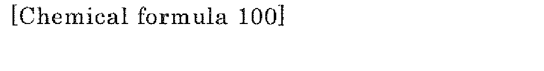

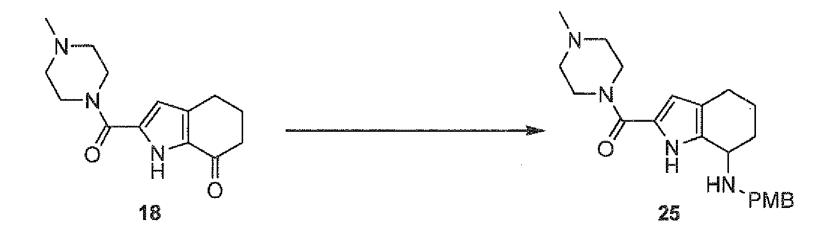

Compound 18 (50 mg, 0.191 mmol), Ti(O-iPr)₄ (0.112 mL, 0.383 mmol) and p-methoxybenzylamine (0.037 mL, 0.287 mmol) were mixed under nitrogen atmosphere and the resulting solution was stirred at 80 °C for 2.5 hours. After cooling to room temperature, ethanol (1.0 mL) was added to the solution to dissolve the residue. Sodium borohydride (21.7 mg, 0.574 mmol) was added to the solution and the resulting mixture was stirred at room temperature for 1hour. Water (2.0 mL) was added to the mixture and extracted with chloroform. The organic layer was washed with saturated brine, dried over anhydrous magnesium sulfate, and the solvent was removed under reduced pressure. The residue was purified by silicagel column chromatography (chloroform-methanol-triethylamine) to give Compound 25 (58 mg, Yield 79 %).
¹H-NMR(DMSO-d₆) δppm: 1.46-1.64 (m, 2H), 1.80-1.94 (m, 2H), 2.18 (s, 3H), 2.29 (br, 4H), 2.40 (br, 2H), 3.62 (t, J = 12.4Hz, 2H), 3.68 (br, 4H), 3.70-3.78 (m, 4H), 6.20 (s, 1H), 6.86 (d, J = 8.4Hz, 2H), 7.29 (d, J = 8.4Hz, 2H), 10.98 (s, 1H)

### 2nd step

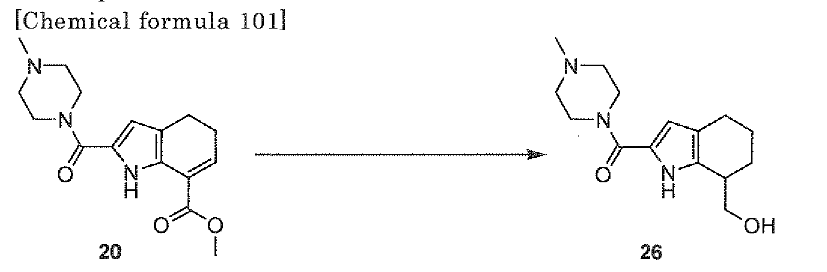

Compound 20 (10 mg, 0.033 mmol) was dissolved in methanol (0.5 mL), lithium borohydride (7.18 mg, 0.33 mmol) was added to the solution, and the resulting mixture was stirred at room temperature for 2hours. Additional lithium borohydride (7.18 mg, 0.33 mmol) was added to the mixture, and the resulting mixture was stirred at room temperature for 30 minutes. Water (10 mL) was added to the mixture and extracted with chloroform. The organic layer was washed with saturated brine, dried over anhydrous magnesium sulfate, and the solvent was removed under reduced pressure. The residue was purified by preparative LCMS (method H) to give Compound 26 (5.0 mg, Yield 45 %).
¹H-NMR(DMSO-d₆) δppm: 1.58 (br, 2H), 1.75 (br, 2H), 2.19 (s, 3H), 2.30 (br, 4H), 2.39 (br, 2H), 2.76 (br, 1H), 3.42 (t, J = 7.6Hz, 2H), 3.66 (br, 4H), 6.21 (s, 1H), 10.56 (s, 1H)

### [Example 13]

### Preparation of I-050 (Compound 30)

### 1st step

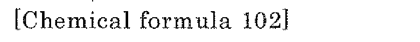

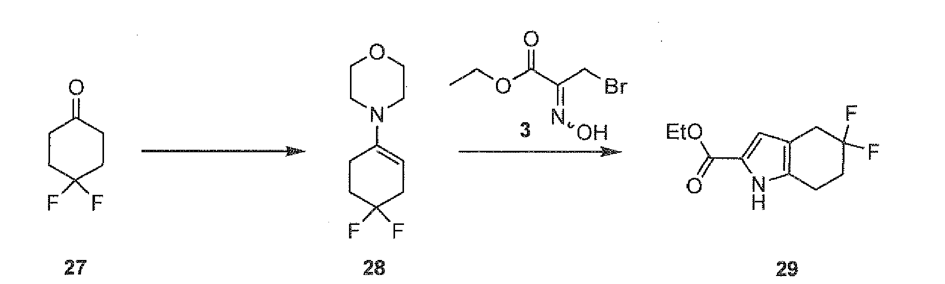

Compound 29 was prepared from a compound 27 according to the method of Example 1, 1st step.
¹H-NMR(CD₃ OD) δppm: 1.28 (t, J = 7.2Hz, 3H), 2.1.7-2.31 (m, 2H), 2.76 (t, J = 6.6Hz, 2H), 3.01 (t, J = 14.4Hz, 2H), 4.21 (q, J = 7.2Hz, 2H), 6.58 (d, J = 3.6Hz, 1H), 11.60

### (br, 1H)

### 2nd step

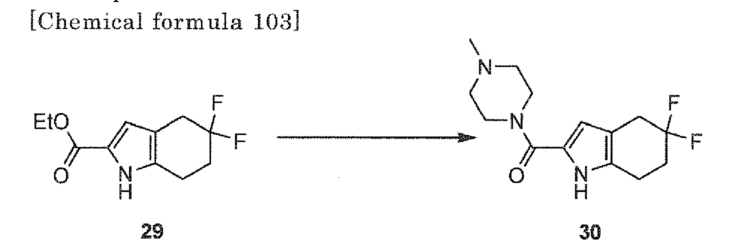

Compound 30 was prepared from the compound 29 according to the method of Example 1, 2nd step.
¹H-NMR(DMSO-d₆) δppm: 2.13-2.24 (br, 5H), 2.30 (br, 4H), 2.71 (br, 2H), 2.98 (t, J = 14.4Hz, 2H), 3.65 (br, 4H), 6.25 (s, 1H), 11.19 (s, 1H)

### [Example 14]

### Preparation of 1-047 (Compound 34)

### 1st step

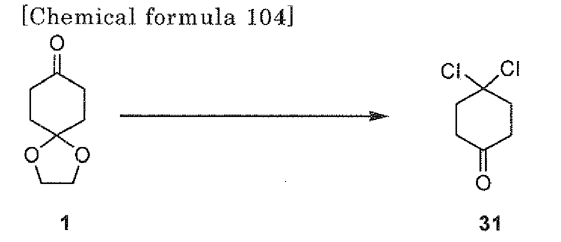

Compound 1(10.0 g, 64.0 mmol) was suspended in toluene (20 mL) under nitrogen atmosphere, 5g each of phosphorous pentachloride (29.3 g, 141 mmol) was added solowly under ice cooling bath, and the resulting mixture was stirred at room temperature for 2 hours. The reaction mixture was poured into ice water (200 mL), the resulting mixture was stirred vigorously for 30 minutes. The reaction mixture was neutralized with sodium hydrogen carbonate, added ethyl acetate and filtered to remove an insoluble material. The organic layer was washed with saturated brine, dried over anhydrous magnesium sulfate, and the solvent was removed under reduced pressure. The obtained residue was purified by silicagel column chromatography (hexane-ethyl acetate) to give Compound 31 (2.45 g, Yield 23 %).
¹H-NMR(DMSO-d₆)δppm: 2.48 (t, J = 6.0Hz, 2H), 2.74 (t, J = 6.0Hz, 2H)

### 2nd step

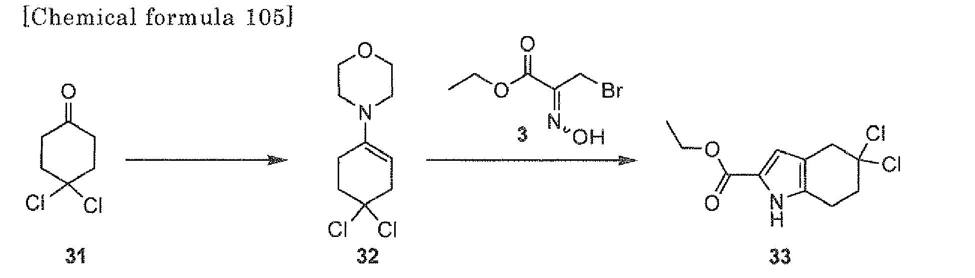

Compound 31 (1.57 g, 9.40 mmol) was dissolved in toluene (15 mL) under nitrogen atmosphere, morpholine (4.91 mL, 56.4 mmol) was added to the solution. Titanium tetrachloride (5 mL in toluene solution, 0.518 mL, 4.70 mmol) was added dropwise under ice cooling bath, and the resulting mixture was allowed to warm up to room temperature and stirred for 5 hours. The reaction mixture was filtered through Celite pad, the filtrate was concentrated *in vacua.* The obtained residue was dissolved in toluene (30 mL), the compound 3(1.0 g, 4.76 mmol) was added to the mixture, and the resulting mixture was stirred at room temperature for hours. The reaction mixture was filtered, and Fe₃(CO)₁₂ (3.60 g, 7.14 mmol) and trifluoroacetic acid (1.10 mL, 14.3 mmol) were added to the filtrate, and the resulting mixture was refluxed for 3 hours. After cooling to room temperature, the reaction mixture was filtered, and the filtrate was concentrated *in vacuo.* The obtained residue was purified by silicagel column chromatography (hexane-ethyl acetate) to give Compound 33(326 mg, Yield 26 %).
¹H-NMR(DMSO-d₆) δppm: 1.26 (t, J = 6.4Hz, 3H), 2.60 (br, 2H), 2.79 (br, 2H), 3.44 (s, 2H), 4.20 (q, J = 6.8Hz, 2H), 6.55 (s, 1H), 11.59 (s, 1H)

### 3rd step

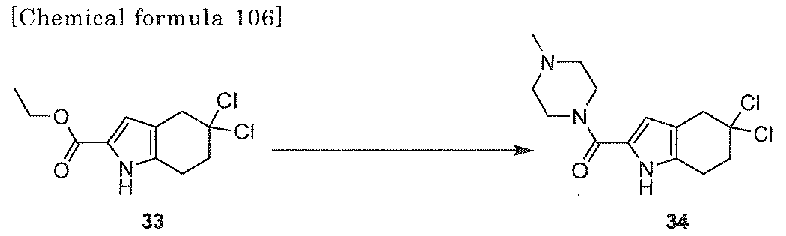

Compound 33 (150 mg, 0.572 mmol) was dissolved in toluene (2.0 mL) under nitrogen atmosphere, 1-methylpiperazine (0.096 mL, 0.858 mmol) was added. Trimethyl aluminum (1.8M in toluene solution, 0.477 mL, 0.858 mmol) was added slowly to the solution under ice cooling bath, and the resulting mixture was refluxed overnight. After cooling to room temperature, saturated Rochelle salt aqueous solution (2.0 mL) was added to the mixture, and the resulting mixture was stirred for 1 hour and extracted with ethyl acetate. The organic layer was washed with brine, dried over anhydrous magnesium sulfate, and the solvent was removed under reduced pressure. The obtained residue was purified by preparative LCMS (method H) to give Compound 34(58 mg, Yield 32 %).
¹H-NMR(DMSO-d₆)δppm: 2.21 (br, 3H), 2.34 (br, 4H), 2.59 (br, 2H), 2.77 (br, 2H), 3.45 (s, 2H), 3.66 (br, 4H), 6.26 (s, 1H), 11.20 (s, 1H)

### [Example 15]

### Preparation of 1-049 (Compound 36)

### 1st step

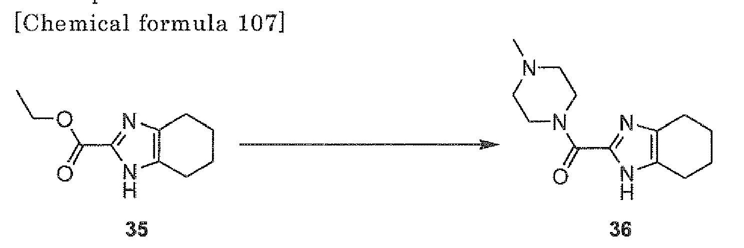

Compound 35 (20.0 mg, 0.103 mmol) was dissolved in toluene (1.0 mL), 1-methylpiperazine (0.017 mL, 0.154 mmol) was added under nitrogen atmosphere. Trimethyl aluminum (1.8M in toluene solution, 0.086 mL, 0.154 mmol) was added slowly to the solution under ice cooling bath, and the resulting mixture was stirred at room temperature for 1 hour. Saturated Rochelle salt aqueous solution (80 mL) was added to the mixture, and the resulting mixture was stirred for 1 hour and extracted with ethyl acetate. The organic layer was washed with saturated brine, dried over anhydrous magnesium sulfate, and the solvent was removed under reduced pressure. The obtained residue was purified by silicagel column chromatography (amino column; chloroform-methanol) to give Compound 36 (9.5 mg, Yield 37 %).
¹H-NMR(DMSO-d₆) δppm: 1.67-1.77 (br, 4H), 2.21 (s, 3H), 2.38 (br, 4H), 2.43-2.51 (br, 2H), 3.30-3.37 (br, 2H), 3.61 (br, 2H), 4.45 (br, 2H), 12.40 (br, 1H)

### [Example 16]

### Preparation of 1-029 (Compound 42)

### 1st step

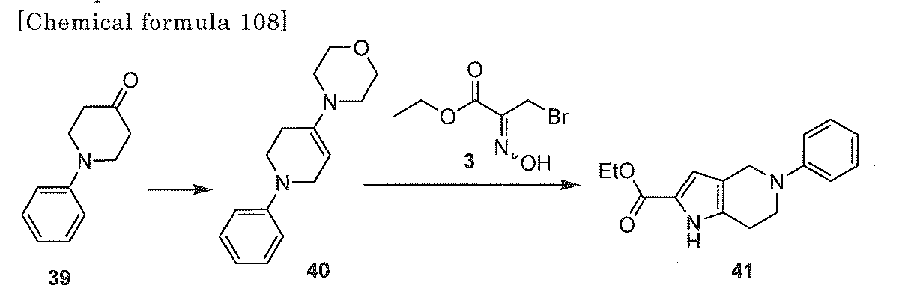

Compound 47 was prepared from a compound 39 according to the method of Example 1, 1st step.
¹H-NMR(DMSO-d₆) δppm: 1.27 (t, J = 7.2Hz, 3H), 2.72 (s, 2H), 3.56 (s, 2H), 4.15 (s, 2H), 4.20 (q, J = 6.8Hz, 2H), 6.64 (s, 1H), 6.74 (t, J = 6.8Hz, 1H), 6.97 (d, J = 7.6Hz, 2H), 7.20 (t, J = 7.2Hz, 2H), 11.56 (s, 1H)

### 2nd step

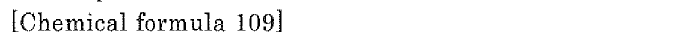

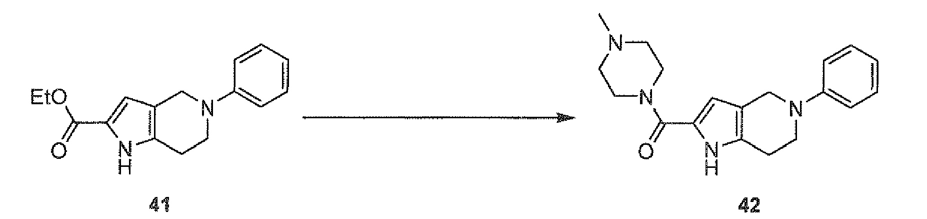

Compound 42 was prepared from the compound 41 according to the method of Example 1, 2nd step.
¹H-NMR(DMSO₋d₆) δppm: 2.19 (s, 3H), 2.31 (br, 4H), 2.69 (s, 2H), 3.56 (s, 2H), 3.68 (br, 4F), 4.16 (s, 2H), 6.32 (s, 1H), 6.72 (br, 1H), 6.98 (br, 2H), 7.20 (br, 2H), 11.15 (s, 1H)

### [Example 17]

### Preparation of 1-026 (Compound 4.7)

### 1st step

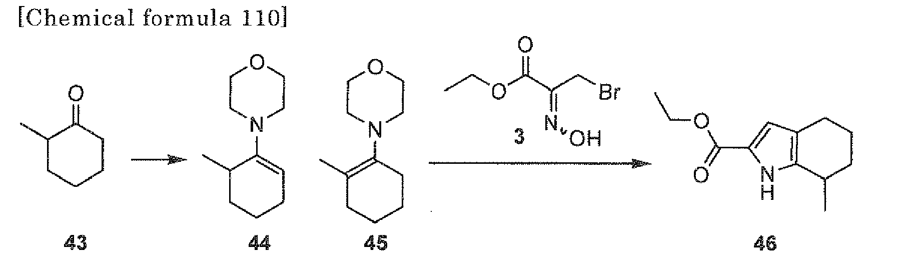

Compound 46 was prepared from a compound 43 according to the method of Example 1, 1st step.
¹H-NMR(DMSO-d₆ ) δppm: 1.18-1.29 (m, 6H), 1.30-1.39 (m, 1H), 1.46-1.60 (m, 1H), 1.69-1.79 (m, 1H), 1.81-1.90 (m, 1H), 2.39 (t, J = 5.2Hz, 2H), 2.76 (q, J = 6.4Hz, 1H), 4.19 (t, J = 7.2Hz, 2H), 6.48 (s, 1H), 11.29 (s, 1H)

### 2nd step

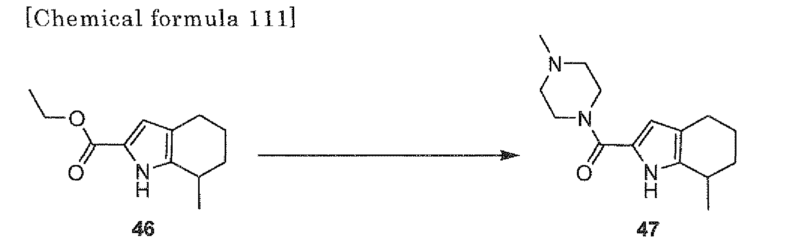

Compound 47 was prepared from the compound 46 according to the method of Example 1, 2nd step.
¹H-NMR(DMSO-d₆) δppm: 1.19 (d, J = 6.0Hz, 3H), 1.28-1.38 (m, 1H), 1.47-1.61 (m, 1H), 1.09-1.80 (m, 1H), 1.80-1.90 (m, 1H), 2.18 (s, 3H), 2.29 (br, 4H), 2.39 (br, 2H), 2.72 (q, J = 5.6Hz, 1H), 3.65 (br, 4H), 6.15 (s, 1H), 10.89 (s, 1H)

### [Example 18]

### Preparation of I-048 (Compound 53)

### 1st step

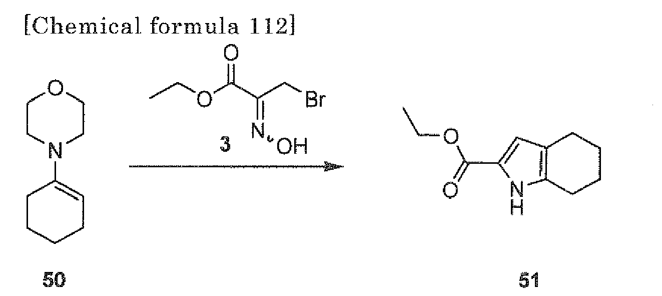

Compound 50 (1.0 g, 6.00 mmol) was dissolved in toluene (30 mL), the compound 3(630 mg, 3.00 mmol) was added to the solution, and the resulting solution was stirred at room temperature for 3 hours. Then, Fe₃(CO)₁₂ (2.27 g, 4.50 mmol) and trifluoroacetic acid (0.693 mL, 9.00 mmol) were added to the solution, and the resulting mixture was refluxed for 3 hours. After cooling to room temperature, the reaction mixture was filtered and the filtrate was concentrated *in vacuo.* The obtained residue was purified by silicagel column chromatography (hexane-ethyl acetate) to give Compound 51 (80 mg, Yield 14 %).
¹H-NMR(DMSO-d₆) δppm: 1,24 (t, J = 6.8Hz, 3H), 1.60-1.75 (m, 4H), 2.40 (t, J 6.0Hz, 2H), 2.49-2.54 (m, 2H), 4.17 (q, J = 6.8Hz, 2H), 6.48 (s, 1H), 11.28 (br, 1H)

### 2nd step

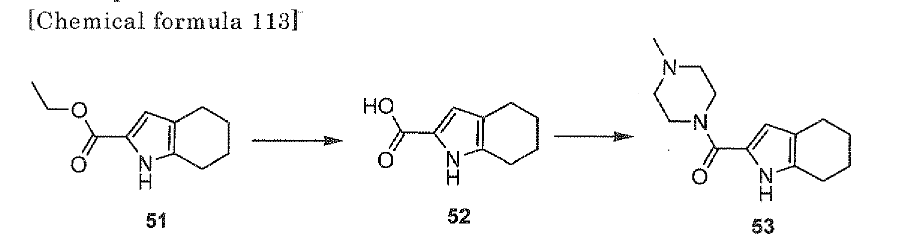

Compound 51(34 mg, 0.177 mmol) was dissolved in THF -methanol (1/1) solution (1.0 mL), 2 mol/L sodium hydroxide aqueous solution (0.266 mL, 0.531 mmol) was added to the solution, and the resulting mixture was stirred at 60 °C for 4 hours. After cooling, the reaction mixture was neutralized with 2 mol/L hydrochloric acid aqueous solution and extracted with ethyl acetate. The organic layer was washed with brine, dried over anhydrous magnesium sulfate, and the solvent was removed under reduced pressure. The obtained residue was dissolved in DMF (1.0 mL), 1-methylpiperazine (0.026 mL, 0.236 mmol), N-methylmorpholine (0.050 mL, 0.454 mmol) and HATU (90 mg, 0.236 mmol) were added to the solution, and the resulting mixture was stirred at room temperature for 2 hours. Water (3 mL) was added to the mixture and extracted with ethyl acetate. The organic layer was washed with saturated brine and dried over anhydrous magnesium sulfate, and the solvent was removed under reduced pressure. The obtained residue was purified by preparative LCMS (method G) to give Compound 53 (14 mg, Yield 31 %).
¹H-NMR(DMSO-d₆,) δppm: 1.60-1.75 (m, 4H), 2.42 (br, 2H), 2.51 (br, 4H), 2.84 (s, 3H), 3.02 (br, 2H), 3.25 (br, 2H), 4.49 (br, 2H), 6.33 (s, 1H), 11.03 (br, 1H)

### [Example 19]

### Preparation of I-011 (Compound 58)

### 1st step

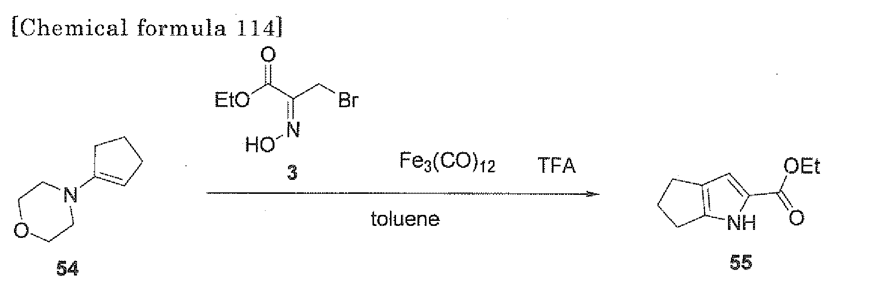

Compound 54 (4.38 g, 28.6 mmol) was dissolved in toluene (80 mL), the compound 3(3 g, 14.3 mmol) was added to the solution, and the resulting mixture was stirred at room temperature for 3.5 hours. Water (40 mL) was added to the mixture, and the organic layer was dried over anhydrous magnesium sulfate.
Fe₃(CO)₁₂ (10.80g, 21.4 mmol) and trifluoroacetic acid (3.3 mL, 42.9 mmol) were added to the organic layer at room temperature, the resulting mixture was stirred at 130 °C for 3 hours. After cooling to room temperature, the reaction mixture was filtered through Celite pad, the filtrated was concentrated *in vacuo.* The obtained residue was purified by silicagel column chromatography (hexane-ethyl acetate) to give Compound 55 (863.3 mg, Yield 34 %).
¹H-NMR(DMSO-d₆, / TMS)δppm: 11.4 (s, 1H), 6.48 (s, 1H), 4.18 (q, J = 7.2Hz, 2H), 2.61 (t, J = 6.9Hz, 2H), 2.50 (t, J = 6.6Hz, 2H), 2.32 (tt, J = 6.6, 6.9Hz, 2H), 1.24 (t, J = 7.2Hz, 3H).

### 2nd step

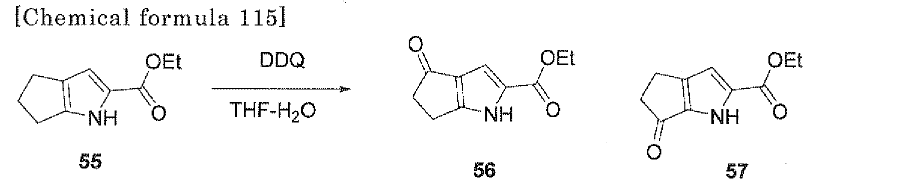

Compound 55 (52 mg, 0.290 mmol) was dissolved in tetrahydrofuran (2 mL) and water (0.3 mL), DDQ was added dropwise to the solution at 0 °C. After addition, the reaction mixture was stirred at 0 °C for 4 hours. Saturated sodium bicarbonate water was added to the mixture and extracted with ethyl acetate. The organic layer was washed with saturated brine, dried over anhydrous magnesium sulfate, and the solvent was removed under reduced pressure. The obtained residue was purified by silicagel column chromatography (hexane-ethyl acetate) to give a compound 57(33 mg, 59%) and Compound 56 (10.6 mg, 19 %).
a compound 57
¹H-NMR(DMSO-d₆ / TMS)δppm: 1.2.4 (s, 1H), 6.77 (s, 1H), 4.26 (q, J = 7.2Hz, 2H), 2.90 (dt, J = 3.3, 4.8Hz, 2H), 2.77 (dt, J = 3.3, 4.8Hz, 2H), 1.26 (t, J = 7.2Hz, 3H).
a compound 56
¹H-NMR(DMSO-d₆ / TMS) δppm: 12.5 (s, 1H), 6.71 (s, 1H), 4.27 (q, J = 7.2Hz, 2H), 2.78 (s, 4H), 1.29 (t, J = 7.2Hz, 3H).

### 3rd step

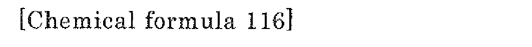

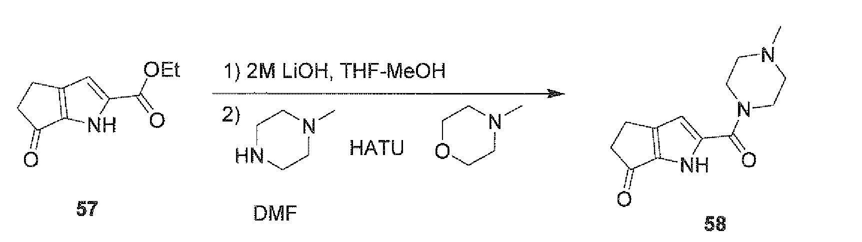

Compound 57 (84.2 mg, 0.436 mmol) was dissolved in tetrahydrofuran (1.5 mL) and methanol (0.8 mL), 2mol/L lithium hydroxide aqueous solution (1.3 mL) was added to the solution, and the resulting mixture was stirred at 70°C for 4 hours. The solvent was removed under reduced pressure, and the residue was washed with ether, and acidified with concentrated hydrochloric acid (pH=2). The precipitated solid was collected by filtration, washed with water and dried to give 6-oxo -1,4,5,6-tetrahydrocyclopenta [b] pyrrol-2-carboxylic acid (62 mg, Yield 86 %).
6-oxo -1,4,5,6-tetrahydrocyclopenta [b] pyrrol-2-carboxylic acid (62 mg, 0.375 mmol) was dissolved in DMF(2 mL), N-methylpiperazine (84µL, 0.751 mmol), HATU(157 mg, 0.413 mmol) and N-methylmorpholine (83µL, 0.751 mmol) were added to the solution, and the resulting mixture was stirred at room temperature for 24 hours. Water was added to the reaction mixture and extracted with chloroform. The organic layer was washed with saturated brine, dried over anhydrous magnesium sulfate, and the solvent was removed under reduced pressure. The obtained residue was purified by silicagel column chromatography (chloroform-methanol) to give Compound 58 (67 mg, 72 %).
¹H-NMR(DMSO-d₆ / TMS) δppm: 12.1 (s, 1H), 6.35 (s, 1H), 3.59 (t, J = 5.1Hz, 4H), 2.79-2.73 (m, 4H), 2.32 (t, J = 5.1Hz, 4H), 2.19 (s, 3H).

### [Example 20]

### Preparation of 1-010 (Compound 61)

### 1st reaction

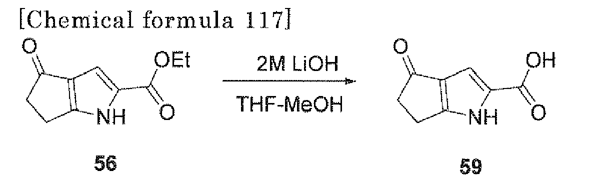

Compound 56 (154.4 mg, 0.799 mmol) was dissolved in tetrahydrofuran (2.5 mL) and methanol (1.3 mL), 2mol/L lithium hydroxide aqueous solution (2.5 mL) was added to the solution, and the resulting mixture was stirred at 70 °C for 4 hours. The solvent was removed under reduced pressure, and the residue was washed with ether, and acidified with concentrated hydrochloric acid (pH=2). The precipitated solid was collected by filtration, washed with water and dried to give Compound 59 (1.07 g, Yield 95 %).
¹H-NMR(DMSO-d₆ /TMS) δppm: 12.8 (brs, 1H), 12.3 (s, 1H), 6.73 (s, 1H), 2.88 (dt, J = 3.0, 5.1Hz, 2H), 2.76 (dt, J = 3.0, 5.1Hz, 2H).

### 2nd step

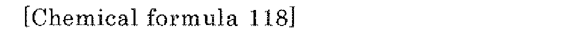

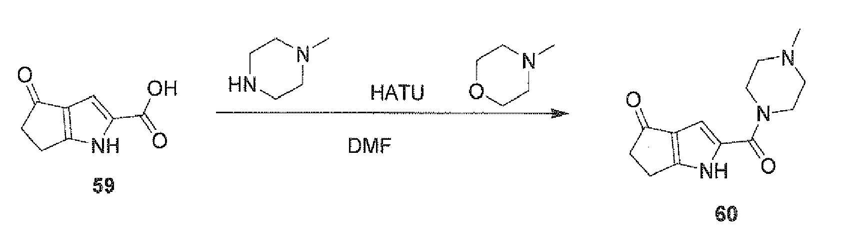

Compound 59 (100 mg, 0.606 mmol) was dissolved in DMF (2 mL), N-methylpiperazine (0.14 mL, 1.21 mmol), HATU(253 mg, 0.666 mmol) and N-methylmorpholine (0.13 mL, 1.21 mmol) were added to the solution, and the resulting mixture was stirred at room temperature for 24 hours. Water was added to the reaction mixture and extracted with chloroform. The organic layer was washed with saturated brine, dried over anhydrous magnesium sulfate, and the solvent was removed under reduced pressure. The obtained residue was purified by silicagel column chromatography (chloroform-methanol) to give Compound 60 (102.7 mg, 69 %).
¹H-NMR(DMSO-d₆ / TMS) δppm: 12.0 (s, 1H), 6.50 (s, 1H), 3.66 (m, 4H), 2.89-2.85 (m, 2H), 2.77-2.73 (m, 2H), 2.32 (t, J = 5.1Hz, 4H), 2.19 (s, 3H).

### 3rd step

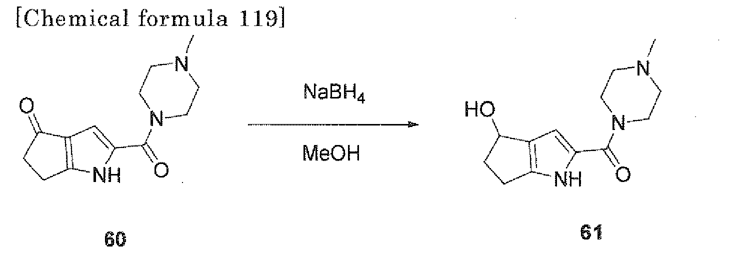

Compound 60 (40 mg, 0.162 mmol) was dissolved in methanol (2 mL), sodium borohydride (12.2 mg, 0.324 mmol) was added to the solution, and the resulting mixture was stirred at 0 °C for 1 hour. Water was added to the mixture and extracted with chloroform. The organic layer was washed with saturated brine, dried over anhydrous magnesium sulfate, and the solvent was removed under reduced pressure. The obtained residue was purified by silicagel column chromatography (chloroform-methanol) to give Compound 61 (36.8 mg, 91 %).
¹H-NMR(DMSO-d₆ / TMS) δppm: 11.1 (s, 1H), 6.27 (s, 1H), 4.88-4.85 (m, 1H), 4.71 (d, J = 6Hz, 1H), 3.66 (t, J = 5.1Hz, 4H), 2.75-2.56 (m, 2H), 2.46-2.41 (m, 1H), 2.31 (t, J = 5.1Hz, 4H), 2.19 (s, 3H), 2.09-2.02 (m, 1H).

### [Example 21]

### Preparation of 1-051 (Compound 66)

### 1st step

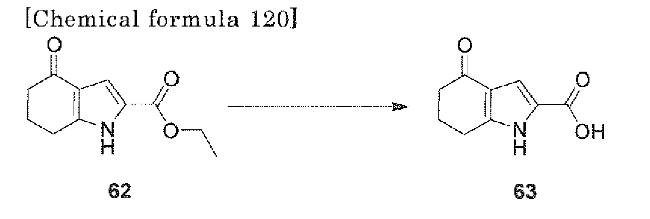

Compound 62 was prepared by the method described in Synthetic Commun.,1995, 25, 507.and Synthetic Commun.,2003,33,3461. Compound 62 (400 mg, 1.93 mmol) was dissolved in THF/MeOH = 2:1 (8.4 mL), 2mol/L lithium hydroxide aqueous solution (5.79 mL, 11.58 mmol) was added to the solution, and the resulting mixture was stirred at 60 °C for 3 hours. The solvent was removed under reduced pressure, water was added to the residue, extracted with diethyl ether and the organic layer was washed with water. Hydrochloric acid was added to the combined aqueous layer, and the pH of the mixture was adjusted to approximately 3 to precipitate colorless solid. The obtained solid was collected by filtration, washed with water and dried to give Compound 63 (334.5 mg, Yield 97 %).
¹H-NMR (DMSO-d₆ / TMS) δppm: 2.01 (quintet, J = 6.2 Hz, 2H), 2.35 (t, J = 6.2 Hz, 2H), 2.77 (t, J = 6.2 Hz, 2H), 6.85 (d, J = 2.3 Hz, 1H), 12.15 (s, 1H), 12.62 (bs, 1H)

### 2nd step

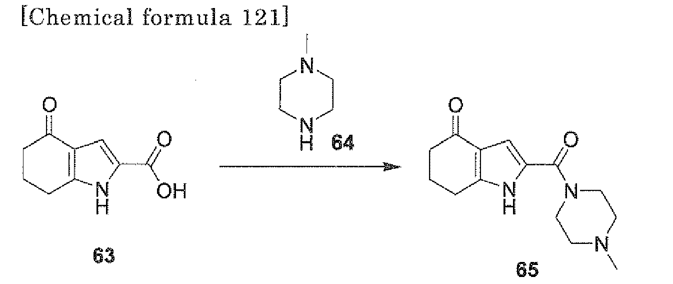

Compound 63(75mg, 0.49 mmol) and HATU (175 mg, 0.46 mmol) were dissolved in DMF (4 mL), Compound 64 (47 µL, 0.49 mmol) and NMM (69µL, 0.63 mmol) were added to the solution, and the resulting mixture was stirred at room temperature for 24 hours. The reaction mixture was diluted with water and extracted with chloroform. The organic layer was washed with saturated brine, dried over sodium sulfate, and the solvent was removed under reduced pressure. The obtained residue was purified by amino column chromatography (chloroform-methanol) to give Compound 65 (63.1 g, Yield 58 %).
¹H-NMR (DMSO-d₆ / TMS) δppm: 2.04 (t, J = 6.0 Hz, 2H), 2.22 (s, 3H), 2.36 (m, 6H), 2.79 (t, J = 6.0 Hz, 2H), 3.98 (s, 4H), 6.61 (s, 1H), 11.91 (s, 1H)

### 3rd step

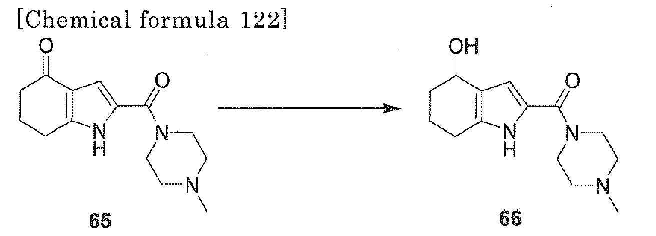

Compound 65 (63mg, 0.24 mmol) was dissolved in methanol (1.7 mL) and cooled under ice cooling bath. NaBH₄ (13.7 mg, 0.36 mmol) was added to the solution, and the resulting mixture was stirred at 0 °C for 2 hours. The reaction mixture was diluted with water and extracted with chloroform. The organic layer was washed with saturated brine, dried over sodium sulfate, and the solvent was removed under reduced pressure to give Compound 66 (41 mg, Yield 65 %).
¹H-NMR (DMSO-d₆ / TMS) δppm: 1.57-1.60 (m, 2H), 1.75-1.85 (m, 2H), 2.19 (s, 3H), 2.30 (t, J = 5.0 Hz, 4H), 2.41-2.45 (m, 2H), 3.66 (t, J = 5.0 Hz, 4H), 4.50 (t, J = 5.5 Hz, 1H), 4.58 (d, J = 5.5 Hz, 1H), 6.34 (s, 1H), 1.0.95 (s, 1H)

### [Example 22]

### Preparation of 1-052 (Compound 75)

### 1st step

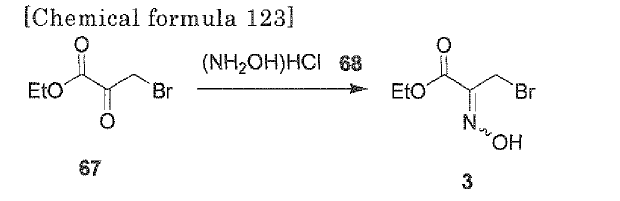

Compound 68 (3.56 g, 51.3 mmol) was dissolved in chloroform (30 mL) and water (30 mL), a compound 67(10 g, 51.3 mmol) was added dropwise to the solution, and the resulting mixture was stirred at room temperature for 18 hours. The solvent was removed under reduced pressure, and the obtained colorless solid was collected by filtration, washed with hexane and dried to give Compound 3 (9.67 g, Yield 90 %). ¹H-NMR (DMSO-d₆ / TMS) δppm: 1.25 (t, J = 7.7 Hz, 3H), 4.20-4.36 (m, 4H), 13.18 (s, 1H)

### 2nd step

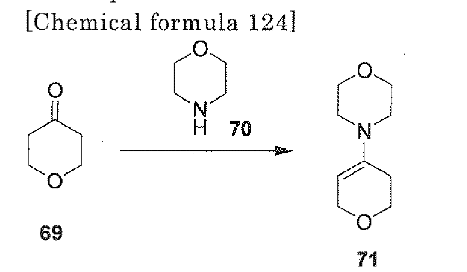

Compound 69 (1 g, 10 mmol) was dissolved in toluene (20 mL), Compound 70(1.32ml, 15 mmol) was added to the solution, and the resulting mixture was refluxed for 7 hours attached with Dean-Stark trap. After cooling to room temperature, the solvent was removed under reduced pressure to give Compound 71 (1.69 g, >99 %).
¹H-NMR (DMSO-d₆ / TMS) δppm: 2.08 (m, 2H), 2.72 (t, J = 4.8 Hz, 4H), 3.61(t, J = 4.8 Hz, 4H), 3.68 (t, J = 5.5 Hz, 4H), 4.08 (d, J = 2.2 Hz, 2H), 4.53 (s, 1H)

### 3rd step

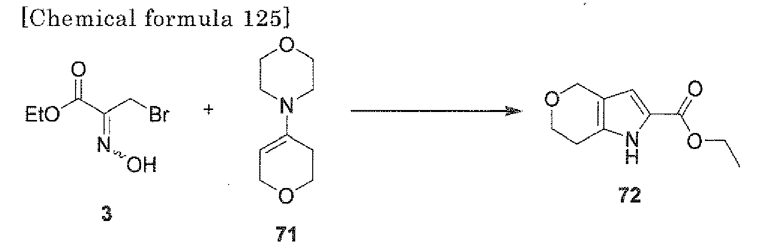

Compound 3 (525 mg, 2.5 mmol) and Compound 71 (846 mg, 5 mmol) were dissolved in toluene (26 mL) and the resulting solution was stirred at room temperature for 3 hours. Fe₃(CO)₁₂ (1.9 g, 3.75 mmol) and TFA(0.58 ml, 7.5 mL) were added to the solution, and the resulting mixture was refluxed for 3 hours. After cooling to room temperature, the reaction mixture was filtered through Celite pad to remove solid, and the solvent was removed reduced pressure. The obtained residue was purified by silicagel column chromatography (hexane - ethyl acetate) to give Compound 72 (128 mg, 26 %).
¹H-NMR (DMSO-d₆ / TMS) δppm: 1.25 (t, J = 6.0 Hz, 3H), 2.62 (t, J = 6.0 Hz, 2H), 3.80 (t, J = 6.0 Hz, 2H), 4.19 (q, J = 6.0 Hz, 2H), 4.50 (s, 2H), 6.50 (s, 1H), 11.56 (bs. 1H)

### 4th step

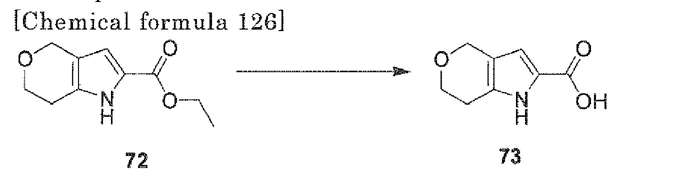

Compound 72 (128 mg, 0.66 mmol) was dissolved in THF/MeOH = 2:1 (8.4 mL), 2mol/L lithium hydroxide aqueous solution (1.97 mL, 3.93 mmol) was added to the solution, and the resulting mixture was stirred at 60 °C for 3 hours. The solvent was removed under reduced pressure, water was added to the residue, extracted with diethyl ether and the organic layer was washed with water. Hydrochloric acid was added to the aqueous layer, and the pH of the mixture was adjusted to approximately 3 to precipitate red solid. The obtained solid was collected by filtration, washed with water and dried to give Compound 73 (43.8 mg, Yield 40 %).
¹H-NMR (DMSO-d₆ / TMS) δppm: 2.61 (t, J = 6.0 Hz, 2H), 3.80 (t, J = 6.0 Hz, 2H), 4.50 (s, 2H), 6.46 (s, 1H), 11.43 (s, 1H), 12.88 (bs, 1H)

### 5th setep

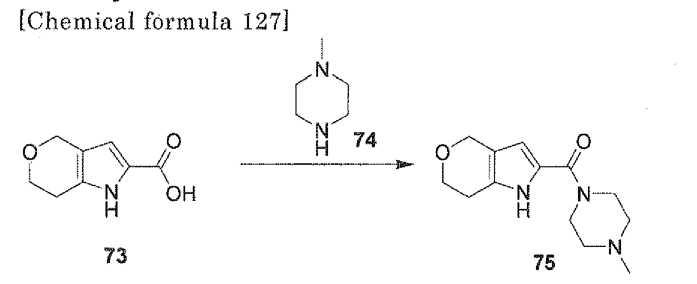

Compound 73 (270 mg, 1.62 mmol) and HATU (676 mg, 1.78 mmol) were dissolved in DMF (27 mL), Compound 74(0.18 mL, 1.62 mmol) and NMM (0.27 mL, 2.42 mmol) were added to the solution, and the resulting mixture was stirred at room temperature for 24 hours. The reaction mixture was diluted with water and extracted with chloroform. The organic layer was washed with saturated brine, dried over sodium sulfate, and the solvent was removed under reduced pressure. The obtained residue was purified by amino column chromatography (chloroform-methanol) to give Compound 75(132 mg, Yield 33 %).
¹H-NMR (DMSO-d₆ / TMS) δppm: 1.08 ( d, J = 6.7 Hz, 3H), 1.71-1.79 (m, 1H), 2.08-2.15 (m, 2H), 2.20 (s, 3H), 2.33 (t, J = 4.9 Hz, 2H), 2.37-2.44 (m, 4H), 2.77-2.81 (m, 2H), 3.66 (t, J = 4.3 Hz, 4H), 6.60 (d, J = 2.4 Hz, 1H), 11.87 (bs, 1H)

### [Example 23]

### Preparation of 1-053 (Compound 83)

### 1st step

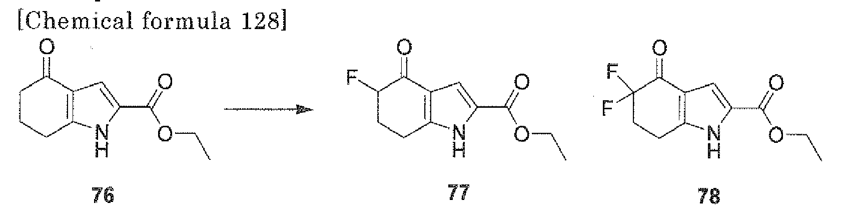

Diisopropylamine (0.55 mL, 3.86 mmol) was dissolved in THF (4 mL), and the solution was cooled under acetone-dry ice bath to -78 °C. n-BuLi (1.6M in hexane solution, 2.4 mL, 3.86 mmol) was added dropwise to the solution, and the resulting mixture was stirred at -78°C for 30 minutes. A compound 76 in THF/Et₂ O = 5:1(15 mL) was added dropwise to the mixture, and the resulting mixture was stirred at -78 °C for 30 minutes and at -40 °C for 1hour. After cooling to -78°C, NFSI(670 mg, 2.1 mmol) in THF(1.5 mL) was added dropwise to the mixture, and the resulting mixture was stirred at -78 °C for 2hours and at room temperature for 4 hours. The reaction mixture was diluted with water, 0.5mol/L hydrochloric acid aqueous solution was added to the mixture and the pH of the mixture was adjusted to 2-3. The mixture was extracted with ethyl acetate, and the organic layer was washed with saturated brine, dried with sodium sulfate, and the solvent was removed under reduced pressure. The obtained residue was purified by silicagel column chromatography (hexane - ethyl acetate) to give Compound 77 (42 mg, Yield 20 %) and Compound 78 (49.2 mg, Yield 21 %).
Compound 77
¹H-NMR (DMSO-d₆ / TMS) δppm: 1.28 (t, J = 7.1Hz, 3H), 2.19-2.44 (m, 2H), 2.93-2.97 (m, 2H), 4.26 (q, J = 7.1 Hz, 2H), 5.20 (dd, J = 11.5, 4.8 Hz, 1H), 6.95 (s, 1H), 12.44 (s, 1H)
Compound 78
¹H-NMR (DMSO-d₆ / TMS) δppm: 1.28 (t, J = 7.1Hz, 3H), 2.55-2.63 (m, 2H), 2.98 (t, J = 6.2 Hz, 2H), 4.25 (q, J = 7.1 Hz, 2H), 7.02 (s, 1H)

### 2nd step

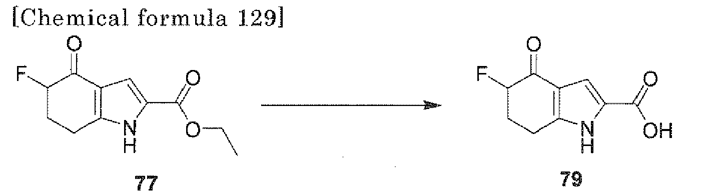

Compound 77 (40 mg, 0.18 mmol) was dissolved in THF/MeOH = 2:1 (1 mL), 2mol/L lithium hydroxide aqueous solution (0.53 mL, 1.01 mmol) was added to the solution, and the resulting mixture was stirred at 60 °C for 3 hours. The solvent was removed under reduced pressure, water was added to the residue, extracted with diethyl ether, and the organic layer was washed with water. Hydrochloric acid was added to the aqueous layer and the pH of the mixture was adjusted to approximately 3, and extracted with chloroform. The organic layer was dried over sodium sulfate, and the solvent was removed under reduced pressure to give Compound 79 (14.8 mg, Yield 42 %).
¹H - NMR (DMSO-d₆ / TMS) δppm: 2.19-2.44 (m, 2H), 2.93-2.97 (m, 2H), 5.12 (dd, J = 11.5, 4.8 Hz, 1H), 5.28 (dd, J = 11.5, 4.8 Hz, 1H), 6.95 (s, 1H), 12.44 (bs, 1H)

### 3rd step

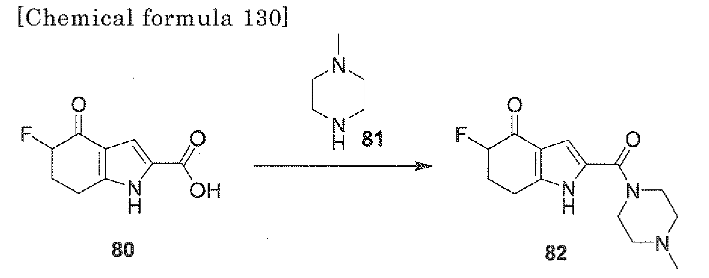

Compound 80 (14 mg, 0.07 mmol) and HATU(59.4 mg, 0.16 mmol) were dissolved in DMF (1 mL), Compound 81 (16 µL, 0.14 mmol) and NMM(23 µL, 0.21 mmol) were added to the solution, and the resulting mixture was stirred at room temperature for 24 hours. The reaction mixture was diluted with water and extracted with chloroform. The organic layer was washed with saturated brine, dried over sodium sulfate, and the solvent was removed under reduced pressure. The obtained residue was purified by amino column chromatography (chloroform - methanol) to give Compound 82 (8 mg, Yield 40 %).
LCMS (method B) retention time: 0.31 min, MS[M-H] = 278

### 4th step

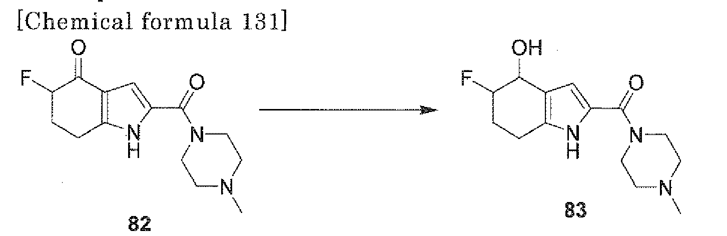

Compound 82 (7 mg, 0.025 mmol) was dissolved in methanol (1 mL) and the solution was cooled under ice cooling bath. NaBH₄ (1.4 mg, 0.038 mmol) was added to the solution, and the resulting mixture was stirred at 0 °C for 2 hours. The reaction mixture was diluted with water and extracted with chloroform. The organic layer was washed with saturated brine, dried over sodium sulfate, and the solvent was removed under reduced pressure to give Compound 83 (7 mg, Yield>99 %).
LCMS (method B) retention time: 0.35 min, MS[M+H]⁺: 282

### [Example 24]

### Preparation of 1-054 (Compound 87)

### 1st step

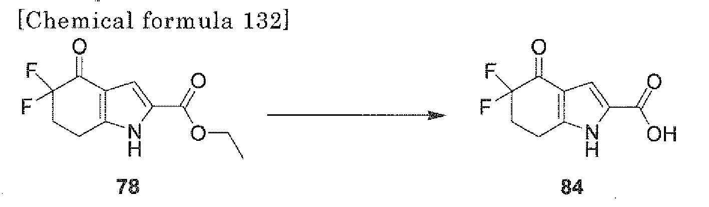

Compound 78 (49 mg, 0.20 mmol) was dissolved in THF/MeOH =2:1 (1 mL), 2mol/L lithium hydroxide aqueous solution (0.6 mL, 1.21 mmol) was added to the solution, and the resulting mixture was stirred at 60 °C for 3 hours. The solvent was removed under reduced pressure, water was added to the residue, extracted with diethyl ether, and the organic lawyer was washed with water. Hydrochloric acid was added to the aqueous layer and the pH of the mixture was adjusted to approximately 3 to give colorless solid. The obtained solid was collected by filtration, washed with water and dried to give Compound 84 (28.6 mg, Yield 66 %).
¹H-NMR (DMSO-d₆ / TMS) δppm: 2.49-2.63 (m, 2H), 2.99 (t, J = 6.1 Hz, 2H), 6.98 (s, 1H), 12.58 (bs, 1H)

### 4th step

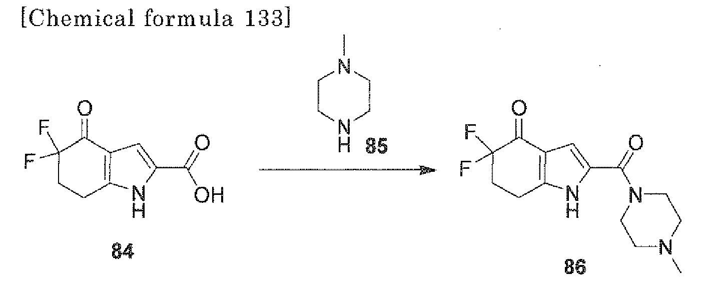

Compound 84(26 mg, 0.12 mmol) and HATU (101 mg, 0.27 mmol) were dissolved in DMF (1 mL), Compound 85(27 µL, 0.24 mmol) and NMM (40 µL, 0.36 mmol) were added to the solution, and the resulting mixture was stirred at room temperature for 24 hours. The reaction mixture was diluted with water and extracted with chloroform. The organic layer was washed with saturated brine, dried over sodium sulfate, and the solvent was removed under reduced pressure. The obtained residue was purified by amino column chromatography (chloroform - methanol) to give Compound 86(29.5 mg, Yield 82 %).
LCMS (method B) retention time: 0.52 min, MS[M+H]⁺: 298

### 5th step

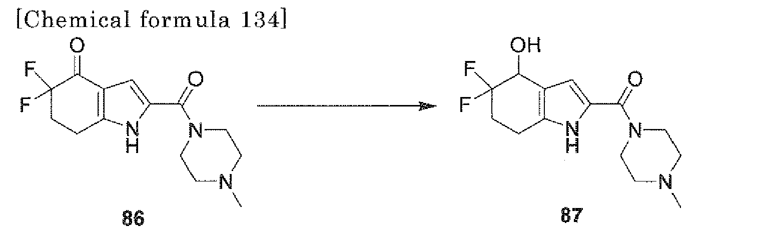

Compound 86 (29 mg, 0.01 mmol) was dissolved in methanol (1 mL) and the solution was cooled under ice cooling bath. NaBH₄ (5.5 mg, 0.15 mmol) was added to the solution, and the resulting mixture was stirred at 0 °C for 2 hours. The reaction mixture was diluted with water and extracted with chloroform. The organic layer was washed with saturated brine, dried over sodium sulfate, the solvent was removed under reduced pressure to give Compound 87 (26.3 mg, Yield 90 %).
¹H-NMR (DMSO-d₆ / TMS) δppm:2.19 (s, 3H), 2.31 (t, J = 4.9 Hz, 4H), 2.60-2.77 (m, 3H), 3.66 (t. J = 4.9 Hz, 4H), 4.0 (dd, J = 15.1, 7.9 Hz, 1H), 5.55 (d, J = 6.6 Hz, 1H), 6.37 (s, 1H), 11.23 (bs, 1H)

### [Example 25]

### Preparation of 1-005 (Compound 90)

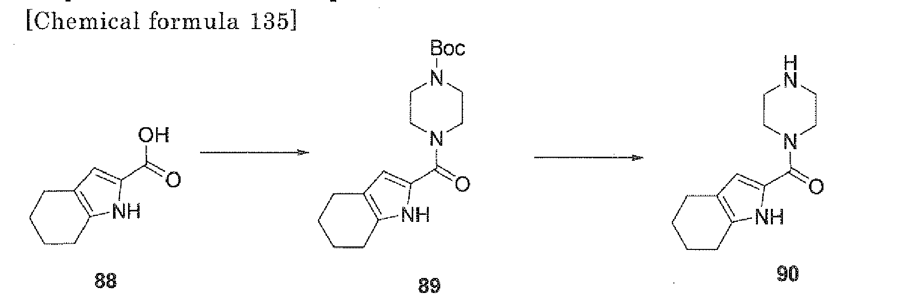

### 1st step

Compound 88 (150 mg, 0.91 mmol) and tert-butyl 1-piperazine carboxylate (507 mg, 2.72 mmol) were dissolved in DMF (6.0 mL), HATU (518 mg, 1.36 mmol) and N-methylmorpholine (0.35 mL, 3.18 mmol) were added to the solution, and the resulting mixture was stirred at room temperature for 3 hours. Water was added to the mixture and extracted with ethyl acetate. The organic layer was washed with water and saturated brine successively, dried over anhydrous magnesium sulfate, and the solvent was removed under reduced pressure. The obtained residue was purified by silicagel column chromatography (hexane - ethyl acetate) to give Compound 89 (233 mg, Yield 77 %).
¹H-NMR (CDCl₃ / TMS) δppm: 1.48 (9H, s), 1.75-1.81 (4H, m), 2.50 (2H, t, J = 6.2 Hz), 2.60 (2H, t, J = 6.2 Hz), 3.48-3.51 (4H, m), 3.78-3.81 (4H, m), 6.26 (1H, d, J = 2.0 Hz), 8.85 (1H, brs).

### 2nd step

Compound 89(230 mg, 0.69 mmol) was dissolved in chloroform (2.3 mL), trifluoroacetic acid (2.3 mL) was added to the solution under ice cooling bath. The resulting mixture was stirred at room temperature for 2 hours and water was added to the mixture. The mixture was neutralized with sodium bicarbonate and extracted with chloroform. The organic layer was washed with saturated brine, dried over anhydrous magnesium sulfate, and the solvent was removed under reduced pressure to give Compound 90 (120 mg, Yield 75 %).
¹H-NMR (CDCl₃ / TMS) δppm: 1.73-1.82 (4H, m), 2.50 (2H, t, J = 5.9 Hz), 2.60 (2H, t, J = 5.9 Hz), 2.91 (4H, t, J = 5.1 Hz), 3.79 (4H, t, J = 5.1 Hz), 6.26 (1H, d, J = 2.4 Hz), 8.89 (1H, brs).

### [Example 26]

### Preparation of 1-007 (Compound 93)

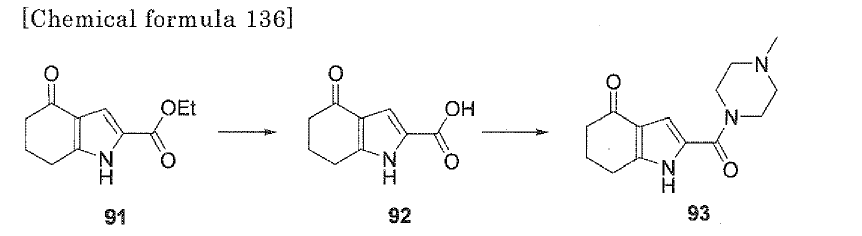

### 1st step

Compound 91 (500 mg, 2.41 mmol) was dissolved in methanol (1.0 mL) and THF (2.0 mL), 5mol/L sodium hydroxide aqueous solution (4.8 mL) was added to the solution, and the resulting mixture was stirred at room temperature for 3 hours. The reaction mixture was neutralized with hydrochloric acid and extracted with chloroform. The organic layer was washed with saturated brine, dried over anhydrous magnesium sulfate. The solvent was removed under reduced pressure to give Compound 92 (414 mg, Yield 96 %).
¹H-NMR(DMSO-d₆ / TMS) δppm: 2.00-2.07 (2H, m), 2.37 (2H, t, J = 6.5 Hz), 2.80 (2H, t, J = 6.2 Hz), 6.88 (1H, d, J = 2.0 Hz), 12.18 (1H, brs).

### 2nd step

Compound 92 (410 mg, 0.69 mmol) was dissolved in DMF (8.2 mL), N-methylpiperazine (0.38 mL, 3.43 mmol), HATU (957 mg, 2.52 mmol) and N-methylmorpholine (0.63 mL, 5.72 mmol) were added to the solution, and the resulting mixture was stirred at room temperature for 3 hours. Water was added to the reaction mixture and extracted with chloroform. The organic layer was washed with saturated brine, dried over anhydrous magnesium sulfate, and the solvent was removed under reduced pressure. The residue was purified by amino silicagel column chromatography (chloroform - methanol) to give Compound 93 (439 mg, Yield 73 %).
¹H-NMR (CDCl₃ / TMS) δppm: 2.15-2.20 (2H, m), 2.33 (3H, s), 2.46 (4H, t, J = 5.2 Hz), 2.49-2.52 (2H, m), 2.84 (2H, t, J = 6.1 Hz), 3.82-3.86 (4H, m), 6.84 (1H, d, J = 2.4 Hz), 9.65 (1H, brs).

### [Example 27]

### Preparation of I-009 (Compound 98)

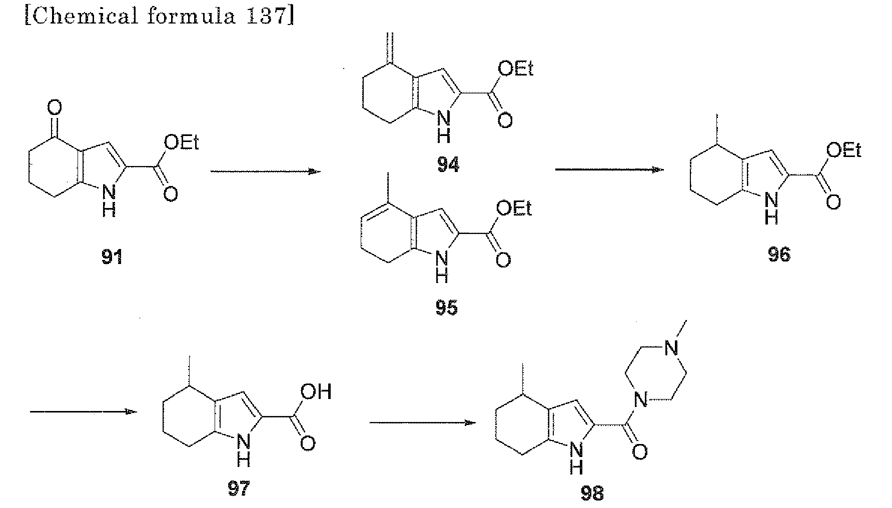

### 1st step

Methyltriphenylphosphonium iodide (585 mg, 1.45 mmol), sodium pentoxide (159 mg, 1.45 mmol) were dissolved in toluene (1.0 mL) and the solution was stirred at room temperature for 30 minutes. Compound 91 (500 mg, 2.41 mmol) in THF (1.0 mL) was added to the solution, and the resulting mixture was refluxed at 110 °C for 3 hours, then cooled to room temperature. Then, the reaction mixture was stirred for 3 hours. Water was added to the reaction mixture and extracted with ethyl acetate, and the organic layer was washed with saturated brine, dried over anhydrous magnesium sulfate. The solvent was removed under reduced pressure and the residue was purified by silicagel column chromatography (hexane - ethyl acetate) to give a mixture of Compound 94 and 95 (30 mg, Yield 30 %).
¹H-NMR (CDCl₃ / TMS) δppm: 1.35 (3H, t, J = 7.1 Hz), 1.88-1.95 (2.5H, m), 2.37-2.46 (2H, m), 2.68-2.73 (2H, m), 4.29-4.33 (2H, m), 4.71 (0.5H, d, J = 1.6 Hz), 5.09 (0.5H, d, J = 1.6 Hz), 5.33-5.36 (0.5H, m), 6.77 (0.5H, d, J = 2.4 Hz), 7.00 (0.5H, d, J = 2.4 Hz), 8.60-8.95 (1H, m).

### 2nd step

The mixture of Compound 94 and 95 (90 mg, 0.44 mmol) was dissolved in methanol (1.8 mL), 5 % palladium -carbon (18 mg) was added to the solution. The atmosphere within the reaction system was replaced with hydrogen gas and the mixture was stirred at room temperature overnight. The reaction mixture was filtered through Celite pad and the filtrate was concentrated *in vacuo.* The obtained residue was purified by silicagel column chromatography (hexane - ethyl acetate) to give Compound 96 (46 mg, Yield 50 %).
¹H-NMR (CDCl₃ / TMS) δppm: 1. 19 (3H, d, J = 6.9 Hz), 1.25-1.28 (1H, m), 1.34 (3H, t, J = 7.1 Hz), 1.69-1.74 (1H, m), 1.88-1.97 (2H, m), 2.57-2.60 (2H, m), 2.66-2.71 (1H, m), 4.29 (2H, q, J = 7.1 Hz), 6.75 (1H, d, J = 2.7 Hz), 8.58 (1H, brs).

### 3rd step

Compound 97 was prepared from Compound 96 according to the Example 26,1st step (Yield 79 %).

### 4th step

Compound 98 was prepared from Compound 97 according to the Example 26, 2nd step (Yield 72 %).
¹H -NMR (CDCl₃ / TMS) δppm: 1.17 (1.5H, s), 1.19 (1.5H, s), 1.26-1.32 (1H, m), 1.67-1.74 (1H, m), 1.89-1.94 (2H, m), 2.33 (3H, s), 2.45 (4H, t, J = 5.0 Hz), 2.57-2.59 (2H, m), 2.70-2.72 (1H, m), 3.85 (4H, t, J = 5.0 Hz), 6.32 (1H, d, J = 2.5 Hz), 9.08 (1H, brs).

### [Example 28]

### Preparation of 1-076

### 1st step

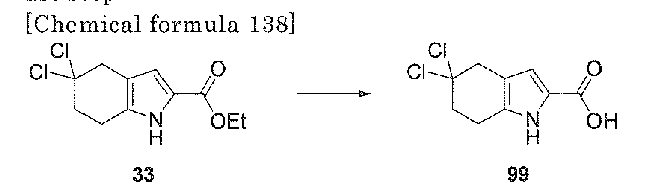

To a solution of Compound 33 (480 mg, 1.83 mmol) in ethanol (5 mL) and tetrahydrofuran (10 mL) was added 1.2 mol/L sodium hydroxide aqueous solution (6 mL), and the resuting mixture was stirred at 60 °C for 5 hours. The reaction mixture was cooled to room temperature, ethanol and tetrahydrofuran was removed under reduced pressure, and the aqueous layer was washed with diethyl ether. The pH of the aqueous layer was adjusted to 2 with 2 mol/L hydrochloric acid aqueous solution. The resulting precipitate was collected by filtration, washed with water, dried *in vacuo* to give a compound 99 (391 mg, 1.67 mmol, 91 %). Compound 99 was used for the next step without further purification.
¹H NMR (DMSO-d6) δ: 2.59 (t, J = 6.2 Hz, 2H), 2.75 (t, J = 6.2 Hz, 2H), 3.43 (s, 2H), 6.47 (d, J = 2.4 Hz, 1H), 11.45 (br, 1H), 12.06 (br, 1H).

### 2nd step

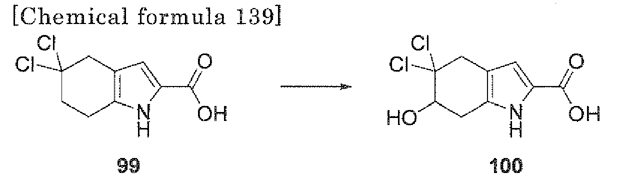

Homogeneous hyphal suspensions of fungal strain, Metarhizium anisopliae RF-5943, were prepared by scratching the surface of 7 agar plate cultures by sterile spatulas adding 5mL of sterile distil water. Seed cultures were prepared by inoculating each suspensions to 7 flasks containing of sterile GSY medium (glucose 2%, soybean meal 0.5%, yeast extract 0.5%, NaCl 0.5%, K₂HPO₄ 0.5%, 100ml tap water, pH7.0, in 500mL conical flask, autoclaving) and incubating 2days on the rotary shaker (28 °C, 200rpm).
Bioconversion reactions were performed as follows. 60 conical flasks (500mL) containing production medium (25g of Brown rice and 50mL of GSY liquid medium) were autoclaved. After cooling, 500µL of compound 99 (300mg, 1.28mmol, DMSO solution (300mg/30ml)) were added homogenously to the each flask. 10mL of seed cultures were inoculated spirally to each flask. These flasks were incubated stationary (28 °C, 6days).
All broth cultures were collected, poured into ethyl acetate (6L), adjusted to pH3 by 5mmol/L HCl, and stirred for 1hour. The organic layer was obtained by decantation. A residual mixture was poured into ethyl acetate (6L), left over night, and then the organic layer was obtained by decantation. Organic layers were combined, evaporated to 300mL, washed with water, and evaporated to give a crude residue. Purification by ODS column (Chromatorex-ODS, 200-300 mesh, 50ϕx100mm, eluent : 0.1 %formic acid-water/0.1% formic acid-acetonitrile) and C30 column (Develosil C30-UG-10, 50ϕx100mm, eluent : 0.1%formic acid-water/0.1%formic acid-acetonitrile) gave Compound 100 (75mg, 0.3mmol, 23%).
The above strain "Metarhizium anisopliae RF-5943" was deposited internationally on 2010/July/20 as "Metarhizium anisopliae RF-5943 (identify code)" with The National Institute of Advanced Industrial Science and Technology (AIST), International Patent Organism Depositary (IPOD) located AIST Tsukuba Central 6, 1-1-1 Higashi, Tsukuba, Ibaraki 305-8566, Japan. The deposit number was FERM ABP-11270, and accession number was PERM BP-11270.
¹H NMR (DMSO-d6) δ : 2.63 (dd, J = 6.5, 16.9 Hz, 1H), 3.00 (dd, J = 5.2, 16.9 Hz, 1H), 3.38 (d, J = 16.5 Hz, 1H), 3.48 (d, J = 16.5 Hz, 1H), 4.13 (m, 1H), 6.11 (d, J = 5.7 Hz, 1H), 6.46 (d, J = 2.4 Hz, 1H), 11.4 (br, 1H), 12.1 (br, 1H).

### 3rd step

Compound 1-076 was prepared from Compound 100 according to the Example 26, 2nd step. The physical property was shown in Table 19.

### [Example 29]

### Preparation of 1-074 (Compound 109)

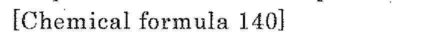

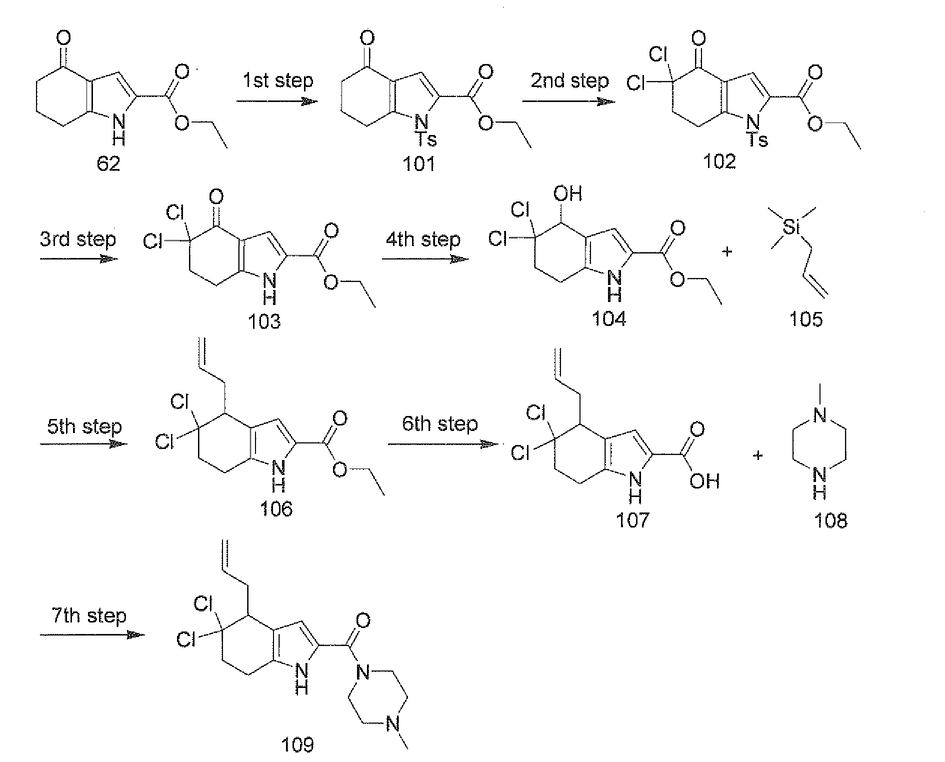

### 1st step

Compound 62 (1.2 g, 5.79 mmol) in DMF (50 mL) was cooled to 0 °C, sodium hydride (301 mg, 7.53 mmol) was added to the solution, and the resulting mixture was stirred at 0 °C for 30 minutes. Tosyl chloride (2.21 g, 11.58 mmol) was added to the mixture, and the resulting mixture was stirred for 18 hours. Water was added to the mixture, extracted with ethyl acetate, washed with water, dried over magnesium sulfate and concentrated *in vacuo.* The residue was purified by silicagel column chromatography eluting with hexane/ethyl acetate=4:1. The eluate was collected and concentrated *in vacuo* to give Compound 101 (1.69 g, Yield 81 %) as colorless solid.
¹H-NMR (DMSO-d₆) δ: 8.03 (d, J = 8.39 Hz, 2H), 7.53 (d, J = 8.08 Hz, 2H), 7.05 (s, 1H), 4.21 (q, J = 7.12 Hz, 2H), 3.17 (t, J = 5.95 Hz, 2H), 2.47-2.45 (m, 2H), 2.44 (s, 3H), 2.14-2.05 (m, 2H), 1.23 (t, J = 7.09 Hz, 3H).

### 2nd step

To a solution of Compound 101(1.69 g, 4.68 mmol) in acetic acid (4 mL)/water (4 mL) was added copper chloride (II) (2.51 g, 18.7 mmol), and the resulting mixture was refluxed for 15 hours. Additional copper chloride (2 g, 4.68 mmol) was added to the mixture, and the resulting mixture was refluxed for 2 hours. Water was added to the mixture, extracted with ethyl acetate, washed with saturated brine, dried over sodium sulfate and concentrated *in vacuo* to give Compound 102. The residue was purified by silicagel column chromatography eluting with hexane/ethyl acetate=10:1. The eluate was collected and concentrated *in vacuo* to give a colorless solid (1.54 g, Yield 76 %).
¹H-NMR (DMSO-d6) δ: 1.23 (t, J = 7.1 Hz, 3H), 2.45 (s, 3H), 3.11 (t, J = 5.8 Hz, 2H), 3.38 (t, J = 6.0 Hz, 2H), 4.23 (q, J = 7.1 Hz, 2H), 7.24 (s, 1H), 7.55 (d, J = 8.5 Hz, 2H), 8.06 (d, J = 8.5 Hz, 2H).

### 3rd step

To a solution of Compound 102(100 mg, 0.232 mmol) in ethanol (3 mL) was added sodium ethoxide (19 mg, 0.279 mmol), and the resulting mixture was stirred at 0 °C for 1 hour. Water was added to the mixture, extracted with ethyl acetate, washed with saturated brine, dried over sodium sulfate and concentrated *in vacuo* to give Compound 103. The obtained product was used for the next step without further purification.
¹H NMR (DMSO-d₆) δ: 1.29 (t, J = 7.1 Hz, 3H), 3.00 (s, 4H), 4.27 (q, J = 7.1 Hz, 2H), 7.05 (d, J = 0.3 Hz, 1H), 12.70 (bs, 1H).

### 4^{th} step

Compound 103 (46 mg, 0.167 mmol) in methanol (3 mL)/tetrahydrofuran (1 mL) was cooled to 0 °C, sodium borohydride (9.45 mg, 0.250 mmol) was added to the solution, and the resulting mixture was stirred at 0 °C for 1 hour. Water was added to the mixture, extracted with ethyl acetate, washed with saturated brine, dried over sodium sulfate and concentrated *in vacua* to give Compound 104. The obtained product was used for the next step without further purification.
¹H-NMR (DMSO-d₆) δ: 11.54 (s, 1H), 6.61 (d, J = 2.35 Hz, 1H), 5.98 (d, J = 7.22 Hz, 1H), 4.66 (d, J = 7.22 Hz, 1H), 4.15 (q, J = 6.99 Hz, 2H), 2.67-2.65 (m, 4H), 1.20 (t, J = 7.13 Hz, 3H).

### 5^{th} step

To a solution of Compound 104 (44 mg, 0.158 mmol) in methylene chloride (2mL) was added a compound 105(0.05 mL, 0.316 mmol). Then, titanium tetrachloride (0.026 mL, 0.237 mmol) was slowly added dropwise, and the resulting mixture was stirred at room temperature for 15 minutes. Water was added to the miture, extracted with methylene chloride, washed with saturated brine, dried over sodium sulfate and concentrated *in vacuo to* give Compound 106.
¹H-NMR (DMSO-d6) δ: 1.24 (td, J = 7.1, 1.4 Hz, 3H), 2.28-2.40 (m, 1H), 2.74 (tt, J = 16.4, 4.4 Hz, 4H), 2.90 (ddd, J = 15.0, 2.5, 1.4 Hz, 1H), 3.38-3.43 (m, 1H), 4.18 (qd, J = 7.1, 1.5 Hz, 2H), 5.13-5.25 (m, 2H), 6.05 (ddt, J = 15.5, 5.7, 1.8 Hz, 1H), 6.64 (s, 1H), 11.61 (bs, 1H).

### 6^{th} step

To a solution of Compound 106 (26 mg, 0.086 mmol) in methanol (0.5 mL)/tetrahydrofuran (1 mL) was added 2 mol/L lithium hydroxide aqueous solution (1.16 mL, 2.318 mmol), and the resulting mixture was stirred at 60 °C for 4 hours. The reaction mixture was concentrated and water (2 mL) was added to the residue, and extracted with diethyl ether. Concentrated hydrochloric acid was added to the aqueous layer and the pH of the mixture was adjusted to approximately 3, extracted with chloroform, dried over sodium sulfate and concentrated *in vacuo* to give Compound 107.
¹H-NMR (DMSO-d6) 8:2.28-2.39 (m, 1H), 2.73 (t, J = 4.4 Hz, 4H), 2.88-2.92 (m, 1H), 3.39-3.42 (m, 1H), 5.19 (dd, J = 21.5, 13.6 Hz, 2H), 6.04-6.06 (m, 1H), 6.61 (s, 1H), 11.47 (s, 1H), 12.07 (bs, 1H).

### 7th step

To a solution of Compound 107 (19 mg, 0.069 mmol) and Compound 108 (0.012 mL, 0.104 mmol) in DMF (2 mL) were added HATU (39.5 mg, 0.104 mmol) and 4-methylmorpholine (0.016 mL, 0.139 mmol), and the resulting mixture was stirred at room temperature for 24 hours. The reaction mixture was poured into water and extracted with ethyl acetate. The organic layer was washed with saturated brine, dried over anhydrous sodium sulfate and concentrated *in vacuo.* The residue was purified by silicagel column chromatography eluting with hexane/ethyl acetate=1:1, the eluate was collected and concentrated *in vacuo* to give Compound 109 (16.1 mg, Yield 65.5 %) as colorless solid.
¹H - NMR (DMSO-d6) δ: 2.19 (s, 3H), 2.30 (t, J = 4.8 Hz, 4H), 2.35-2.46 (m, 1H), 2.65-2.79 (m, 4H), 2.86-2.92 (m, 1H), 3.39-3.42 (m, 1H), 3.62 (t, J = 4.8 Hz, 4H), 5.11-5.25 (m, 2H), 5.18 (dt, J = 22.7, 9.6 Hz, 1H), 6.35 (d, J = 2.8 Hz, 1H), 11.22 (bs, 1H).

### [Example 30]

### Preparation of I-059 (Compound 110)

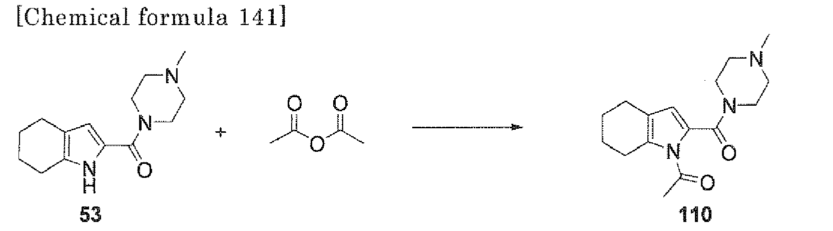

To a solution of Compound 53 (40 mg, 0.162 mmol) in dimethylformamide (2 mL) was added sodium hydride under ice cooling bath, and the resulting mixture was stirred at room temperature for 1 hour. Then, acetic acid anhydride (0.031 mL, 0.323 mmol) was added to the mixture, and the resulting mixture was stirred for 16 hours. The reaction mixture was poured into ice water and extracted with ethyl acetate. The organic layer was washed with water and saturated brine successively, dried over anhydrous sodium sulfate and concentrated *in vacuo.* The residue was purified by silicagel column chromatography eluting with chloroform/methanol=1/10 and the eluate was collected and concentrated *in vacuo* to give Compound 110 (18.2 mg, Yield 39 %) as yellow oil.
¹H NMR (DMSO-d6) δ: 1.62-1.68 (m, 4H), 2.08 (s, 3H), 2.18 (s, 3H), 2.29 (t, J = 4.5 Hz, 4H), 2.39-2.50 (m, 4H), 3.59 (t, J = 4.5 Hz 4H), 6.13 (s, 1H).

### [Example 31]

### Preparation of 1-060 (Compound 111)

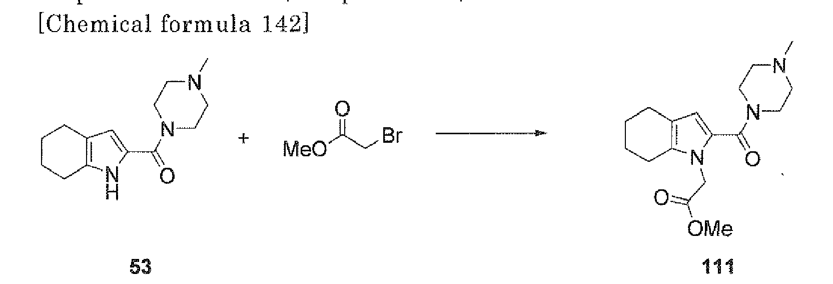

To a solution of Compound 53 (90.8 mg, 0.367 mmol) in dimethylformamide (2 mL) were added cesium carbonate (144 mg, 0.441 mmol) and 2-bromomethyl acetate (0.047 mL, 0.477 mmol) under ice cooling bath, and the resulting mixture was stirred at room temperature for 20 hours. The reaction mixture was poured into ice bath and extracted with ethyl acetate. The organic layer was washed with water and saturated brine successively, dried over anhydrous sodium sulfate and concentrated *in vacuo.* The residue was purified by silicagel column chromatography eluting with ethyl acetate 100 % and the eluate was collected and concentrated *in vacuo* to give Compound 111 (3.5 mg, Yield. 3 %) as colorless oil.
¹H NMR (DMSO-d6) δ: 1.64-1.69 (m, 4H), 2.08 (s, 3H), 2.31 (t, J = 4.5 Hz, 4H), 2.40-2.42 (m, 2H), 2.50-2.51 (m, 2H), 3.59 (t, J = 4.5 Hz 4H), 4.01 (s, 3H), 4.87 (s, 2H), 6.33 (s, 1H).

### [Example 32]

### Preparation of I-065

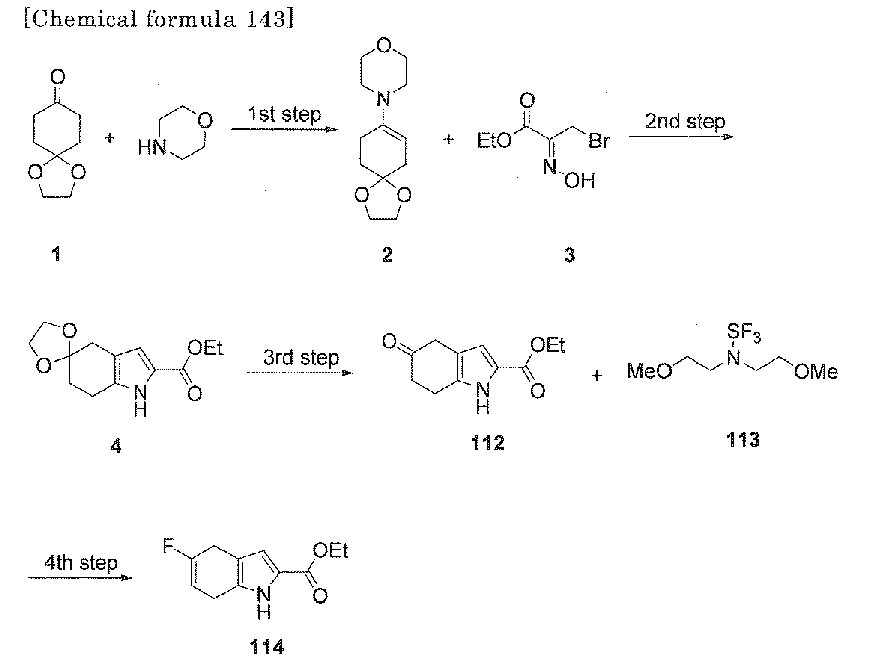

### 1st step

To a solution of Compound 1(17 g, 109 mmol) in toluene was added morpholine (14.2 mL, 163 mmol), and the resulting mixture was refluxed for 8 hours. The reaction mixture was concentrated *in vacuo.* The obtained Compound 2 was used for the next step without further purification.
¹H -NMR (CDCl3) δ: 1.81 (t, J = 6.6 Hz, 2H), 2.01 (t, J = 6.9 Hz, 2H), 2.30 (t, J = 6.3 Hz,, 4H), 2.51 (t, J = 6.9 Hz, 2H), 3.97-4.02 (m, 8H), 4.54 (t, J = 3.9 Hz, 1H).

### 2nd step

To a solution of Compound 2 (11.4 g, 50.5 mmol) in toluene (100 mL) was added (E)-ethyl 3-bromo-2-(hydroxyimino)propanoate (5.3 g, 25.2 mmol) at room temperature, and the resulting mixture was stirred for 3.5 hours. The reaction mixture was poured into ice bath, the organic layer was washed with saturated brine, dried over anhydrous magnesium sulfate and concentrated *in vacuo.* To a solution of the obtained residue in toluene (100 mL) were added Fe₃(CO)₁₂ (19.1 g, 37.9 mmol) and trifluoroacetic acid (5.8 mL, 76 mmol) at room temperature, and the resulting mixture was refluxed for 3 hours. The reaction mixture was cooled to room temperature, filtered through Celite pad and the filtrate was concentrated *in vacuo.* The residue was purified by silicagel column chromatography eluting with hexane/ethyl acetate= 1/5. The eluate was collected and concentrated *in vacuo* to give a pale yellow Compound 4 (1.52 g, Yield 24 %).
¹H NMR (DMSO-d6) δ: 1.24 (t, J = 6.9 Hz, 3H), 1.83 (t, J = 6.3 Hz, 2H), 2.49-2.63 (m, 4H), 3.90 (s, 4H), 4.18 (q, J = 6.9 Hz, 2H), 6.46 (d, J = 2.1 Hz, 1H), 11.4 (s, 1H).

### 3rd step

To a solution of Compound 4 (560 mg, 2.23 mmol) in acetone (12 mL) was added hydrochloric acid (1 mol/L, 2.2 mL, 2.23 mmol) at room temperature, and the resulting mixture was stirred at 50 °C for 2.5 hours. The reaction mixture was concentrated *in vacuo,* and the residue was neutralized with saturated sodium hydrogen carbonate aqueous solution and extracted with ethyl acetate. The organic layer was washed with saturated brine, dried over anhydrous sodium sulfate and concentrated *in vacuo.* The residue was purified by silicagel column chromatography eluting with hexane/ethyl acetate= 2/5. The eluate was collected and concentrated *in vacuo* to give a colorless Compound 112 (392 mg, Yield 85 %).
¹H NMR (DMSO-d6) δ: 1.25 (t, J = 6.9 Hz, 3H), 2.58 (t, J = 6.9 Hz, 2H), 2.92 (t, J = 6.9 Hz, 2H), 3.32 (s 2H), 4.20 (q, J = 6.9 Hz, 2H), 6.54 (d, J = 2.1 Hz, 1H), 11.6 (s, 1H).

### 4th step

To a solution of Compound 112 (50 mg, 0.241 mmol) in methylene chloride (3 mL) was added bis (2-methoxyethyl) aminosulfur trifluoride (113) (0.10 mL, 0.555 mmol) under ice cooling bath, and the resulting mixture was stirred at room temperature for 3 hours. The reaction mixture was poured into saturated sodium bicarbonate aqueous solution under ice cooling bath and extracted with ethyl acetate. The organic layer was washed with saturated brine, dried over anhydrous sodium sulfate and concentrated *in vacuo.* The residue was purified by silicagel column chromatography eluting with hexane/ethyl acetate= 1/5. The eluate was collected and concentrated *in vacuo* to give a colorless Compound 114 (8.6 mg, Yield 17 %)
¹H NMR (DMSO-d6) δ: 1.26 (t, J = 6.9 Hz, 3H), 3.26-3.33 (m, 4H), 4.20 (q, J = 6.9 Hz, 2H), 5.46 (d, J = 18.0 Hz, 1H), 6.59 (d, J = 2.1 Hz, 1H), 11.6 (s, 1H).

### 5th step

A compound I-065 was prepared from Compound 114 according to the Example 26,1st step and 2nd step. The physical property was shown in Table 17.

### [Example 33]

### Preparation of I-075 (Compound 124)

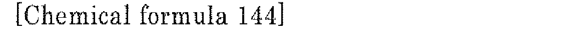

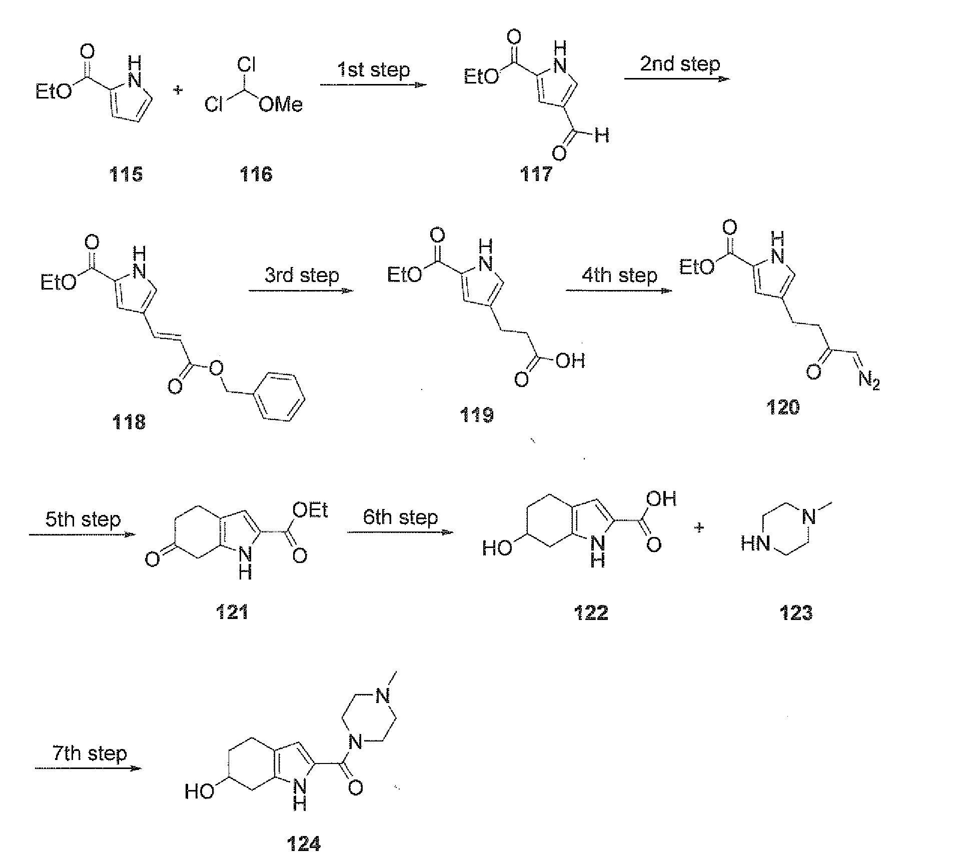

### 1st step

To a solution of Compound 115 (5 g, 35.9 mmol) and aluminum chloride (11.5 g, 86.0 mmol) in methylene chloride/nitromethane (1:1, 150 mL) was added dropwise dichloro methoxymethane (116)(3.8 mL, 43.1 mmol) at -20 °C over 30 minutes. The reaction mixture was stirred for 1.5 hours and poured to ice water and extracted with methylene chloride. The organic layer was washed with saturated brine, dried over anhydrous magnesium sulfate and concentrated *in vacuo.* The obtained brown residue was recrystallized from hexane/ethyl acetate to give a light brown Compound 117 (4.95 g, Yield 82 %).
¹H NMR (DMSO-d6) δ: 1.29 (t, J = 6.9 Hz, 3H), 4.27 (q, J = 6.9 Hz, 2H), 7.11 (d, J = 0.15 Hz, 1H), 7.81 (d, J = 1.8 Hz, 1H), 9.75 (s, 1H), 12.7 (brs, 1H).

### 2nd step

To a solution of Compound 117 (3 g, 18.0 mmol) in methylene chloride (70 mL) was added (benzyloxycarbonyl)triphenylphosphonium bromide (17.6 g, 35.9 mmol) under ice cooling bath, followed by addition of triethylamine (7.5 mL, 53.8 mmol) over 10 minutes. The reaction mixture was stirred at room temperature for 20 hours and concentrated *in vacuo.* The residue was dissolved in methylene chloride. Diethyl ether was added to the solution to precipitate solid. After collection of the solid by filtration, the mother liquor was concentrated *in vacuo* and the residue was purified by silicagel column chromatography eluting with hexane/ethyl acetate=1/1. The eluate was collected and concentrated *in vacuo* to give a yellow Compound 118 (3.71 g, Yield 69 %).
¹H NMR (DMSO-d6) δ: 1.28 (t, J = 6.9 Hz, 3H), 4.24 (q, J = 6.9 Hz, 2H), 5.17(s, 2H), 6.35 (dd, J = 0.6, 15.9 Hz, 1H), 7.18 (s, 1H), 7.31-7.39(m, 5H), 7.50 (s, 1H), 7.58 (d, J = 15.9 Hz, 1H).

### 3rd step

To a solution of Compound 118 (700 mg, 2.34 mmol) in ethyl acetate (15 mL) was added palladium/carbon (10 wt%, 249 mg, 0.234 mmol) at room temperature, and the resulting mixture was stirred for 4 hours under hydrogen gas atmosphere. The reaction mixture was filtered through Celite pad and the filtrate was concentrated *in vacuo.* The obtained Compound 119 was used for the next step without further purification.
¹H NMR (DMSO-d6) δ: 1.26 (t, J = 6.9 Hz, 3H), 2.42-2.65 (m, 4H), 4.18 (q, J = 6.9 Hz, 2H), 6.62 (d, J = 1.8 Hz, 1H), 6.81 (s, 1H), 11.6 (s, 1H), 12.1 (brs, 1H).

### 4th step

To a solution of Compound 119 (200 mg, 0.947 mmol) in methylene chloride (10 mL) were added oxalyl chloride (0.1 mL, 1.14 mmol) and dimethylformamide (7 - L, 0.1 mmol) under ice cooling bath, and the resulting mixture was stirred for 2 hours. The reaction mixture was concentrated *in vacuo* and the residue was dissolved in tetrahydrofuran (10 mL). Trimethylsilyldiazomethane (2 mol/L, 1.2 mL, 2.37 mmol) was added to the mixture under ice cooling bath, and the resulting mixture was stirred at room temperature for 16 hours. The reaction mixture was concentrated *in vacuo* and the residue was dissolved in methylene chloride/methanol (1:1, 30 mL). Silicagel (70-230 mesh, 6 g) was added to the mixture, and the resulting mixture was stirred at room temperature for 30 hours. The reaction mixture was filtered through Celite pad and the filtrate was concentrated *in vacuo.* The residue was purified by silicagel column chromatography eluting with hexane/ethyl acetate=1/1. The eluate was collected and concentrated *in vacuo* to give Compound 120 (139.7 mg, Yield 63 %) as yellow oil.
¹H NMR (DMSO-d6) δ: 1.26 (t, J = 6.9 Hz, 3H), 2.53-2.63 (m, 4H), 4.19 (q, J = 6.9 Hz, 2H), 6.09 (brs, 1H), 6.62 (s, 1H), 6.80 (s, 1H), 11.6 (brs, 1H).

### 5th step

To a solution of rhodium acetate dimer (201 mg, 0.455 mmol) in methylene chloride (20 mL) was added dropwise a solution of Compound 120 (2.14 g, 9.10 mmol) in methylene chloride (30 mL) at room temperature over 30 minutes. The reaction mixture was stirred for 10 minutes, poured into ice water and extracted with ethyl acetate. The organic layer was washed with water, dried over anhydrous sodium sulfate and concentrated *in vacuo.* The residue was purified by silicagel column chromatography eluting with hexane/ethyl acetate=1/1. The eluate was collected and concentrated *in vacuo* to give a colorless Compound 121 (443.5 mg, Yield 23 %).
¹H NMR (DMSO-d6) δ: 1.27 (t, J = 6.9 Hz, 3H), 2.54(t, J = 6.6 Hz, 2H), 2.75 (t, J= 6.6 Hz, 2H), 3.44 (s, 2H), 4.20 (q, J = 6.9 Hz, 2H), 6.59 (d, J = 2.1, 1H), 11.5 (s, 1H).

### 6th step

To a solution of Compound 121 (443.5 mg, 2.14 mmol) in methanol (20 mL) was added sodium borohydride (121 mg, 3.21 mmol) under ice cooloing bath, and the resulting mixture was stirred for 2 hours. The reaction mixture was poured into ice water and extracted ethyl acetate. The organic layer was washed with water, dried over anhydrous sodium sulfate and concentrated *in vacuo.* The residue was dissolved in ethanol (20 mL) and sodium hydroxide aqueous solution (2 mol/L, 10 mL) was added to the solution at room temperature, and the resulting mixture was stirred for 12 hours. The reaction mixture was concentrated *in vacuo* and the residue was washed with ethyl ether. The residue was acidified with concentrated hydrochloric acid (pH=3) and extracted with ethyl acetate. The organic layer was washed with saturated brine, dried over anhydrous magnesium sulfate and concentrated *in vacuo.* The obtained Compound 122 was used for the next step without further purification. ¹H NMR (DMSO-d6) δ: 1.14-1.23 (m, 1H), 1.50-1.60 (m, 1H), 2.32-2.50 (m, 3H),2.78 (dd, J = 6.0, 18.0 Hz, 1H), 3.87-3.88 (m, 1H), 4.76 (d, J = 3 Hz, 1H), 6.40 (d, J = 3 Hz, 1H), 11.1 (s, 1H), 11.8 (brs, 1H).

### 7th step

To a solution of Compound 122 (52 mg, 0.287 mmol) in dimethylformamide (2 mL) were added N-methylpiperazine (123) (0.04 mL, 0.373 mmol), HATU (120 mg, 0.316 mmol) and N-methylmorpholine (0.05 mL, 0.430 mmol) successively at room temperature, and the resulting mixture was stirred for 7 hours. The reaction mixture was poured into ice water and extracted with chloroform. The organic layer was washed with water, dried over anhydrous sodium sulfate and concentrated *in vacuo.* The residue was purified by silicagel column chromatography eluting with chloroform/methanol=1/8. The eluate was collected and concentrated *in vacuo* to give a colorless Compound 124 (11.6 mg, Yield 15 %).
¹H NMR (DMSO-d6) δ: 1.53-1.60 (m, 1H), 1.80-1.83 (m, 1H), 2.18 (s, 3H), 2.28(t, J = 5.1 Hz, 4H), 2.36-2.51 (m, 3H), 2.78 (dd, J = 6.0, 18.0 Hz, 1H), 3.64 (t, J = 5.1 Hz, 1H), 3.58-3.59 (m, 1H), 4.76 (d, J = 4.2 Hz, 1H), 6.16 (d, J = 2.1 Hz, 1H), 10.9 (s, 1H).

### [Example 34]

### Preparation of I-067 (Compound 129)

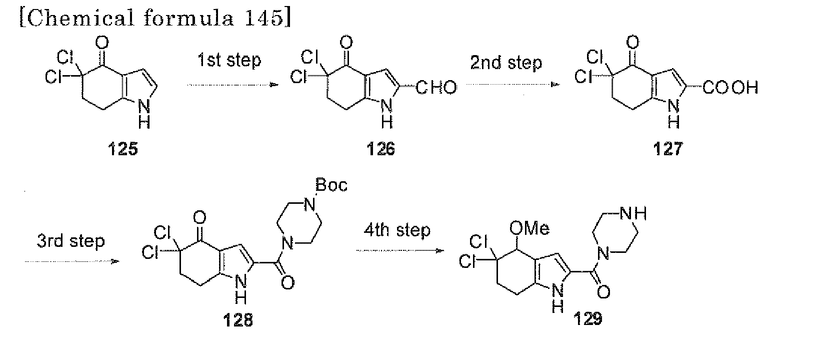

### 1st step

To a solution of Compound 125 (300 mg, 1.47 mmol) in N,N-dimethylformamide (3 mL) was added phosphoryl chloride (0.205 mL, 2.21 mmol), and the resulting mixture was stirred at 80 °C for 1 hour. The reaction mixture was poured into ice water, extracted with ethyl acetate, and the organic layer was washed with saturated brine. The organic layer was dried over anhydrous magnesium sulfate and the residue was recrystallized from hexane/ethyl acetate to give a colorless Compound 126 (197 mg, Yield 58 %).
¹H NMR (DMSO-d6) δ: 3.02 (s, 4H), 7.41 (s, 1H), 9.57 (s, 1H).

### 2nd step

To a solution of Compound 126 (195 mg, 0.84 mmol) in tert- butanol (7 mL)/tetrahydrofuran (7 mL) was added 2-methyl-2-butene(0.89 mL, 8.4 mmol) under ice cooling bath. A mixed solution of sodium dihydrogenorthophosphate (1.01 g, 8.4 mmol) and sodium chlorite (228 mg, 2.52 mmol) (1.75 mL) was added to the mixture and the resulting mixture was stirred at room temperature for 4 hours. 1 mol/L hydrochloric acid aqueous solution was added to the reaction mixture, extracted with ethyl acetate, and the organic layer was washed with saturated brine. The organic layer was dried over anhydrous magnesium sulfate and concentrated *in vacuo.* The residue was washed with diethyl ether to give Compound 127 (177 mg, Yield 85 %) as colorless solid.
¹H NMR (DMSO-d6) δ: 2.99 (s, 4H), 6.99 (s, 1H), 12.55 (s, 1H).

### 3rd step

To a solution of Compound 127 (175 mg, 0. 71 mmol) in N-methylpyrrolidone (2 mL) were added (7-azabenzotriazol-1-yl)-N,N,N',N'-tetramethyluronium hexafluorophosphate (402 mg, 1.06 mmol), tert-butylpiperazine -1-carboxylate (197 mg, 1.06 mmol) and N-methylmorpholine (0.194 mL, 1.76 mmol) under ice cooling bath, and the resulting mixture was stirred at room temperature for 4 hours. The reaction mixture was poured into water, extracted with ethyl acetate, and the organic layer was washed with water, saturated sodium bicarbonate aqueous solution and saturated brine. The organic layer was dried over anhydrous magnesium sulfate and concentrated *in vacuo.* The residue was purified by silicagel column chromatography eluting with chloroform/methanol =95/5. The eluate was collected and concentrated *in vacuo* to give a colorless Compound 128 (169 mg, Yield 58 %).
¹H NMR (DMSO-d6) δ: 1.44 (s, 9H), 3.01 (s, 4H), 3.42 (t, J = 5 Hz, 4H), 3.70 (s, 4H), 6.88 (s, 1H), 12.36 (s, 1H).

### 4th step

To a solution of Compound 128 (215 mg, 0.54 mmol) in ethyl acetate (10 mL) was added 4 mol/L hydrochloric acid in ethyl acetate (1.29 mL, 5.4 mmol), and the resulting mixture was stirred at room temperature for 1 hour. The precipitated solid was collected by filtration, washed with ethyl acetate to afford colorless solid. Methanol was added to the solid, and the resulting mixture was heated, then collected by filtration to give Compound 129 (74 mg, Yield 43 %) as colorless solid. ¹H NMR (DMSO-d6) δ: 2.49-2.82 (m, 8H), 3.49 (s, 3H), 3.67 (brs, 4H), 6.48 (s, 1H), 8.22 (s, 1H), 11.34 (s, 1H).

### [Example 35]

### Preparation of 1-055 (Compound 131)

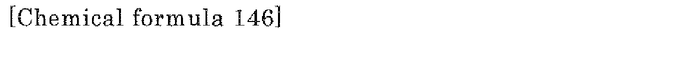

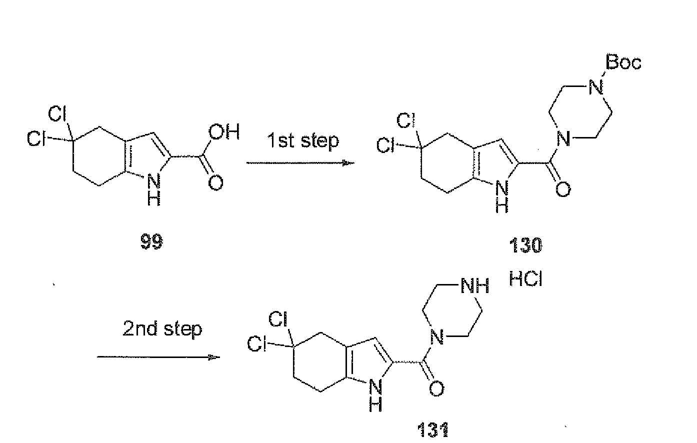

### 1st step

Compound 99 (80 mg, 0.342 mmol) was dissolved in DMF (1 mL), 1-Boc-piperazine (83 mg, 0.444 mmol), NMM (0.094 mL, 0.854 mmol) and HATU (169 mg , 0.444 mmol) were added to the solution, and the resulting mixture was stirred at room temperature for 6 hours. Water was added to the mixture and extracted with ethyl acetate/methanol (10/1). The organic layer was washed with saturated brine, dried over anhydrous magnesium sulfate and concentrated *in vacuo*. The residue was purified by silicagel column chromatography(chloroform-methanol) to give Compound 130(55 mg, 40 %).
¹H-NMR(DMSQ-d₆) δppm:1.42 (s, 9H), 2.60 (br, 2H), 2.77 (br, 2H), 3.35-3.50 (m, 6H), 3.64 (br, 4H), 6.31 (s, 1H), 11.23 (s, 1H)

### 2nd step

Hydrochloric acid in ethyl acetate (4 mol/L, 1.0 mL) was added to Compound 130 (20 mg, 0.05 mmol), and the resulting mixture was stirred at room temperature for 40 minutes. The mixture was concentrated *in vacuo* and the obtained powder was washed with ethyl acetate to give Compound 131(14 mg, Yield 83 %). ¹H-NMR(DMSO-d₆) δppm:2.60 (s, 2H), 2.78 (s, 2H), 3.12 (s, 4H), 3.45 (s, 2H) 3.89 (s, 4H), 6.37 (s, 1H), 9.54 (s, 2H), 11.32 (s, 1H)

The following compounds were prepared in a manner similar to the general procedures and Examples. The chemical structure of the compounds and the physical properties of them are described below.

### (Method of identification fo the compound)

LC/MS data of compound of the present invention were measured under any one of the following 10 conditions (Method A to J), and a retention time and [M+H]⁺ are shown.

### (Method A)

Column: Xbridge C18 (5 µm, i.d.4.6 x 50 mm) (Waters)
Flow rate: 3 mL/min
UV detection wavelength: 254 nm
Mobile phase: [A] is 0.1% formic acid-containing aqueous solution, and [B] is 0.1% formic acid-containing acetonitrile solution.
Gradient: Linear gradient of 10% to 100% solvent[B] for 3 minutes was performed, and 100% solvent [B] was maintained for 1 minute.

### (Method B)

Column: Shim-pack XR-ODS (2.2 µm, i.d.50 x 3.0 mm) (Shimadzu)
Flow rate: 1.6 mL/min
UV detection wavelength: 254 nm
Mobile phase: [A] is 0.1% formic acid-containing aqueous solution, and [B] is 0.1% formic acid-containing acetonitrile solution.
Gradient: Linear gradient of 10% to 100% solvent[B] for 3 minutes was performed, and 100% solvent [B] was maintained for 1 minute.

### (Method C)

Column: Luna C18(2) (5µm, i.d. 4.6 x 50 mm)(Phenomenex)
Flow rate: 3 mL/min
UV detection wavelength: 254 nm
Mobile phase: [A] is 0.1% formic acid-containing aqueous solution, and [B] is 0.1% formic acid-containing acetonitrile solution.
Gradient: Linear gradient of 10% to 100% solvent[B] for 3 minutes was performed, and 100% solvent [B] was maintained for 1 minute.

### (Method D)

Column: Gemini-NX (5µm, i.d. 4.6 x 50 mm) (Phenomenex)
Flow rate: 3 mL/min
UV detection wavelength: 254 nm
Mobile phase: [A] is 0.1% formic acid-containing aqueous solution, and [B] is 0.1% formic acid-containing methanol solution.
Gradient: Linear gradient of 5% to 100% solvent[B] for 3.5 minutes was performed, and 100% solvent [B] was maintained for 0.5 minute.

### (Method E)

Column: XBridge C18 (5µm, i.d. 4.6 x 50 mm) (Waters)
Flow rate: 2 mL/min
UV detection wavelength: 254 nm
Mobile phase: [A] is 10 mM ammonium bicarbonate-containing aqueous solution, and [B] is acetonitrile.
Gradient: Linear gradient of 10% to 100% solvent[B] for 3 minutes was performed, and 100% solvent [B] was maintained for 1 minute.

### (Method F)

Column: Gemini-NX (5µm, i.d. 4.6 x 50 mm) (Phenomenex)
Flow rate: 3 mL/min
UV detection wavelength: 254 nm
Mobile phase: [A] is 10 mM ammonium bicarbonate-containing aqueous solution, and [B] is methanol.
Gradient: Linear gradient of 5% to 100% solvent[B] for 3.5 minutes was performed, and 100% solvent [B] was maintained for 0.5 minute.

### (Method G)

Column: X-Bridge Prep C18(5µm OBD, i.d.19 x 50 mm) (Waters)
Flow rate: 25 mL/min
UV detection wavelength: 254 nm
Mobile phase: [A] is 0.3% formic acid-containing aqueous solution, and [B] is 0.3% formic acid-containing acetonitrile solution.
Gradient: Linear gradient of 10% to 100% solvent[B] for 5 minutes was performed, and 100% solvent [B] was maintained for 1 minute.

### (Method H)

Column: Gemini-NX (C18, 5µm AXIA Packed, i.d. 21.2 x 50 mm) (Phenomenex) Flow rate: 25 mL/min
UV detection wavelength: 254 nm
Mobile phase: [A] is 0.1% formic acid-containing aqueous solution, and [B] is 0.1% formic acid-containing methanol solution.
Gradient: Linear gradient of 10% to 100% solvent[B] for 5 minutes was performed, and 100% solvent [B] was maintained for 1 minute.

### (Method I)

Column: X-Bridge Prep C18 (C18 5µm OBD, i.d.19 x 50 mm) (Waters)
Flow rate: 25 mL/min
UV detection wavelength: 254 nm
Mobile phase: [A] is 10 mM ammonium bicarbonate-containing aqueous solution, and [B] is acetonitrile.
Gradient: Linear gradient of 10% to 100% solvent[B] for 5 minutes was performed, and 100% solvent [B] was maintained for 1 minute.

### (Method J)

Column: Gemini-NX (C18, 5µm AXIA Packed, i.d. 21.2 x 50 mm) (Phenomenex) Flow rate: 25 mL/min
UV detection wavelength: 254 nm
Mobile phase: [A] is 10 mM ammonium bicarbonate-containing aqueous solution, and [B] is methanol.
Gradient: Linear gradient of 10% to 100% solvent[B] for 5 minutes was performed, and 100% solvent [B] was maintained for 1 minute.

**[Table 7]**

| Compound No. | Structure | LC/MS Retention time (min) | MS[M+H]⁺ | LC method |
|---|---|---|---|---|
| I-001 | 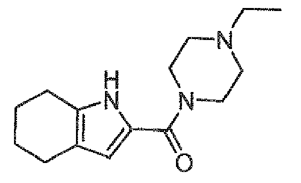 | 1.48 | 262 | E |
| I-002 | 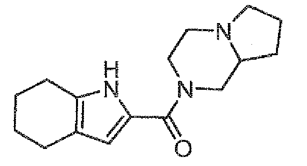 | 1.52 | 274 | E |
| I-003 | 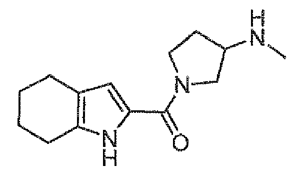 | 0.91 | 248 | B |
| I-004 | 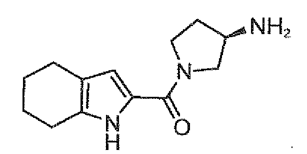 | 0.87 | 234 | B |
| I-005 | 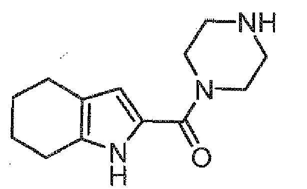 | 0.86 | 234 | B |
| I-006 | 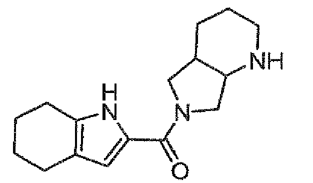 | 0.98 | 274 | B |

**[Table 8]**

| Compound No. | Structure | LC/MS Retention time (min) | MS[M+H]⁺ | LC method |
|---|---|---|---|---|
| I-007 | 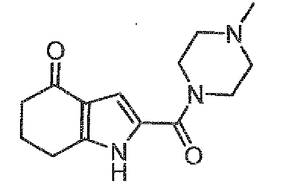 | 0.38 | 262 | B |
| I-008 | 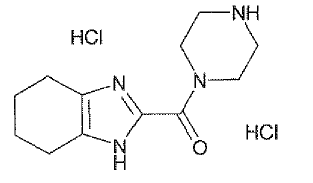 | 0.38 | 235 | B |
| I-009 | 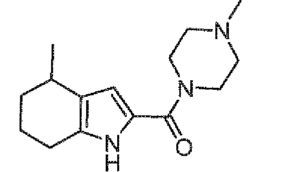 | 1.02 | 262 | B |
| I-010 | 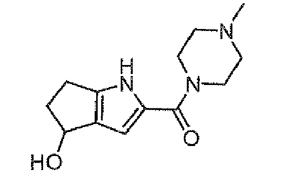 | 0.37 | 250 | B |
| I-011 | 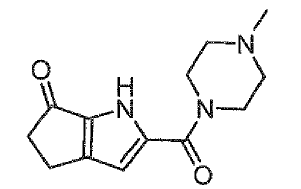 | 0.35 | 248 | B |
| I-012 | 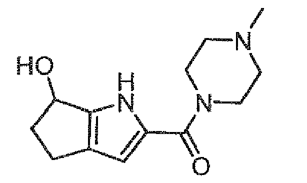 | 0.35 | 250 | B |

**[Table 9]**

| Compound No. | Structure | LC/MS Retention time (min) | MS[M+H]⁺ | LC method |
|---|---|---|---|---|
| I-013 | 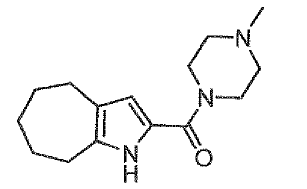 | 0.95 | 262 | A |
| I-014 | 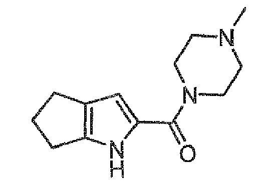 | 0.65 | 234 | A |
| I-015 | 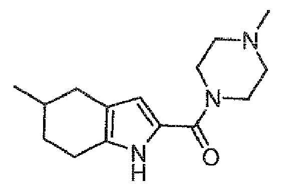 | 1.04 | 262 | A |
| I-016 | 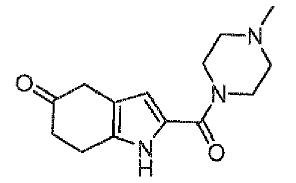 | 0.28 | 262 | A |
| I-017 | 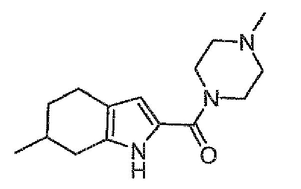 | 1.00 | 262 | A |
| I-018 | 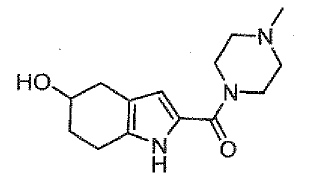 | 0.64 | 264 | E |

**[Table 10]**

| Compound No. | Structure | LC/MS Retention time (min) | MS[M+H]⁺ | LC method |
|---|---|---|---|---|
| I-019 | 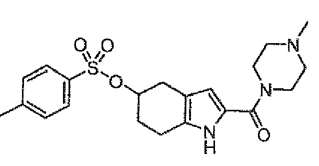 | 1.22 | 418 | A |
| I-020 | 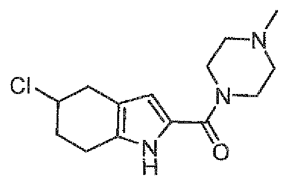 | 0.76 | 282 | C |
| I-021 | 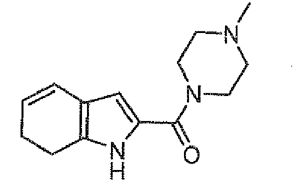 | 0.67 | 246 | C |
| I-022 | 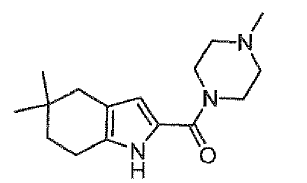 | 1.06 | 276 | A |
| I-023 | 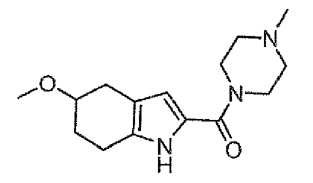 | 0.63 | 278 | A |
| I-024 | 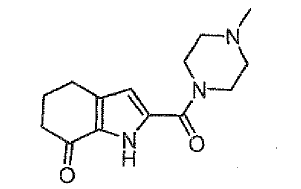 | 0.44 | 262 | C |

**[Table 11]**

| Compound No. | Structure | LC/MS Retention time (min) | MS[M+H]⁺ | LC method |
|---|---|---|---|---|
| I-025 | 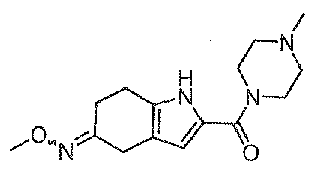 | 0.57 | 291 | C |
| I-026 | 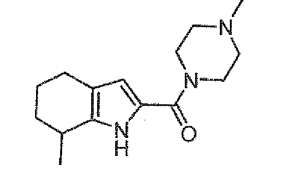 | 0.80 | 262 | C |
| I-027 | 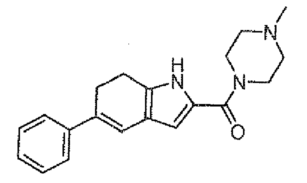 | 1.76 | 322 | C |
| I-028 | 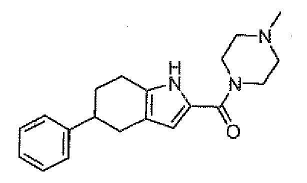 | 1.80 | 324 | E |
| I-029 | 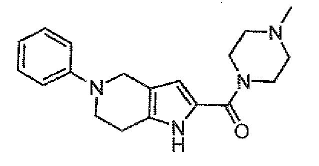 | 0.59 | 325 | C |
| I-030 | 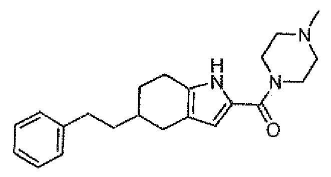 | 1.27 | 352 | C |

**[Table 12]**

| Compound No. | Structure | LC/MS Retention time (min) | MS[M+H]⁺ | LC method |
|---|---|---|---|---|
| I-031 | 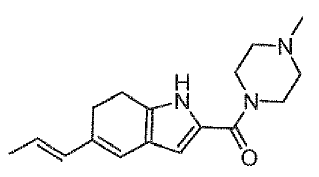 | 1.05 | 286 | C |
| I-032 | 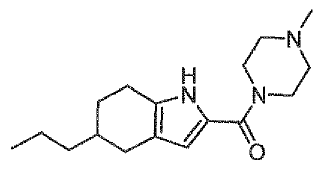 | 1.19 | 290 | C |
| I-033 | 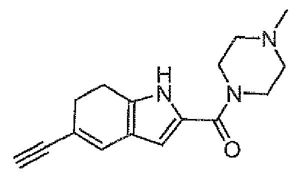 | 0.70 | 270 | C |
| I-034 | 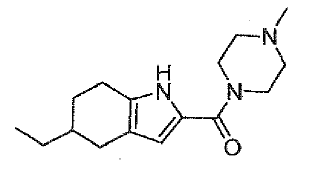 | 1.73 | 276 | E |
| I-035 | 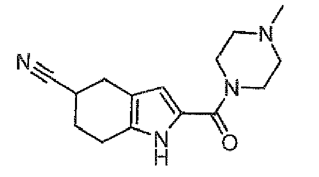 | 0.42 | 273 | A |
| I-036 | 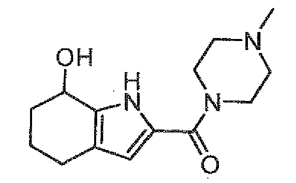 | 0.40 | 264 | C |

**[Table 13]**

| Compound No. | Structure | LC/MS Retentiontime (min) | MS[M+H]⁺ | LC method |
|---|---|---|---|---|
| I-037 | 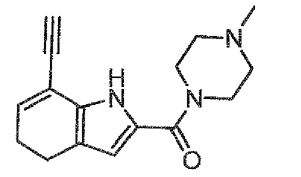 | 1.28 | 270 | D |
| I-038 | 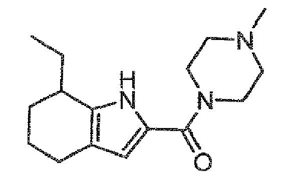 | 1.55 | 276 | D |
| I-039 | 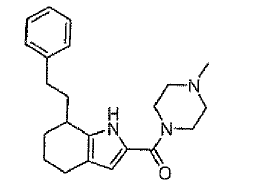 | 2.11 | 352 | D |
| I-040 | 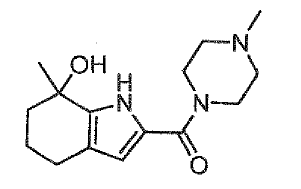 | 1.00 | 278 | E |
| I-041 | 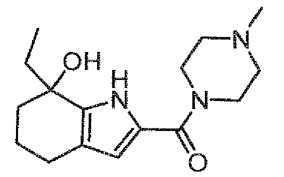 | 1.15 | 292 | E |
| I-042 | 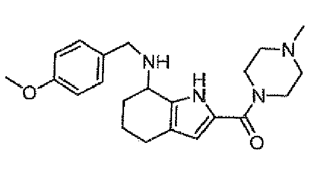 | 1.60 | 383 | E |

**[Table 14]**

| Compound No. | Structure | LC/MS Retention time (min) | MS[M+H]⁺ | LC method |
|---|---|---|---|---|
| I-043 | 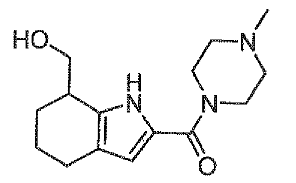 | 0.89 | 278 | D |
| I-044 | 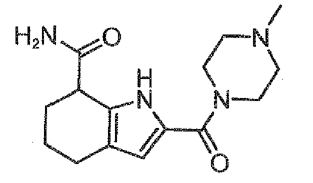 | 1.76 | 291 | F |
| I-045 | 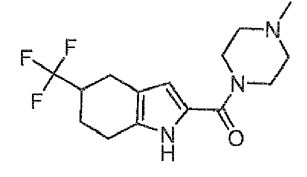 | 1.38 | 316 | D |
| I-046 | 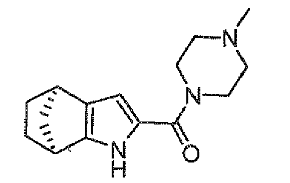 | 2.42 | 260 | F |
| I-047 | 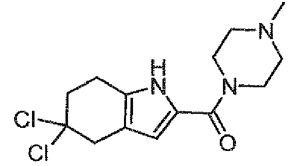 | 1.31 | 316 | D |
| I-048 | 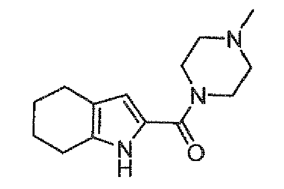 | 0.78 | 248 | A |

**[Table 15]**

| Compound No. | Structure | LC/MS Retention time (min) | MS[M+H]⁺ | LC method |
|---|---|---|---|---|
| I-049 | 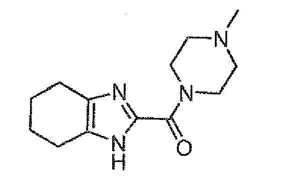 | 0.96 | 249 | C |
| I-050 | 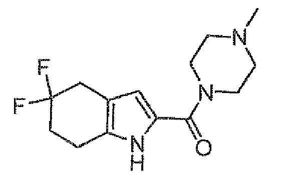 | 0.77 | 284 | C |
| I-051 | 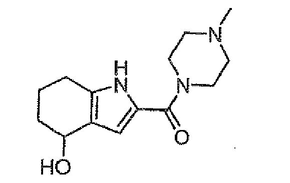 | 0.36 | 264 | B |
| I-052 | 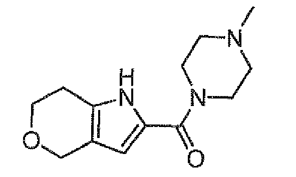 | 0.54 | 250 | B |
| I-053 | 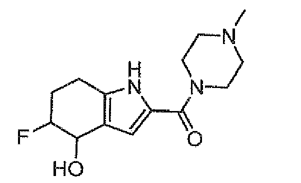 | 0.35 | 282 | B |
| I-054 | 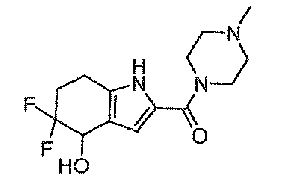 | 0.43 | 300 | B |

**[Table 16]**

| Compound No. | Structure | LC/MS Retention time (min) | MS[M+H]⁺ | LC method |
|---|---|---|---|---|
| I-055 | 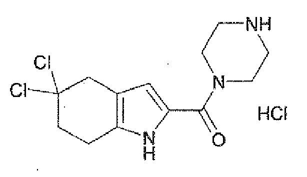 | 2.26 | 302 | F |
| I-056 | 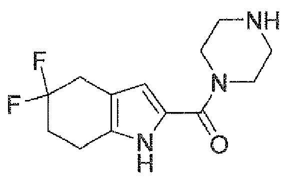 | 0.71 | 270 | B |
| I-057 | 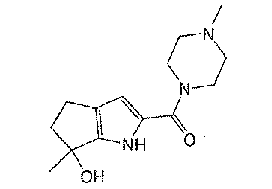 | 0.44 | 264 | B |
| I-058 | 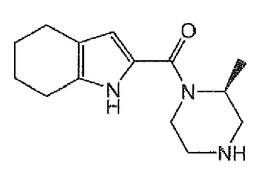 | 0.91 | 248 | B |
| I-059 | 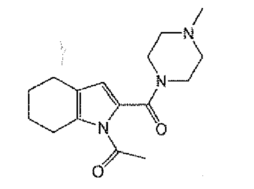 | 0.91 | 290 | B |
| I-060 | 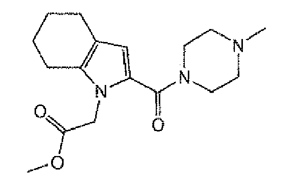 | 1.10 | 320 | B |

**[Table 17]**

| Compound No. | Structure | LC/MS Retention time (min) | MS[M+H]⁺ | LC method |
|---|---|---|---|---|
| I-061 | 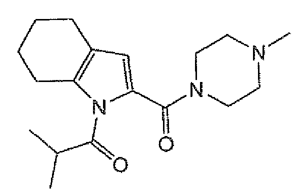 | 1.15 | 318 | B |
| I-062 | 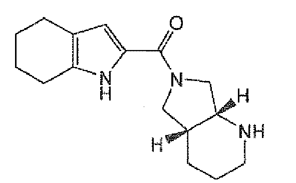 | 1.38 | 274 | B |
| I-063 | 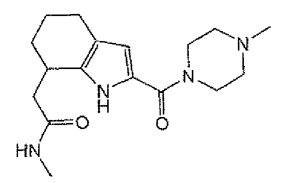 | 2.14 | 319 | F |
| I-064 | 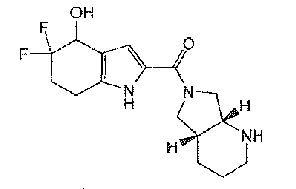 | 0.61 | 326 | B |
| I-065 | 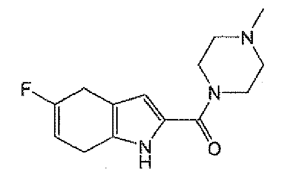 | 0.75 | 264 | B |
| I-066 | 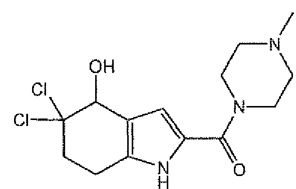 | 0.37 | 332, 334 | A |

**[Table 18]**

| Compound No. | Structure | LC/MS Retention time (min) | MS[M+H]⁺ | LC method |
|---|---|---|---|---|
| I-067 | 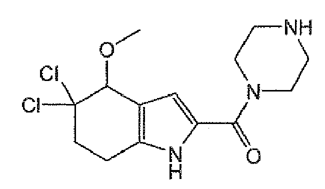 | 0.92 | 332, 334 | A |
| I-068 | 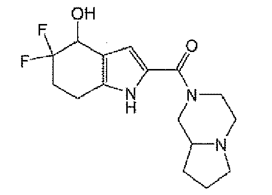 | 0.48 | 326 | B |
| I-069 | 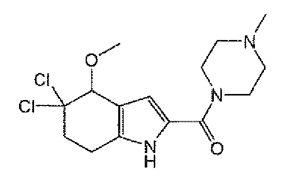 | 0.96 | 346, 348 | A |
| I-070 | 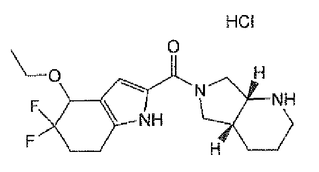 | 0.98 | 354 | B |
| I-071 | 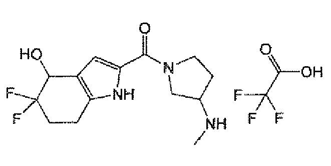 | 0.48 | 300 | B |
| I-072 | 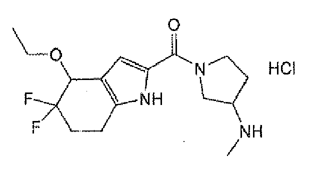 | 0.95 | 328 | B |

**[Table 19]**

| Compound No. | Structure | LC/MS Retention time (min) | MS[M+H]⁺ | LC method |
|---|---|---|---|---|
| I-073 | 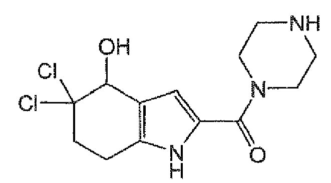 | 1.69 | 318, 320 | F |
| I-074 | 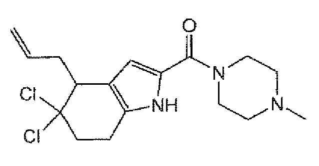 | 1.33 | 356 | B |
| I-075 | 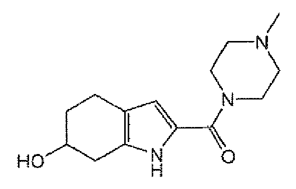 | 0.36 | 264 | B |
| I-076 | 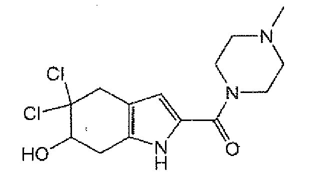 | 0.66 | 333 | A |

In a general formula (I-α), the compounds having the following group may be prepared in a manner similar to the above reaction.

A formula (I-α):

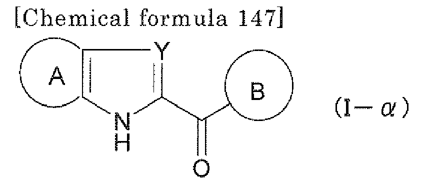

**[Table 20]**

| | A | | A |
|---|---|---|---|
| A1 | 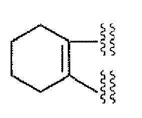 | A5 | 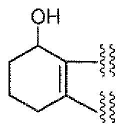 |
| A2 | 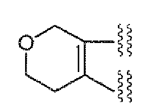 | A6 | 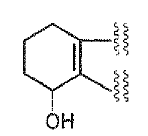 |
| A3 | 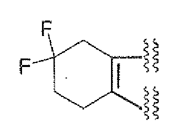 | A7 | 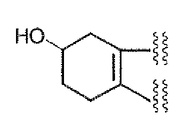 |
| A4 | 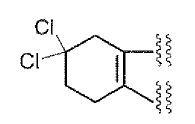 | | |

**[Table 21]**

| | B | | B |
|---|---|---|---|
| B1 | 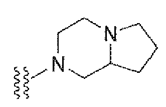 | B5 | 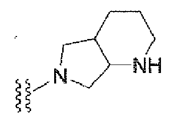 |
| B2 | 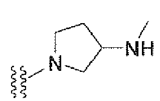 | B6 | 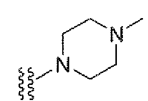 |
| B3 | 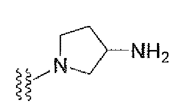 | B7 | 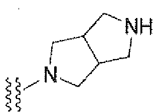 |
| B4 | 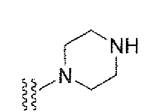 | | |

**[Table 22]**

| | Y |
|---|---|
| Y1 | N |
| Y2 | CH |

The compounds having the following (A, B, Y) in the combination of A, B, Y. (A,B,Y)=(A1,B1,Y1),(A1,B1,Y2),(A1,B2,Y1),(A1,B2,Y2),(A1,B3,Y1),(A1,B3,Y2),(A1,B4, Y1),(A1,B4,Y2),(A1,B5,Y1),(A1,B5,Y2),(A.1,B6,Y1),(A1,B6,Y2),(A1,B7,Y1),(A1,B7,Y2),( A2,B1,Y1),(A2,B1,Y2),(A2,B2,Y1),(A2,B2,Y2),(A2,B3,Y1),(A2,B3,Y2),(A2,B4,Y1),(A2,B 4,Y2),(A2,B5,Y1),(A2,B5,Y2),(A2,B6,Y1),(A2,B6,Y2),(A2,B7,Y1),(A2,B7,Y2),(A3,B1,Y1) ,(A3,B1,Y2),(A3,B2,Y1),(A,3,B2,Y2),(A3,B3,Y1),(A3,B3,Y2),(A3,B4,Y1),(A3,B4,Y2),(A3, B5,Y1),(A3,B5,Y2),(A3,B6,Y1),(A3,B6,Y2),(A3,B7,Y1),(A3,B7,Y2),(A4,B1,Y1),(A4,B1,Y 2),(A4,B2,Y1),(A4,B2,Y2),(A4,B3,Y1),(A4,B3,Y2),(A4,B4,Y1),(A4,B4,Y2),(A4,B5,Y1),(A 4,B5,Y2),(A4,B6,Y1),(A4,B6,Y2),(A4,B7,Y1),(A4,B7,Y2),(A5,B1,Y1),(A5,B1,Y2),(A5,B2, Y1),(A5,B2,Y2),(A5,B3,Y1),(A5,B3,Y2),(A5,B4,Y1)/A5,B4,Y2),(A5,B5,Y1),(A5,B5,Y2),( A5,86,Y1),(A5,86,Y2),(A5,B7,Y1),(A5,87,Y2),(A6,B1,Y1),(A6,B1,Y2),(A6,B2,Y1),(A6,B 2,Y2),(A6,B3,Y1),(A6,B3,Y2),(A6,B4,Y1),(A6,B4,Y2),(A6,B5,Y1),(A6,B5,Y2),(A6,B6,Y1) ,(A6,B6,Y2),(A6,B7,Y1),(A6,B7,Y2),(A7,B1,Y1),(A7,B1,Y2),(A7,B2,Y1),(A7,B2,Y2),(A7, B3,Y1),(A7,B3,Y2),(A7,B4,Y1),(A7,B4,Y2),(A7,B5,Y1),(A7,B5,Y2),(A7,B6,Y1),(A7,B6,Y 2),(A7,B7,Y1),(A7,B7,Y2)

### Histamine H4 receptor binding test

Membrane fraction of CHO-K1 stably expressing human HRH4 was purchased from PerkinElmer. Radioligand binding assay was performed in 96 well polypropylene assay plates in a total assay volume of 150 µL. Membrane suspension (15 µg of protein) was mixed with [3H]-Histamine (PerkinElmer) at 15 nM and compounds at various concentration in 50mM Tris-HCl pH7.4, 5mM EDTA buffer. Assays were incubated for 60 min at room temperature, then the reactions were terminated by rapid filtration over 96-well GF/B filter plates (PerkinElmer) presoaked in 0.5% polyethyleneimine. Unbound radioacitivity was eliminated by six times of wash with 350 µL of ice-cold 50 mM Tris pH7.4 buffer. When the filter plates were dried, Microscint-MS liquid scintillation fluid (PerkinElmer) was added to each well and retained radioactivity plates were counted in a Topcount liquid scintillation counter (PerkinElmer). Specific binding was calculated by subtracting non-specific binding determined in the presence of 100 µM unlabeled histamine from total count. The resulting data were analyzed for IC₅₀ using the non-linear regression on XLfit. Affinity values (Ki) were then determined by Cheng-Prusoff equation.

The results of the compounds of the present invention in histamine H4 receptor binding test are shown in the following table.

**[Table 23]**

| Compound No. | H4 Ki (nM) | Compound No. | H4 Ki (nM) |
|---|---|---|---|
| I-003 | 680 | I-046 | 290 |
| I-005 | 250 | I-047 | 14 |
| I-006 | 230 | I-048 | 110 |
| I-008 | 420 | I-049 | 150 |
| I-009 | 150 | I-050 | 63 |
| I-012 | 680 | I-051 | 250 |
| I-013 | 300 | I-052 | 380 |
| I-015 | 86 | I-053 | 140 |
| I-016 | 370 | I-054 | 230 |
| I-017 | 570 | I-055 | 23 |
| I-019 | 200 | I-056 | 130 |
| I-020 | 22 | I-057 | 440 |
| I-021 | 120 | I-059 | 270 |
| I-022 | 70 | I-061 | 210 |
| I-023 | 990 | I-062 | 260 |
| I-024 | 710 | I-064 | 180 |
| I-026 | 100 | I-065 | 35 |
| I-027 | 760 | I-066 | 350 |
| I-033 | 320 | I-067 | 480 |
| I-035 | 150 | I-068 | 520 |
| I-036 | 88 | I-069 | 220 |
| I-040 | 180 | I-070 | 270 |
| I-041 | 590 | I-073 | 980 |
| I-043 | 270 | I-075 | 20 |
| I-045 | 910 | I-076 | 25 |

### CYP3A4 fluorescent MBI test

The CYP3A4 fluorescent MBI test is a test of investigating enhancement of CYP3A4 inhibition of a compound by a metabolism reaction, and the test was performed using, as CYP3A4 enzyme expressed in Escherichia coli and employing, as an index, a reaction in which 7-benzyloxytrifluoromethylchmarin (BFC) is debenzylated by the CYP3A4 enzyme to produce a metabolite, 7-hydroxytrifluoromethylchmarin (HFC) emitting fluorescent light.

The reaction conditions were as follows: substrate, 5.6 µmol/L 7-BFC; prereaction time, 0 or 30 minutes; reaction time, 15 minutes; reaction temperature, 25°C (room temperature); CYP3A4 content (expressed in Escherichia coli ), at pre-reaction 62.5 pmol/mL, at reaction 6.25 pmol/mL (at 10-fold dilution); test drug concentration, 0.625, 1.25, 2.5, 5, 10, 20 µmol/L (six points).

An enzyme in a K-Pi buffer (pH 7.4) and a test drug solution as a pre-reaction solution were added to a 96-well plate at the composition of the pre-reaction, a part of it was transferred to another 96-well plate so that it was 1/10 diluted by a substrate in a K-Pi buffer, NADPH as a co-factor was added to initiate a reaction as an index (without preincubation) and, after a predetermined time of a reaction, acetonitrile/0.5 mol/L Tris (trishydroxyaminomethane) = 4/1 was added to stop the reaction. In addition, NADPH was added to a remaining preincubation solution to initiate a preincubation (with preincubation) and, after a predetermined time of a preincubation, a part was transferred to another plate so that it was 1/10 diluted with a substrate and a K-Pi buffer to initiate a reaction as an index. After a predetermined time of a reaction, acetonitrile/0.5 mol/L Tris (trishydroxyaminomethane) = 4/1 was added to stop the reaction. For the plate on which each index reaction had been performed, a fluorescent value of 7-HFC which is a metabolite was measured with a fluorescent plate reader. (Ex = 420 nm, Em = 535 nm).

Addition of only DMSO which is a solvent dissolving a drug to a reaction system was adopted as a control (100%), remaining activity (%) was calculated at each concentration of a test drug added as the solution, and IC₅₀ was calculated by reversepresumption by a logistic model using a concentration and an inhibition rate. When a difference between IC₅₀ values is 5 µM or more, this was defined as (+) and, when the difference is 3 µM or less, this was defined as (-).

### (Result)

I-010: (-)
I-050: (-)

### CYP inhibition test

Using commercially available pooled human hepatic microsome, and employing, as markers, 7-ethoxyresorufin O-deethylation (CYP1A2), tolbutamide methylhydroxylation (CYP2C9), mephenytoin 4'-hydroxylation (CYP2C19), dextromethorphan O-demethylation (CYP2D6), and terfenedine hydroxylation as typical substrate metabolism reactions of human main five CYP enzyme forms (CYP1A2, 2C9, 2C19, 2D6, 3A4), an inhibitory degree of each metabolite production amount by a test compound was assessed.

The reaction conditions were as follows: substrate, 0.5 µmol/L ethoxyresorufin (CYP1A2), 100 µmol/L tolbutamide (CYP2C9), 50 µmol/L S-mephenitoin (CYP2C19), 5µmol/L dextromethorphan (CYP2D6), 1 µmol/L terfenedine (CYP3A4); reaction time, 15 minutes; reaction temperature, 37°C; enzyme, pooled human hepatic microsome 0.2 mg protein/mL; test drug concentration, 1, 5, 10, 20 µmol/L (four points).

Each five kinds of substrates, human hepatic microsome, or a test drug in 50 mM Hepes buffer as a reaction solution was added to a 96-well plate at the composition as described above, NADPH, as a cofactor was added to initiate metabolism reactions as markers and, after the incubation at 37°C for 15 minutes, a methanol/acetonitrile = 1/1 (v/v) solution was added to stop the reaction. After the centrifugation at 3000 rpm for 15 minutes, resorufin (CYP1A2 metabolite) in the supernatant was quantified by a fluorescent multilabel counter and tributamide hydroxide (CYP2CP metabolite), mephenytoin 4' hydroxide (CYP2C19 metabolite), dextromethorphan (CYP2D6 metabolite), and terfenadine alcohol (CYP3A4 metabolite) were quantified by LC/MS/MS.

Addition of only DMSO being a solvent dissolving a drug to a reaction system was adopted as a control (100%), remaining activity (%) was calculated at each concentration of a test drug added as the solution and IC₅₀ was calculated by reverse presumption by a logistic model using a concentration and an inhibition rate.

### (Result)

1-050 : five kinds>20µmol/L

### Metabolism Stability Test

Using commercially available pooled human hepatic microsomes, prepared in accordance with Japanese journal of pharmacology, 33 (1), p.41-56, Feb 1983, a test compound was reacted for a constant time, a remaining rate was calculated by comparing a reacted sample and an unreacted sample, thereby, a degree of metabolism in liver was assessed.

A reaction was performed (oxidative reaction) at 37°C for 0 minute or 30 minutes in the presence of 1 mmol/L NADPH in 0.2 mL of a buffer (50 mmol/L tris-HCl pH 7.4, 150 mmol/L potassium chloride, 10 mmol/L magnesium chloride) containing 0.5 mg protein/mL of human liver microsomes. After the reaction, 50 µL of the reaction solution was added to 100 µL of a methanol/acetonitrile = 1/1 (v/v), mixed and centrifuged at 3000 rpm for 15 minutes. The test compound in the supernatant was quantified by LC/MS/MS, and a remaining amount of the test compound after the reaction was calculated, letting a compound amount at 0 minute reaction time to be 100%. Hydrolysis reaction was performed in the absence of NADPH and glucuronidation reaction was in the presence of 5 mM UDP-glucuronic acid in place of NADPH, followed by similar operations.

### (Result)

I-073(test compound concentration ; 2 µmol/L): (human)99%, (rat)79%
I-012(test compound concentration ; 0.5 µmol/L): (human)94%, (rat)92%

Bioavailability (BA) test and total body clearance test
Materials and methods for studies on oral absorption
(1) Animal: mice or rats
(2) Breeding conditions: mice and rats are allowed to freely take solid feed and sterilized tap water
(3) Dose and grouping: orally or intravenously administered at a predetermined dose; grouping is as follows (Dose depends on the compound)
   Oral administration: 1 to 30 mg/kg (n=2 to 3)
   Intravenous administration: 0.5 to 10 mg/kg (n=2 to 3)
(4) Preparation of dosing solution: for oral administration, in a solution or a suspension state; for intravenous administration, in a solubilized state
(5) Administration method: in oral administration, forcedly administer into ventriculus with oral probe; in intravenous administration, administer from caudal vein with a needle-equipped syringe
(6) Evaluation items: blood is collected over time, and the plasma concentration of drug is measured by LC/MS/MS
(7) Statistical analysis: regarding the transition of the plasma concentration, the area under the plasma concentration-time curve (AUC) and total body clearance (Clt) are calculated by non-linear least squares program WinNonlin (Registered trademark), and the bioavailability (BA) is calculated from the AUCs of the oral administration group and intravenous administration group
   (Result) The following values show the rat BA of the compounds on oral 1mg/kg dosage.
   BA:
   I-073: 59%
   I-035: 31%
   C1t:
   I-073: 24 mL/min/kg

### FAT Test

20 µL of freezing-stored rat typhoid bacillus (Salmonella typhimurium TA98 strain, TA100 strain) was inoculated on 10 mL of a liquid nutrient medium (2.5% Oxoid nutrient broth No.2), and this was cultured before shaking at 37°C for 10 hours. 9 mL of a bacterial solution of the TA98 strain was centrifuged (2000 x g, 10 minutes) to remove a culturing solution, the bacteria was suspended in 9 mL of a Micro F buffer (K₂HPO₄: 3.5 g/L, KH₂PO₄: 1 g/L, (NH₄)₂SO₄: 1 g/L, trisodium citrate dehydrate: 0.25 g/L, MgSO₄ • 7H₂O: 0.1 g/L), the suspension was added to 110 mL of an Exposure medium (Micro F buffer containing Biotin: 8 µg/mL, histidine: 0.2 µg/mL, glucose: 8 mg/mL), and the TA100 strain was added to 120 mL of the Exposure medium relative to 3.16 mL of the bacterial solution to prepare a test bacterial solution. Each 12 µL of a test substance DMSO solution (8 stage dilution from maximum dose 50 mg/mL at 2-fold ratio), DMSO as a negative control, 50 µg/mL of 4-nitroquinoline-1-oxide DMSO solution for the TA98 strain, 0.25 µg/mL of 2-(furyl)-3-(5-nitro-2-furyl)acrylamide DMSO solution for the TA100 strain under the non-metabolism activating condition, 40 µg/mL of 2-aminoanthracene DMSO solution for the TA98 strain, 20 µg/mL of 2-aminoanthracene DMSO solution for the TA100 strain under the metabolism activating condition as a positive control, and 588 µL of the test bacterial solution (a mixed solution of 498 µL of the test bacterial solution
and 90 µL of S9 mix under the metabolism activating condition) were mixed, and this was shaking-cultured at 37°C for 90 minutes. 460 µL of the bacterial solution exposed to the test substance was mixed with 2300 µL of an Indicator medium (Micro F buffer containing biotin: 8 µg/mL, histidine: 0.2 µg/mL, glucose: 8 mg/mL, Bromo Cresol Purple: 37.5 µg/mL), each 50 µL was dispensed into microplate 48 wells/dose, and this was subjected to stationary culturing at 37°C for 3 days. Since a well containing a bacterium which has obtained the proliferation ability by mutation of an amino acid (histidine) synthesizing enzyme gene turns from purple to yellow due to a pH change, the bacterium proliferation well which has turned to yellow in 48 wells per dose is counted, and was assessed by comparing with a negative control group.

### Solubility test

The solubility of a compound is determined under a condition in which 1% DMSO is added. 10 mM compound solution is prepared using DMSO, and then 6 µL of the compound solution is added to 594 µL of artificial intestinal juice in pH 6.8 (to 250 mL of 0.2 mol/L potassium dihydrogen phosphate reagent solution is added 118 mL of 0.2 mol/L NaOH reagent solution and water to provide a final volume of 1000 mL). After standing at 25 °C for 16 hours, the mixed solution is filtrated with suction. The filtrate is diluted twice with methanol/water (1/1), and then a concentration in the filtration is measured with HPLC or LC/MS/MS by the absolute calibration method.

### (Result)

I-050: >50 µmol/L

### hERG Test

For the purpose of assessing risk of an electrocardiogram QT interval prolongation, effects on delayed rectifier K⁺ current (I_{Kr}), which plays an important role in the ventricular repolarization process, was studied using HEK293 cells expressing human ether-a-go-go related gene (hERG) channel.
After a cell was retained at a membrane potential of -80 mV by whole cell patch clamp method using an automated patch clamp system (PatchXpress 7000A, Axon Instruments Inc.), I_{Kr} induced by depolarization pulse stimulation at +50 mV for 2 seconds and, further, repolarization pulse stimulation at -50 mV for 2 seconds was recorded. After the generated current was stabilized, extracellular solution (NaCl: 135 mmol/L, KCl: 4 mmol/L, CaCl₂ • 2H₂O: 1.8 mmol/L, MgCl₂ • 6H₂O: 1 mmol/L, glucose: 10 mmol/L, HEPES (4-(2-hydroxyethyl)-1-piperazineethanesulfonic acid): 10 mmol/L, pH=7.4) in which the test compound had been dissolved at an objective concentration was applied to the cell under the room temperature condition for 10 minutes. From the recording I_{Kr}, an absolute value of the tail peak current was measured based on the current value at the resting membrane potential using an analysis software (DataXpress ver.1, Molecular Devices Corporation). Further, the % inhibition relative to the tail peak current before application of the test substance was calculated, and compared with the vehicle-applied group (0.1% dimethyl sulfoxide solution) to assess influence of the test substance on I_{Kr}.
(Result) The following values show the % inhibition of the compounds at 1 µmol/L.
I-035: 0%
I-043: 0%

### Formulation Example 1

A granule containing the following ingredient is prepared.

| | | |
|---|---|---|
| Ingredient | Compound represented by the formula (I) | 10 mg |
| | Lactose | 700 mg |
| | Corn starch | 274 mg |
| | HPC-L | 16 mg |

The compound represented by the formula (I) and lactose are passed through a 60 mesh sieve. Corn starch is passed through a 120 mesh sieve. These are mixed with a V-type mixing machine. An aqueous solution of HPC-L (low viscosity hydroxypropylcellulose) is added to a mixed powder, and this is kneaded, granulated (extrusion granulation, pore diameter 0.5 to I mm), and dried. The resulting dry granule is sieved with a vibration sieve (12/60 mesh) to obtain a granule.

### Formulation Example 2

A powder for filling into a capsule containing the following ingredients is prepared.

| | | |
|---|---|---|
| Ingredient | Compound represented by the formula (I) | 15 mg |
| | Lactose | 90 mg |
| | Corn starch | 42 mg |
| | HPC-L | 3 mg |

The compound represented by the formula (I), and lactose are passed through a 60 mesh sieve. Corn starch is passed through a 120 mesh sieve. These ingredients are mixed with each other. An aqueous solution of HPC-L is added to a mixed powder, and this is kneaded, granulated, and dried. The dried granules obtained are subjected to sizing, and 150 mg of the sized granules are capsulated into a size #4 hard gelatin capsule.

### Formulation Example 3

A tablet containing the following ingredients is prepared.

| | | |
|---|---|---|
| Ingredient | Compound represented by the formula (I) | 10 mg |
| | Lactose | 90 mg |
| | Microcrystaline cellulose | 30 mg |
| | CMC-Na | 15 mg |
| | Magnesium stearate | 5 mg |

The compound represented by the formula (Ia), lactose, microcrystalline cellulose, CMC-Na (carboxymethylcellulose sodium salt) are passed through a 60 mesh sieve, and mixed. Magnesium stearate is mixed into a mixed powder to obtain a mixture powder for tabletting. The present mixed powder is directly compressed to obtain a 150 mg tablet.

### Formulation Example 4

The following ingredients are warmed, mixed, and sterilized to obtain an injectable.

| | | |
|---|---|---|
| Ingredient | Compound represented by the formula (I) | 3 mg |
| | Nonionic surfactant | 15 mg |
| | Purified water for injection | 1 mL |

### Formulation Example 5

A cataplasm containing the following ingredients is prepared.

| | | |
|---|---|---|
| Ingredient | Compound represented by the formula (I) | 50 mg |
| | aqueous-based (5% ethanol/5% butylene glycol/90% purified water) | 950 mg |
| | glycerin | |
| | kaoline | |
| | aqueous polyvinyl alcohol | |

The compound represented by the formula (I) is added to aqueous-based. The mixture is irradiated by ultrasonic for 15 minutes and then is sufficiently stirred to obtain a solution. 5 part of glycerin, 1 part of kaoline and 5 part of aqueous polyvinyl alcohol are homogeneously mixed and 1 part of the resulting solution is added to the above solution including the compound represented by the formula (I). The obtained solution is mixed and to give a paste form and the resulting paste is applied to an onwoven fabric. The resulting composition is covered by polyester film to give a cataplasm.
The present application claims priority to Japanese Patent Application No.2009-178508, which was filed in Japan, and which are herein incorporated by reference in their entirety.

### [INDUSTRIAL APPLICABILITY]

The compound of the present invention has histamine H4 receptor modulating effect and is useful in the treatment of deseases or conditions associated with histamine H4, for example, respiratory disease such as bronchial asthma, allergic rhinitis and chronic obstructive pulmonary disease(COPD); inflammatory disease such as chronic rheumatoid arthritis, atopic dermatitis, allergic conjunctivitis, psoriasis, inflammatory bowel disease, ulcerative colitis, lupus, atherosclerotic cardiovascular disease; pain relief for neurogenic pain and nociceptive pain.

## Claims

1. A compound represented by a formula (I): 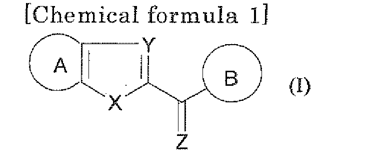 wherein
A ring is a ring represented by the following formula: 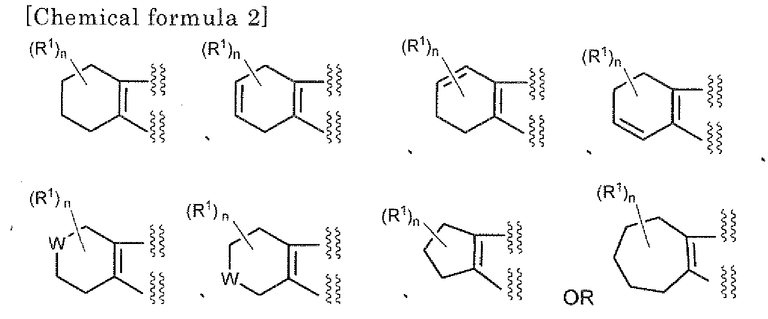 wherein
R¹ is each independently one or more group(s) selected from the group consisting of following group a) to group e) defined below;
a) halogen, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, hydroxy, formyl, carboxy, substituted or unsubstituted alkyloxycarbonyl, substituted or unsubstituted carbamoyl, substituted or unsubstituted sulfamoyl, substituted or unsubstituted alkylsulfonyloxy, substituted or unsubstituted arylsulfonyloxy, substituted or unsubstituted alkylsulfinyl, substituted or unsubstituted arylsulfinyl, cyano, nitro, substituted or unsubstituted amino, substituted or unsubstituted cycloalkyl, substituted or unsubstituted cycloalkenyl, a substituted or unsubstituted non-aromatic heterocyclic group, substituted or unsubstituted aryl, substituted or unsubstituted heteroaryl, substituted or unsubstituted cycloalkyloxy, substituted or unsubstituted cycloalkenyloxy, substituted or unsubstituted non-aromatic heterocyclic ring-oxy, substituted or unsubstituted aryloxy, and substituted or unsubstituted heteroaryloxy,
b) two R¹ on the same carbon atom are taken together =O and =NR³,
c) two R¹ on the same carbon atom are taken together -(CR^{4a}R^{4b})ₓ-,
d) two R¹ on the adjacent carbon atom are taken together -(CR^{5a}R^{5b})_{y}-, and
e) two R¹ on the non-adjacent carbon atom are taken together -(CR^{6a}R^{6b})_{z}-, provided that R¹ is not selected from the group consisting of group c), group d) and group e) at the same time;
W is -O-, -N(R²)- or -S(O)ₘ -;
R² is hydrogen, substituted or unsubstituted alkyl, substituted or unsubstituted alkenyl, substituted or unsubstituted alkynyl, acyl, substituted or unsubstituted cycloalkyl, substituted or unsubstituted cycloalkenyl, a substituted or unsubstituted non-aromatic heterocyclic group, substituted or unsubstituted aryl, or substituted or unsubstituted heteroaryl;
m is an integer of 0 to 2;
R³ is hydrogen, substituted or unsubstituted alkyl, hydroxy or substituted or unsubstituted alkyloxy;
R^{4a}, R^{4b}, R^{5a}, R^{5b}, R^{6a} and R^{6b} are each independently hydrogen, halogen or substituted or unsubstituted alkyl;
n is an integer of 0 to 6;
x is an integer of 2 to 7;
y is an integer of 1 to 5;
z is an integer of 1 to 3;
X is N(R⁷)- or -O-;
R⁷ is hydrogen, substituted or unsubstituted alkyl, substituted or unsubstituted cycloalkyl, substituted or unsubstituted acyl or substituted or unsubstituted alkyloxycarbonyl;
Y is -C(R⁸)= or -N=;
R⁸ is hydrogen, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylthio, acyl, hydroxy, formyl, carboxy, substituted or unsubstituted alkyloxycarbonyl, substituted or unsubstituted carbamoyl, substituted or unsubstituted sulfamoyl, cyano, nitro, or substituted or unsubstituted amino;
Z is =O, =S, or =NR⁹;
R⁹ is hydrogen, substituted or unsubstituted alkyl, hydroxy or substituted or unsubstituted alkyloxy;
B ring is a ring represented by the following formula: 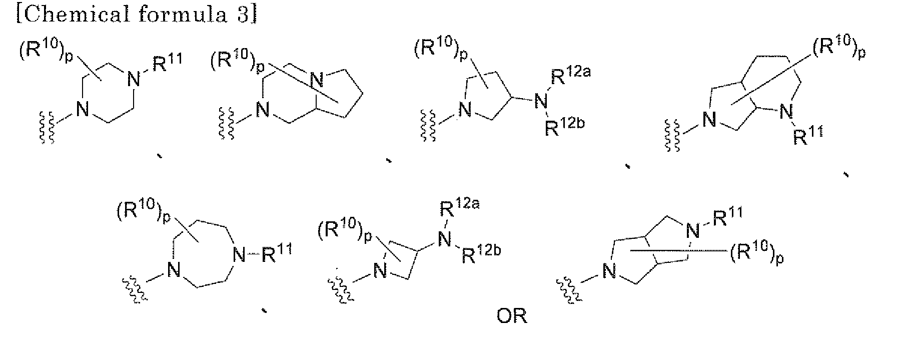 wherein
R¹⁰ is each independently substituted or unsubstituted alkyl, halogen, hydroxy, substituted or unsubstituted alkyloxy, substituted or unsubstituted amino;
R¹¹ is hydrogen or substituted or unsubstituted alkyl;
R^{12a} and R^{12b} are each independently hydrogen or substituted or unsubstituted alkyl;
p is an integer of 0 to 4;
provided that R⁸ is not methyl when A ring is a ring represented by the following formula: 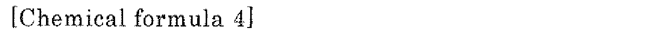 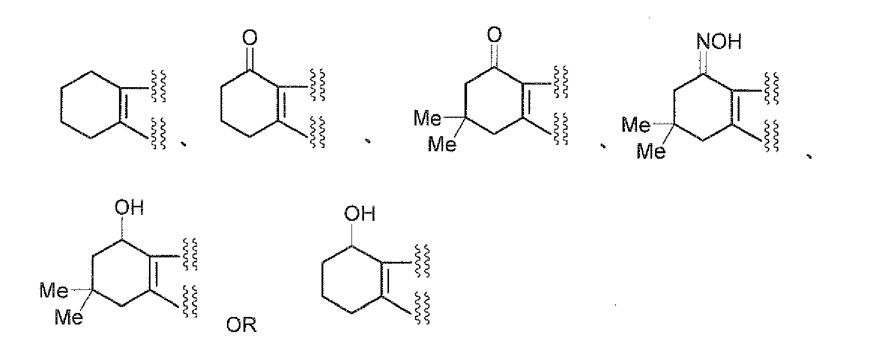 , or its pharmaceutically acceptable salt, or a solvate thereof.

2. The compound according to claim 1, wherein Z is =O, or its pharmaceutically acceptable salt, or a solvate thereof.

3. The compound according to claim 1 or 2, wherein R¹ is one or more group(s) selected from the group consisting of group a) and group b), or its pharmaceutically acceptable salt, or a solvate thereof.

4. The compound according to any one of claims 1 to 3, wherein B ring is a ring represented by the following formula: 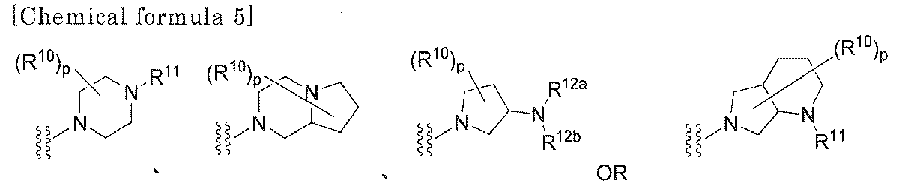 wherein R¹⁰, R¹¹, R^{12a}, R^{12b} and p are as defined in claim 1, or its pharmaceutically acceptable salt, or a solvate thereof.

5. The compound according to any one of claims 1 to 4, wherein W is -O- or -N(R²)-, wherein R² is as defined in claim 1, or its pharmaceutically acceptable salt, or a solvate thereof.

6. The compound according to any one of claims 1 to 5, wherein A ring is a ring represented by the following formula: 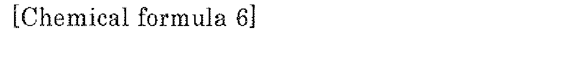 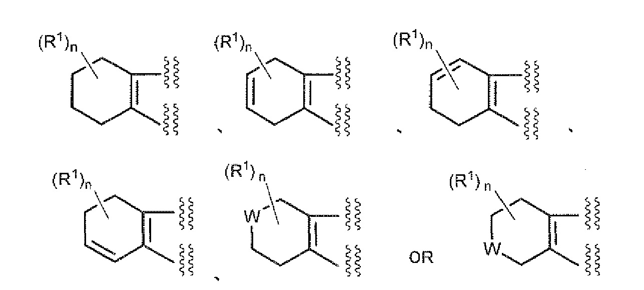 wherein R¹ is one or more group(s) selected from the group consisting of group a) and group b) in claim 1, W and n are as defined in clam 1, or its pharmaceutically acceptable salt, or a solvate thereof.

7. The compound according to any one of claims 1 to 6, wherein X is -N(R⁷)-, wherein R⁷ is as defined in claim 1, or its pharmaceutically acceptable salt, or a solvate thereof.

8. A pharmaceutical composition comprising the compound according to any one of claims 1 to 7, or its pharmaceutically acceptable salt, or a solvate thereof.

9. The pharmaceutical composition according to claim 8, wherein the composition is for histamine H4 modulator.

10. A method of treating and/or preventing a disease or condition related to histamine H4 receptor comprising administrating the compound according to any one of claims 1 to 7, or its pharmaceutically acceptable salt, or a solvate thereof.

11. The compound, or its pharmaceutically acceptable salt, or a solvate thereof according to any one of claims 1 to 7 for treating and/or preventing a disease or condition related to histamine H4 receptor.
